(19) Europäisches Patentamt
European Patent Office
Office européen des brevets

(11) EP 2 594 554 A1

(12) EUROPEAN PATENT APPLICATION
published in accordance with Art. 153(4) EPC

(43) Date of publication:
**22.05.2013 Bulletin 2013/21**

(21) Application number: **11806765.1**

(22) Date of filing: **12.07.2011**

(51) Int Cl.:
***C07C 311/08*** *(2006.01)* ***A61K 31/167*** *(2006.01)*
***A61K 31/18*** *(2006.01)* ***A61K 31/216*** *(2006.01)*
***A61K 31/235*** *(2006.01)* ***A61K 31/277*** *(2006.01)*
***A61K 31/341*** *(2006.01)* ***A61K 31/351*** *(2006.01)*
***A61K 31/381*** *(2006.01)* ***A61K 31/397*** *(2006.01)*
***A61K 31/40*** *(2006.01)* ***A61K 31/4015*** *(2006.01)*
***A61K 31/403*** *(2006.01)* ***A61K 31/407*** *(2006.01)*
***A61K 31/41*** *(2006.01)* ***A61K 31/415*** *(2006.01)*
***A61K 31/4164*** *(2006.01)* ***A61K 31/4196*** *(2006.01)*
***A61K 31/421*** *(2006.01)* ***A61K 31/4245*** *(2006.01)*
***A61K 31/426*** *(2006.01)*

(86) International application number:
**PCT/JP2011/065857**

(87) International publication number:
**WO 2012/008435 (19.01.2012 Gazette 2012/03)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.07.2010 JP 2010159036**

(71) Applicant: **Dainippon Sumitomo Pharma Co., Ltd.**
**Osaka 541-8524 (JP)**

(72) Inventors:
- **KATAYAMA, Seiji**
  **Osaka-shi**
  **Osaka 554-0022 (JP)**
- **HORI, Seiji**
  **Osaka-shi**
  **Osaka 554-0022 (JP)**
- **HASEGAWA, Futoshi**
  **Osaka-shi**
  **Osaka 554-0022 (JP)**
- **SUZUKI, Kuniko**
  **Osaka-shi**
  **Osaka 554-0022 (JP)**

(74) Representative: **Webster, Jeremy Mark et al**
**Mewburn Ellis LLP**
**33 Gutter Lane**
**London**
**EC2V 8AS (GB)**

# (54) BIARYL AMIDE DERIVATIVE OR PHARMACEUTICALLY ACCEPTABLE SALT THEREOF

(57) Disclosed is a novel biaryl amide derivative represented by formula (1) and having an affinity for the aldosterone receptor; also disclosed is a pharmaceutically acceptable salt thereof. (In the formula, A is any of the groups represented by formula (a); L is -CONH-, etc.; $R^1$ is a substitutable aminosulfonyl group, etc.; $R^2$ is a hydrogen atom, etc.; $R^3$ is a hydrogen atom, etc.; $R^4$ is a hydrogen atom, a halogen atom, hydroxy group, a substitutable amino group, a substitutable $C_{1-6}$ alkoxy group, a substitutable 4- to 7-membered cyclic amino group, etc.; $R^{5a}$, $R^{5b}$ and $R^{5c}$ are each independently hydrogen atoms, etc.; $R^6$ is a halogen atom, a cyano group, etc.; $R^7$ and $R^8$ are each independently a hydrogen atom, etc.; and m is an integer such as 0.)

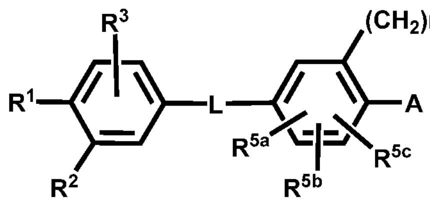
(1)

A = 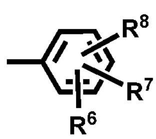
(a)

EP 2 594 554 A1

## Description

TECHNICAL FIELD

The present invention relates to novel biaryl amide derivatives with affinity for an aldosterone receptor, or pharmaceutically acceptable salts thereof. It also relates to a therapeutic or preventive agent for various diseases involving the receptor, comprising biaryl amide derivatives, or pharmaceutically acceptable salts thereof.

BACKGROUND ART

Nuclear receptors are a transcriptional regulator where low-molecular physiologically active substances are a ligand starting with steroid hormones, and forms a gene superfamily. Mineralcorticoid receptors, glucocorticoid receptors, androgen receptors, progesterone receptors as well as estrogen receptors are known as receptors where steroid hormones are a ligand. The receptors play an important role in the adjustment of physiological functions via the expression control of various genes.
Aldosterone is a physiological ligand of mineralcorticoid receptors. The receptor is also referred to as an aldosterone receptor. Aldosterone controls egestion of electrolyte via aldosterone receptors in kidney, while it is known that an excess activation of aldosterone receptors causes various diseases such as hypertension and heart failure. Hence, compounds (i.e., an antagonist and a partial agonist) which suppress the excess activation of aldosterone receptors are expected to be a preventive or therapeutic agent for the above diseases.
So far, steroid compounds such as spironolactone and eplerenone are known as an aldosterone receptor antagonist. These compounds are broadly demonstrated to be useful as a medicament. However, they are known to be associated with serious by-effects such as gynecomastia, sex dysfunction, etc. derived from effects on other steroidal hormone receptors such as androgen receptors or progesterone receptors or hyperpotassemia derived from excess antagonism against aldosterone receptors, and are restricted to be used. Hence, development of high safety non-steroidal compounds which reduce these by-effects is desired.
Recently, non-steroidal compounds having an affinity for or an antagonistic activity on aldosterone receptors have been studied. So far, compounds characterized by various monocyclic / fused heterocycle structures have been reported (Patent documents 1 to 5). For example, pyrrole derivatives in Patent document 1 or Patent document 5, 2H-benzo[b][1,4]oxazin-3(4H)-one derivatives, etc. in Patent document 2, tricyclic fused pyrazoline derivatives in Patent document 3, diphenylmethylimidazole derivatives, etc. in Patent document 4 are disclosed.
As a biaryl amide derivative, a compound of the following formula (I):
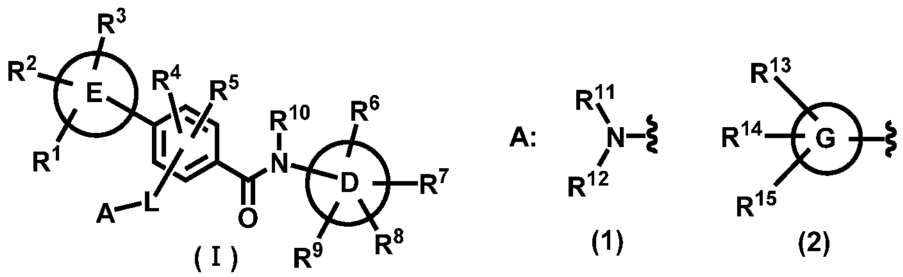

[wherein A is a group of the above formula (1) or (2);
L is lower alkylene;
Ring D and Ring E are the same or different and monocyclic or bicyclic hydrocarbon ring, or monocyclic or bicyclic heteroaromatic ring;
Ring G is monocyclic or bicyclic heterocycle;
$R^1$ to $R^9$ are the same or different and hydrogen atom, halogen atom, lower alkyl, halogen-substituted lower alkyl, -O-lower alkyl, $-SO_2$-lower alkyl, $-SO_2NH_2$, etc.;
$R^{10}$ is hydrogen atom, or lower alkyl;
$R^{11}$ to $R^{15}$ are the same or different and hydrogen atom, halogen atom, lower alkyl, halogen-substituted lower alkyl, etc.] is known (Patent document 6).
However, Patent document 6 does not disclose or indicate any compounds such as a compound of the present invention wherein Ring D is monocyclic phenyl ring, and Ring D and Ring E are a specific combination having any substituents. Hence, biaryl amide derivatives such as a compound of the present invention have not known so far. Further, it has not known so far that a compound group of the present invention has a high binding affinity for an

aldosterone receptor.

[Patent document 1] WO 06/012642 pamphlet
[Patent document 2] WO 07/077961 pamphlet
[Patent document 3] WO 08/053300 pamphlet
[Patent document 4] WO 08/118319 pamphlet
[Patent document 5] WO 08/126831 pamphlet
[Patent document 6] WO 04/110986 pamphlet

SUMMARY OF INVENTION

PROBLEMS TO BE SOLVED BY THE INVENTION

The problem to be solved by the present invention is to provide compounds with high affinity for an aldosterone receptor. Further, it is to provide non-steroidal compounds with reduced by-effects (e.g., hormonal-like by-effects, hyperpotassemia, etc.) compared to the conventional steroidal aldosterone receptor antagonists.

MEANS OF SOLVING THE PROBLEMS

As a result of extensive studies, the inventors of the present invention have found that a compound of the following formula (1) or a pharmaceutically acceptable salt thereof (also referred to as "the compound of the present invention" hereinafter) has a high affinity for and antagonistic activity or partial agonistic activity against an aldosterone receptor. Additionally, the inventors have found that the compound may reduce much by-effects compared to the conventional aldosterone receptor antagonists, and have achieved the present invention.

Specifically, the present invention is as follows. Item 1: A compound of formula (1), or a pharmaceutically acceptable salt thereof.

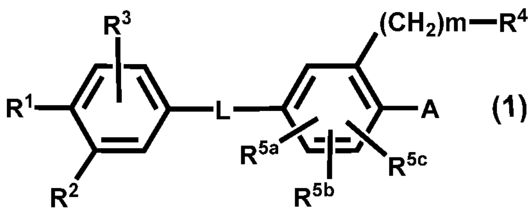

(1)

[wherein A is any one of groups of the following formulae (a) to (e):

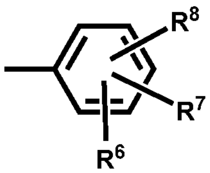
(a)

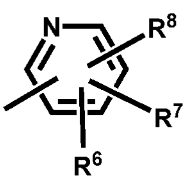
(b)

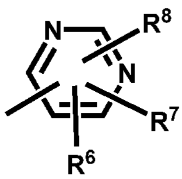
(c)

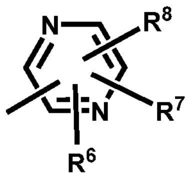
(d)

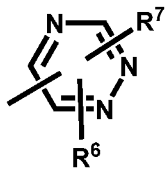
(e)

;

L is -CONH-, or -NHCO-;

$R^1$ is optionally substituted aminosulfonyl group, optionally substituted $C_{1-6}$ alkylsulfonyl group, or optionally substituted $C_{1-6}$ alkylsulfonylamino group;

$R^2$ is hydrogen atom, halogen atom, hydroxy group, optionally substituted $C_{1-6}$ alkyl group, optionally substituted $C_{1-6}$ alkoxy group, or optionally substituted 5- or 6-membered monocyclic heteroaryl group;

$R^3$ is hydrogen atom, or halogen atom;

$R^4$ is hydrogen atom, halogen atom, hydroxy group, cyano group, nitro group, formyl group, carboxyl group, optionally substituted $C_{1-6}$ alkyl group, optionally substituted amino group, optionally substituted $C_{1-6}$ alkoxy group, optionally substituted $C_{1-6}$ alkoxycarbonyl group, optionally substituted 4-to 7-membered heterocyclic group, optionally substituted 5- or 6-membered monocyclic heteroaryl group, optionally substituted $C_{7-14}$ aralkyloxy group, optionally substituted $C_{1-6}$ alkylcarbonyloxy group, optionally substituted $C_{1-6}$ alkylsulfonyloxy group, optionally substituted 4- to 8-membered cyclic

amino group, optionally substituted 4- to 7-membered saturated heterocyclic oxy group, optionally substituted $C_{1-6}$ alkylcarbonylamino group, optionally substituted $C_{3-10}$ cycloalkylcarbonylamino group, optionally substituted $C_{1-6}$ alkoxycarbonylamino group, optionally substituted 5- or 6-membered monocyclic heteroarylcarbonylamino group, optionally substituted 4- to 7-membered saturated heterocyclic carbonylamino group, or optionally substituted aminocarbonylamino group;

$R^{5a}$, $R^{5b}$ and $R^{5c}$ are each independently hydrogen atom, halogen atom, optionally substituted amino group, or optionally substituted $C_{1-6}$ alkyl group;

$R^6$ is halogen atom, cyano group, optionally substituted $C_{1-6}$ alkyl group, amino group (in which the amino may be optionally substituted with the same or different 1 to 2 groups selected from the group consisting of $C_{1-6}$ alkyl, $C_{3-10}$ cycloalkyl, $C_{3-10}$ cycloalkyl-$C_{1-4}$ alkyl, saturated heterocycle which may be optionally substituted with $C_{1-6}$ alkyl, 5- or 6-membered saturated heterocyclic-$C_{1-4}$ alkyl, 5- or 6-membered monocyclic heteroaryl, and 5- or 6-membered monocyclic heteroaryl-$C_{1-4}$ alkyl), hydroxy group, optionally substituted $C_{1-6}$ alkoxy group, optionally substituted $C_{3-10}$ cycloalkyl group, optionally substituted $C_{3-10}$ cycloalkoxy group, optionally substituted $C_{1-6}$alkylthio group, optionally substituted $C_{1-6}$ alkoxycarbonyl group, optionally substituted 4- to 7-membered cyclic amino group, optionally substituted 4- to 7-membered cyclic aminocarbonyl group, optionally substituted $C_{6-10}$ aryl group, optionally substituted 5- to 10-membered monocyclic or polycyclic heteroaryl group, optionally substituted 5- or 6-membered monocyclic heteroaryloxy group, optionally substituted 4- to 7-membered saturated heterocyclic group, or optionally substituted 4- to 7-membered saturated heterocyclic oxy group;

$R^7$ and $R^8$ are each independently hydrogen atom, halogen atom, hydroxy group, cyano group, nitro group, amino group (in which the amino may be optionally substituted with the same or different 1 to 2 groups selected from the group consisting of $C_{1-6}$ alkyl, $C_{3-10}$ cycloalkyl, $C_{3-10}$cycloalkyl-$C_{1-4}$ alkyl, 5-or 6-membered saturated heterocycle, 5- or 6-membered saturated heterocyclic-$C_{1-4}$ alkyl, 5- or 6-membered monocyclic heteroaryl, and 5- or 6-membered monocyclic heteroaryl-$C_{1-4}$ alkyl), optionally substituted $C_{1-6}$ alkyl group, optionally substituted $C_{1-6}$ alkoxy group, optionally substituted $C_{1-6}$ alkylthio group, optionally substituted $C_{1-6}$ alkoxycarbonyl group, or optionally substituted $C_{1-6}$ alkylcarbonyloxy group, or;

any one of $R^6$, $R^7$ and $R^8$ is hydrogen atom, and the remaining two groups are adjacent each other and may combine each other together with the ring atoms to which they bind to form $C_{3-7}$cycloalkyl ring, 5- or 6-membered saturated heterocycle, or 5- or 6-membered heteroaryl ring;

m is an integer of 0 to 6;

provided that 4-(methylsulfonyl)-N-[3'-(trifluoromethyl)biphenyl-4-yl]benzamide, and 2'-methyl-5'-(5-methyl-1,3,4-oxadiazol-2-yl)-N-[4-[(methylsulfonyl)amino]phenyl]biphenyl-4-carboxamide are excluded.]

Item 2: The compound of Item 1, wherein A is a group of formula (a), or a pharmaceutically acceptable salt thereof.

Item3: The compound of Item 2, or a pharmaceutically acceptable salt thereof, wherein a group of formula (a) is a group of the following formula (a1) or (a2):

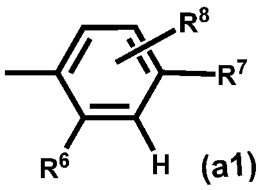

(a1)

or

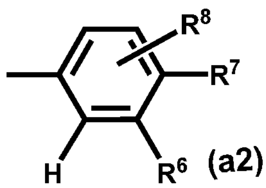

(a2).

Item 4: The compound of Item 3, or a pharmaceutically acceptable salt thereof, wherein a group of formula (a) is a group of the following formula (a1):

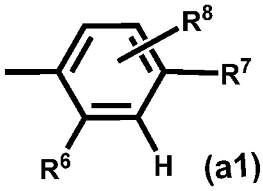

(a1).

Item 5: The compound of Item 3, or a pharmaceutically acceptable salt thereof, wherein a group of formula (a) is a group of the following formula (a2):

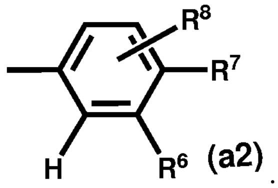

Item 6: The compound of any one of Items 1 to 5, or a pharmaceutically acceptable salt thereof, wherein L is -NHCO-.

Item 7: The compound of any one of Items 1 to 5, or a pharmaceutically acceptable salt thereof, wherein L is -CONH-.

Item 8: The compound of any one of Items 1 to 7, or a pharmaceutically acceptable salt thereof, wherein $R^1$ is

1: aminosulfonyl group (in which the amino may be optionally substituted with the same or different 1 to 2 groups selected from the group consisting of

(a) $C_{1-6}$ alkyl (in which the group may be optionally substituted with

(i) amino (in which the amino may be optionally substituted with the same or different 1 to 2 $C_{1-6}$alkyl),
(ii) $C_{1-6}$alkoxy, or
(iii) 4- to 7-membered cyclic amino),

(b) $C_{1-6}$alkylcarbonyl,
(c) aminocarbonyl, and
(d) -C(=NH)-$NH_2$),

2: $C_{1-6}$ alkylsulfonyl group, or
3: $C_{1-6}$ alkylsulfonylamino group.

Item 9: The compound of Item 8, or a pharmaceutically acceptable salt thereof, wherein $R^1$ is aminosulfonyl group.

Item 10: The compound of Item 8, or a pharmaceutically acceptable salt thereof, wherein $R^1$ is $C_{1-6}$ alkylsulfonyl group.

Item 11: The compound of any one of Items 1 to 10, or a pharmaceutically acceptable salt thereof, wherein $R^2$ is hydrogen atom, halogen atom, hydroxy group, $C_{1-6}$ alkyl group, or optionally substituted $C_{1-6}$ alkoxy group.

Item 12: The compound of Item 11, or a pharmaceutically acceptable salt thereof, wherein $R^2$ is hydrogen atom, hydroxy group, or $C_{1-6}$alkoxy group.

Item 13: The compound of Item 12, or a pharmaceutically acceptable salt thereof, wherein $R^2$ is $C_{1-6}$alkoxy group.

Item 14: The compound of any one of Items 1 to 13, or a pharmaceutically acceptable salt thereof, wherein $R^3$ is hydrogen atom.

Item 15: The compound of any one of Items 1 to 14, or a pharmaceutically acceptable salt thereof, wherein $R^4$ is

1: hydrogen atom,
2: halogen atom,
3: hydroxy group,
4: cyano group,
5: nitro group,
6: formyl group,
7: carboxyl group,
8: amino group (in which the amino may be optionally substituted with the same or different 1 to 2 groups selected from the group consisting of

(a) $C_{1-6}$ alkyl (in which the group may be optionally substituted with

(i) 1 to 3 fluorine atoms,

(ii) cyano,
(iii) hydroxy,
(iv) amino (in which the amino may be optionally substituted with the same or different 1 to 2 $C_{1-6}$ alkyl),
(v) $C_{1-6}$alkoxy,
(vi) $C_{6-10}$aryloxy,
(vii) $C_{7-14}$aralkyl (in which the group may be optionally substituted with $C_{1-4}$alkoxy), or
(viii) aminocarbonyl),

(b) $C_{3-10}$cycloalkyl (in which the group may be optionally substituted with cyano, or $C_{1-4}$ alkyl),
(c) $C_{3-10}$cycloalkyl-$C_{1-4}$ alkyl,
(d) $C_{6-10}$ aryl (in which the group may be optionally substituted with $C_{1-6}$alkyl),
(e) $C_{7-14}$aralkyl (in which the group may be optionally substituted with halogen atom, or $C_{1-4}$alkoxy),
(f) 4- to 7-membered saturated heterocycle (in which the heterocycle may be optionally substituted with $C_{1-6}$alkyl),
(g) 4- to 7-membered saturated heterocycfic-$C_{1-4}$ alkyl (in which the heterocycle may be optionally substituted with $C_{1-4}$alkyl),
(h) 5- or 6-membered monocyclic heteroaryl (in which the group may be optionally substituted with the same or different 1 to 2 groups selected from the group consisting of cyano and $C_{1-4}$alkyl), and
(i) 5- or 6-membered monocyclic heteroaryl-$C_{1-4}$ alkyl (in which the heteroaryl may be optionally substituted with $C_{1-4}$alkyl)),

9: $C_{1-6}$alkoxy group (in which the group may be optionally substituted with

(a) 1 to 2 hydroxy,
(b) 1 to 3 fluorine atoms,
(c) amino (in which the amino may be optionally substituted with the same or different 1 to 2 groups selected from the group consisting of

(i) $C_{1-6}$alkyl (in which the group may be optionally substituted with hydroxy, $C_{1-4}$alkoxy, or 5- or 6-membered monocyclic heteroaryl),
(ii) $C_{3-6}$cycloalkyl, and
(iii) 4- to 7-membered saturated heterocycfic-$C_{1-4}$ alkyl),

(d) 1 to 2 $C_{1-6}$alkoxy,
(e) 4- to 7-membered cyclic amino (in which the ring may be optionally substituted with the same or different 1 to 2 groups selected from the group consisting of

(i) hydroxy,
(ii) cyano,
(iii) 1 to 4 fluorine atoms,
(iv) $C_{1-6}$alkyl (in which the group may be optionally substituted with 1 to 3 fluorine atoms, or $C_{1-6}$ alkoxy),
(v) $C_{1-6}$alkoxy,
(vi) formyl,
(vii) $C_{1-6}$ alkylcarbonyl,
(viii) $C_{1-6}$ alkylsulfonyl, and
(ix) oxo),

(f) 4- to 7-membered saturated heterocycle (in which the ring may be optionally substituted with the same or different group(s) selected from the group consisting of (i) to (ix) in the above (e)),
(g) 5- or 6-membered monocyclic heteroaryl (in which the ring may be optionally substituted with $C_{1-6}$alkyl), or
(h) $C_{7-14}$aralkyloxy),

10: $C_{1-6}$alkoxycarbonyl group,
11: 4- to 7-membered heterocyclic group (in which the ring may be optionally substituted with the same or different group(s) selected from the group consisting of (i) to (ix) in the above (e)),
12: 5- or 6-membered monocyclic heteroaryl group (in which the ring may be optionally substituted with cyano, or $C_{1-6}$ alkyl),
13: $C_{7-14}$aralkyloxy group,
14: $C_{1-6}$ alkylcarbonyloxy group,

15: $C_{1-6}$alkylsulfonyloxy group,
16: 4- to 8-membered cyclic amino group (in which the ring may be optionally substituted with the same or different group(s) selected from the group consisting of (i) to (ix) in the above (e)),
17: saturated heterocyclic oxy group (in which the ring may be optionally substituted with the same or different 1 to 2 groups selected from the group consisting of $C_{1-6}$ alkyl which may be optionally substituted with 1 to 3 fluorine atoms, and oxo),
18: $C_{1-6}$alkylcarbonylamino group (in which the alkyl may be optionally substituted with

(a) hydroxy,
(b) 1 to 3 fluorine atoms,
(c) $C_{1-6}$alkoxy, or
(d) $C_{1-6}$alkylcarbonyloxy),

19: $C_{3-10}$cycloalkylcarbonylamino group,
20: $C_{1-6}$alkoxycarbonylamino group,
21: 5- or 6-membered monocyclic heteroarylcarbonylamino group (in which the ring may be optionally substituted with $C_{1-6}$alkyl),
22: 4- to 7-membered saturated heterocyclic carbonylamino group (in which the ring may be optionally substituted with $C_{1-6}$alkyl which may be optionally substituted with 1 to 3 fluorine atoms),
23: mono- or di-$C_{1-6}$ alkylaminocarbonylamino group, or
24: $C_{1-6}$alkyl group (in which the group may be optionally substituted with group(s) selected from 1 to 3 fluorine atoms, and hydroxy).

Item 16: The compound of Item 15, or a pharmaceutically acceptable salt thereof, wherein $R^4$ is

1: hydrogen atom,
2: halogen atom,
3: hydroxy group,
4: cyano group,
5: nitro group,
6: formyl group,
7: carboxyl group,
8: amino group (in which the amino may be optionally substituted with the same or different 1 to 2 groups selected from the group consisting of

(a) $C_{1-6}$alkyl (in which the group may be optionally substituted with

(i) 1 to 3 fluorine atoms,
(ii) cyano,
(iii) hydroxy,
(iv) amino (in which the amino may be optionally substituted with the same or different 1 to 2 $C_{1-6}$ alkyl),
(v) $C_{1-6}$alkoxy,
(vi) $C_{6-10}$aryloxy, or
(vii) aminocarbonyl),

(b) $C_{3-10}$ cycloalkyl (in which the group may be optionally substituted with $C_{1-4}$alkyl),
(c) $C_{3-10}$cycloalkyl-$C_{1-4}$ alkyl,
(d) $C_{6-10}$ aryl,
(e) $C_{7-14}$aralkyl (in which the group may be optionally substituted with halogen atom, or $C_{1-4}$alkoxy),
(f) 4- to 7-membered saturated heterocycle (in which the heterocycle may be optionally substituted with $C_{1-6}$alkyl),
(g) 4- to 7-membered saturated heterocyclic-$C_{1-4}$alkyl (in which the heterocycle may be optionally substituted with $C_{1-4}$alkyl),
(h) 5- or 6-membered monocyclic heteroaryl (in which the group may be optionally substituted with the same or different 1 to 2 groups selected from the group consisting of cyano and $C_{1-4}$alkyl), and
(i) 5- or 6-membered monocyclic heteroaryl-$C_{1-4}$ alkyl (in which the heteroaryl may be optionally substituted with $C_{1-4}$alkyl)),

9: $C_{1-6}$alkoxy group (in which the group may be optionally substituted with

(a) 1 to 2 hydroxy,
(b) amino (in which the amino may be optionally substituted with 1 or 2 $C_{1-6}$alkyl (in which the group may be optionally substituted with hydroxy)),
(c) 1 to 2 $C_{1-6}$alkoxy,
(d) 4- to 7-membered cyclic amino (in which the ring may be optionally substituted with $C_{1-6}$alkyl or oxo),
(e) 4- to 7-membered saturated heterocycle (in which the group may be optionally substituted with $C_{1-4}$alkyl),
(f) 5- or 6-membered monocyclic heteroaryl (in which the ring may be optionally substituted with $C_{1-6}$alkyl), or
(g) $C_{7-14}$aralkyloxy),

10: $C_{1-6}$alkoxycarbonyl group,
11: 4- to 7-membered heterocyclic group (in which the ring may be optionally substituted with $C_{1-4}$alkyl, or oxo),
12: 5- or 6-membered monocyclic heteroaryl group (in which the ring may be optionally substituted with $C_{1-6}$alkyl),
13: $C_{7-14}$aralkyloxy group,
14: $C_{1-6}$alkylcarbonyloxy group,
15: 4- to 8-membered cyclic amino group (in which the ring may be optionally substituted with the same or different 1 to 2 groups selected from the group consisting of

(a) hydroxy,
(b) $C_{1-4}$alkyl (in which the group may be optionally substituted with $C_{1-6}$ alkoxy),
(c) cyano,
(d) 1 to 4 fluorine atoms,
(e) $C_{1-6}$alkoxy,
(f) formyl,
(g) $C_{1-6}$alkylcarbonyl,
(h) $C_{1-6}$alkylsulfonyl, and
(i) oxo),

16: saturated heterocyclic oxy group (in which the ring may be optionally substituted with the same or different 1 to 2 groups selected from the group consisting of $C_{1-6}$ alkyl and oxo), or 17: $C_{1-6}$alkoxycarbonylamino group.

Item 17: The compound of Item 16, or a pharmaceutically acceptable salt thereof, wherein $R^4$ is amino group (in which the amino is substituted with $C_{1-6}$ alkyl which is substituted with 1 to 3 fluorine atoms).
Item 18: The compound of Item 17, or a pharmaceutically acceptable salt thereof, wherein $R^4$ is 2,2-difluoroethylamino.
Item 19: The compound of any one of Items 1 to 18, or a pharmaceutically acceptable salt thereof, wherein $R^{5a}$, $R^{5b}$, and $R^{5c}$ are each independently

1: hydrogen atom,
2: halogen atom,
3: amino group (in which the amino may be optionally substituted with the same or different 1 to 2 groups selected from the group consisting of

(a) $C_{1-6}$alkyl,
(b) $C_{3-10}$cycloalkyl, and
(c) $C_{3-10}$cycloalkyl-$C_{1-4}$ alkyl), or

4: $C_{1-6}$alkyl group.

Item 20: The compound of Item 19, or a pharmaceutically acceptable salt thereof, wherein $R^{5a}$, $R^{5b}$, and $R^{5c}$ are each independently

1: hydrogen atom, or
2: $C_{1-6}$alkyl group.

Item 21: The compound of Item 20, or a pharmaceutically acceptable salt thereof, wherein all of $R^{5a}$, $R^{5b}$, and $R^{5c}$ are hydrogen atom.
Item 22: The compound of any one of Items 1 to 21, or a pharmaceutically acceptable salt thereof, wherein $R^6$ is

1: halogen atom,
2: cyano group,
3: $C_{1-6}$alkyl group (in which the group may be optionally substituted with

(a) hydroxy,
(b) cyano,
(c) oxo,
(d) 1 to 4 fluorine atoms (provided that if $R^6$ is $C_1$ alkyl, it is 1 to 3 fluorine atoms),
(e) amino (in which the amino may be optionally substituted with the same or different 1 to 2 groups selected from the group consisting of

(i) $C_{1-6}$ alkyl (in which the group may be optionally substituted with 1 to 3 fluorine atoms, or $C_{1-6}$ alkoxy),
(ii) $C_{3-10}$cycloalkyl, and
(iii) $C_{3-10}$cycloalkyl-$C_{1-4}$ alkyl),

(f) 4- to 7-membered cyclic amino (in which the ring may be optionally substituted with

(i) 1 to 3 fluorine atoms,
(ii) $C_{1-6}$alkyl, or
(iii) $C_{1-6}$alkoxy),

(g) $C_{1-6}$ alkoxy (in which the group may be optionally substituted with 1 to 3 fluorine atoms),
(h) $C_{3-10}$cycloalkoxy,
(i) 5- or 6-membered monocyclic heteroaryl,
(j) 5- or 6-membered monocyclic heteroaryloxy,
(k) 4- to 7-membered saturated heterocyclic oxy,
(l) $C_{1-6}$alkylcarbonyl, or
(m) $C_{1-6}$alkoxycarbonyl),

4: amino group (in which the amino may be optionally substituted with the same or different 1 to 2 groups selected from the group consisting of

(a) $C_{1-6}$alkyl,
(b) $C_{3-10}$cycloalkyl,
(c) $C_{3-10}$cycloalkyl-$C_{1-4}$ alkyl, and
(d) 4- to 7-membered saturated heterocycle (in which the ring may be optionally substituted with $C_{1-6}$ alkyl)),

5: hydroxy group,
6: $C_{1-6}$alkoxy group (in which the group may be optionally substituted with

(a) hydroxy,
(b) cyano,
(c) 1 to 6 fluorine atoms (provided that if $R^6$ is $C_1$ alkoxy, it is 1 to 3 fluorine atoms, and if $R^6$ is $C_2$ alkoxy, it is 1 to 5 fluorine atoms),
(d) amino (in which the amino may be optionally substituted with the same or different 1 to 2 groups selected from the group consisting of

(i) $C_{1-6}$alkyl,
(ii) $C_{3-10}$cycloalkyl, and
(iii) $C_{3-10}$cycloalkyl-$C_{1-4}$ alkyl),

(e) 4- to 7-membered cyclic amino (in which the ring may be optionally substituted with

(i) 1 to 3 fluorine atoms,
(ii) $C_{1-6}$alkyl, or
(iii) $C_{1-6}$alkoxy),

(f) $C_{1-6}$ alkoxy (in which the group may be optionally substituted with 1 to 3 fluorine atoms),

(g) $C_{3-10}$cycloalkyl, or
(h) 5- or 6-membered monocyclic heteroaryl),

7: $C_{3-10}$cycloalkyl group (in which the group may be optionally substituted with $C_{1-6}$ alkyl, or cyano),
8: $C_{3-10}$cycloalkoxy group,
9: $C_{1-6}$alkylthio group,
10: $C_{1-6}$alkoxycarbonyl group,
11: 4- to 7-membered cyclic amino group (in which the ring may be optionally substituted with

(a) 1 to 3 fluorine atoms,
(b) $C_{1-6}$alkyl, or
(c) $C_{1-6}$alkoxy),

12: 4- to 7-membered cyclic aminocarbonyl group,
13: $C_{6-10}$aryl group,
14: 5- or 6-membered monocyclic heteroaryl group (in which the ring may be optionally substituted with $C_{1-6}$alkyl),
15: 5- or 6-membered monocyclic heteroaryloxy group,
16: 4- to 7-membered saturated heterocyclic group, or
17: 4- to 7-membered saturated heterocyclic oxy group.

Item 23: The compound of Item 22, or a pharmaceutically acceptable salt thereof, wherein $R^6$ is

1: halogen atom,
2: cyano group,
3: $C_{1-6}$alkyl group (in which the group may be optionally substituted with

(a) hydroxy,
(b) cyano,
(c) 1 to 4 fluorine atoms,
(d) amino (in which the amino may be optionally substituted with 1 to 2 $C_{1-6}$ alkyl),
(e) 4- to 7-membered cyclic amino (in which the ring may be optionally substituted with $C_{1-6}$ alkyl),
(f) $C_{1-6}$ alkoxy (in which the group may be optionally substituted with 1 to 3 fluorine atoms),
(g) 5- or 6-membered monocyclic heteroaryl,
(h) $C_{1-6}$alkylcarbonyl, or
(i) $C_{1-6}$alkoxycarbonyl),

4: amino group (in which the amino may be optionally substituted with the same or different 1 to 2 groups selected from the group consisting of

(a) $C_{1-6}$alkyl,
(b) $C_{3-10}$cycloalkyl,
(c) $C_{3-10}$cycloalkyl-$C_{1-4}$ alkyl, and
(d) 4- to 7-membered saturated heterocycle (in which the ring may be optionally substituted with $C_{1-6}$ alkyl)),

5: $C_{1-6}$ alkoxy group (in which the group may be optionally substituted with 1 to 6 fluorine atoms, or 4- to 7-membered cyclic amino),
6: $C_{3-10}$cycloalkyl group,
7: $C_{3-10}$cycloalkoxy group,
8: $C_{1-6}$alkylthio group,
9: $C_{1-6}$alkoxycarbonyl group,
10: 4- to 7-membered cyclic amino group (in which the ring may be optionally substituted with $C_{1-6}$ alkyl),
11: 4- to 7-membered cyclic aminocarbonyl group,
12: $C_{6-10}$aryl group, or
13: 5- or 6-membered monocyclic heteroaryl group (in which the ring may be optionally substituted with $C_{1-6}$alkyl).

Item 24: The compound of Item 23, or a pharmaceutically acceptable salt thereof, wherein $R^6$ is halogen atom.
Item 25: The compound of any one of Items 1 to 24, or a pharmaceutically acceptable salt thereof, wherein $R^7$ is

1: hydrogen atom,
2: halogen atom,
3: hydroxy group,
4: cyano group,
5: nitro group,
6: amino group (in which the amino may be optionally substituted with the same or different 1 to 2 groups selected from the group consisting of

(a) $C_{1-6}$alkyl,
(b) $C_{3-10}$cycloalkyl, and
(c) $C_{3-10}$cycloalkyl-$C_{1-4}$ alkyl),

7: $C_{1-6}$alkyl group (in which the group may be optionally substituted with 1 to 3 fluorine atoms),
8: $C_{1-6}$alkoxy group (in which the group may be optionally substituted with

(a) hydroxy,
(b) carboxyl,
(c) 1 to 3 fluorine atoms,
(d) amino (in which the amino may be optionally substituted with the same or different 1 to 2 $C_{1-6}$ alkyl),
(e) $C_{1-6}$alkoxycarbonyl, or
(f) $C_{1-6}$alkylcarbonyloxy),

9: $C_{1-6}$alkylthio group,
10: $C_{1-6}$alkoxycarbonyl group, or
11: $C_{1-6}$alkylcarbonyloxy group.

Item 26: The compound of Item 25, or a pharmaceutically acceptable salt thereof, wherein $R^7$ is

1: hydrogen atom,
2: halogen atom,
3: hydroxy group,
4: nitro group,
5: amino group,
6: $C_{1-6}$alkyl group,
7: $C_{1-6}$alkoxy group (in which the group may be optionally substituted with

(a) hydroxy,
(b) carboxyl,
(c) 1 to 3 fluorine atoms,
(d) amino (in which the amino may be optionally substituted with the same or different 1 to 2 $C_{1-6}$ alkyl),
(e) $C_{1-6}$alkoxycarbonyl, or
(f) $C_{1-6}$alkylcarbonyloxy),

8: $C_{1-6}$alkylthio group,
9: $C_{1-6}$ alkoxycarbonyl group, or
10: $C_{1-6}$alkylcarbonyloxy group.

Item 28: The compound of Item 26, or a pharmaceutically acceptable salt thereof, wherein $R^7$ is

1: hydrogen atom,
2: halogen atom,
3: hydroxy group,
4: $C_{1-6}$alkyl group, or
5: $C_{1-6}$alkoxy group.

Item 29: The compound of Item 28, or a pharmaceutically acceptable salt thereof, wherein $R^7$ is

1: hydrogen atom,

2: halogen atom, or 3: $C_{1-6}$alkyl group.

Item 30: The compound of Item 29, or a pharmaceutically acceptable salt thereof, wherein $R^7$ is hydrogen atom.
Item 31: The compound of any one of Items 1 to 30, or a pharmaceutically acceptable salt thereof, wherein $R^8$ is

1: hydrogen atom,
2: halogen atom, or
3: $C_{1-6}$alkoxy group.

Item 32: The compound of Item 31, or a pharmaceutically acceptable salt thereof, wherein $R^8$ is hydrogen atom.
Item 33: The compound of any one of Items 1 to 32, or a pharmaceutically acceptable salt thereof, wherein m is 0 or 1.
Item 34: The compound of Item 33, or a pharmaceutically acceptable salt thereof, wherein m is 0.
Item 35: The compound of Item 33, or a pharmaceutically acceptable salt thereof, wherein m is 1.
Item 36: The compound of Item 1, or a pharmaceutically acceptable salt thereof, which is any one selected from the following group:

2-(hydroxymethyl)-*N*-[4-(methylsulfonyl)phenyl]-2'-(trifluorophenyl)biphenyl-4-carboxamide,
2-(hydroxymethyl)-*N*-(3-methoxy-4-sulfamoylphenyl)-2'-(trifluorophenyl)biphenyl-4-carboxamide,
2-(hydroxymethyl)-*N*-(3-methoxy-4-sulfamoylphenyl)-2'-(trifluoromethoxy)biphenyl-4-carboxamide,
2'-ethyl-2-(hydroxymethyl)-*N*-(3-methoxy-4-sulfamoylphenyl)biphenyl-4-carboxamide,
4'-fluoro-2-(hydroxymethyl)-*N*-(3-methoxy-4-sulfamoylphenyl)-2'-(trifluorophenyl)biphenyl-4-carboxamide,
2-(hydroxymethyl)-4'-methoxy-*N*-(3-methoxy-4-sulfamoylphenyl)-2'-(trifluorophenyl)biphenyl-4-carboxamide,
2-(hydroxymethyl)-*N*-[4-(methylsulfonyl)phenyl]-2'-(trifluoromethoxy)biphenyl-4-carboxamide,
2-[(3,3-difluoropyrrolidin-1-yl)methyl]-*N*-[4-(methylsulfonyl)phenyl]-2'-(trifluoromethoxy)biphenyl-4-carboxamide,
2-[(3,3-difluoropiperidin-l-yl)methyl]-*N*-[4-(methylsulfonyl)phenyl]-2'-(trifluoromethoxy)biphenyl-4-carboxamide,
2-{[(2-methoxyethyl)amino]methyl{-*N*-[4-(methylsulfonyl)phenyl]-2'-(trifluoromethoxy)biphenyl-4-carboxamide,
2-[(diethylamino)methyl]-*N*-[4-(methylsulfonyl)phenyl]-2'-(trifluoromethoxy)biphenyl-4-carboxamide, 2-{[(3*S*)-3-fluoropyrrolidin-1-yl]methyl{-*N*-[4-(methylsulfonyl)phenyl]-2'-(trifluoromethoxy)biphenyl-4-carboxamide,
2-{[(2,2-difluoroethyl)amino]methyl{-*N*-[4-(methylsulfonyl)phenyl]-2'-(trifluoromethoxy)biphenyl-4-carboxamide,
2-{[bis(2-methoxyethyl)amino]methyl{-*N*-[4-(methylsulfonyl)phenyl]-2'-(trifluoromethoxy)biphenyl-4-carboxamide,
*N*-[4-(methylsulfonyl)phenyl]-2-{[(2,2,2-trifluoroethyl)amino]methyl}-2'-(trifluoromethoxy)biphenyl-4-carboxamide,
2-{[(2,2-difluoroethyl)amino]methyl{-*N*-(3-methoxy-4-sulfamoylphenyl)-2'-(trifluoromethoxy)biphenyl-4-carboxamide,
2-(hydroxymethyl)-*N*-(3-methoxy-4-sulfamoylphenyl)-2'-(1,1,2,2-tetrafluoroethoxy)biphenyl-4-carboxamide,
2-{[(2,2-difluoroethyl)amino]methyl}-*N*-(3-methoxy-4-sulfamoylphenyl)-2'-(trifluorophenyl)biphenyl-4-carboxamide,
2-{[(2,2-difluoroethyl)amino]methyl{-4'-methoxy-*N*-(3-methoxy-4-sulfamoylphenyl)-2'-(trifluorophenyl)biphenyl-4-carboxamide,
*N*-[2-(hydroxymethyl)-2'-(trifluorophenyl)biphenyl-4-yl]-3-methoxy-4-sulfamoylbenzamide,
*N*-[2-(hydroxymethyl)-2'-(trifluoromethoxy)biphenyl-4-yl]-3-methoxy-4-sulfamoylbenzamide,
*N*-[2-{[(2,2-difluoroethyl)amino]methyl{-2'-(trifluorophenyl)biphenyl-4-yl]-3-methoxy-4-sulfamoylbenzamide,
2-{[(2,2-difluoroethyl)amino]methyl}-2',4'-difluoro-*N*-(3-methoxy-4-sulfamoylphenyl)biphenyl-4-carboxamide,
2-{[(2,2-difluoroethyl)amino]methyl}-*N*-(3-methoxy-4-sulfamoylphenyl)-2'-methylbiphenyl-4-carboxamide,
2-{[(2,2-difluoroethyl)amino]methyl}-2'-fluoro-*N*-(3-methoxy-4-sulfamoylphenyl)biphenyl-4-carboxamide,
2- {[(2,2-difluoroethyl)amino]methyl}-2',5'-difluoro-*N*-(3-methoxy-4-sulfamoylphenyl)biphenyl-4-carboxamide,
5'-chloro-2- {[(2,2-difluoroethyl)amino]methyl}-2'-fluoro-*N*-(3-methoxy-4-sulfamoylphenyl)biphenyl-4-carboxamide,
2'-chloro-2-{[(2,2-difluoroethyl)amino]methyl}-4'-fluoro-*N*-(3-methoxy-4-sulfamoylphenyl)biphenyl-4-carboxamide,
2- {[(2,2-difluoroethyl)amino]methyl}-*N*-(3-methoxy-4-sulfamoylphenyl)-4'-methyl-2'-(trifluorophenyl)biphenyl-4-carboxamide,
2'-chloro-2- {[(2,2-difluoroethyl)amino]methyl}-*N*-(3-methoxy-4-sulfamoylphenyl)biphenyl-4-carboxamide,
2',4'-dichloro-2- {[(2,2-difluoroethyl)amino]methyl}-*N*-(3-methoxy-4-sulfamoylphenyl)biphenyl-4-carboxamide,
2', 5'-dichloro-2- {[(2,2-difluoroethyl)amino]methyl}-*N*-(3-methoxy-4-sulfamoylphenyl)biphenyl-4-carboxamide,
2'-cyclopropyl-2- {[(2,2-difluoroethyl)amino]methyl}-4'-fluoro-*N*-(3-methoxy-4-sulfamoylphenyl)biphenyl-4-carboxamide,
4'-chloro-2- {[(2,2-difluoroethyl)amino]methyl}-*N*-(3-methoxy-4-sulfamoylphenyl)-2'-(trifluorophenyl)-biphenyl-4-carboxamide,
2- {[(2,2-difluoroethyl)amino]methyl}-4'-fluoro-*N*-(3-methoxy-4-sulfamoylphenyl)-2'-(trifluorophenyl)biphenyl-4-carboxamide,

*N*-(2'-chloro-2- {[(2,2-difluoroethyl)amino]methyl}-4'-fluorobiphenyl-4-yl)-3-methoxy-4-sulfamoylbenzamide,
*N*-[2-{[(2,2-difluoroethyl)amino]methyl{-4'-fluoro-2'-(trifluorophenyl)biphenyl-4-yl]-3-methoxy-4-sulfamoylbenzamide,
2-{[(3R)-3-fluoropyrrolidin-1-yl]methyl}-*N*-[4-(methylsulfonyl)phenyl]-2'-(trifluoromethoxy)biphenyl-4-carboxamide,
2-{[(2-fluoroethyl)amino]methyl}-*N*-[4-(methylsulfonyl)phenyl]-2'-(trifluoromethoxy)biphenyl-4-carboxamide,
2-[(cyclopropylamino)methyl]-*N*-[4-(methylsulfonyl)phenyl] -2'-(trifluoromethoxy)biphenyl-4-carboxamide,
3-methoxy-4-sulfamoyl-*N*-[2-{[(2,2,2-trifluoroethyl)amino]methyl}-2'-(trifluorophenyl)biphenyl-4-yl]benzamide,
3-methoxy-4-sulfamoyl-*N*-[2-{[(2,2,2-trifluoroethyl)amino]methyl}-2'-(trifluoromethoxy)biphenyl-4-yl]benzamide,
2'-chloro-2-(hydroxymethyl)-*N*-(3-methoxy-4-sulfamoylphenyl)biphenyl-4-carboxamide,
2-(hydroxymethyl)-*N*-(3-methoxy-4-sulfamoylphenyl)-2'-methylbiphenyl-4-carboxamide,
2'-cyclopropyl-2-(hydroxymethyl)-*N*-(3-methoxy-4-sulfamoylphenyl)biphenyl-4-carboxamide,
2-(hydroxymethyl)-4'-methoxy-*N*-(3-methoxy-4-sulfamoylphenyl)-2'-(trifluoromethoxy)biphenyl-4-carboxamide,
2'-(cyclopropyloxy)-2-(hydroxymethyl)-*N*-(3-methoxy-4-sulfamoylphenyl)biphenyl-4-carboxamide,
*N*-[2- {[(2,2-difluoroethyl)amino]methyl}-4'-methoxy-2'-(trifluorophenyl)biphenyl-4-yl]-3-methoxy-4-sulfamoylbenzamide,
2- {[(2,2-difluoroethyl)amino]methyl}-4'-methoxy-*N*-(3-methoxy-4-sulfamoylphenyl)-2'-methylbiphenyl-4-carboxamide,
2- {[(2,2-difluoroethyl)amino]methyl}-4'-fluoro-2'-methoxy-*N*-(3-methoxy-4-sulfamoylphenyl)biphenyl-4-carboxamide, and
2-{[(2,2-difluoroethyl)amino]methyl{-4'-methoxy-*N*-(3-methoxy-4-sulfamoylphenyl)-2'-(trifluoromethoxy)biphenyl-4-carboxamide.

Item 37: The compound of Item 36, or a pharmaceutically acceptable salt thereof, which is any one selected from the following group:

2-(hydroxymethyl)-*N*-(3-methoxy-4-sulfamoylphenyl)-2'-(trifluorophenyl)biphenyl-4-carboxamide,
2-{[(2,2-difluoroethyl)amino]methyl}-*N*-(3-methoxy-4-sulfamoylphenyl)-2'-(trifluoromethoxy)biphenyl-4-carboxamide,
*N*-[2-(hydroxymethyl)-2'-(trifluoromethoxy)biphenyl-4-yl]-3-methoxy-4-sulfamoylbenzamide,
2-{[(2,2-difluoroethyl)amino]methyl{-*N*-(3-methoxy-4-sulfamoylphenyl)-2'-methylbiphenyl-4-carboxamide,
2-{[(2,2-difluoroethyl)amino]methyl}-2'-fluoro-*N*-(3-methoxy-4-sulfamoylphenyl)biphenyl-4-carboxamide,
2'-chloro-2-{[(2,2-difluoroethyl)amino]methyl}-4'-fluoro-*N*-(3-methoxy-4-sulfamoylphenyl)biphenyl-4-carboxamide,
2'-chloro-2-{[(2,2-difluoroethyl)amino]methyl}-*N*-(3-methoxy-4-sulfamoylphenyl)biphenyl-4-carboxamide,
4'-chloro-2-{[(2,2-difluoroethyl)amino]methyl{-*N*-(3-methoxy-4-sulfamoylphenyl)-2'-(trifluorophenyl)biphenyl-4-carboxamide, and
2-{[(2,2-difluoroethyl)amino]methyl}-4'-fluoro-*N*-(3-methoxy-4-sulfamoylphenyl)-2'-(trifluorophenyl)-biphenyl-4-carboxamide.

Item 38: The compound of Item 36, or a pharmaceutically acceptable salt thereof, which is any one selected from the following group:

2-(hydroxymethyl)-*N*-[4-(methylsulfonyl)phenyl]-2'-(trifluorophenyl)biphenyl-4-carboxamide,
2-(hydroxymethyl)-*N*-[4-(methylsulfonyl)phenyl]-2'-(trifluoromethoxy)biphenyl-4-carboxamide,
2-{[(2,2-difluoroethyl)amino]methyl}-*N*-[4-(methylsulfonyl)phenyl]-2'-(trifluoromethoxy)biphenyl-4-carboxamide, and
2-{[(2-fluoroethyl)amino]methyl}-*N*-[4-(methylsulfonyl)phenyl]-2'-(trifluoromethoxy)biphenyl-4-carboxamide.

Item 39: A pharmaceutical composition, comprising the compound of any one of Items 1 to 38 or a pharmaceutically acceptable salt thereof.

Item 40: A medicament, comprising the compound of any one of Items 1 to 38 or a pharmaceutically acceptable salt thereof as the active ingredient.

Item 41: A preventive or therapeutic agent for hypertension, stroke, arrhythmia, heart failure, cardiomegaly, arteriosclerosis, vascular restenosis, renal fibrosis, cardiac infarction, diabetes complication, renal disease, edema, primary aldosteronism, inflammation, insulin resistance, sleep apnea syndrome, non-alcoholic steatohepatitis, or Cushing's syndrome, which comprises the compound of any one of Items 1 to 38, or a pharmaceutically acceptable salt thereof.

Item 42: A method for preventing or treating hypertension, stroke, arrhythmia, heart failure, cardiomegaly, arteriosclerosis, vascular restenosis, renal fibrosis, cardiac infarction, diabetes complication, renal disease, edema, primary aldosteronism, inflammation, insulin resistance, sleep apnea syndrome, non-alcoholic steatohepatitis, or Cushing's syndrome, which comprises administering an effective amount of the compound of any one of Items 1 to 38, or a pharmaceutically acceptable salt thereof to a patient in need.

Item 43: Use of the compound of any one of Items 1 to 38 or a pharmaceutically acceptable salt thereof in the manufacture of a medicament for preventing or treating hypertension, stroke, arrhythmia, heart failure, cardiomegaly, arteriosclerosis, vascular restenosis, renal fibrosis, cardiac infarction, diabetes complication, renal disease, edema, primary aldosteronism, inflammation, insulin resistance, sleep apnea syndrome, non-alcoholic steatohepatitis, or Cushing's syndrome.

EFFECT OF THE INVENTION

The compound of the present invention has a high binding affinity for and antagonistic activity against an aldosterone receptor. Thus, the compound is useful for prevention and/or treatment for various diseases involving a mineralcorticoid receptor (MR) including hypertension, stroke, arrhythmia, heart failure, cardiomegaly, arteriosclerosis, vascular restenosis, renal fibrosis, cardiac infarction, diabetes complication, renal disease, edema, primary aldosteronism, inflammation, insulin resistance, sleep apnea syndrome, non-alcoholic steatohepatitis, Cushing's syndrome, etc. The compound of the present invention may also reduce side effects (e.g., hormonal-like side effects, hyperpotassemia, etc.) compared to the conventional aldosterone receptor antagonist (e.g., spironolactone and eplerenone, etc.).

DESCRIPTION OF EMBODIMENTS

The present invention is explained in more detail as follows.
The number of substituents in groups defined by using "optionally substituted" or "substituted" herein is not limited as long as the substituents may bind, and is 1 or multiple. Unless otherwise specified, each explanation of each group is applicable to a group which is a part of or a substituent of other group.

Herein, "halogen atom" includes, for example, fluorine atom, chlorine atom, bromine atom, or iodine atom, etc. Preferable one is fluorine atom, or chlorine atom.

"$C_{1-6}$ alkyl group" is straight- or branched-chain saturated hydrocarbon group with 1 to 6 carbon atoms. Preferable one includes $C_{1-4}$alkyl group, etc. A concrete example of "$C_{1-6}$alkyl group" includes, for example, methyl, ethyl, propyl, 1-methylethyl, butyl, 2-methylpropyl, 1-methylpropyl, 1,1-dimethylethyl, pentyl, 3-methylbutyl, 2-methylbutyl, 2,2-dimethylpropyl, 1-ethylpropyl, 1,1-dimethylpropyl, hexyl, 4-methylpentyl, 3-methylpentyl, 2-methylpentyl or 1-methylpentyl, etc.

"$C_{1-6}$alkyl" moiety of "$C_{1-6}$alkylthio group" is the same as defined in the above "$C_{1-6}$alkyl". "$C_{1-6}$ alkylthio group" preferably includes "$C_{1-4}$ alkylthio group", etc. A concrete example of "$C_{1-6}$alkylthio group" includes, for example, methylthio, ethylthio, propylthio, 1-methylethylthio, butylthio, 2-methylpropylthio, 1-methylpropylthio, 1,1-dimethylethylthio, pentylthio, 3-methylbutylthio, 2-methylbutylthio, 2,2-dimethylpropylthio, 1-ethylpropylthio, 1,1-dimethylpropylthio, hexylthio, 4-methylpentylthio, 3-methylpentylthio, 2-methylpentylthio or 1-methylpentylthio, etc.

"$C_{3-10}$ cycloalkyl group" is cyclic saturated hydrocarbon group with 3 to 10 carbon atoms. Preferable one is "$C_{3-7}$ cycloalkyl group", and more preferable one includes "$C_{3-6}$ cycloalkyl group", etc. A concrete example of "$C_{3-10}$ cycloalkyl group" includes, for example, cyclopropyl, cyclobutyl, cyclopentyl, cyclohexyl or cycloheptyl, etc. The "$C_{3-10}$ cycloalkyl group" includes fused ring compounds with aromatic ring. As a concrete example, the following groups, etc. are included, for example.

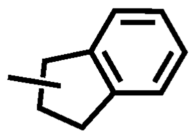

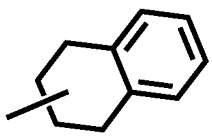

"$C_{1-6}$alkyl" moiety of "$C_{1-6}$alkoxy group" is the same as defined in the above "$C_{1-6}$alkyl". "$C_{1-6}$ alkoxy group" preferably includes "$C_{1-4}$alkoxy group", etc. A concrete example of "$C_{1-6}$alkoxy group" includes, for example, methoxy, ethoxy, propoxy, 1-methylethoxy, butoxy, 2-methylpropoxy, 1-methylpropoxy, 1,1-dimethylethoxy, pentyloxy, 3-methylbutoxy, 2-methylbutoxy, 2,2-dimethylpropoxy, 1-ethylpropoxy, 1,1-dimethylpropoxy, hexyloxy, 4-methylpentyloxy, 3-methylpentyloxy, 2-methylpentyloxy, 1-methylpentyloxy, 3,3-dimethylbutoxy, 2,2-dimethylbutoxy, 1,1-dimethylbutoxy or 1,2-dimethylbutoxy, etc.

"$C_{3-10}$cycloalkyl" moiety of "$C_{3-10}$ cycloalkoxy group" is the same as defined in the above "$C_{3-10}$ cycloalkyl". Preferable one is "$C_{3-7}$ cycloalkoxy group", and more preferable one is "$C_{3-6}$ cycloalkoxy group", etc. A concrete example of "$C_{3-10}$ cycloalkoxy group" includes, for example, cyclopropoxy, cyclobutoxy, cyclopentyloxy, cyclohexyloxy or cy-

cloheptyloxy, etc.

"$C_{1-6}$alkoxy" moiety of "$C_{1-6}$alkoxycarbonyl group" is the same as defined in the above "$C_{1-6}$ alkoxy". A concrete example of "$C_{1-6}$ alkoxycarbonyl group" includes, for example, straight- or branched-chain alkoxycarbonyl group with 2 to 7 carbon atoms. A concrete example includes, for example, methoxycarbonyl, ethoxycarbonyl, propoxycarbonyl, 2-methylethoxycarbonyl, butoxycarbonyl, 2-methylpropoxycarbonyl, 1-methylpropoxycarbonyl or 1,1-dimethylethoxycarbonyl, etc..

"$C_{1-6}$alkyl" moiety of "$C_{1-6}$alkylcarbonyl group" is the same as defined in the above "$C_{1-6}$alkyl". A concrete example of "$C_{1-6}$alkylcarbonyl group" includes, for example, methylcarbonyl, ethylcarbonyl, propylcarbonyl, 1-methylethylcarbonyl, butylcarbonyl, 2-methylpropylcarbonyl, 1-methylpropylcarbonyl or 1,1-dimethylethylcarbonyl, etc.

"$C_{1-6}$alkylcarbonyl" moiety of "$C_{1-6}$alkylcarbonyloxy group" is the same as defined in the above "$C_{1-6}$alkylcarbonyl". "$C_{1-6}$alkylcarbonyloxy group" preferably includes "$C_{1-4}$ alkylcarbonyloxy group", etc. A concrete example of "$C_{1-6}$alkylcarbonyloxy group" includes, for example, methylcarbonyloxy, ethylcarbonyloxy, propylcarbonyloxy, 1-methylethylcarbonyloxy, butylcarbonyloxy, 2-methylpropylcarbonyloxy, 1-methylpropylcarbonyloxy, 1,1-dimethylethylcarbonyloxy, pentylcarbonyloxy, 3-methylbutylcarbonyloxy, 2-methylbutylcarbonyloxy, 2,2-dimethylpropylcarbonyloxy, 1-ethylpropylcarbonyloxy, 1,1-dimethylpropylcarbonyloxy, hexylcarbonyloxy, 4-methylpentylcarbonyloxy, 3-methylpentylcarbonyloxy, 2-methylpentylcarbonyloxy or 1-methylpentylcarbonyloxy, etc.

"$C_{1-6}$alkyl" moiety of "$C_{1-6}$alkylsulfonyl group" is the same as defined in the above "$C_{1-6}$alkyl". A concrete example of "$C_{1-6}$alkylsulfonyl group" includes, for example, methanesulfonyl, ethanesulfonyl, propylsulfonyl, 1-methylethylsulfonyl, 2-methylethylsulfonyl, 1-methylpropylsulfonyl, 2-methylpropylsulfonyl, 1,1-dimethylethylsulfonyl or butylsulfonyl, etc.

"$C_{1-6}$alkylsulfonyl" moiety of "$C_{1-6}$alkylsulfonylamino group" is the same as defined in the above "$C_{1-6}$alkylsulfonyl". A concrete example of "$C_{1-6}$alkylsulfonylamino group" includes, for example, methanesulfonylamino, ethanesulfonylamino, propylsulfonylamino, 1-methylethylsulfonylamino, 2-methylethylsulfonylamino, 1-methylpropylsulfonylamino, 2-methylpropylsulfonylamino, 1,1-dimethylethylsulfonylamino or butylsulfonylamino, etc.

"$C_{1-6}$alkylsulfonyl" moiety of "$C_{1-6}$alkylsulfonyloxy group" is the same as defined in the above "$C_{1-6}$alkylsulfonyl". A concrete example of "$C_{1-6}$alkylsulfonyloxy group" includes, for example, methanesulfonyloxy, ethanesulfonyloxy, propylsulfonyloxy, 1-methylethylsulfonyloxy, 2-methylethylsulfonyloxy, 1-methylpropylsulfonyloxy, 2-methylpropylsulfonyloxy, 1,1-dimethylethylsulfonyloxy or butylsulfonyloxy, etc.

"$C_{3-10}$ cycloalkyl-$C_{1-4}$ alkyl group" is a group wherein the above "$C_{3-10}$ cycloalkyl" is substituted on "$C_{1-4}$ alkyl". A concrete example of "$C_{3-10}$ cycloalkyl-$C_{1-4}$ alkyl group" includes, for example, cyclopropylmethyl, cyclobutylmethyl, cyclopentylmethyl, cyclohexylmethyl, cycloheptylmethyl, cyclohexylethyl, cyclohexylpropyl or cyclohexylbutyl, etc. Preferable one includes (cycloalkyl with 3 to 6 carbon atoms)-$C_{1-4}$alkyl group ($C_3$-$_6$cycloalkyl-$C_{1-4}$ alkyl group).

"$C_{6-10}$ aryl group" is aromatic hydrocarbon group with 6 to 10 carbon atoms. A concrete example of "$C_{6-10}$ aryl group" includes, for example, phenyl, 1-naphthyl or 2-naphthyl, etc.

"Heteroaryl group" includes, for example, 5- to 10-membered monocyclic or polycyclic group, etc., and the group contains the same or different one or more (e.g. 1 to 4) heteroatoms selected from nitrogen atom, sulfur atom or oxygen atom. Preferable one includes, for example, 5- or 6-membered monocyclic group, etc. A concrete example of "heteroaryl group" includes pyrrolyl, thienyl, benzothienyl, benzofuranyl, benzoxazolyl, benzthiazolyl, furyl, oxazolyl, thiazolyl, isoxazolyl, isothiazolyl, benzisoxazolyl, benzisothiazolyl, imidazolyl, pyrazolyl, pyridyl, pyrazyl, pyrimidyl, pyridazyl, quinolyl, isoquinolyl, triazolyl, triazinyl, tetrazolyl, indolyl, imidazo[1,2-a]pyridyl, pyrazolo[1,5-a]pyridyl, [1,2,4]triazolo[1,5-a]pyridyl, benzimidazolyl, quinoxalyl, cinnolyl, quinazolyl, indazolyl, naphthylidyl, quinolinolyl or isoquinolinolyl, etc.

"Heterocyclic group" includes, for example, 3- to 7-membered heterocyclic group with the same or different 1 to 3 atoms selected from nitrogen atom, oxygen atom or sulfur atom, etc. Preferable one is 4- to 7-membered group, and more preferable one is 5- or 6-membered group. All of the above nitrogen atom, oxygen atom and sulfur atom are ring-constituent atom. The heterocyclic group may be either saturated or partially unsaturated heterocyclic group, more preferably saturated heterocyclic group. A concrete example of "heterocyclic group" includes pyranyl, tetrahydrofuryl, pyrrolidinyl, imidazolidinyl, piperidinyl, morpholinyl, thiomorpholinyl, dioxothiomorpholinyl, hexamethyleneiminyl, oxazolidinyl, thiazolidinyl, imidazolidinyl, oxoimidazolidinyl, dioxoimidazolidinyl, oxooxazolidinyl, dioxooxazolidinyl, dioxothiazolidinyl, tetrahydrofuranyl or tetrahydropyridinyl, etc. The group includes heterocyclic group with a bridged structure. In the group, ring-constituent nitrogen atom may not be a binding point of the "group". In other words, the group does not include, for example, a concept such as pyrrolidino group.

The above "heterocyclic group" may form a fused ring with 6-membered aromatic hydrocarbon or 6-membered heteroaryl. For example, bicyclic "heterocycle" with 11 or 12 ring-constituent atoms wherein the above 5- or 6-membered "heterocyclic group" is fused with 6-membered aromatic hydrocarbon or 6-membered heteroaryl is included. The 6-membered aromatic hydrocarbon includes benzene, etc. The 6-membered unsaturated heterocycle includes pyridine, pyrimidine or pyridazine, etc. A concrete example of the fused ring includes dihydroindolyl, dihydroisoindolyl, dihydropurinyl, dihydrothiazolopyrimidinyl, dihydrobenzodioxanyl, isoindolinyl, indazolyl, pyrrolidinyl, tetrahydroquinolinyl, decahydroquinolinyl, tetrahydroisoquinolinyl, decahydroisoquinolinyl, tetrahydronaphthylidinyl or tetrahydropyridoazepinyl, etc.

"$C_{7-14}$ aralkyl group" is a group wherein the above "$C_{6-10}$ aryl group" is substituted on the above "$C_{1-4}$alkyl group". Preferable one includes "$C_{7-10}$ aralkyl group" ($C_6$ aryl-$C_{1-4}$ alkyl group). A concrete example of "$C_{7-14}$ aralkyl group" includes, for example, benzyl, phenethyl, phenylpropyl or naphthylmethyl, etc.

"Saturated heterocyclic" moiety of "saturated heterocyclic carbonyl group" is the same as defined in the above "saturated heterocycle". The "saturated heterocyclic" preferably includes 4- to 7-membered saturated heterocyclic group, etc. A concrete example of "saturated heterocyclic carbonyl group" includes, for example, azetidinecarbonyl, oxetanecarbonyl, tetrahydropyranecarbonyl, tetrahydropyridinecarbonyl, pyrrolidinecarbonyl, oxopyrrolidinecarbonyl, tetrahydrofuranecarbonyl, piperidinecarbonyl, piperazinecarbonyl, morpholinecarbonyl, thiomorpholinecarbonyl, dioxoimidazolidinecarbonyl, azepanecarbonyl or oxoazepanecarbonyl, etc.

"Heteroaryl" moiety of "heteroarylcarbonyl group" is the same as defined in the above "heteroaryl". The "heteroaryl" preferably includes 5- or 6-membered monocyclic group, etc. A concrete example of "heteroarylcarbonyl group" includes, for example, pyrrolecarbonyl, thiophenecarbonyl, furanecarbonyl, oxazolecarbonyl, thiazolecarbonyl, isoxazolecarbonyl, isothiazolecarbonyl, imidazolecarbonyl, pyrazolecarbonyl, pyridinecarbonyl, pyrazinecarbonyl, pyrimidinecarbonyl, pyridazinecarbonyl, triazolecarbonyl, triazinecarbonyl or tetrazolecarbonyl, etc.

"Heteroaryloxy group" is a group wherein the above "heteroaryl group" is substituted on oxygen atom. The "heteroaryl" moiety preferably includes 5- or 6-membered monocyclic group, etc. A concrete example of "heteroaryloxy group" includes, for example, pyrrolyloxy, thienyloxy, benzothienyloxy, benzofuranyloxy, benzoxazolyloxy, benzthiazolyloxy, furyloxy, oxazolyloxy, thiazolyloxy, isoxazolyloxy, isothiazolyloxy, imidazolyloxy, pyrazolyloxy, pyridyloxy, pyrazyloxy, pyrimidyloxy, pyridazyloxy, quinolyloxy, isoquinolyloxy, triazolyloxy, triazinyloxy, tetrazolyloxy, indolyloxy, imidazo [1,2-a]pyridyloxy, dibenzofuranyloxy, benzimidazolyloxy, quinoxalyloxy, cinnolyloxy, quinazolyloxy, indazolyloxy, naphthylidyloxy, quinolinolyloxy or isoquinolinolyloxy, etc.

"Saturated heterocyclic oxy group" is a group wherein the above "saturated heterocycle" is substituted on oxygen atom. Preferable one is "4- to 7-membered saturated heterocyclic oxy group". A concrete example of "saturated heterocyclic oxy group" includes, for example, azetidinyloxy, oxetanyloxy, tetrahydropyranyloxy, tetrahydropyridinyloxy, pyrrolidinyloxy, oxopyrrolidinyloxy, tetrahydrofuranyloxy, piperidinyloxy, azepanyloxy or oxoazepanyloxy, etc.

"$C_{7-14}$ aralkyloxy group" is a group wherein the above "$C_{7-14}$ aralkyl group" is substituted on oxygen atom. Preferable one includes $C_{7-10}$ aralkyloxy group, etc. A concrete example of "$C_{7-14}$ aralkyloxy group" includes, for example, benzyloxy, phenethyloxy, phenylpropyloxy or naphthylmethyloxy, etc.

"5- or 6-Membered monocyclic heteroaryl-$C_{1-4}$ alkyl group" is a group wherein the above "5- or 6-membered monocyclic heteroaryl" is substituted on the above "$C_{1-4}$alkyl". A concrete example of "5-or 6-membered monocyclic heteroaryl-$C_{1-4}$ alkyl group" includes, for example, pyrrolylmethyl, thienylmethyl, furylmethyl, etc.

"5- or 6-Membered saturated heterocyclic-$C_{1-4}$ alkyl group" is a group wherein the above "5- or 6-membered saturated heterocycle" is substituted on the above "$C_{1-4}$ alkyl". A concrete example of "5- or 6-membered saturated heterocyclic-$C_{1-4}$ alkyl group" includes, for example, tetrahydropyranylmethyl, pyrrolidinylmethyl, etc.

"Optionally substituted amino group" includes, for example, amino, mono- or di-substituted amino, 4- to 8-membered cyclic amino, etc.

A substituent of "mono- or di-substituted amino" includes, for example, "$C_{1-6}$alkyl", "$C_{3-10}$ cycloalkyl", "$C_{3-10}$ cycloalkyl-$C_{1-4}$ alkyl", "5- or 6-membered saturated heterocycle", "5- or 6-membered saturated heterocyclic-$C_{1-4}$ alkyl", "5- or 6-membered monocyclic heteroaryl", "5- or 6-membered monocyclic heteroaryl-$C_{1-4}$ alkyl", etc.

A concrete example of "mono-substituted amino" includes, for example,"mono-$C_{1-6}$ alkylamino" (e.g., methylamino, ethylamino, propylamino, 1-methylethylamino, butylamino, 2-methylpropylamino, 1-methylpropylamino, 1,1-dimethylethylamino, etc.),

"$C_{3-10}$ cycloalkylamino" (e.g., cyclopropylamino, cyclobutylamino, cyclopentylamino, cyclohexylamino, cycloheptylamino, etc.),

"($C_{3-10}$ cycloalkyl-$C_{1-4}$ alkyl)amino" (e.g., cyclopropylmethylamino, cyclobutylmethylamino, cyclopentylmethylamino, cyclohexylmethylamino, cycloheptylmethylamino, etc.),

"5- or 6-membered saturated heterocyclic-amino" (e.g., tetrahydropyranylamino, tetrahydropyridinylamino, pyrrolidinylamino, oxopyrrolidinylamino, tetrahydrofuranylamino, piperidinylamino, etc.),

"(5- or 6-membered saturated heterocyclic-$C_{1-4}$ alkyl)amino" (e.g., tetrahydropyranylmethylamino, tetrahydropyridinylmethylamino, pyrrolidinylmethylamino, oxopyrrolidinylmethylamino, tetrahydrofuranylmethylamino, piperidinylmethylamino, piperazinylmethylamino, morpholinylmethylamino, etc.),

"(5- or 6-membered monocyclic heteroaryl)amino" (e.g., pyrrolylamino, thienylamino, furylamino, oxazolylamino, thiazolylamino, isoxazolylamino, isothiazolylamino, imidazolylamino, pyrazolylamino, triazolylamino, oxadiazolylamino, thiadiazolylamino, tetrazolylamino, pyridylamino, pyrazylamino, pyrimidylamino, pyridazylamino, triazylamino, etc.),

"(5- or 6-membered monocyclic heteroaryl-$C_{1-4}$ alkyl)amino" (e.g., pyrrolylmethylamino, thienylmethylamino, furylmethylamino, oxazolylmethylamino, thiazolylmethylamino, isoxazolylmethylamino, isothiazolylmethylamino, imidazolylmethylamino, pyrazolylmethylamino, triazolylmethylamino, oxadiazolylmethylamino, thiadiazolylmethylamino, tetrazolylmethylamino, pyridylmethylamino, pyrazylmethylamino, pyrimidylmethylamino, pyridazylmethylamino, triazylmethylamino, etc.), etc.

A concrete example of "di-substituted amino" includes , for example,
"di-$C_{1-6}$ alkylamino" (e.g., dimethylamino, diethylamino, dipropylamino, di-1-methylethylamino, dibutylamino, di-2-methylpropylamino, di-1-methylpropylamino, di-1,1-dimethylethylamino, etc.),
"N-($C_{1-6}$ alkyl)-N-($C_{3-10}$ cycloalkyl)amino" (e.g., methylcyclopropylamino, methylcyclobutylamino, methylcyclopentylamino, methylcyclohexylamino, methylcycloheptylamino, etc.),
"N-($C_{1-6}$alkyl)-N-(5- or 6-membered saturated heterocyclic)amino" (e.g., methyltetrahydropyranylamino, methyltetrahydropyridinylamino, methylpyrrolidinylamino, methyloxopyrrolidinylamino, methyltetrahydrofuranylamino, methylpiperidinylamino, etc.), etc.

"4- to 8-Membered cyclic amino group" includes, for example, 4- to 8-membered monocyclic cyclic amino group with the same or different 1 to 3 heteroatoms selected from nitrogen atom, oxygen atom or sulfur atom. Preferable one is 4- to 7-membered monocyclic cyclic amino group. In "4- to 8-membered cyclic amino group", a nitrogen atom in the ring is directly a bonding site of the "group". A concrete example of "4- to 8-membered cyclic amino group" includes, for example, azetidino, pyrrolidino, imidazolidino, oxazolidino, thiazolidino, piperazino, piperidino, morpholino, thiomorpholino, azepano or oxoazepano, etc. The group also includes a cyclic amino group wherein a ring contains partially unsaturated bonds.

"4- to 8-Membered cyclic amino group" or "4- to 7-membered cyclic amino group" may form a fused ring with $C_{3-6}$ cycloalkyl, 6-membered aromatic hydrocarbon or 5- or 6-membered heterocycle. A concrete example of the fused ring includes the following "group", etc.

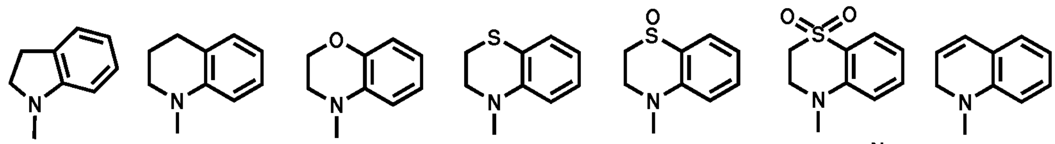

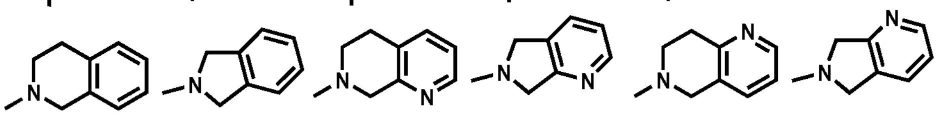

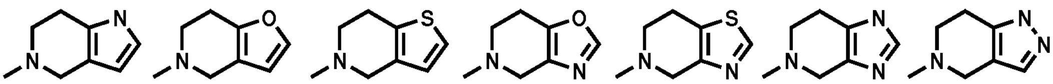

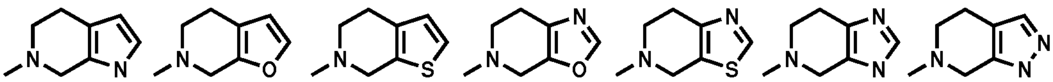

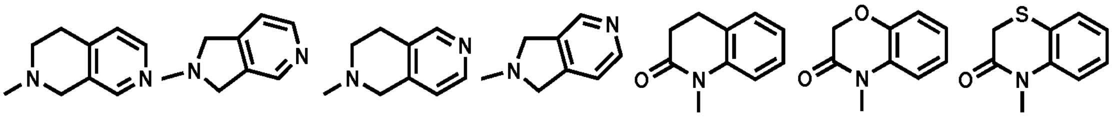

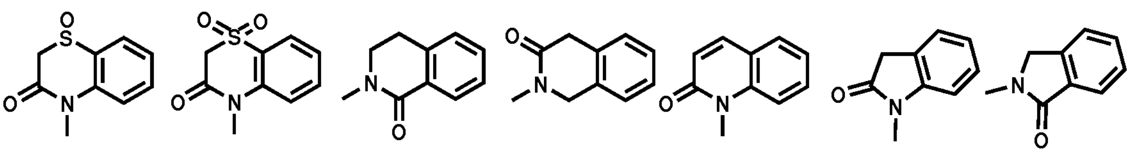

"4- to 7-Membered cyclic amino" moiety of "4- to 7-membered cyclic aminocarbonyl group" is the same as defined in the above "4- to 7-membered cyclic amino". "4- to 7-Membered cyclic aminocarbonyl group" includes, for example, 4- to 7-membered monocyclic cyclic aminocarbonyl group with the same or different 1 to 3 heteroatoms selected from nitrogen atom, oxygen atom or sulfur atom. A concrete example includes, for example, azetidinocarbonyl, pyrrolidinocarbonyl, imidazolidinocarbonyl, oxazolidinocarbonyl, thiazolidinocarbonyl, piperazinocarbonyl, piperidinocarbonyl, morpholinocarbonyl, thiomorpholinocarbonyl, azepanocarbonylor oxoazepanocarbonyl, etc..

"$C_{1-6}$alkylcarbonyl" moiety of "$C_{1-6}$alkylcarbonylamino group" is the same as defined in the above

"$C_{1-6}$alkylcarbonyl". "$C_{1-6}$ alkylcarbonylamino group" preferably includes "$C_{1-4}$ alkylcarbonylamino group". A concrete example of "$C_{1-6}$alkylcarbonylamino group" includes, for example, methylcarbonylamino, ethylcarbonylamino, propylcarbonylamino, 1-methylethylcarbonylamino, butylcarbonylamino, 2-methylpropylcarbonylamino, 1-methylpropylcarbonylamino, 1,1-dimethylethylcarbonylamino, pentylcarbonylamino, 3-methylbutylcarbonylamino, 2-methylbutylcarbonylamino, 2,2-dimethylpropylcarbonylamino, 1-ethylpropylcarbonylamino, 1,1-dimethylpropylcarbonylamino, hexylcarbonylamino, 4-methylpentylcarbonylamino, 3-methylpentylcarbonylamino, 2-methylpentylcarbonylamino or 1-methylpentylcarbonylamino, etc.

"$C_{3-10}$ cycloalkyl" moiety of "$C_{3-10}$ cycloalkylcarbonylamino group" is the same as defined in the above "$C_{3-10}$ cycloalkyl". Preferable one includes "$C_{3-7}$ cycloalkylcarbonylamino group", and more preferable one includes "$C_{3-6}$ cycloalkylcarbonylamino group". A concrete example of "$C_{3-10}$ cycloalkylcarbonylamino group" includes, for example, cyclopropylcarbonylamino, cyclobutylcarbonylamino, cyclopentylcarbonylamino, cyclohexylcarbonylamino or cycloheptylcarbonylamino, etc.

"Saturated heterocyclic" moiety of "saturated heterocyclic carbonylamino group" is the same as defined in the above "saturated heterocycle". Preferable one includes "4- to 7-membered saturated heterocyclic carbonylamino group", etc. A concrete example of "saturated heterocyclic carbonylamino group" includes, for example, azetidinecarbonylamino, oxetanecarbonylamino, tetrahydropyranecarbonylamino, tetrahydropyridinecarbonylamino, pyrrolidinecarbonylamino, oxopyrrolidinecarbonylamino, tetrahydrofuranecarbonylamino, piperidinecarbonylamino, piperazinecarbonylamino, morpholinecarbonylamino, thiomorpholinecarbonylamino, dioxoimidazolidinecarbonylamino, azepanecarbonylamino or oxoazepanecarbonylamino, etc.

"$C_{1-6}$alkoxy" moiety of "$C_{1-6}$alkoxycarbonylamino group" is the same as defined in the above "$C_{1-6}$ alkoxy". A concrete example of "$C_{1-6}$alkoxycarbonylamino group" includes, for example, methoxycarbonylamino, ethoxycarbonylamino, propoxycarbonylamino, 2-methylethoxycarbonylamino, butoxycarbonylamino, 2-methylpropoxycarbonylamino, 1-methylpropoxycarbonylamino or 1,1-dimethylethoxycarbonylamino, etc.

"Heteroaryl" moiety of "heteroarylcarbonylamino group" is the same as defined in the above "heteroaryl". Preferable one is "5- or 6-membered monocyclic heteroarylcarbonylamino group". A concrete example of "heteroarylcarbonylamino group" includes, for example, pyrrolecarbonylamino, thiophenecarbonylamino, furanecarbonylamino, oxazolecarbonylamino, thiazolecarbonylamino, isoxazolecarbonylamino, isothiazolecarbonylamino, imidazolecarbonylamino, pyrazolecarbonylamino, pyridylcarbonylamino, pyrazinecarbonylamino, pyrimidinecarbonylamino, pyridazinecarbonylamino, triazolecarbonylamino, triazinecarbonylamino or tetrazolecarbonylamino, etc.

A substituent in "optionally substituted $C_{1-6}$alkyl group" includes, for example,

(a) halogen atom,
(b) cyano group,
(c) hydroxy group,
(d) formyl group,
(e) $C_{1-6}$alkylcarbonyl group,
(f) $C_{1-6}$alkylcarbonyloxy group,
(g) carboxyl group,
(h) amino (in which the amino may be optionally substituted with the same or different 1 to 2 groups selected from the group consisting of

 (h1) $C_{1-6}$alkyl (in which the alkyl may be optionally substituted with

  (h11) hydroxy,
  (h12) cyano,
  (h13) halogen atom,
  (h14) amino (in which the amino may be optionally substituted with the same or different 1 to 2 $C_{1-6}$alkyl groups),
  (h15) $C_{1-6}$alkoxy,
  (h16) 5- or 6-membered monocyclic heteroaryl, or
  (h17) 4- to 7-membered saturated heterocycle),

 (h2) $C_{3-10}$cycloalkyl (in which the ring may be optionally substituted with $C_{1-6}$alkyl),
 (h3) $C_{3-10}$cycloalkyl-$C_{1-4}$ alkyl,
 (h4) 4- to 7-membered saturated heterocycle (in which the ring may be optionally substituted with halogen atom, or $C_{1-6}$ alkyl),
 (h5) 4- to 7-membered saturated heterocyclic-$C_{1-4}$ alkyl,
 (h6) $C_{6-10}$ aryl (in which the ring may be optionally substituted with halogen atom, $C_{1-6}$ alkyl or $C_{1-6}$ alkoxy),

(h7) $C_{7-14}$aralkyl (in which the ring may be optionally substituted with halogen atom, $C_{1-6}$ alkyl or $C_{1-6}$ alkoxy),
(h8) 5- or 6-membered monocyclic heteroaryl (in which the ring may be optionally substituted with halogen atom, or $C_{1-6}$ alkyl), and
(h9) 5- or 6-membered monocyclic heteroaryl-$C_{1-4}$ alkyl),

(i) $C_{1-6}$ alkoxy (in which the group may be optionally substituted with

(i1) hydroxy,
(i2) $C_{1-6}$ alkoxy (in which the group may be optionally substituted with 1 to 3 fluorine atoms),
(i3) $C_{3-10}$cycloalkyl,
(i4) 4- to 7-membered cyclic amino (in which the ring may be optionally substituted with

(i41) hydroxy,
(i42) cyano,
(i43) 1 to 4 fluorine atoms,
(i44) $C_{1-6}$ alkyl (in which the alkyl may be optionally substituted with 1 to 3 fluorine atoms, or $C_{1-6}$alkoxy),
(i45) $C_{1-6}$alkoxy,
(i46) formyl,
(i47) $C_{1-6}$alkylcarbonyl,
(i48) $C_{1-6}$alkylsulfonyl, or
(i49) oxo),

(i5) 4- to 7-membered saturated heterocycle (in which the heterocycle may be optionally substituted with group(s) selected from the above (i41) to (i49)),
(i6) 5- or 6-membered monocyclic heteroaryl (in which the ring may be optionally substituted with halogen atom, or $C_{1-6}$ alkyl),
(i7) $C_{6-10}$aryl (in which the group may be optionally substituted with halogen atom, $C_{1-4}$ alkyl, or $C_{1-4}$ alkoxy),
(i8) $C_{1-6}$alkylcarbonylamino,
(i9) amino (in which the amino may be optionally substituted with the same or different 1 to 2 groups selected from the group consisting of $C_{1-6}$ alkyl (in which the group may be optionally substituted with hydroxy), $C_{3-10}$cycloalkyl, and $C_{3-10}$cycloalkyl-$C_{1-4}$ alkyl),
(i10) mono- or di-$C_{1-6}$alkylaminocarbonyl,
(i11) halogen atom, or
(i12) $C_{7-14}$aralkyloxy),

(j) $C_{3-7}$cycloalkoxy group (in which the ring may be optionally substituted with $C_{1-6}$ alkyl),
(k) oxo group,
(l) 5- or 6-membered monocyclic heteroarylgroup (in which the ring may be optionally substituted with halogen atom, or $C_{1-6}$ alkyl),
(m) 4- to 7-membered saturated heterocyclic group (in which the heterocycle may be optionally substituted with group(s) selected from the above (i41) to (i49)),
(n) optionally substituted aminocarbonyl group,
(o) optionally substituted aminosulfonyl group,
(p) optionally substituted aminocarbonyloxy group,
(q) $C_{7-14}$ aralkyloxy group
(r) 5- or 6-membered monocyclic heteroaryloxy group (in which the ring may be optionally substituted with halogen atom, or $C_{1-6}$ alkyl),
(s) 4- to 7-membered saturated heterocyclic oxy group (in which the ring may be optionally substituted with $C_{1-6}$ alkyl which may be optionally substituted with 1 to 3 halogen atoms),
(t) $C_{1-6}$alkylsulfonyl group,
(v) $C_{1-6}$alkoxycarbonyl group,
(u) $C_{1-6}$alkylsulfonyloxy group,
(v) $C_{1-6}$alkoxycarbonylamino group,
(x) $C_{1-6}$ alkylcarbonylamino group (in which the group may be optionally substituted with

(x1) hydroxy,
(x2) halogen atom,
(x3) $C_{1-6}$alkoxy, or

(x4) $C_{1-6}$alkylcarbonyloxy),

(y) 5- or 6-membered monocyclic heteroarylcarbonylamino group (in which the group may be optionally substituted with $C_{1-6}$ alkyl),
(z) 4- to 7-membered saturated heterocyclic carbonylamino group (in which the group may be optionally substituted with $C_{1-6}$ alkyl which may be optionally substituted with 1 to 3 fluorine atoms. ), (aa) mono- or di-$C_{1-6}$alkylaminocarbonylamino group, or
(ab) 4- to 7-membered cyclic amino group (in which the ring may be optionally substituted with group(s) selected from the above (i41) to (i49)), etc.

"Optionally substituted amino" moiety in "optionally substituted aminocarbonyl group", "optionally substituted aminosulfonyl group", "optionally substituted aminocarbonylamino group", and "optionally substituted aminocarbonyloxy group" is mono-substituted amino group, or di-substituted amino group. Substituents of mono- or di-substituted amino are the same as defined in the above substituents (h1) to (h9). A concrete example of "optionally substituted amino" moiety is the same as defined in the above "mono-substituted amino" and "di-substituted amino".

Substituents in "optionally substituted $C_{1-6}$ alkylcarbonyl group", "optionally substituted $C_{1-6}$ alkylsulfonyl group", "optionally substituted $C_{1-6}$ alkylthio group", "optionally substituted $C_{1-6}$ alkylcarbonyloxy group", "optionally substituted $C_{1-6}$ alkylsulfonylamino group", "optionally substituted $C_{1-6}$ alkoxy group", "optionally substituted $C_{1-6}$ alkoxycarbonyl group", "optionally substituted $C_{1-6}$ alkylcarbonylamino group" and "optionally substituted $C_{1-6}$ alkoxycarbonylamino group" include, for example, one group selected from the group consisting of the above (a) to (ab) which are an exemplification of substituents in "optionally substituted $C_{1-6}$alkyl group", etc.

Substituents in "optionally substituted $C_{3-10}$ cycloalkyl group", "optionally substituted $C_{3-10}$ cycloalkoxy group", "optionally substituted 4- to 8-membered cyclic amino group", "optionally substituted 4- to 7-membered cyclic aminocarbonyl group", "optionally substituted 4- to 7-membered saturated heterocyclic group", "optionally substituted 4- to 7-membered saturated heterocyclic oxy group", "optionally substituted 4- to 7-membered saturated heterocyclic carbonylamino group", and "optionally substituted $C_{3-10}$ cycloalkylcarbonylamino group" includes, for example, 1 group selected from the group consisting of the above (a) to (ab) which are an exemplification of substituents in the above "optionally substituted $C_{1-6}$ alkyl group", and $C_{1-4}$ alkyl, etc. A ring in the substituents (e.g. cycloalkyl, cyclic amino, etc.) may be optionally substituted with oxo or thioxo.

Substituents in "optionally substituted $C_{6-10}$ aryl group", "optionally substituted 5- or 6-membered monocyclic heteroaryl group", "optionally substituted 5- or 6-membered monocyclic heteroaryloxy group", "optionally substituted 5- or 6-membered monocyclic heteroarylcarbonylamino group", "optionally substituted $C_{7-14}$aralkyl group" and "optionally substituted $C_{7-14}$aralkyloxy group" include, for example,

(a2) halogen atom,
(b2) cyano group,
(c2) optionally substituted $C_{1-6}$ alkyl group,
(d2) $C_{1-6}$ alkylsulfonyl group (in which the group may be optionally substituted with

(d21) halogen atom,
(d22) hydroxy,
(d23) $C_{1-6}$alkoxy,
(d24) $C_{3-10}$cycloalkyl,
(d25) $C_{3-10}$cycloalkoxy,
(d26) di-$C_{1-6}$alkylamino,
(d27) 4- to 7-membered cyclic amino, or
(d28) 4- to 7-membered saturated heterocycle),

(e2) amino group (in which the amino may be optionally substituted with the same or different 1 to 2 groups selected from the group consisting of the above (h1) to (h9)),
(f2) aminosulfonyl group (in which the amino may be optionally substituted with the same or different 1 to 2 $C_{1-6}$ alkyl groups (in which the alkyl may be optionally substituted with halogen atom, hydroxy, $C_{1-4}$alkoxy, or di-$C_{1-6}$ alkylamino)),
(g2) 4- to 7-membered cyclic amino group (in which the ring may be optionally substituted with the above (i1) to (i12)),
(h2) aminocarbonyl group (in which the amino may be optionally substituted with the same or different 1 to 2 groups selected from the above (h1) to (h9)),
(i2) 4- to 7-membered saturated heterocycle (in which the ring may be optionally substituted with group(s) selected from the above (i1) to (i12)),

(j2) carboxyl group,
(k2) $C_{1-6}$alkoxy group (in which the group may be optionally substituted with group(s) selected from the above (i1) to (i12)),
(12) $C_{3-10}$cycloalkoxy group,
(m2) 4- to 7-membered saturated heterocyclic oxy group (in which the ring may be optionally substituted with group(s) selected from the above (i1) to (i12)),
(n2) $C_{7-14}$aralkyloxy group,
(o2) $C_{1-6}$ alkoxycarbonyl group (in which the group may be optionally substituted with group(s) selected from the above (i1) to (i12)),
(p2) $C_{1-6}$ alkylcarbonylamino group (in which the amino may be optionally substituted with $C_{1-6}$ alkyl, and the alkyl may be optionally substituted with group(s) selected from the above (d21) to (d28)),
(q2) $C_{3-10}$cycloalkylcarbonylamino group (in which the amino may be optionally substituted with $C_{1-6}$ alkyl),
(r2) 5- or 6-membered monocyclic heteroarylcarbonylamino group (in which the amino may be optionally substituted with $C_{1-6}$ alkyl),
(s2) 4- to 7-membered saturated heterocyclic carbonylamino group (in which the amino may be optionally substituted with $C_{1-6}$ alkyl, and the ring may be optionally substituted with group(s) selected from the above (i1) to (i12)),
(t2) mono- or di-$C_{1-6}$ alkylaminocarbonylamino group (in which the amino may be optionally substituted with $C_{1-6}$alkyl),
(u2) $C_{1-6}$ alkoxycarbonylamino group (in which the amino may be optionally substituted with $C_{1-6}$ alkyl, and the alkoxy may be optionally substituted with group(s) selected from the above (i1) to (i12)), (v2) $C_{6-10}$aryl group, or
(w2) 5- or 6-membered monocyclic heteroaryl group, etc.

"Any one of $R^6$, $R^7$ and $R^8$ is hydrogen atom, and the remaining two groups are adjacent each other and may combine each other together with the ring atoms to which they bind to form $C_{3-7}$cycloalkyl ring, 5- or 6-membered saturated heterocycle, or 5- or 6-membered heteroaryl ring" means that aromatic ring of "A" in formula (1) combine together with any two of the adjacent $R^6$, $R^7$ and $R^8$ to form a bicyclic group. Herein, "$C_{3-7}$ cycloalkyl ring" is cyclic alkyl ring with 3 to 7 carbon atoms. "5- or 6-Membered saturated heterocycle" is 5- or 6-membered heterocycle with the same or different 1 to 3 atoms selected from nitrogen atom, oxygen atom or sulfur atom. "5- or 6-Membered heteroaryl ring" is heteroaromatic ring containing the same or different one or more (e.g. 1 to 4) heteroatoms selected from nitrogen atom, sulfur atom or oxygen atom. A concrete example of "$C_{3-7}$ cycloalkyl ring", "5- or 6-membered saturated heterocycle" and "5- or 6-membered heteroaryl ring" in the definition includes, for example, the following groups:
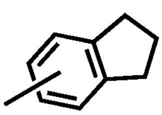 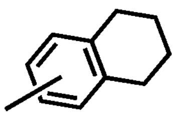 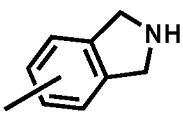 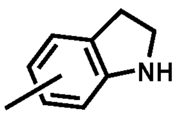 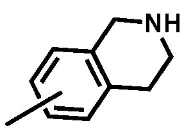 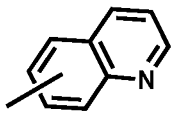

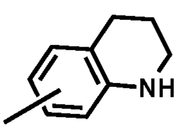 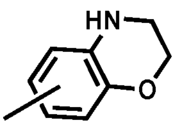 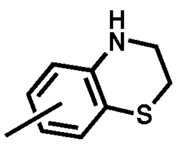 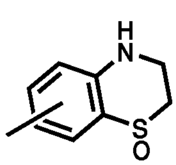 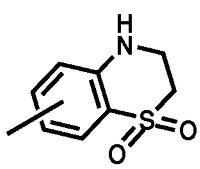 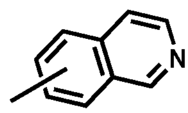 .

Said $C_{3-7}$ cycloalkyl ring, or 5- or 6-membered saturated heterocycle may be optionally substituted with oxo group.
A compound wherein 4- to 7-membered cyclic amino group is substituted with oxo group includes a compound wherein oxo is protected by substituion of ethylene glycol, etc.
A compound of formula (1) includes a compound wherein hydrogen atom is replaced with hydroxy in "$-(CH_2)_m-R^4$".
A preferable embodiment of the present invention is further illustrated.
"A" is preferably a group of formula (a) or formula (b):

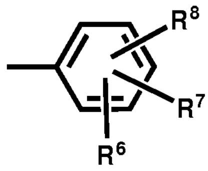

(a)

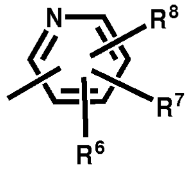

(b)

,

more preferably a group of formula (a).

"$R^1$" is preferably

1: aminosulfonyl group (in which the amino may be optionally substituted with the same or different 1 to 2 groups selected from the group consisting of

(a) $C_{1-6}$alkyl,
(b) $C_{1-6}$alkylcarbonyl,
(c) aminocarbonyl, and
(d) -C(=NH)-$NH_2$),

2: $C_{1-6}$ alkylsulfonyl group, or
3: $C_{1-6}$alkylsulfonylamino group.

More preferable one is

1: aminosulfonyl group (in which the amino may be optionally substituted with the same or different 1 to 2 groups selected from the group consisting of

(a) $C_{1-6}$alkyl,
(b) $C_{1-6}$alkylcarbonyl,
(c) aminocarbonyl, and
(d) -C(=NH)-$NH_2$), or

2: $C_{1-6}$alkylsulfonyl group.

"$R^1$" is more preferably aminosulfonyl group.
"$R^2$" is preferably hydrogen atom, hydroxy group, or $C_{1-6}$alkoxy group, more preferably $C_{1-6}$ alkoxy group.
"$R^3$" is preferably hydrogen atom.
"$R^4$" is preferably

1: hydrogen atom,
2: halogen atom,
3: hydroxy group,
4: cyano group,
5: nitro group,
6: formyl group,
7: carboxyl group,
8: amino group (in which the amino may be optionally substituted with the same or different 1 to 2 groups selected from the group consisting of

(a) $C_{1-6}$ alkyl (in which the group may be optionally substituted with

(i) 1 to 3 fluorine atoms,
(ii) cyano,
(iii) hydroxy,
(iv) amino (in which the amino may be optionally substituted with the same or different 1 to 2 $C_{1-6}$ alkyl),
(v) $C_{1-6}$alkoxy,
(vi) $C_{6-10}$aryloxy, or

(vii) aminocarbonyl),

(b) $C_{3-10}$cycloalkyl (in which the group may be optionally substituted with $C_{1-4}$alkyl),
(c) $C_{3-10}$cycloalkyl-$C_{1-4}$ alkyl,
(d) $C_{6-10}$aryl,
(e) $C_{7-14}$aralkyl (in which the group may be optionally substituted with halogen atom, or $C_{1-4}$alkoxy),
(f) 4- to 7-membered saturated heterocycle (in which the heterocycle may be optionally substituted with $C_{1-6}$alkyl),
(g) 4- to 7-membered saturated heterocycfic-$C_{1-4}$alkyl (in which the heterocycle may be optionally substituted with $C_{1-6}$alkyl),
(h) 5- or 6-membered monocyclic heteroaryl (in which the group may be optionally substituted with the same or different 1 to 2 groups selected from the group consisting of cyano and $C_{1-4}$alkyl), and
(i) 5- or 6-membered monocyclic heteroaryl-$C_{1-4}$ alkyl (in which the heteroaryl may be optionally substituted with $C_{1-4}$alkyl)),

9: $C_{1-6}$alkoxy group (in which the group may be optionally substituted with

(a) 1 to 2 hydroxy,
(b) amino (in which the amino may be optionally substituted with 1 or 2 $C_{1-6}$alkyl (in which the group may be optionally substituted with hydroxy)),
(c) 1 to 2 $C_{1-6}$alkoxy,
(d) 4- to 7-membered cyclic amino (in which the ring may be optionally substituted with $C_{1-6}$alkyl or oxo),
(e) 4- to 7-membered saturated heterocycle (in which the group may be optionally substituted with $C_{1-4}$alkyl),
(f) 5- or 6-membered monocyclic heteroaryl (in which the group may be optionally substituted with $C_{1-6}$alkyl), or
(g) $C_{7-14}$aralkyloxy),

10: $C_{1-6}$alkoxycarbonyl group,
11: 4- to 7-membered heterocyclic group (in which the ring may be optionally substituted with $C_{1-4}$alkyl or oxo),
12: 5- or 6-membered monocyclic heteroaryl group (in which the ring may be optionally substituted with $C_{1-6}$alkyl),
13: $C_{7-14}$aralkyloxy group,
14: $C_{1-6}$alkylcarbonyloxy group,
15: 4- to 8-membered cyclic amino group (in which the ring may be optionally substituted with the same or different 1 to 2 groups selected from the group consisting of

(a) hydroxy,
(b) $C_{1-4}$alkyl (in which the group may be optionally substituted with $C_{1-6}$alkoxy),
(c) cyano,
(d) 1 to 4 fluorine atoms,
(e) $C_{1-6}$alkoxy,
(f) formyl,
(g) $C_{1-6}$alkylcarbonyl,
(h) $C_{1-6}$alkylsulfonyl, and
(i) oxo),

16: saturated heterocyclic oxy group (in which the ring may be optionally substituted with the same or different 1 to 2 groups selected from $C_{1-6}$ alkyl or oxo), or
17: $C_{1-6}$alkoxycarbonylamino group.

"$R^4$" is more preferably amino group (in which the amino is substituted with $C_{1-6}$alkyl which is substituted with 1 to 3 fluorine atoms), particularly preferably 2,2-difluoroethylamino.

"$R^{5a}$", "$R^{5b}$" and "$R^{5c}$" are each independently and preferably

1: hydrogen atom, or
2: $C_{1-6}$ alkyl group, and more preferably, all of them are hydrogen atom.

"$R^6$" is preferably

1: halogen atom,
2: cyano group,

3: $C_{1-6}$alkyl group (in which the group may be optionally substituted with

(a) hydroxy,
(b) cyano,
(c) 1 to 4 fluorine atoms,
(d) amino (in which the amino may be optionally substituted with 1 to 2 $C_{1-6}$ alkyl),
(e) 4- to 7-membered cyclic amino (in which the ring may be optionally substituted with $C_{1-6}$ alkyl),
(f) $C_{1-6}$ alkoxy (in which the group may be optionally substituted with 1 to 3 fluorine atoms),
(g) 5- or 6-membered monocyclic heteroaryl,
(h) $C_{1-6}$alkylcarbonyl, or
(i) $C_{1-6}$alkoxycarbonyl),

4: amino group (in which the amino may be optionally substituted with the same or different 1 to 2 groups selected from the group consisting of

(a) $C_{1-6}$alkyl,
(b) $C_{3-10}$cycloalkyl,
(c) $C_{3-10}$cycloalkyl-$C_{1-4}$ alkyl, and
(d) 4- to 7-membered saturated heterocycle (in which the ring may be optionally substituted with $C_{1-6}$ alkyl)),

5: $C_{1-6}$ alkoxy group (in which the group may be optionally substituted with 1 to 6 fluorine atoms, or 4- to 7-membered cyclic amino),
6: $C_{3-10}$cycloalkyl group,
7: $C_{3-10}$ cyloalkoxy group,
8: $C_{1-6}$ alkylthio group,
9: $C_{1-6}$ alkoxycarbonyl group,
10: 4- to 7-membered cyclic amino group (in which the ring may be optionally substituted with $C_{1-6}$ alkyl),
11: 4- to 7-membered cyclic aminocarbonyl group,
12: $C_{610}$ aryl group, or
13: 5- or 6-membered monocyclic heteroaryl group (in which the ring may be optionally substituted with $C_{1-6}$ alkyl).
More preferable one is halogen atom, particularly preferably fluorine atom.

"$R^7$" is preferably

1: hydrogen atom,
2: halogen atom,
3: hydroxy group,
4: nitro group,
5: amino group,
6: $C_{1-6}$ alkyl group,
7: $C_{1-6}$ alkoxy group (in which the group may be optionally substituted with

(a) hydroxy,
(b) carboxyl,
(c) 1 to 3 fluorine atoms,
(d) amino (in which the amino may be optionally substituted with the same or different 1 to 2 $C_{1-6}$ alkyl),
(e) $C_{1-6}$ alkoxycarbonyl, or
(f) $C_{1-6}$ alkylcarbonyloxy),

8: $C_{1-6}$ alkylthio group,
9: $C_{1-6}$ alkoxycarbonyl group, or
10: $C_{1-6}$ alkylcarbonyloxy group.

"$R^7$" is preferably

1: hydrogen atom,
2: halogen atom,
3: hydroxy group,

4: $C_{1-6}$ alkyl group, or
5: $C_{1-6}$ alkoxy group. More preferable one is hydrogen atom, halogen atom, or $C_{1-6}$ alkyl group, particularly preferably hydrogen atom.

"$R^8$" is preferably hydrogen atom.
"m" is preferably 0 or 1, more preferably 1.
More preferable embodiment of the compound of the present invention includes compounds of the following formulae (1a) to (1d).

(1) A compound of the following formula (1a), or a pharmaceutically acceptable salt thereof.

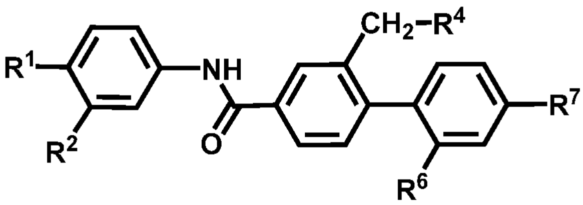
(1a)

[In the formula, each symbol is the same as defined in Item 1.]

(2) A compound of the following formula (1b), or a pharmaceutically acceptable salt thereof.

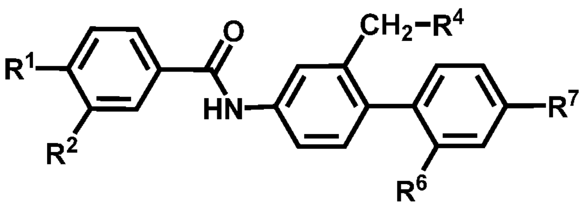
(1b)

[In the formula, each symbol is the same as defined in Item 1.]

(3) A compound of the following formula (1c), or a pharmaceutically acceptable salt thereof.

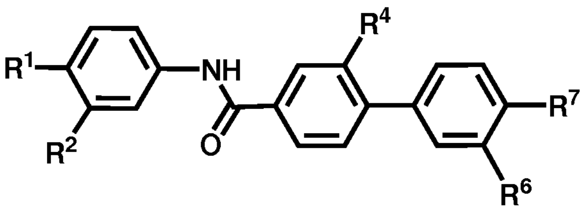
(1c)

[In the formula, each symbol is the same as defined in Item 1.]

(4) A compound of the following formula (1d), or a pharmaceutically acceptable salt thereof.

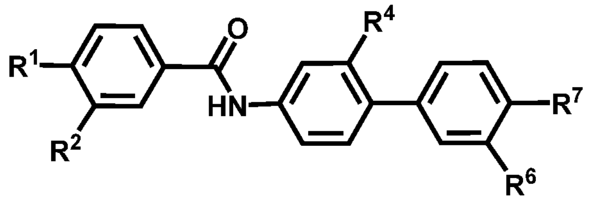

(1d)

[In the formula, each symbol is the same as defined in Item 1.]

Each preferable embodiment of each symbol in compounds of the above formulae (1a) to (1d) is the same as a preferable embodiment in a compound of formula (1).

A "pharmaceutically acceptable salt" includes an acid addition salt and a base addition salt. For example, the acid addition salt includes an inorganic acid salt such as hydrochloride, hydrobromide, sulfate, hydroiodide, nitrate, phosphate, or an organic acid salt such as citrate, oxalate, acetate, formate, propionate, benzoate, trifluoroacetate, maleate, tartrate, methanesulfonate, benzenesulfonate, paratoluenesulfonate, and the base addition salt includes an inorganic base salt such as sodium salt, potassium salt, calcium salt, magnesium salt, ammonium salt, or an organic base salt such as triethyl ammonium salt, triethanol ammonium salt, pyridinium salt, diisopropyl ammonium salt, and further, an amino acid salt including a basic or acidic amino acid such as arginine, aspartic acid, or glutamic acid.

The present invention encompasses a compound of formula (1) or a prodrug thereof, or a pharmaceutically acceptable salt thereof. It also encompasses a solvate thereof such as a hydrate or an ethanolate, etc. Further, the present invention encompasses every tautomer, every existing stereoisomer and every crystalline form of the compound of the present invention (1).

The term "prodrug of a cmpound of formula (1)" herein means a compound which is converted to a compound of formula (1) by reaction(s) by enzyme or gastric acid, etc. under the physiological condition *in vivo,* e.g. a compound which is converted to a compound of formula (1) by enzymatic oxidization, reduction, hydrolysis, etc.; a compound which is converted to a compound of formula (1) by hydrolysis by gastric acid, etc.

The compound of the present invention may have at least one asymmetric carbon atom. Hence, the present invention encompasses not only racemates of the compound of the present invention, but also optically-active compounds thereof. The compound of the present invention may have stereoisomers thereof when the compound has two or more asymmetric carbon atoms. Hence, the present invention encompasses the stereoisomers and a mixture thereof.

Preparation methods for a compound of formula (1) in the present invention are illustrated as below, but the present invention is not intended to be limited thereto.

A compound of formula (1) may be synthesized by a combination of known synthetic methods from known compounds. For example, it may be synthesized by the following methods.

A compound of formula (1) or a salt thereof may be prepared, for example, by any of the following methods of Step 1 to Step 6.

<u>Preparation 1</u>

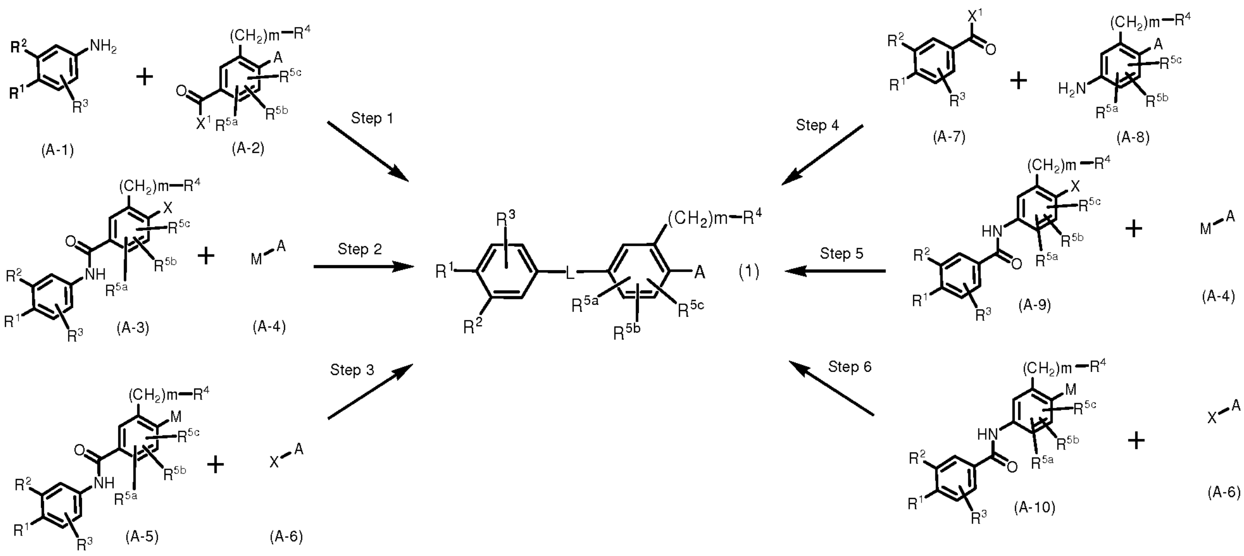


[In the scheme, $X^1$ is hydroxy group or a leaving group (e.g. halogen atom such as chlorine atom, bromine atom or iodine atom, etc.). X is a leaving group (e.g. halogen atom such as bromine atom or iodine atom, or sulfonyloxy group such as trifluoromethanesulfonyloxy, etc.). M is boronic acid ($B(OH)_2$) or boronic acid ester, or organotin (e.g. $Sn(n\text{-}Bu)_4$, etc.), or other alkaline-earth metals which form appropriate organometal compounds (e.g. magnesium, zinc, etc.). Each of other symbols is the same as defined in the above Item 1.]

Step 1:

It may be synthesized, if necessary, by activating a compound of formula (A-2) wherein $X^1$ is hydroxy group or a salt thereof in an inactive solvent to react with a compound of formula (A-1) or a salt thereof in the presence of a base, if necessary. A method for activating a compound of formula (A-2) or a salt thereof includes, for example, a method for converting its carboxy group to an acid halide, a mixed acid anhydride, etc., or a method using a condensing agent, etc.

When Compound (A-2) is activated into an acyl halide (acyl-halide method), Compound (A-2) wherein $X^1$ is hydroxy group is reacted with a halogenating agent such as oxalyl chloride, thionyl chloride, phosphorous oxychloride, phosphorous pentachloride etc., in the presence or absence of an additive in an inactive solvent, if necessary, to give Compound (A-2) wherein $X^1$ is halogen such as chlorine as an acyl halide.

The additive includes N,N-dimethylformamide, N,N-diethylformamide etc. The inactive solvent includes a halogenated hydrocarbon solvent such as dichloromethane, dichloroethane, or chloroform, an aromatic hydrocarbon solvent such as toluene, xylene etc., esters such as ethyl acetate etc. The reaction temperature is in the range from -80°C to heating to reflux, usually in the range from -10°C to 80°C. The reaction time is usually in the range from 10 minutes to 48 hours. After finishing the reaction, the reaction mixture may be concentrated under reduced pressure in the presence of a hydrocarbon solvent such as benzene or toluene, if necessary, and then the resulted acyl halide may be reacted with Compound (A-1) or a salt thereof in an inactive solvent in the presence of a base, if necessary, to give a compound of formula (1).

The base includes, for example, organic bases such as triethylamine, diisopropylethylamine, tributylamine, 1,5-diazabicyclo[4.3.0]non-5-ene (DBN), 1,4-diazabicyclo[2.2.2]octane (DABCO), 1,8-diazabicyclo[5.4.0]undec-7-ene (DBU), pyridine, dimethylaminopyridine, picoline or N-methylmorpholine (NMM), etc., or inorganic bases such as sodium bicarbonate, potassium hydrogen carbonate, sodium carbonate, potassium carbonate, sodium hydroxide, potassium hydroxide, etc. The base is usually used in an amount of 1 to 20 equivalents to Compound (A-2) wherein $X^1$ is halogen. Any inactive solvents may be used if they may not react under the reaction condition of the present step, and include, for example, a halogenated hydrocarbon solvent such as dichloromethane, chloroform, 1,2-dichloroethane, carbon tetrachloride, an ether solvent such as diethyl ether, diisopropylether, tetrahydrofurane, 1,4-dioxane, etc., an aromatic hydrocarbon solvent such as benzene, toluene, xylene, etc., esters such as ethyl acetate, methyl acetate, etc., or a mixed solvent thereof. The reaction temperature is in the range from -80°C to heating to reflux, usually in the range from -10°C to 60°C. The reaction time is usually in the range from 30 minutes to 48 hours.

When Compound (A-2) is activated into a mixed acid anhydride (mixed acid-anhydride method), Compound (A-2) wherein $X^1$ is hydroxy group may be reacted with an acyl halide in the presence of a base to give a mixed acid anhydride, followed by reacting with Compound (A-1) or a salt thereof to give a compound of formula (1).

The reaction temperature is in the range from -80°C to heating to reflux, usually in the range from -10°C to room

temperature. The reaction time is usually in the range from 30 minutes to 48 hours. The acyl halide includes, for example, methoxycarbonyl chloride, ethoxycarbonyl chloride, isopropyloxycarbonyl chloride, isobutyloxycarbonyl chloride, para-nitrophenoxycarbonyl chloride or t-butylcarbonyl chloride, etc. The base includes, for example, organic bases such as triethylamine, diisopropylethylamine, tributylamine, 1,5-diazabicyclo[4.3.0]non-5-ene (DBN), 1,4-diazabicyclo[2.2.2]octane (DABCO), 1,8-diazabicyclo[5.4.0]undec-7-ene (DBU), pyridine, dimethylaminopyridine, picoline or N-methylmorpholine (NMM), etc., or inorganic bases such as sodium bicarbonate, potassium hydrogen carbonate, sodium carbonateor potassium carbonate, etc. Any inactive solvents may be used if they may not react under the reaction condition of the present step, and include, for example, a halogenated hydrocarbon solvent such as dichloromethane, chloroform, 1,2-dichloroethane, carbon tetrachloride, etc., an ether solvent such as diethyl ether, diisopropylether, tetrahydrofurane, 1,4-dioxane, etc., an aromatic hydrocarbon solvent such as benzene, toluene, or xylene, etc., esters such as ethyl acetate, methyl acetate, etc., or a mixed solvent thereof.

Alternatively, when Compound (A-2) is activated by using various condensing agents, Compound (A-2) wherein $X^1$ is hydroxy group may be reacted with Compound (A-1) or a salt thereof in an inactive solvent in the presence of a base, if necessary, to give a compound of formula (1). Phase transfer catalysts or other additives may be also optionally used.

The condensing agent includes substances described in The Experimental Chemistry (editted by The Chemical Society of Japan, Maruzen) vol. 22. For example, it includes , for example, phosphate esters such as diethyl cyanophosphonate, diphenylphosphoryl azide, etc., carbodiimides such as 1-ethyl-3-(3-dimethylaminopropyl)-carbodiimide hydrochloride (WSC•HCl), dicyclohexylcarbodiimide (DCC), etc., a combination of disulfides such as 2,2'-dipyridyldisulfide, etc. and phosphines such as triphenylphosphine, etc., phosphorus halides such as N,N'-bis(2-oxo-3-oxazolidinyl)phosphinic chloride (BOPC1), etc., a combination of azodicarboxylic acid diester such as diethyl azodicarboxylate, etc. and phosphine such as triphenylphosphine, etc., 2-halo-1-lower alkylpyridinium halides such as 2-chloro-1-methylpyridinium iodide, 1,1'-carbonyldiimidazole (CDI), diphenylphosphoryl azide (DPPA), diethylphosphorylcyanide (DEPC), 2-(1H-benzotriazol-1-yl)-1,1,3,3-tetramethyluronium tetrafluoroborate (TBTU), 2-(1H-benzotriazol-1-yl)-1,1,3,3-tetramethyluronium hexafluorophosphate (HBTU), benzotriazol-1-yloxytris(dimethylamino)phosphonium hexafluorophosphate (BOP), benzotriazol-1-yloxytris(pyrrolidino)phosphonium hexafluorophosphate (PYBOP), 2-(7-aza-1H-benzotriazol-1-yl)-1,1,3,3-tetramethyluronium hexafluorophosphate (HATU), or 2-chloro-1,3-dimethylimidazolidinium tetrafluoroborate (CIB), etc.

Any inactive solvents may be used, but not limited to, if they may not react under the reaction condition of the present step, and include, for example, the same solvent as described in the above acylhalide method as well as an aprotic polar solvent such as N,N-dimethylformamide, N,N-dimethylacetamide, N-methyl-2-pyrrolidinone, 1,3-dimethyl-2-imidazolidinone, dimethyl sulfoxide, etc., water, or a mixed solvent thereof.

The base includes, but not limited to, organic bases such as triethylamine, diisopropylethylamine, tributylamine, 1,5-diazabicyclo[4.3.0]non-5-ene (DBN), 1,4-diazabicyclo[2.2.2]octane (DABCO), 1,8-diazabicyclo[5.4.0]undec-7-ene (DBU), pyridine, dimethylaminopyridine, picolineor N-methylmorpholine (NMM), etc., or inorganic bases such as sodium bicarbonate, potassium hydrogen carbonate, sodium carbonate, potassium carbonate, cesium carbonate, sodium hydroxide, or sodium hydride, etc.

The phase transfer catalyst includes, for example, a quaternary ammonium salt such as tetrabutylammonium bromide or benzyltriethylammonium bromide, etc., or a crown ether such as 18-crown-6-ether, etc., and is used when the base is an inorganic base.

The other additives include, for example, 1-hydroxybenzotriazole (HOBt), 1-hydroxy-7-azabenzotriazole (HOAt), etc., and are used when the condensing agent is carbodiimides such as 1-ethyl-3-(3-dimethylaminopropyl)-carbodiimide hydrochloride (WSC•HCl), etc.

The reaction temperature is in the range from -80°C to heating to reflux, usually in the range from -10°C to about 60°C. The reaction time depends on the condition such as reaction temperature, starting materials and solvent to be used, but is usually in the range from 30 minutes to 48 hours.

Step 2:

Compound (A-3) or a salt thereof may be treated in a coupling reaction with organometal compound (A-4) such as boronic acid or boronic acid ester, or organotin, magnesium or zinc to give a compound of formula (1). The reaction may be carried out in the presence of a transition metal catalyst and in the presence of a ligand, a base, an additive, if necessary, in the appropriate inactive solvent. The reaction temperature is usually in the range from -10°C to a boiling temperature of a solvent to be used. The reaction time depends on the condition such as reaction temperature, catalysts, starting materials and solvent to be used, but is usually in the range from 10 minutes to 48 hours.

The transition metal catalyst includes, for example, palladium (II) acetate, palladium (II) chloride, tris(dibenzylideneacetone)dipalladium (0), tetrakis(triphenylphosphine)palladium (0), bis(triphenylphosphine)palladium (II) chloride, bis(tri-O-tolylphosphine) dichloropalladium (II), bis(tri-tert-butylphosphine)palladium (0), or [1,1'-bis(diphenylphosphino) ferrocene]dichloropalladium (II), etc.

The ligand includes, for example, triphenylphosphine, tri-O-tolylphosphine, tri-tert-butylphosphine, tri-2-furylphosphine, tri-cyclohexylphosphine, triphenylarsine, 1,1'-bis(diphenylphosphino)ferrocene (dppf), 2-dicyclohexylphosphino-2',6'-dimethoxybiphenyl (SPhos), 2-dicyclohexylphosphino-2',4',6'-triisopropylbiphenyl (XPhos), etc.
The base includes, for example, an organic base such as triethylamine, diisopropylethylamine, etc., an inorganic base such as sodium carbonate, sodium bicarbonate, potassium carbonate, cesium carbonate, potassium phosphate, etc.
The additive includes, for example, an inorganic salt such as lithium chloride, cesium fluoride, copper (I) iodide, copper (I) bromide, etc.
The inactive solvent includes, for example, water, acetonitrile, a halogenated hydrocarbon solvent such as chloroform, dichloromethane, etc., an alcohol solvent such as methanol, ethanol, 2-propanol, etc., an ether solvent such as 1,2-dimethoxyethane, tetrahydrofurane, 1,4-dioxane, etc., an aromatic hydrocarbon solvent such as benzene, toluene, xylene, etc., an aprotic polar solvent such as N,N-dimethylformamide, N,N-dimethylacetamide, N-methylpyrrolidone, etc., or a mixed solvent thereof.

Step 3:

Organometal compound (A-5) and Compound (A-6) or a salt thereof may be treated in the similar manner to the method of Step 2 to give a compound of formula (1).

Step 4:

Compound (A-7) or a salt thereof and Compound (A-8) or a salt thereof may be treated in the similar manner to the mehod of Step 1 to give a compound of formula (1).

Step 5:

Organometal compound (A-4) and Compound (A-9) or a salt thereof may be treated in the similar manner to the method of Step 2 to give a compound of formula (1).

Step 6:

Organometal compound (A-10) and Compound (A-6) or a salt thereof may be treated in the similar manner to the method of Step 2 to give a compound of formula (1).
Compound (A-2) or a salt thereof may be prepared, for example, according to the following mehod.

<u>Preparation 2</u>

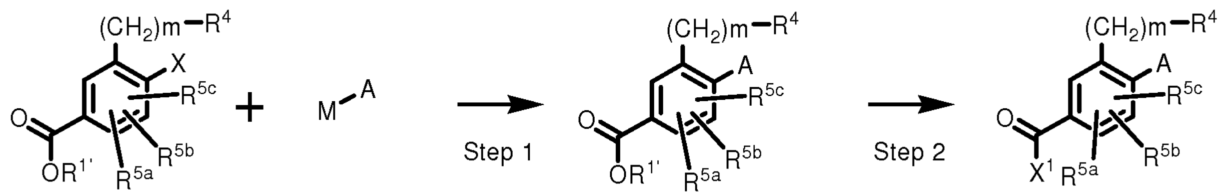


(A-11) (A-4) (A-12) (A-2)

(In the scheme, $R^{1'}$ is hydrogen atom, methyl group, ethyl group, tert-butyl group or benzyl group, etc., and other symbols are the same as defined above.)

Step 1:

Organometal compound (A-4) and Compound (A-11) or a salt thereof may be treated in the similar manner to the method of Step 2 in Preparation 1 to give Compound (A-12). When $R^{1'}$ is hydrogen, Compound (A-12) is the same as Compound (A-2) wherein $X^1$ is hydroxy group, and in that case, Step 2 is abbreviated.

Step 2:

The present step is a step for converting Compound (A-12) wherein $R^{1'}$ is not hydrogen atom to a carboxylic compound of Compound (A-2) wherein $X^1$ is hydroxy group by deprotecting an ester group. Compound (A-2) wherein $X^1$ is hydroxy group may be also treated in the similar manner to the method of Step 1 in Preparation 1 to give an acid halide of formula (A-2) wherein $X^1$ is halogen such as chlorine.
The present step may be carried out according to the general method of a literature (e.g., Protective Groups in Organic Synthesis, 3rd ed., T. W. Greene, John Wiley & Sons Inc. (1999), etc.).
Specifically, the following method is carried out, for example.

(A) When $R^{1'}$ is methyl group, ethyl group, etc., Compound (A-12) may be converted to a carboxylic acid by alkali hydrolysis, or acidic hydrolysis. That is, in case of alkali hydrolysis, for example, Compound (A-12) may be treated with water in the presence of a hydroxide of alkali metal or alkaline-earth metal such as sodium hydroxide, potassium hydroxide, lithium hydroxide, magnesium hydroxide and in the presence or absence of an alcohol solvent such as methanol, ethanol, 2-propanol, butanol, an ether solvent such as diethyl ether, diisopropylether, tetrahydrofurane, 1,4-dioxane, an aromatic hydrocarbon solvent such as benzene, toluene, xylene in the range from room temperature to heating to reflux, usually for 30 minutes to 48 hours to give Compound (A-2) wherein $X^1$ is hydroxy group.
In case of acidic hydrolysis, for example, Compound (A-12) may be treated with inorganic strong acid and water in an appropriate inactive solvent, if necessary, to give Compound (A-2) wherein $X^1$ is hydroxy group. The reaction temperature is usually in the range from room temperature to a boiling point of solvent. The reaction time depends on conditions such as reaction temperature, starting materials, and solvents, and is usually in the range from 10 minutes to 48 hours. The inorganic strong acid includes, for example, hydrochloric acid, bromic acid, sulfuric acid, etc. The inactive solvent includes, for example, an alcohol solvent such as methanol, ethanol, 1-propanol, ethylene glycol, etc., an ether solvent such as 1,4-dioxane, acetic acid, etc..

(B) When $R^{1'}$ is tert-butyl group, Compound (A-12) may be converted to a carboxylic acid by acidic hydrolysis. That is, for example, Compound (A-12) may be usually treated with Bronsted acid such as hydrochloric acid, sulfuric acid, nitric acid, phosphoric acid, formic acid, trifluoroacetic acid, toluenesulfonic acid, etc., or Lewis acid such as aluminum chloride, zinc bromide, boron trifluoride, etc. in the range from -20°C to room temperature, usually, in an inactive solvent or without any solvent to give Compound (A-2) wherein $X^1$ is hydroxy group. The inactive solvent includes, for example, an ether solvent such as tetrahydrofurane, diethyl ether, dioxane, 1,2-dimethoxyethane, etc., hydrocarbons such as toluene, benzene, etc., a halogenated hydrocarbon solvent such as dichloromethane, chloroform, 1,2-dichloroethane, etc., a mixed solvent thereof. When Bronsted acid is used, water may be used as a solvent alone or in combination with the above inactive solvents. The reaction time is usually in the range from 30 minutes to 48 hours.

(C) When $R^{1'}$ is benzyl group, Compound (A-12) may be converted to a carboxylic acid by hydrogenation. That is, for example, Compound (A-12) may be treated in an inactive solvent in the presence of a metal catalyst such as palladium/carbon, palladium oxide, nickel, etc. in the range from - 20°C to room temperature, usually, under hydrogen gas atmosphere at normal pressure to 10 atm to give Compound (A-2) wherein $X^1$ is hydroxy group. The inactive solvent includes an alcohol solvent such as methanol, ethanol, 2-propanol, butanol, etc., an ether solvent such as diethyl ether, diisopropylether, tetrahydrofurane, 1,4-dioxane, etc., an aromatic hydrocarbon solvent such as benzene, toluene, xylene, etc., esters such as ethyl acetate, methyl acetate, etc., an organic acid such as acetic acid, etc., water, or a mixed solvent thereof. The reaction time is usually in the range from 30 minutes to 48 hours.

Compound (A-12) or a salt thereof may be prepared according to the following method, for example.

Preparation 3

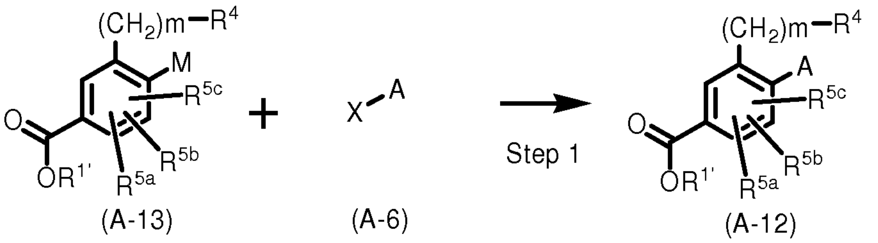

(In the scheme, symbols are the same as defined above.)

Step 1:

Organometal compound (A-13) and Compound (A-6) or a salt thereof may be treated in the similar manner to the method of Step 2 in Preparation 1 to give Compound (A-12).

Compound (A-3) or a salt thereof may be prepared, for example, according to the following method.

Preparation 4

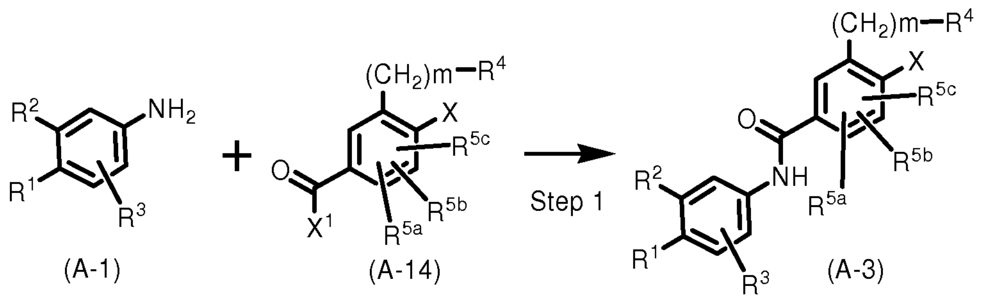

(In the scheme, symbols are the same as defined above.)

Step 1:

Compound (A-1) or a salt thereof and Compound (A-14) or a salt thereof may be prepared in the similar manner to the method of Step 1 in Preparation 1 to give Compound (A-3).

Compound (A-5) or a salt thereof may be prepared, for example, according to the following method.

Preparation 5

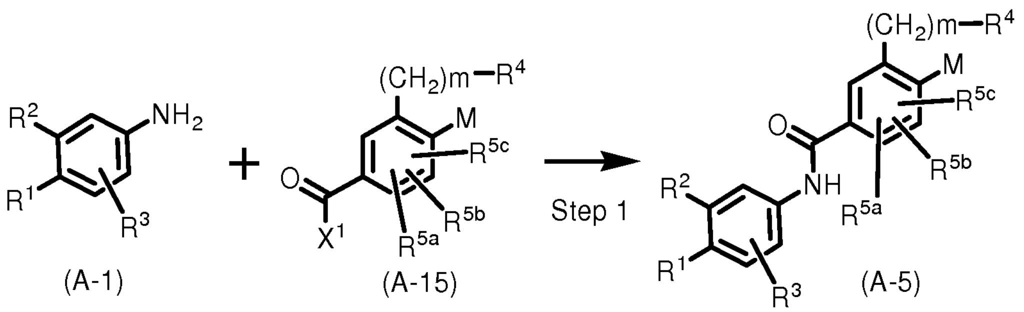

(In the scheme, symbols are the same as defined above.)

Step 1:

Compound (A-1) or a salt thereof and Compound (A-15) or a salt thereof may be treated in the similar manner to the method of Step 1 in Preparation 1 to give Compound (A-5).

Compound (A-8) or a salt thereof may be prepared, for example, according to the following method.

Preparation 6

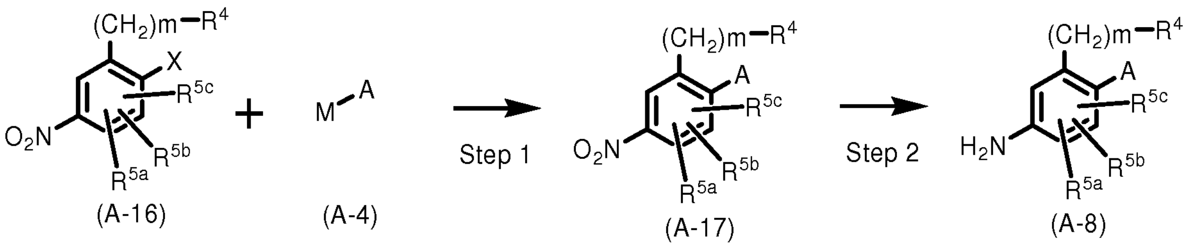


(A-16) (A-4) (A-17) (A-8)

(In the scheme, symbols are the same as defined above.)

Step 1:

Organometal compound (A-4) and Compound (A-16) or a salt thereof may be treated in the similar manner to the method of Step 2 in Preparation 1 to give Compound (A-17).

Step 2:

The present step is a step for converting Compound (A-17) to Compound (A-8) by reducing nitro group to amino group.
The present step may be carried out according to the general method of a literature (e.g., Comprehensive Organic Transformations, R. C. Larock, VCH publisher Inc. (1989), etc.), for example.
Specifically, for example, Compound (A-17) may be treated by hydrogenation in the similar manner to the above method (Step 2, (C) in Preparation 2), or treated in the presence of a metal reducing agent in an appropriate inactive solvent in the range from about 0°C to a boiling point of a solvent to be used for 10 minutes to 48 hours, usually, to give Compound (A-8). The metal reducing agent includes, for example, tin (II) chloride, reduced iron or titanium (III) trichloride, etc. The inactive solvent includes, for example, water, diluted hydrochloric acid, acetic acid, acetone, acetonitrile, an alcohol solvent such as methanol, ethanol, 2-propanol, etc., an ether solvent such as tetrahydrofurane, 1,2-dimethoxyethane, etc., esters solvent such as ethyl acetate, etc., an aprotic polar solvent such as N,N-dimethylformamide, etc., or a mixed solvent thereof.

Compound (A-17) or a salt thereof may be prepared according to the following method, for example.

Preparation 7

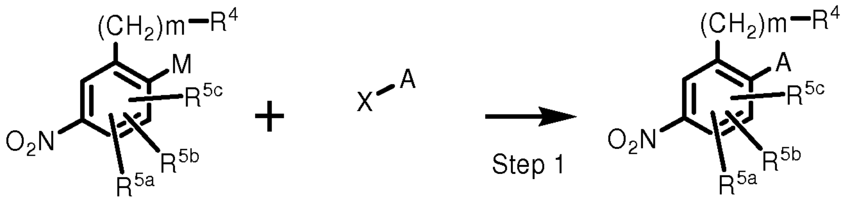


(A-18) (A-6) (A-17)

(In the scheme, symbols are the same as defined above.)

Step 1:

Organometal compound (A-18) and Compound (A-6) or a salt thereof may be treated in the similar manner to the method of Step 2 in Preparation 1 to give Compound (A-17).

Compound (A-9) or a salt thereof may be prepared, for example, according to the following method.

Preparation 8

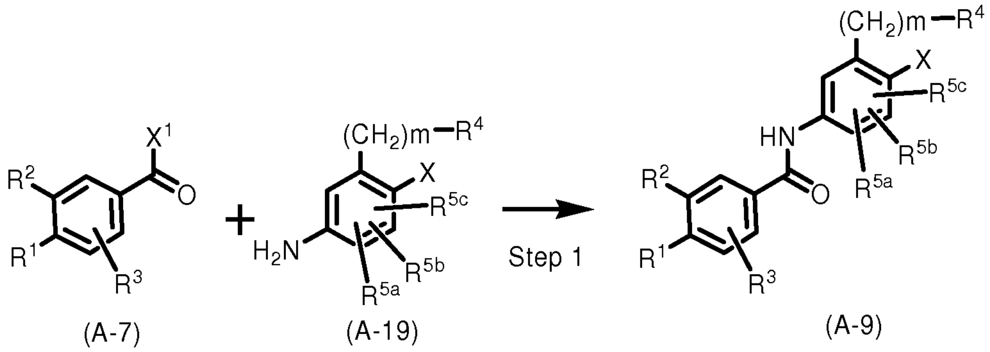


(A-7) (A-19) (A-9)

(In the scheme, symbols are the same as defined above.)

Step 1:

Compound (A-7) or a salt thereof and Compound (A-19) or a salt thereof may be treated in the similar manner to the method of Step 1 in Preparation 1 to give Compound (A-9).

Compound (A-10) or a salt thereof may be also prepared, for example, according to the following method.

Preparation 9

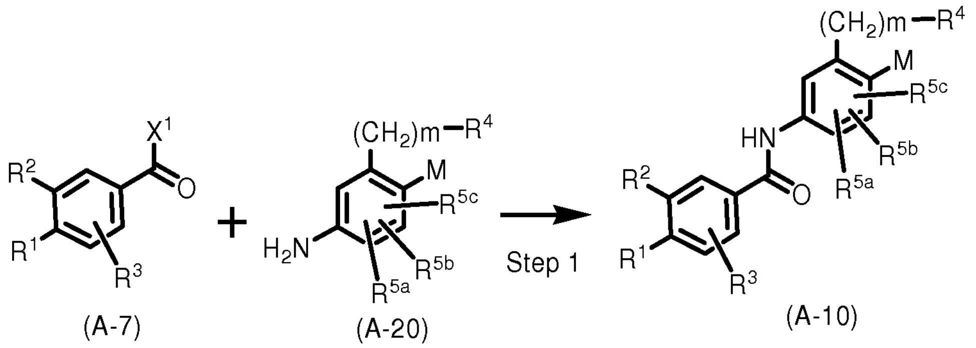


(A-7) (A-20) (A-10)

(In the scheme, symbols are the same as defined above.)

Step 1:

Compound (A-7) or a salt thereof and Compound (A-20) or a salt thereof may be treated in the similar manner to the method of Step 1 in Preparation 1 to give Compound (A-10).

Among starting materials and intermediates in each preparation illustrated above, compounds of which preparations are not specifically described are commercially available, or may be prepared from commercially available compounds according to known methods for a skilled person or equivalent methods thereof. A part of useful preparation is illustrated as below.

Preparation of Compound (A-1)

Compound (A-1) wherein a moiety corresponding to $R^1$ is alkyl sulfone or sulfonamide may be prepared according to the method of a literature (WO 06/012642 pamphlet) or an equivalent method thereof, for example.
Among Compound (A-7), Compound (A-23) may be prepared, for example, according to the following method.

Preparation 10

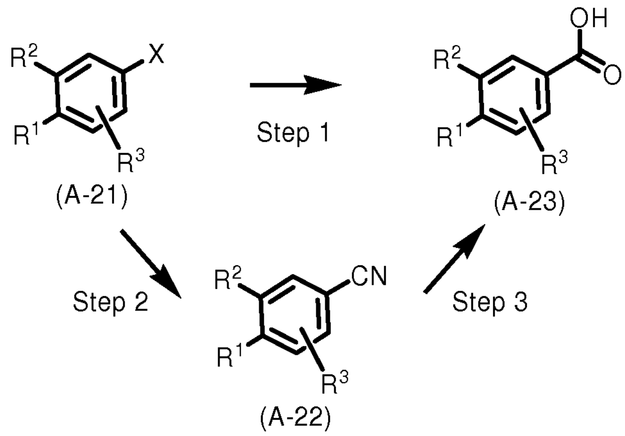


(In the scheme, symbols are the same as defined above.)

Step 1:

Compound (A-21) wherein X is halogen atom such as bromine atom or iodine atom, or a salt thereof may be treated in halogen-metal exchange reaction by an organometallic reagent, followed by treatment by carbon dioxide (gas or dry ice), and optionally, treatment in the presence of a chelate reagent in an appropriate inactive solvent in the range from about -100°C to a boiling point of solvent to be used for 10 minutes to 48 hours to give Compound (A-23).
The organometallic reagent includes, for example, an organolithium reagent such as methyllithium, n-butyllithium, sec-butyllithium or tert-butyllithium, etc.
The chelate reagent includes, for example, N,N,N',N'-tetramethylethylenediamine, etc.
The inactive solvent includes, for example, an aromatic hydrocarbon solvent such as benzene or toluene, etc., an aliphatic hydrocarbon solvent such as pentane or hexane, etc., an ether solvent such as diethyl ether or tetrahydrofurane, etc., or a mixed solvent thereof.

Step 2:

The present step is a step for converting Compound (A-21) or a salt thereof to Compound (A-22) by cyanation. The combination of the present step and Step 3 is an alternative step to Step 1.
The present step may be carried out, for example, according to the method of a literature (e.g., the above mentioned Comprehensive Organic Transformations, etc.).
Specifically, Compound (A-21) may be reacted with metal cyanide in the presence of a transition metal catalyst, a ligand, an additive, etc., if necessary, optionally in the presence of a base, in an appropriate inactive solvent in the present step. The reaction temperature is usually in the range from room temperature to a boiling point of a solvent to be used. The reaction time depends on the condition such as reaction temperature, catalysts, starting materials, and solvents to be used, and is usually in the range from 10 minutes to 48 hours.
The transition metal catalyst, ligand, additive, base, and inactive solvent include, for example, those described in Step 2 in Preparation 1. The additive also includes, for example, zinc, zinc chloride, diethylzinc, norbornene, tri-n-butyltin chloride.
The metal cyanide includes, for example, sodium cyanide, potassium cyanide, copper (I) cyanide, zinc cyanide, trimethylsilyl cyanide, and potassium ferrocyanide (II), etc.

Step 3:

The present step is a step for converting Compound (A-22) to a carboxylic acid of Compound (A-23) by hydrolysis of cyano group.
The present step may be carried out, for example, according to the method of a literature (e.g., the above mentioned Comprehensive Organic Transformations, etc.).
Specifically, Compound (A-22) may be reacted with inorganic strong acid or strong base and water, in an appropriate inactive solvent, if necessary, in the present step. The reaction temperature is usually in the range from room temperature to a boling point of a solvent to be used. The reaction time depends on the condition such as reaction temperature, starting materials, and solvents, and is usually in the range from 10 minutes to 48 hours.
The inorganic strong acid includes hydrochloric acid, bromic acid, sulfuric acid, etc., and the inorganic strong base includes sodium hydroxide, potassium hydroxide, etc.
The inactive solvent includes an alcohol solvent such as methanol, ethanol, 1-propanol, ethylene glycol, etc., an ether solvent such as 1,4-dioxane, etc., acetic acid.
Among Compound (A-19), Compound (A-26) may be prepared, for example, according to the following method.

Preparation 11

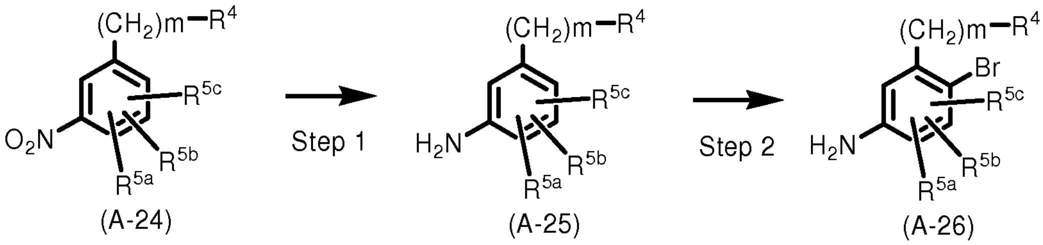


(In the scheme, symbols are the same as defined above.)

Step 1:

The present step is a step for converting Compound (A-24) to Compound (A-25) by reduction of nitro group to amino group.
The present step may be carried out according to the general method of a literature (e.g., Comprehensive Organic Transformations, R. C. Larock, VCH publisher Inc. (1989), etc.), for example, and specifically, include the similar method to the above mentioned process (i.e. Step 2 in Preparation 6).

Step 2:

The present step is a step for converting Compound (A-25) to Compound (A-26) by selective bromination of amino group in para position.
Specifically, Compound (A-25) may be treated in the presence of a brominating agent in the presence of an additive, if necessary, in an appropriate inactive solvent in the range from about 0°C to a boiling point of a solvent to be used for 10 minutes to 48 hours, usually, to give Compound (A-26), for example. The brominating agent includes, for example, N-bromosuccinimide, bromine, pyridinium bromide perbromide, etc.
The additive includes, for example, sodium acetate, sodium chloride, hydrogen peroxide, etc.
The inactive solvent includes, for example, water, diluted hydrochloric acid, acetic acid, acetonitrile, an alcohol solvent such as methanol or 2-propanol, etc., an ether solvent such as tetrahydrofurane, etc., or an aprotic polar solvent such as N,N-dimethylformamide, dimethyl sulfoxide, etc., a halogenated hydrocarbon solvent such as carbon tetrachloride, or chloroform, etc., or a mixed solvent thereof.
The organometal compounds (A-4), (A-5), (A-10), (A-13), (A-15), (A-18) and (A-20) may be prepared , if necessary, by protection or deprotection of the corresponding halide such as bromide or iodide (e.g., Compound (A-6), (A-3), (A-9), (A-11), (A-14), (A-16) and (A-19), respectively) in their metal moieties (referred to as "M" in the scheme), for example, followed by the following method.
When the organometal compound is boronic acid, the corresponding halide may be treated in halogen-metal exchange reaction with an organometallic reagent, followed by treatment with boronic acid trialkyl ester, and optionally

reaction in the presence of a chelate reagent, in an appropriate inactive solvent in the range from about -100°C to a boiling point of a solvent to be used for 10 minutes to 48 hours, followed by hydrolysis by diluted hydrochloric acid to give the organometal compound.
The organometallic reagent, chelate reagent, and inactive solvent include, for example, those described in Step 1 in Preparation 10.
The boronic acid trialkyl ester includes, for example, trimethyl borate, triethyl borate, tributyl borate or triisopropyl borate, etc.

When the organometal compound is boronic acid ester (e.g., 4,4,5,5-tetramethyl-1,3,2-dioxaborolane derivatives, etc.), for example, the corresponding halide may be reacted with 4,4,5,5-tetramethyl-1,3,2-dioxaborolane or bis (pinacolato)diborane in the presence of a transition metal catalyst, optionally in the presence of a ligand, a base, etc., in an appropriate inactive solvent in the range from about 20°C to a boiling point of a solvent to be used, usually, for 10 minutes to 48 hours to give the organometal compound.
The transition metal catalyst includes, for example, palladium (II) acetate, [1,1'-bis(diphenylphosphino)ferrocene]dichloropalladium (II), bis(triphenylphosphine)palladium (II) chloride, etc.
The ligand includes, for example, 1,1'-bis(diphenylphosphino)ferrocene (dppf), etc.
The base includes, for example, an organic base such as triethylamine, etc., an inorganic base such as potassium acetate, etc.
The inactive solvent includes, for example, an ether solvent such as 1,4-dioxane, etc., or an aprotic polar solvent such as N,N-dimethylformamide or dimethyl sulfoxide, etc., or a mixed solvent thereof.

When the organometal compound is organotin derivatives, the corresponding halide may be treated in halogen-metal exchange reaction with an organometallic reagent under the similar condition to the above case of boronic acid, followed by a coupling reaction with an organotin reagent to give the organometal compound. The organotin reagent includes, for example, tri-n-butyltin chloride or trimethyltin chloride.

The organotin derivatives may be also prepared by reacting the corresponding halide with an organotin reagent in the presence of a transition metal catalyst, optionally in the presence of a ligand, a base, etc., in an appropriate inactive solvent in the range from about 20°C to a boiling point of a solven to be used for 10 minutes to 48 hours. The organotin reagent in this case includes hexa-n-butylditin or hexamethylditin.
The transition metal catalyst includes, for example, tetrakis(triphenylphosphine)palladium (0), bis(triphenylphosphine) palladium (II) chloride, etc. The base includes, for example, an organic base such as triethylamine, etc., an inorganic base such as potassium acetate, etc. The inactive solvent includes an aromatic hydrocarbon solvent such as toluene, etc., or an aprotic polar solvent such as N,N-dimethylformamide or N-methylpyrrolidone, etc., or a mixed solvent thereof.

The protective groups, condensing agents, etc. may be referred to as abbreviations by conventional IUPAC-IUB (biochemical nomenclature committiee) in the technical field throughout the description.
Preferable salts and pharmaceutically acceptable salts of the starting compounds and the desired compounds are conventional non-toxic salts, and include an acid addition salt such as an organic acid salt (e.g. acetate, trifluoroacetate, maleate, fumarate, citrate, tartrate, methanesulfonate, benzenesulfonate, formate or toluenesulfonate, etc.) and an inorganic acid salt (e.g. hydrochloride, hydrobromide, hydroiodide, sulfate, nitrate or phosphate, etc.), a salt with amino acid (e.g. arginine, aspartic acid or glutamic acid, etc.), a metal salt such as an alkali metal salt (e.g. sodium salt or potassium salt, etc.) and an alkaline-earth metal salt (e.g. calcium salt or magnesium salt, etc.), ammonium salt, or an organic base salt (e.g. trimethylamine salt, triethylamine salt, pyridine salt, picoline salt, dicyclohexylamine salt or N,N'-dibenzylethylenediamine salt, etc.), and a skilled person may optionally select them.

In each reaction of the above described Preparations, in addition to the case where the use of protective groups are specified, any sites other than reaction sites may be optionally protected and then deprotected after the reaction or a sequence of reactions to give the desired compound when any functional groups other than reaction sites may be changed under the reaction condition or may be inappropriate for carrying out the reaction. The protective group includes the conventional groups described in a literature (e.g. the above mentioned Protective Groups in Organic Synthesis, etc.), and specifically the protective group for amino group includes benzyloxycarbonyl, tert-butoxycarbonyl, acetyl or benzyl, and the protective group for hydroxy group includes trialkylsilyl group such as trimethylsilyl or tert-butyldimethylsilyl, acetyl or benzyl.
An introduction and deprotection of the protective group may be carried out according to the conventional method in the organic synthetic chemistry (e.g. the above mentioned Protective Groups in Organic Synthesis) or equivalent methods thereof.

The intermediates or final products in the above preparations may be also optionally reacted by conversion of their functional groups, particularly extension of various side chains in view of amino group, hydroxy group, carbonyl group, halogen group, etc., as well as the above protection and deprotection, if necessary, to give other compounds encompassed in the present invention (e.g. conversion of $R^4$, etc.). The conversion of functional group and the extension of side chains may be carried out according to the conventional method (e.g., the above mentioned Comprehensive Organic Transformations, etc.).

The intermediates and the desired compound in the above each Preparation may be isolated and purified by the conventional purification in the organic synthetic chemistry, for example, neutralization, filtration, extraction, washing, drying, concentration, recrystalization, various kinds of chromatography, etc. The intermediates may be also used in the next reaction without purification.

Some compounds in the present invention (1) may have optical isomers based on the optically active center, atropisomers based on axial or planar chirality caused by restriction of intramolecular rotation, other stereoisomers, tautomers, and geometric isomers, etc., and the present invention encompasses all possible isomers and a mixture thereof including these isomers.

Particularly, optical isomers and atropisomers may be obtained as a racemate, or an optically-active compound when optically active starting materials or intermediates are used, respectively. If necessary, the corresponding racemates of the starting materials, intermediates or final products may be physically or chemically resolved into their optical enantiomers thereof by known separation method such as a method using optically active column, fractional crystallization in an appropriate stage of the above Preparations. Specifically, two kinds of diastereomers are obtained from racemates by reaction using optically active resolving agents in the diastereomer method. The different diastereomers may be generally resolved by known methods such as fractional crystallization due to the difference of physical properties.

When a pharmaceutically acceptable salt of a compound of formula (1) is desired, a pharmaceutically acceptable salt of Compound (1) may be directly purified when Compound (1) is obtained in the form of the pharmaceutically acceptable salt, and a free form of Compound (1) may be dissolved or suspended in an appropriate organic solvent, followed by addition of acid or base to form a salt in the conventional manner when the free form of Compound (1) is obtained. Compound (1) and a pharmaceutically acceptable salt thereof may also exist in the form of adduct with water or various solvents, and the present invention also encompasses the adduct.

The compound of the present invention has high binding affinity for aldosterone receptors, and shows pharmacological effects such as antagonistic activities or partial agonistic activities as an aldosterone receptor regulator. Thus, it is usuful for preventing and/or treating diseases such as hypertension (including essential hypertension, secondary hypertension, resistant hypertension), stroke, arrhythmia, heart failure, cardiomegaly, arteriosclerosis, vascular restenosis, renal fibrosis, cardiac infarction, diabetes complication, renal disease, edema, primary aldosteronism, inflammation, insulin resistance, sleep apnea syndrome, non-alcoholic steatohepatitis, Cushing's syndrome.

For the purpose of medical care, the compound of the present invention may be used in the form of a pharmaceutical formulation in combination with pharmaceutically acceptable carriers such as solid or liquid organic or inorganic excipients which is appropriate for oral, parenteral administration or external application, including local, enteral, intravenous, intramuscular, inhalation, nasal, intra-articular, intrathecal, transtracheal or ocular administration. The pharmaceutical formulation includes solid, semisolid or liquid such as capsule, tablet, pellet, sugar-coated tablet, powder, granule, suppository, ointment, cream, lotion, inhalation, injection, cataplasm, gel, tape, eye drop, liquid, syrup, aerozol, suspension, emulsion. These formulations may be prepared by the conventional method. Optionally, adjuvant, stabilizer, wetting agent or emulsifying agent, buffering agent and other conventional additives may be added to these formulations.

Dosage amounts of the compound of the present invention vary depending on ages and conditions of patients, and about 0.1 mg, 1 mg, 10 mg, 50 mg, 100 mg, 250 mg, 500 mg and 1,000 mg for average single dose of Compound (1) are effective on circulatory disease such as hypertension, stroke, arrhythmia, heart failure, cardiomegaly, arteriosclerosis, vascular restenosis, renal fibrosis, cardiac infarction, diabetes complication, renal disease, edema, primary aldosteronism, inflammation. Generally, 0.1 mg/person to about 1,000 mg/person per day, preferably 1 mg/person to about 100 mg/person per day may be adminstered for human.

The compound of the present invention may be used in combination with a drug such as therapeutic agent for diabetes, therapeutic agent for diabetic complication, antihyperlipidemic agent, antihypertensive drug, anti-obesity agent, diuretic (referred to as "concomitant drug" hereinafter) for the purpose of enhancement of its effect. The timings of administration of the compound of the present invention and the concomitant drug to subjects are not limited, and may be simultaneous administration or administration at an interval. A combination drug of the compound of the present invention and the concomitant drug may be administered. Dosage amounts of the concomitant drug may be optionally adjusted based on the clinically-used dose. The ratios of combination of the compound of the present invention and the concomitant drug may be optionally adjusted depending on administration subjects, administration routes, target diseases, conditions, combinations, etc. For example, 0.01 to 100 parts by weight of the concomitant drug may be used to 1 part by weight of the compound of the present invention for human administration subjects.

The therapeutic agent for diabetes includes insulin preparations (e.g., animal insulin preparation extracted from pancreas of bovine, swine, etc.; human insulin preparation genetically-engineered using Escherichia coli, yeast, etc.), insulin resistance-improving drugs (e.g., pioglitazone or a hydrochloride thereof, troglitazone, rosiglitazone or a maleate thereof, GI-262570, JTT-501, MCC-555, YM-440, KRP-297, CS-011, etc.), $\alpha$-glucosidase inhibitors (e.g., voglibose, acarbose, miglitol, emiglitate, etc.), biguanides (e.g., metformin, etc.), insulin secretagogues (e.g., sulfonylurea such as tolbutamide, glibenclamide, gliclazide, chlorpropamide, tolazamide, acetohexamide, glyclopyramide, glimepiride; repaglinide, senaglinide, nateglinide, mitiglinide, etc.), GLP-1, GLP-1 analogs (e.g., exenatide, liraglutide, SUN-E7001,

AVE010, BIM-51077, CJC1131, etc.), protein tyrosine phosphatase inhibitors (e.g., vanadic acid, etc.), β3 agonists (e.g., GW-427353B, N-5984, etc.), DPPIV inhibitors (e.g., sitagliptin, vildagliptin, saxagliptin, SYR-322, etc.).

The therapeutic agent for diabetic complication includes aldose reductase inhibitors (e.g., tolrestat, epalrestat, zenarestat, zopolrestat, minarestat, fidarestat, SK-860, AS-3201, etc.), neurotrophic factors (e.g., NGF, NT-3, BDNF, etc.), PKC inhibitors (e.g., LY-333531, etc.), AGE inhibitors (e.g., ALT946, pimagedine, pyratoxatin, N-phenacylthiazolium bromide (ALT766), etc.), active oxygen scavengers (e.g., thioctic acid, etc.), cerebral vasodilators (e.g., tiapride, mexiletine, etc.).

The antihyperlipidemic agent includes HMG-CoA reductase inhibitors (e.g., pravastatin, simvastatin, lovastatin, atorvastatin, fluvastatin, itavastatin or sodium salts thereof, etc.), squalene synthetase inhibitors, ACAT inhibitors.

The antihypertensive drug includes angiotensin-converting enzyme inhibitors (e.g., captopril, enalapril, alacepril, delapril, lisinopril, imidapril, benazepril, cilazapril, temocapril, trandolapril, etc.), angiotensin II antagonists (e.g., olmesartan medoxomil, candesartan, cilexetil, losartan, eprosartan, valsartan, telmisartan, irbesartan, tasosartan, etc.), calcium antagonists (e.g., nicardipine hydrochloride, manidipine hydrochloride, nisoldipine, nitrendipine, nilvadipine, amlodipine, etc.), renin inhibitor (e.g., aliskiren, etc.), ACE/NEP inhibitor (e.g., omapatrilat, fasidotril, etc.), P blockers (e.g., atenolol, bisoprolol, betaxolol, metoprolol, etc.), α blockers (e.g., urapidil, terazosin, doxazosin, bunazosin, etc.), αβblockers (e.g., amosulalol, arotinolol, labetalol, carvedilol, etc.).

The anti-obesity agent includes central anti-obesity agents (e.g., phentermine, sibutramine, amfepramone, dexamphetamine, mazindol, SR-141716A, etc.), pancreatic lipase inhibitors (e.g., orlistat, etc.), peptidic appetite suppressors (e.g., leptin, CNTF (ciliary neurotrophic factors), etc.), cholecystokinin agonists (e.g., lintitript, FPL-15849, etc.).

The diuretic includes xanthine derivatives (e.g., theobromine sodium salicylate, theobromine calcium salicylate. etc.), thiazide preparations (e.g., ethiazide, cyclopenthiazide, trichloromethiazide, hydrochlorotiazide, hydroflumethiazide, bentylhydrochlorothiazide, penflutizide, polythiazide, methychlothiazide, etc.), antialdosterone preparations (e.g., spironolactone, triamterene, etc.), carbonate dehydratase inhibitors (e.g., acetazolamide, etc.), chlorbenzenesulfonamide preparations (e.g., chlorthalidone, mefruside, indapamide, etc.), azosemide, isosorbide, ethacrynic acid, piretanide, bumetanide, furosemide.

The above two or more concomitant drugs may be combined in optional ratios.

When the compound of the present invention is used in combination with a concomitant drug, dosage amounts of these drugs may be lessened within the safe range in view of by-effects of the drugs. Accordingly, any possible by-effects caused by these drugs may be safely suppressed.

## EXAMPLES

The present invention is illustrated in more detail by the following Reference Examples, Examples and Test Examples but should not be construed to be limited thereto.

In Reference Examples and examples, the following abbreviations may be used.

| | |
|---|---|
| TLC: | thin layer chromatography |
| THF: | tetrahydrofuran |
| $NaBH(OAc)_3$: | sodium triacetoxyborohydride |
| $(Boc)_2O$: | di-tert-butyl dicarbonate |
| $Pd(OH)_2$: | palladium hydroxide |
| DMF: | N,N-dimethylformamide |
| DIEA: | N-ethyldiisopropylamine |
| WSCI: | 1-(3-dimethylaminopropyl)-3-ethyl carbodiimide |
| WSCI•HCl: | 1-(3-dimethylaminopropyl)-3-ethyl carbodiimide hydrochloride |
| HOBt: | 1-hydroxybenzotriazole |
| HOBt•$H_2O$: | 1-hydroxybenzotriazole monohydrate |
| Me: | methyl |
| Et: | ethyl |
| DMF | N,N-dimethylformamide |
| NMP: | 1-methyl-2-pyrrolidinone |
| Boc: | tert-butoxycarbonyl |
| Cbz or Z: | benzyloxycarbonyl |
| N: | normal (e.g., 2N HCl means 2 normal concentration of aqueous solution of hydrochloric acid) |
| M: | mole concentration (mol/L) (e.g., 2M methyl amine means 2 mol/L of methyl amine solution) |
| $t_R$: | retention time |

Purification using preparative reversed phase HPLC was carried out as follows:

Purification was carried out by using Gilson HPLC System. Column was YMC CombiPrep ODS-A column (5μm, 50×20 minI.D.), and a solvent was a mixture of $CH_3CN$ containing 0.035% TFA and water containing 0.05% TFA. Samples were detected by UV absorption at each of 210nm, 220nm and 254nm.

Condition for elution was as follows:

Fractionation system: Gilson HPLC System
Column: YMC CombiPrep ODS-A 50×20 minI.D.
Solvent: $CH_3CN$ containing 0.035% TFA and water containing 0.05% TFA
Flow rate: 35 mL/min
Gradient: linear gradient from 1:99 (v/v) $CH_3CN$/water to 95:5 (v/v) $CH_3CN$/water, within 13 minutes at 35 mL/min

Condition for LC/MS analysis for identification of a compound is as follows:

Analysing method SB 1:
Detector: API 150EX LC/MS mass spectrometer (Applied Biosystems)
HPLC: Shimadzu LC 10ATVP
Column: Shiseido CAPCELL PAK $C_{18}$ ACR (S-5um, 4.6 x 50mm)
Solvent: solution A: 0.035% TFA/$CH_3CN$, solution B: 0.05% TFA/$H_2O$
Gradient 0.0-0.5 min: A 10%, 0.5-4.8 min: linear gradient, A 10% to 99%, 4.8-5.0 min: A 99%
Flow Rate: 3.5 mL/min
Detection: UV absorption at 220nm and 254nm

Analysing method SB2:

Gradient: 0.0-0.5 min: A 25%, 0.5-4.8 min: linear gradient, A 25% to 99%, 4.8-5.0 min: A 99%
Other conditions are the same as SB 1.

Analysing method SB3:

Gradient: 0.0-0.5 min: A 40%, 0.5-4.8 min: linear gradient, A 40% to 99%, 4.8-5.0 min: A 99% Other conditions are the same as SB 1.

Analysing method SA1:

Detector: API 150EX LC/MS mass spectrometer (Applied Biosystems)
HPLC: Agilent 1100 for API Series
Column: YMC CombiScreen ODS-A (S-5 μm, 12 nm, 4.6x50mm)
Solvent: solution A: 0.05% TFA/$H_2O$, solution B: 0.035% TFA/MeCN
Gradient: 0.0-1.0 min: A 99%, 1.0-4.7 min: linear gradient, A 99% to 1%, 4.7-5.7 min: A 1%, 5.7-6.1 min: linear gradient, A 1% to 99%, 6.1-7.1 min: linear gradient, A 99% to 100%, 7.1-7.2 min: A 100%
Flow Rate: 3.5 mL/min
Detection: UV absorption at 220nm

Analysing method SA2:

Gradient: 0.0-1.0 min: A 90%, 1.0-4.7 min: linear gradient, A 90% to 1%, 4.7-5.7 min: A 1%, 5.7-6.1 min: linear gradient, A 1% to 90%, 6.1-7.1 min: linear gradient, A 90% to 100%, 7.1-7.2 min: A 100%
Other conditions are the same as SA1.

Analysing method SA3:

Solvent: solution A: 0.05% TFA/$H_2O$, solution B: 0.035% TFA/MeOH
Gradient: 0.0-1.0 min: A 75%, 1.0-4.7 min: linear gradient, A 75% to 1%, 4.7-5.7 min: A 1%, 5.7-6.1 min: linear gradient, A 1% to 75%, 6.1-7.1 min: linear gradient, A 75% to 100%, 7.1-7.2 min: A 100%
Other conditions are the same as SA1.

Analysing method SA4:

Gradient: 0.0-1.0 min: A 60%, 1.0-4.7 min: linear gradient, A 60% to 1%, 4.7-5.7 min: A 1%, 5.7-6.1 min: linear gradient, A 1% to 60%, 6.1-7.1 min: linear gradient, A 60% to 100%, 7.1-7.2 min: A 100%
Other conditions are the same as SA3.

Analysing method SC1:

Detector: ACQUITY SQ Detector (Waters)
HPLC: ACQUITY UPLC system
Column: Waters ACQUITY UPLC BEH C18 (1.7um, 2.1mm X 50mm)
Solvent: solvent A: 0.05% $HCO_2H/H_2O$, solvent B: 0.05% $HCO_2H$/MeCN
Gradient: 0.0-1.3 min: linear gradient A 95% to 5%
Flow Rate: 0.75 mL/min
Detection: UV absorption at 220 nm and 254 nm

Analysing method SC2:

Solvent: solvent A: 0.05% $HCO_2H/H_2O$, solvent B: 0.05% $HCO_2H$/MeOH
Gradient: 0.0-1.3 min: linear gradient A 75% to 1%
Other conditions are the same as SC1.

Analysing method SC3:

Detector: ACQUITY SQ Detector (Waters)
HPLC: ACQUITY UPLC system
Column: Waters ACQUITY UPLC BEH C18 (1.7um, 2.1mm X 50mm)
Solvent: solvent A: 0.05% $HCO_2H/H_2O$, solvent B: 0.05% $HCO_2H$/MeCN
Gradient: 0.0-1.3 min: linear gradient A 90% to 1%

Analysing method SD1:

Detector: API 2000, Q-TRAP (Applied Biosystems) and 3100 (Waters) etc. were used.
HPLC: Shimadzu LC 10ATVP, LC2010, Agilent 1100, 1200 and Waters ACQUITY etc. were used.
Column: Phenomenex GEMINI 5μm C18 110A (50 x 4.6 mm)
Solvent: solution A: 0.05%TFA/$H_2O$, solution B: MeCN
Gradient: 0.00-0.01 min: B 10%, 0.01-1.5 min: linear gradient 10% to 30%, 1.5-3.0 min: linear gradient B 30% to 90%, 3.0-4.0 min: B 90%, 4.0-5.0 min: linear gradient B 90% to 10%
Flow rate: 1.2 mL/min
Detection: UV absorption at 220nm and 260 nm

Analysing method SD2:

Solvent: solution A: 0.05% $HCO_2H/H_2O$, solution B: MeCN
Other conditions are the same as SD 1.

Analysing method SD2:

Solvent: solution A: 10mM $NH_4OAc/H_2O$, solution B: MeCN
Other conditions are the same as SD 1.

Reference Example 1
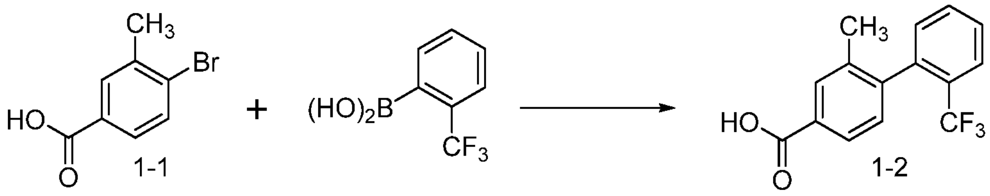

1-1

1-2

To a solution of 4-bromo-3-methylbenzoic acid (2.15 g; Compound 1-1) in 1,2-dimethoxyethane (80 mL) were added tetrakis(triphenylphosphine)palladium (0) (580 mg), 2N aqueous solution of sodium carbonate (40 mL) and 2-(trifluoromethyl)phenylboronic acid (3.80 g), and the mixture was stirred at 95°C to 100°C for 46 hours. After being cooled to room temperature, diethyl ether and water were added to the reaction solution and the mixture was separated. The aqueous layer was acidified by addition of aqueous solution of hydrochloric acid and then extracted with a mixture of ethyl acetate-tetrahydrofuran (2:1). The organic layer was dried over anhydrous magnesium sulfate, filtered, and then concentrated under reduced pressure. The resulting residue was purified with silica gel column chromatography (hexane/ethyl acetate) to give Compound 1-2 (1.72 g).

$^1$H-NMR(DMSO-$d_6$) δ 2.01(s, 3H), 7.22(d, J = 7.9 Hz, 1H), 7.33(d, J = 7.5 Hz, 1H), 7.64(t-like, J = 7.6 Hz, 1H), 7.73(d, J = 7.5 Hz, 1H), 7.77-7.80(m, 2H), 9.49(bs, 1H).

Reference Example 2

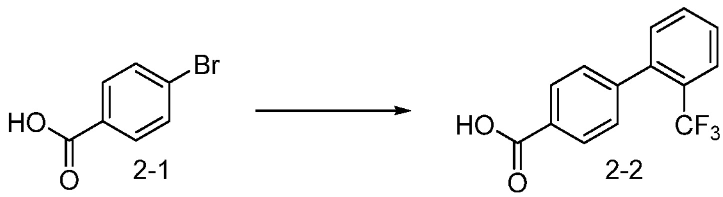

2-1

2-2

Compound 2-2 was prepared from 4-bromobenzoic acid (Compound 2-1) in the same manner as Reference Example 1.

$^1$H-NMR(DMSO-$d_6$) δ 7.42-7.45(m, 2H), 7.65(t-like, J = 7.8 Hz, 1H), 7.74(t-like, J = 7.2 Hz, 1H), 7.86(d, J = 7.3 Hz, 1H), 7.98-8.01(m, 2H), 13.06(bs, 1H).

Reference Example 3

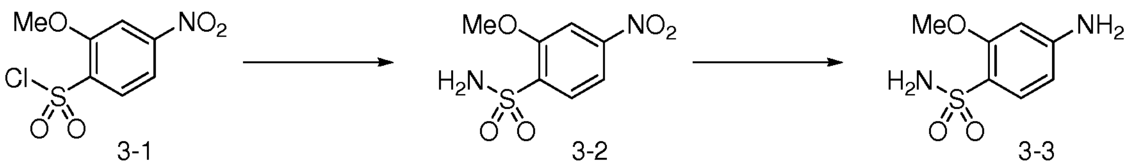

3-1

3-2

3-3

2-Methoxy-4-nitrobenzenesulfonyl chloride (252 mg: compound 3-1) was added to conc. ammonia water (25%, 2.8 mL) with stirring, and the mixture was stirred at room temperature for 3 days. Aqueous solution of ammonium chloride was added to the reaction mixture and then extracted with a mixture of ethyl acetate-tetrahydrofuran (2:1). The organic layer was dried over anhydrous magnesium sulfate, filtered and then concentrated under reduced pressure to give a residue, which was purified with silica gel column chromatography (chloroform/methanol) to give compound 3-2 (188 mg). The compound 3-2 (94 mg) was dissolved in a mixture (6 mL) of ethyl acetate-methanol (5:1) and stirred in the presence of 10% palladium-carbon (containing 50% water) (110 mg) under 0.3MPa of hydrogen atmosphere at room temperature for 2 hours. After finishing the reaction, palladium-carbon was filtered off and washed with a mixture of methanol-tetrahydrofuran (1:1). The filtrate and washing were combined and concentrated under reduced pressure to give compound 3-3 (82 mg).

Compound 3-2: $^1$H-NMR(DMSO-$d_6$) δ 4.03(s, 3H), 7.47(s, 2H), 7.68-7.93(m, 2H), 7.97(d, J = 8.3 Hz, 1H).

Compound 3-3: $^1$H-NMR(DMSO-$d_6$) δ 3.76(s, 3H), 5.81(s, 2H), 6.11(d, J = 8.4 Hz, 1H), 6.23(s, 1H), 6.57(s, 2H), 7.32 (d, J = 8.4 Hz, 1H).

Reference Example 4

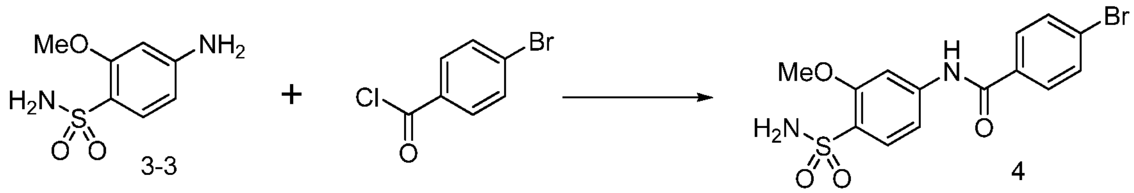

3-3 4

Compound 3-3 (71 mg) of Reference Example 3 and 4-bromobenzoyl chloride (77 mg) were mixed in tetrahydrofuran (1.5 mL), diisopropylethylamine (67 μL) was added thereto, and the mixture was stirred in a sealed tube at 80°C for 4 hours. The reaction mixture was cooled to room temperature, concentrated under reduced pressure to give a residue, which was purified with silica gel column chromatography (hexane/ethyl acetate) and compound 4 (114 mg) was prepared as a white solid.

$^1$H-NMR(DMSO-$d_6$) δ 3.88(s, 3H), 7.00(s, 2H), 7.47(dd, J = 8.6, 1.7 Hz, 1H), 7.67-7.70(m, 2H), 7.75-7.78(m, 2H), 7.90-7.93(m, 2H), 10.56(s, 1H).

Reference Example 5

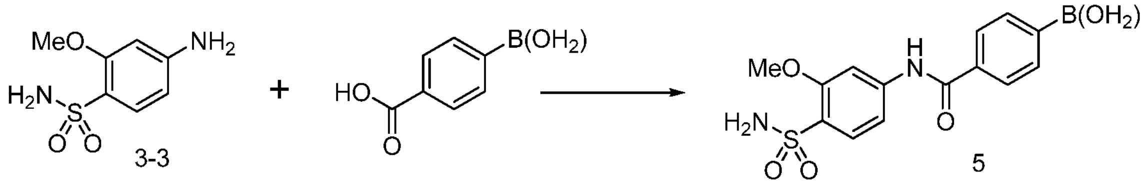

3-3 5

Compound 3-3 (114 mg) of Reference Example 3, 4-carboxyphenylboronic acid (103 mg) and (benzotriazol-1-yloxy) tripyrrolidinophosphonium hexaphosphate (PyBOP) (352 mg) were mixed in DMF (2.8 mL), diisopropylethylamine (120 μL) was added thereto, and the mixture was stirred at room temperature for 16 hours. Water was added to the reaction mixture which was extracted with ethyl acetate. The organic layer was washed with saturated brine, dried over anhydrous sodium sulfate, filtered and then concentrated under reduced pressure. The resulting residue was purified with silica gel column chromatography (chloroform/methanol = 30/1) to give compound 5 (208 mg) as a brown solid. $^1$H-NMR(DMSO-$d_6$) δ 3.88(s, 3H), 6.99(s, 2H), 7.51(dd, J = 8.7, 1.8 Hz, 1H), 7.682(d, J = 8.5 Hz, 1H), 7.73(d, J=1.7 Hz, 1H), 7.92(s-like, 4H), 8.27(s, 2H), 10.49(s, 1H).

Reference Example 6

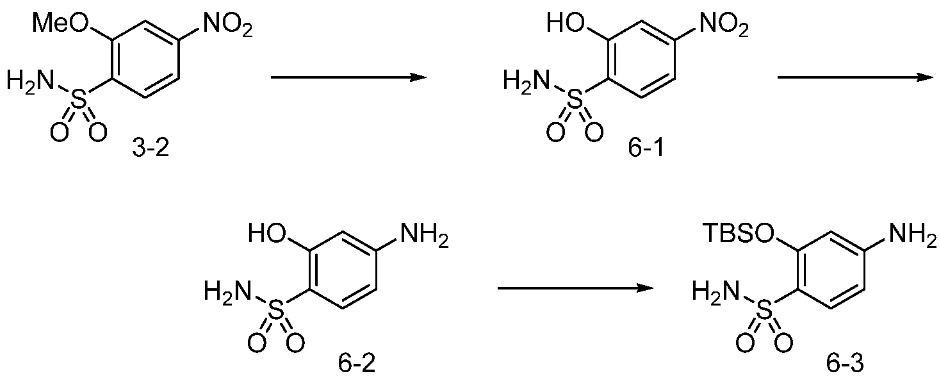

3-2 6-1 6-2 6-3

Compound 3-2 (2.0 g), which is an intermediate of Reference Example 3, was suspended in dichloromethane (57 mL) and 1M solution of boron tribromide in dichloromethane (69 mL) was added thereto at -78°C. The mixture was warmed up to room temperature and stirred for 16 hours. After finishing the reaction, water was added to the reaction mixture at 0°C, which was extracted with ethyl acetate. The organic layer was washed with saturated brine, dried over anhydrous sodium sulfate, filtered and concentrated under reduced pressure. The resulting residue was purified with silica gel column chromatography (hexane/ethyl acetate) to give compound 6-1 (1.72 g). Compound 6-1 (1.47 g) was hydrogenated in the presence of 10% palladium-carbon in the same manner as Reference Example 3 to give compound 6-2 (1.2 g). Imidazole (803 mg) and tert-butyldimethylsilyl chloride (1.07 g) were added to a solution of compound 6-2 (1.1 g) in DMF (6 mL) and the mixture was stirred at room temperature for 3 days. Then, water was added to the reaction mixture which

was extracted with ethyl acetate. The organic layer was washed with saturated brine, dried over anhydrous sodium sulfate, filtered and then concentrated under reduced pressure. The resulting residue was purified with silica gel column chromatography (chloroform/methanol) to give compound 6-3 (1.26 g).

Compound 6-1: $^1$H-NMR(DMSO-$d_6$) δ 7.31(s, 2H), 7.72-7.75(m, 2H), 7.88-7.90(m, 1H).

Compound 6-2: $^1$H-NMR(DMSO-$d_6$) δ 5.67(bs, 2H), 6.02(dd, J = 8.5, 2.0 Hz, 1H), 6.09(d, J = 2.2 Hz, 1H), 7.24(d, J = 8.5 Hz, 1H), 7.94(s, 2H).

Compound 6-3: $^1$H-NMR(DMSO-$d_6$) δ 0.25(s, 6H), 0.97(s, 9H), 5.78(s, 2H), 6.14(d, J = 8.5 Hz, 1H), 6.20(s, 1H), 6.32 (s, 2H), 7.33(d, J = 8.3 Hz, 1H).

Reference Example 7

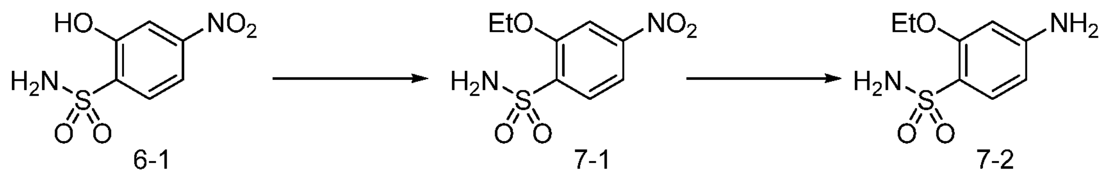

6-1 7-1 7-2

Compound 6-1 (180 mg), which is an intermediate of Reference Example 6, was dissolved in DMF (2.8 mL) contained 1% water, sodium hydrogencarbonate (208 mg) and ethyl iodide (100 μL) were added thereto, and the mixture was stirred at room temperature for 24 hours. The reaction mixture was filtered, and then water was added to the filtrate and the mixture was extracted with ethyl acetate. The organic layer was washed with saturated brine, dried over anhydrous sodium sulfate, filtered and then concentrated under reduced pressure. The resulting residue was purified with silica gel column chromatography (hexane/ethyl acetate) to give compound 7-1 (63 mg). Compound 7-1 (63 mg) was hydrogenated in the presence of 10% palladium-carbon in the same manner as Reference Example 3 to give compound 7-2 (55 mg).

Compound 7-1: $^1$H-NMR(DMSO-$d_6$) δ 1.40(t, J = 7.0 Hz, 3H), 4.35(q, J = 7.0 Hz, 2H), 7.34(s, 2H), 7.89(dd, J = 8.5, 2.1 Hz, 1H), 7.91(d, J = 2.2 Hz, 1H), 7.98(d, J = 8.5 Hz, 1H).

Compound 7-2: $^1$H-NMR(DMSO-$d_6$) δ 1.35(t, J = 7.0 Hz, 3H), 4.04(q, J = 7.0 Hz, 2H), 5.78(s, 2H), 6.10(dd, J = 8.5, 2.0 Hz, 1H), 6.23(d, J = 2.0 Hz, 1H), 6.42(s, 2H), 7.33(d, J = 8.5 Hz, 1H).

Reference Example 8

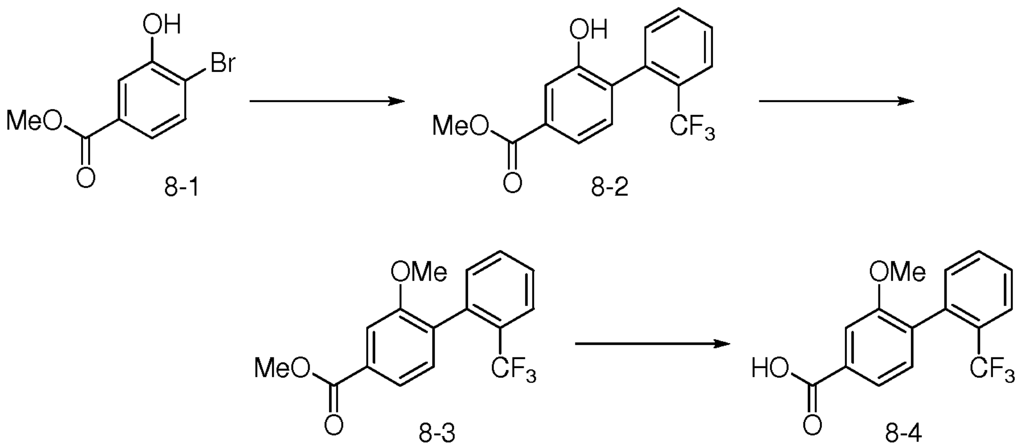

8-1 8-2 8-3 8-4

A mixture of methyl 4-bromo-3-hydroxybenzoate (500 mg: compound 8-1), [1,1'-bis(diphenylphosphino)ferrocene]dichloropalladium (II) (316 mg), potassium carbonate (1.19 g) and 2-(trifluoromethyl)phenylboronic acid (455 mg) was stirred in a mixed solvent (11 mL) of DMF-water (10:1) at 80°C for 18 hours. The mixture was cooled to room temperature, and then aqueous solution of ammonium chloride was added thereto and the mixture was extracted with ethyl acetate. The organic layer was washed with saturated brine, dried over anhydrous sodium sulfate, filtered and then concentrated under reduced pressure to give a residue, which was purified with silica gel column chromatography (hexane/ethyl acetate) to give compound 8-2 (258 mg). Sodium hydride (60%, 22.7 mg) was suspended in DMF (2 mL) and the suspension was added dropwise to compound 8-2 (140 mg) in DMF (2 mL) at 0°C and then the mixture was stirred at

0°C for 20 minutes. Then, iodomethane (35 μL) was added thereto at 0°C and the mixture was stirred at room temperature for 18 hours. Water was added to the reaction mixture and the mixture was extracted with ethyl acetate. The organic layer was washed with saturated brine, dried over anhydrous sodium sulfate, filtered and then concentrated under reduced pressure to give a residue, which was purified with silica gel column chromatography (hexane/ethyl acetate) to give compound 8-3 (130 mg). Compound 8-3 (130 mg) was dissolved in DMF (12 mL), 5N aqueous solution of sodium hydroxide (12 mL) was added thereto and the mixture was stirred at room temperature for 10 minutes. The reaction mixture was acidified by addition of 2N aqueous solution of hydrochloric acid at 0°C and extracted with ethyl acetate. The organic layer was washed with saturated brine, dried over anhydrous sodium sulfate, filtered and concentrated under reduced pressure to give compound 8-4 (119 mg).

Compound 8-2: $^1$H-NMR(DMSO-$d_6$) δ 3.84(s, 3H), 7.17(d, J = 7.8 Hz, 1H), 7.32(d, J = 7.6 Hz, 1H), 7.43(dd, J = 7.9, 1.4 Hz, 1H), 7.51(d, J = 1.5 Hz, 1H), 7.59(t-like, J = 7.6 Hz, 1H), 7.68(t-like, J = 7.6 Hz, 1H), 7.80(d, J = 7.8 Hz, 1H), 9.95(s, 1H).

Compound 8-3: $^1$H-NMR(DMSO-$d_6$) δ 3.74(s, 3H), 3.88(s, 3H), 7.27(d, J = 8.0 Hz, 1H), 7.33(d, J = 7.6 Hz, 1H), 7.47 (dd, J = 8.0, 2.0 Hz, 1H), 7.53-7.62(m, 2H), 7.68-7.72(m, 1H), 7.81(d, J = 7.6 Hz, 1H). Compound 8-4: $^1$H-NMR(DMSO-$d_6$) δ 3.72(s, 3H), 7.24(d, J = 7.8 Hz, 1H), 7.33(d, J = 8.0 Hz, 1H), 7.43-7.45(m, 1H), 7.53-7.62(m, 2H), 7.68-7.72(m, 1H), 7.80(d, J = 7.8 Hz, 1H), 13.05(bs, 1H).

Reference Example 9

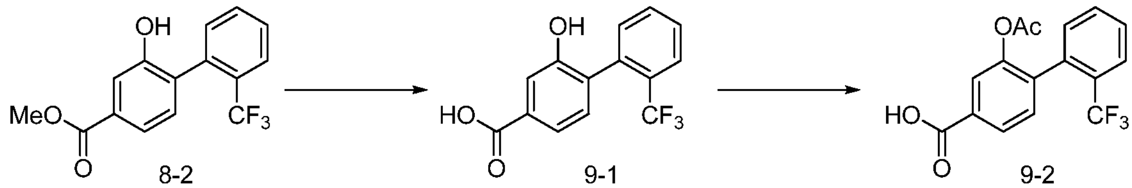

Compound 8-2 (2.0 g), which was an intermediate of Reference Example 8, was dissolved in a mixed solvent (33 mL) of tetrahydrofuran-methanol (1:1), 5N aqueous solution of sodium hydroxide (10 mL) was added dropwise thereto and the mixture was stirred at 80°C for 16 hours. The reaction solution was cooled to 0°C, and then acidified by addition of 2N aqueous solution of hydrochloric acid and extracted with ethyl acetate. The organic layer was washed with saturated brine, dried over anhydrous sodium sulfate, filtered and concentrated under reduced pressure to give compound 9-1 (1.5 g) as a crude product. The crude product of compound 9-1 (473 mg) was dissolved in tetrahydrofuran (5.6 mL), 4,4-dimethylaminopyridine (205 mg) and acetic anhydride (174 μL) were added thereto successively and the mixture was stirred at room temperature for 2 hours. The reaction solution was acidified by addition of 1N aqueous solution of hydrochloric acid and extracted with ethyl acetate. The organic layer was washed with 1N aqueous solution of hydrochloric acid and saturated brine successively, dried over anhydrous sodium sulfate, filtered and concentrated under reduced pressure to give a residue, which was purified with silica gel column chromatography (hexane/ethyl acetate) to give compound 9-2 (417 mg).

Compound 9-2: $^1$H-NMR(DMSO-$d_6$) δ 1.92(s, 3H), 7.25(d, J = 7.3 Hz, 1H), 7.44(d, J = 7.9 Hz, 1H), 7.63-7.79(m, 3H), 7.83-7.89(m, 2H), 13.3(bs, 1H).

Reference Example 10

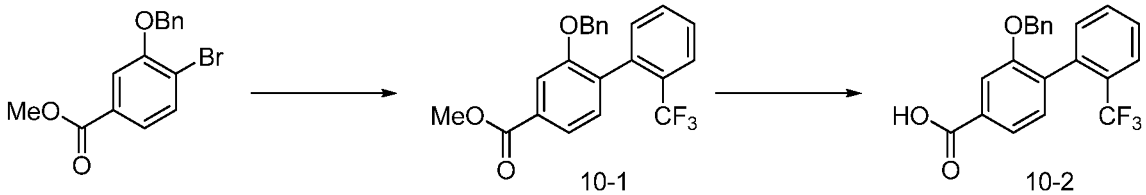

Compound 10-1 (2.6 g) was prepared from methyl 3-(benzyloxy)-4-bromobenzoate (4.6 g) and 2-(trifluoromethyl)phenylboronic acid (3.0 g) in the same manner as the first step of Reference Example 8. Compound 10-1 (1.3 g) was dissolved in a mixed solvent of tetrahydrofuran-methanol (1:1) (20 mL), 1N aqueous solution of sodium hydroxide (5 mL) was added dropwise thereto, and the mixture was stirred at 80°C for 16 hours. The reaction solution was cooled to 0°C, and then acidified by addition of 2N aqueous solution of hydrochloric acid and concentrated under reduced pressure. Then, after addition of ethyl acetate and 1N aqueous solution of hydrochloric acid, an organic layer was extracted. The

organic layer was washed with saturated brine, dried over anhydrous sodium sulfate, filtered and concentrated under reduced pressure to give compound 10-2 (1.2 g) as a white solid.

Compound 10-1: $^1$H-NMR(DMSO-$d_6$) $\delta$ 3.87(s, 3H), 5.14(AB q, $J_{AB}$ = 32.2 Hz, 2H), 7.16(d, J = 7.6 Hz, 2H), 7.23-7.32(m, 4H), 7.39(d, J = 7.6 Hz, 1H), 7.60-7.66(m, 3H), 7.71(t-like, J = 7.4 Hz, 1H), 7.83(d, J = 7.6 Hz, 1H).
Compound 10-2: $^1$H-NMR(DMSO-$d_6$) $\delta$ 5.13(AB q, $J_{AB}$ = 26.1 Hz, 2H), 7.15-7.50(m, 7H), 7.58-7.74(m, 4H), 7.82(d, J = 7.7 Hz, 1H), 13.12(bs, 1H).

Reference Example 11

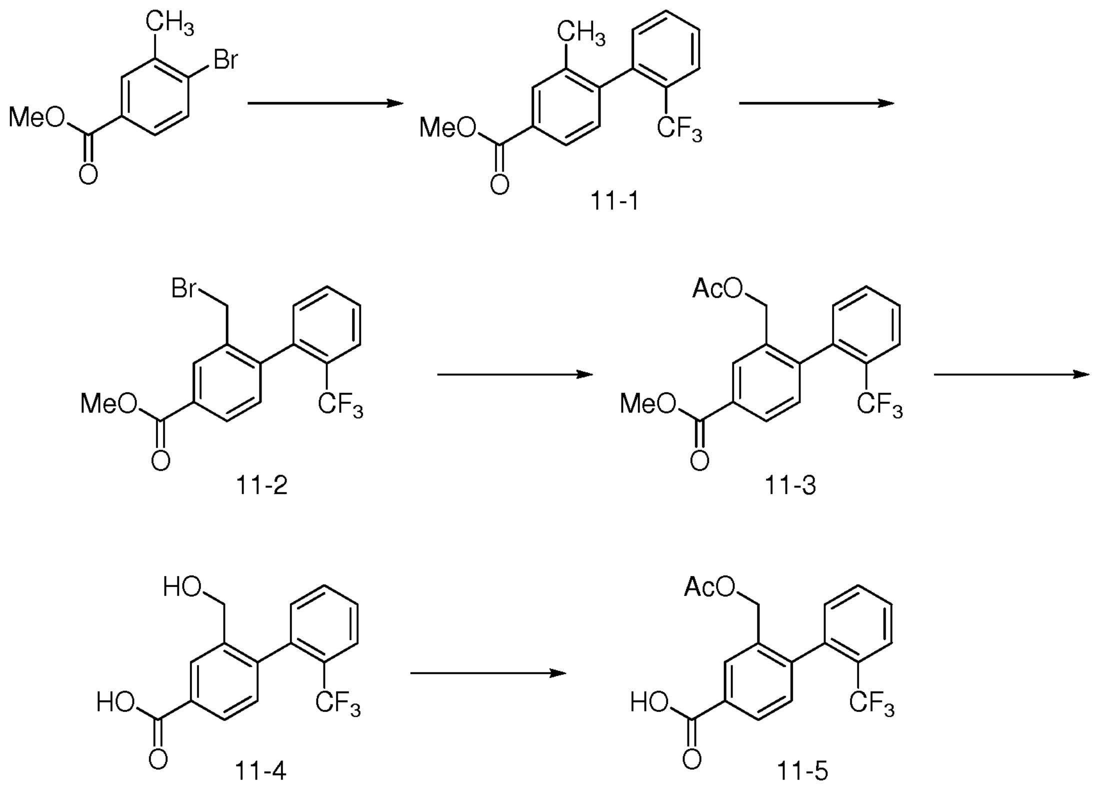

11-1

11-2

11-3

11-4

11-5

A mixture of methyl 4-bromo-3-methylbenzoate (4.58 g), [1,1'-bis(diphenylphosphino)-ferrocene]dichloropalladium (II) (1.46 g), potassium carbonate (11.0 g) and 2-(trifluoromethyl)phenylboronic acid (7.60 g) was stirred in DMF (100 mL) at 80°C for 24 hours. After cooling to room temperature, the reaction mixture was diluted with a mixed solvent of ethyl acetate-toluene (1:1) and filtered. Water was added to the filtrate and the mixture was extracted with ethyl acetate. The organic layer was washed with water and saturated brine successively, dried over anhydrous sodium sulfate, filtered and concentrated under reduced pressure to give a residue, which was purified with silica gel column chromatography (hexane/ethyl acetate) to give compound 11-1 (5.61 g). N-Bromosuccinimide (2.06 g) and benzoyl peroxide (133 mg) were added to a solution of compound 11-1 (3.07 g) in carbon tetrachloride (47 mL) and the mixture was heated to reflux for 6 hours. After cooling to room temperature, the reaction mixture was filtered. The filtrate was concentrated under reduced pressure and the resulting residue was purified with silica gel column chromatography (hexane/ethyl acetate) to give compound 11-2 (4.05 g) as a crude product. The crude product (3.78 g) of compound 11-2 was dissolved in acetic acid (73 mL), sodium acetate (4.17 g) was added thereto, and the mixture was heated to reflux for 16 hours. After cooling to room temperature, the reaction solution was concentrated under reduced pressure to give a residue, to which water was added and the mixture was extracted with ethyl acetate. The organic layer was washed with water and saturated brine successively, dried over anhydrous magnesium sulfate, filtered and concentrated under reduced pressure to give a residue, which was purified with silica gel column chromatography (hexane/ethyl acetate) to give compound 11-3 (2.39 g). Compound 11-3 (2.04 g) was dissolved in a mixed solvent of tetrahydrofuran-methanol (1:1) (40 mL), 1N aqueous solution of sodium hydroxide (23 mL) was added dropwise thereto, and the mixture was stirred at room temperature for 25 hours. The reaction solution was acidified by addition of 2N aqueous solution of hydrochloric acid, concentrated under reduced pressure and ethyl acetate and 1N aqueous solution of hydrochloric acid were added. The organic layer was extracted, washed with saturated brine, dried over anhydrous magnesium sulfate, filtered and concentrated under reduced pressure to give a residue, which was azeotropically distilled with toluene to give compound 11-4 (1.69 g). Compound 11-5 (89 mg) was prepared from compound 11-4 (94 mg) in the same manner

as the second step of Reference Example 9.

Compound 11-1: $^1$H-NMR($CDCl_3$) δ 2.08(s, 3H), 3.94(s, 3H), 7.22(d, J = 8.1 Hz, 2H), 7.50(t-like, J = 7.6 Hz, 1H), 7.59(t-like, J = 7.1 Hz, 1H), 7.77(d, J = 7.7 Hz, 1H), 7.88(md, J = 7.9 Hz, 1H), 7.94(m, 1H).

Compound 11-2: $^1$H-NMR($CDCl_3$) δ 3.96(s, 3H), 4.04(d, J = 10.4 Hz, 1H), 4.40(d, J = 10.4 Hz, 1H), 7.27(d, J = 8.1 Hz, 2H), 7.43(d, J = 7.3 Hz, 1H), 7.52-7.64(m, 2H), 7.79(d, J = 7.3 Hz, 1H), 7.99(dd, J = 8.0, 1.7 Hz, 1H), 8.21 (d, J = 1.7 Hz, 1H).

Compound 11-3: $^1$H-NMR($CDCl_3$) δ 2.00(s, 3H), 3.94(s, 3H), 4.81(AB q, $J_{AB}$ = 22.8 Hz, 2H), 7.28(d, J = 7.9 Hz, 2H), 7.48-7.59(m, 2H), 7.76(d, J = 7.2 Hz, 1H), 8.00(dd, J = 8.0, 1.7 Hz, 1H), 8.14(d, J = 1.3 Hz, 1H).

Compound 11-4: $^1$H-NMR($CDCl_3$) δ 4.42(AB q, $J_{AB}$ = 23.0 Hz, 2H), 7.29(d, J = 8.1 Hz, 2H), 7.50-7.61(m, 2H), 7.78(d, J = 7.5 Hz, 1H), 8.05(dd, J = 8.0, 1.6 Hz, 1H), 8.34(s, 1H).

Compound 11-5: $^1$H-NMR($CDCl_3$) δ 4.83(AB q, $J_{AB}$ = 20.4 Hz, 2H), 7.27(d, J = 7.5 Hz, 1H), 7.32(d, J = 8.0 Hz, 1H), 7.50-7.61(m, 2H), 7.77(d, J = 7.2 Hz, 1H), 8.08(dd, J = 7.9, 1.8 Hz, 1H), 8.21(d, J = 1.7 Hz, 1H).

Reference Example 12

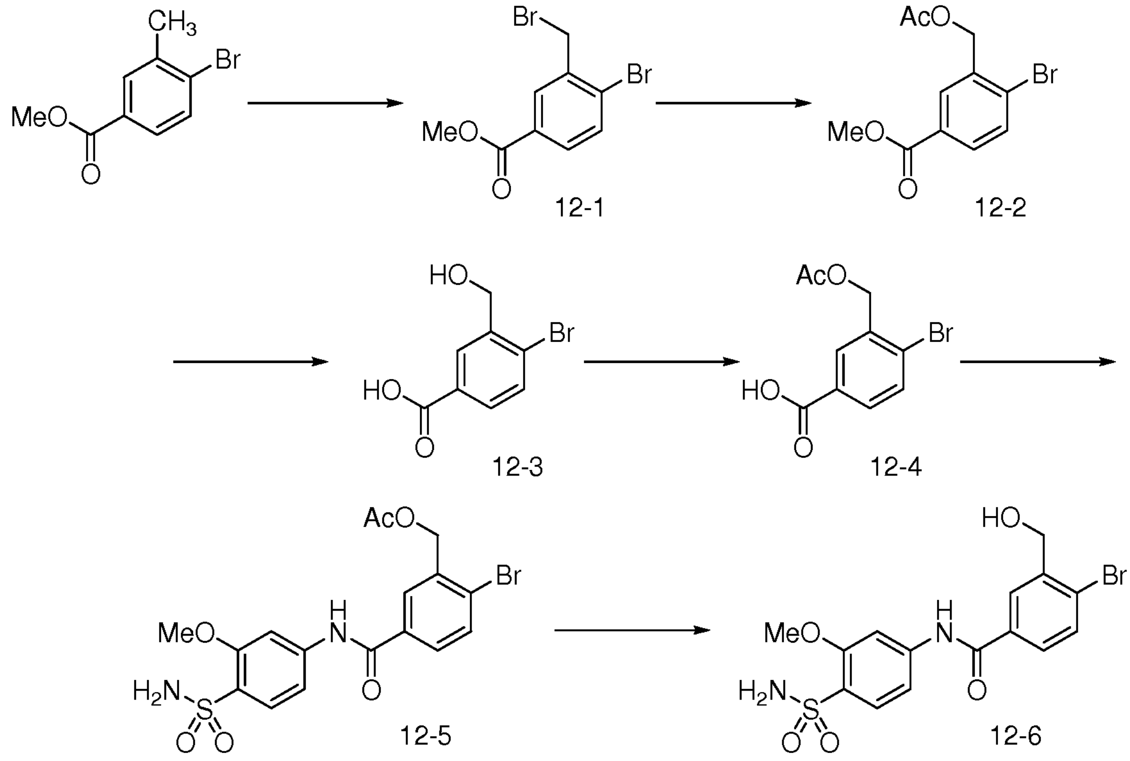

12-1 12-2 12-3 12-4 12-5 12-6

Compound 12-4 (1.00 g) was prepared from methyl 4-bromo-3-methylbenzoate (2.29 g) via compound 12-1, compound 12-2 and compound 12-3 in the same manner as the second step to the fifth step in Reference Example 11. Thionyl chloride (648 μL) and DMF (23 μL) were added dropwise to a suspension of compound 12-4 (1.00 g) in toluene (12 mL) and the mixture was stirred at 95°C for 2 hours. After cooling to room temperature, the reaction solution was concentrated under reduced pressure, azeotropically distilled with toluene and dissolved in tetrahydrofuran (30 mL). Compound of Reference Example 3 (658 mg) and diisopropylethylamine (773 μL) were added thereto and the mixture was stirred at room temperature for 20 hours. 2N Aqueous solution of hydrochloric acid (150 mL) was added to the reaction mixture with stirring and the resulting precipitate was filtered. The precipitate was washed with water and dried under reduced pressure to give compound 12-5 (1.40 g). Compound 12-5 (700 mg) was dissolved in a mixed solution (15 mL) of tetrahydrofuran-methanol (1:1), 1N aqueous solution of sodium hydroxide (2.3 mL) was added dropwise thereto, and the mixture was stirred at room temperature for 30 minutes. The reaction mixture was acidified with 2N aqueous solution of hydrochloric acid (1.2 mL), the solvent was evaporated under reduced pressure, water (8 mL) was added thereto with stirring and the precipitate was filtered. The precipitate was washed with water and dried under reduced pressure to give compound 12-6 (560 mg).

Compound 12-1: $^1$H-NMR($CDCl_3$) δ 3.91(s, 3H), 4.60(s, 2H), 7.64(d, J = 8.3 Hz, 1H), 7.79(dd, J = 8.3, 2.1 Hz, 1H), 8.09(d, J = 2.0 Hz, 1H).

Compound 12-2: $^1$H-NMR($CDCl_3$) δ 2.15(s, 3H), 3.91(s, 3H), 5.20(s, 2H), 7.64(d, J = 8.3 Hz, 1H), 7.83(dd, J =

8.3, 2.1 Hz, 1H), 8.04(d, J = 2.2 Hz, 1H).

Compound 12-3: $^1$H-NMR(DMSO-$d_6$) δ 4.54(s, 2H), 5.60(br, 1H), 7.70(d, J = 7.7 Hz, 1H), 7.74(dd, J = 8.2, 2.0 Hz, 1H), 8.11(m, 1H), 13.14(br, 1H).

Compound 12-4: $^1$H-NMR(DMSO-$d_6$) δ 2.11(s, 3H), 5.17(s, 2H), 7.79-7.84(m, 2H), 8.01(m, 1H), 13.30(br, 1H).

Compound 12-5: $^1$H-NMR(DMSO-$d_6$) δ 2.12(s, 3H), 3.89(s, 3H), 5.19(s, 2H), 7.01(s, 2H), 7.46(dd, J = 8.6, 1.7 Hz, 1H), 7.68-7.71(m, 2H), 7.84-7.91(m, 2H), 8.03(m, 1H), 10.62(s, 1H).

Compound 12-6: $^1$H-NMR(DMSO-$d_6$) δ 3.88(s, 3H), 4.58(d, J = 4.8 Hz, 2H), 5.63(t, J = 5.2 Hz, 1H), 7.01(br, 1H), 7.50(dd, J = 8.6, 1.8 Hz, 1H), 7.67-7.82(m, 6H), 8.12(m, 1H).

Reference Example 13

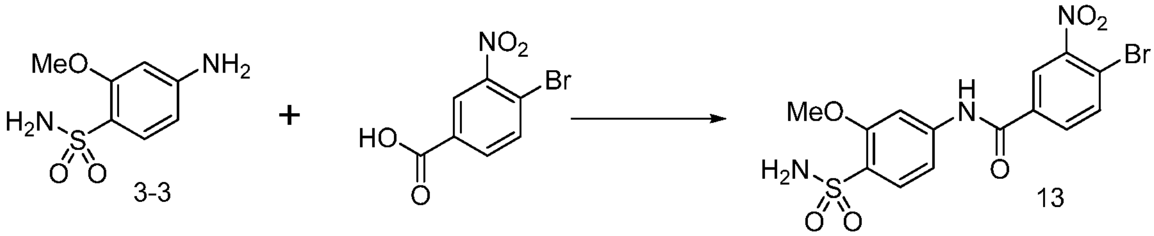

Compound 13 (407 mg) was prepared from 4-bromo-3-nitrobenzoic acid (246 mg) and compound 3-3 (222 mg) of Reference Example 3 in the same manner as the fifth step of Reference Example 12.

Compound 13: $^1$H-NMR(DMSO-$d_6$) δ 3.89(s, 3H), 7.03(s, 2H), 7.47(dd, J = 8.6, 1.7 Hz, 1H), 7.66(d, J = 1.7 Hz, 1H), 7.72(d, J = 8.4 Hz, 1H), 8.11-8.18(m, 2H), 8.58(d, J = 1.7 Hz, 1H), 10.76(s, 1H).

Reference Example 14

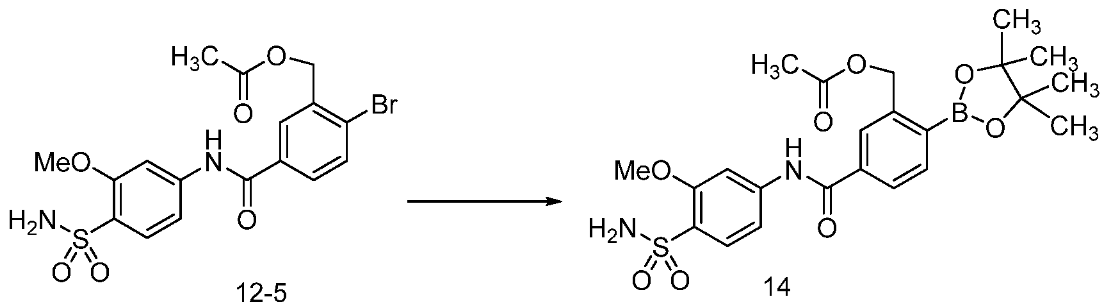

Compound 12-5 (4.0 g), which was an intermediate of Reference Example 12, was dissolved in dimethylsulfoxide (40 mL), [1,1'-bis(diphenylphosphino)ferrocene]dichloropalladium (II) (1.92 g), potassium acetate (3.1 g) and bispinacolato diboron (6.04 g) were added thereto, and the mixture was stirred at 85°C overnight. After cooling to room temperature, ice water was added to the reaction mixture and the mixture was extracted with ethyl acetate. The organic layer was washed with saturated brine, dried over anhydrous sodium sulfate, filtered and concentrated under reduced pressure to give a residue, which was recrystallized from chloroform, filtered and dried under reduced pressure to give compound 14 (2.96 g).

Compound 14: $^1$H-NMR(DMSO-$d_6$) δ 1.31(s, 12H), 2.05(s, 3H), 3.89(s, 3H), 5.30(s, 2H), 7.00(s, 2H), 7.50(d, J = 8.0 Hz, 1H), 7.69(d, J = 8.0 Hz, 1H), 7.84(d, J = 8.0 Hz, 1H), 7.94(d, J = 8.0 Hz, 1H), 10.6(s, 1H).

Reference Example 15

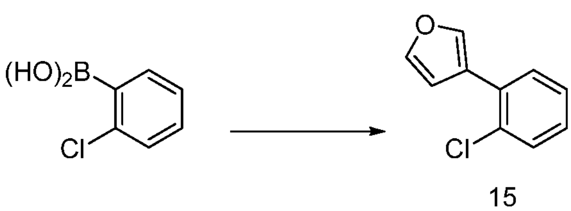

2-(3-Furanyl)chlorobenzene 15 (329 mg) was prepared from 2-chlorophenylboronic acid (625 mg) and 3-bromofuran (293 mg) in the same manner as the first step of Reference Example 11. Compound 15: $^1$H-NMR(DMSO-$d_6$) δ 6.90(d, J = 4.0 Hz, 1H), 7.29-7.41(m, 2H), 7.51-7.60(m, 2H), 7.78(t, J = 4.0 Hz, 1H), 8.11-8.12(m, 1H).

Reference Example 16

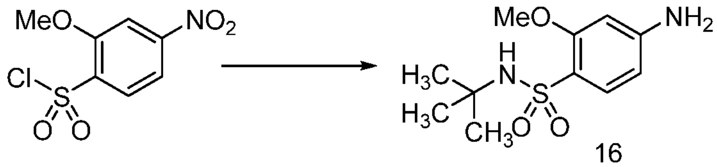

16

Compound 16 (24 g) was prepared from 2-methoxy-4-nitrobenzenesulfonyl chloride (25 g) in the same manner as Reference Example 3, wherein tert-butylamine was used in place of aqueous solution of ammonia.
Compound 16: $^1$H-NMR(DMSO-$d_6$) δ 1.01(s, 9H), 3.76(s, 3H), 5.85(s, 2H), 6.12(d, J = 8.0 Hz, 1H), 6.21(s, 1H), 6.35(s, 1H), 7.31(d, J = 8.0 Hz, 1H).

Reference Example 17

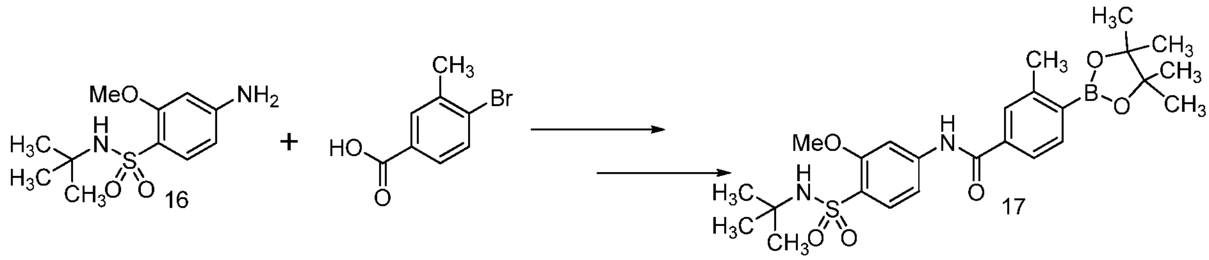

16 17

Compound 17 (411 mg) was prepared from the compound obtained in Reference Example 16 (1.0 g) and 4-bromo-3-methylbenzoic acid (0.69 g) through the same reactions as the fifth step of Reference Example 12 and Reference Example 14.
Compound 17: $^1$H-NMR(DMSO-$d_6$) δ 1.05(s, 9H), 1.32(s, 12H), 3.89(s, 3H), 6.87(s, 1H), 7.51(d, J = 12.0 Hz, 1H), 7.68-7.76(m, 5H), 10.5(s, 1H).

Reference Example 18

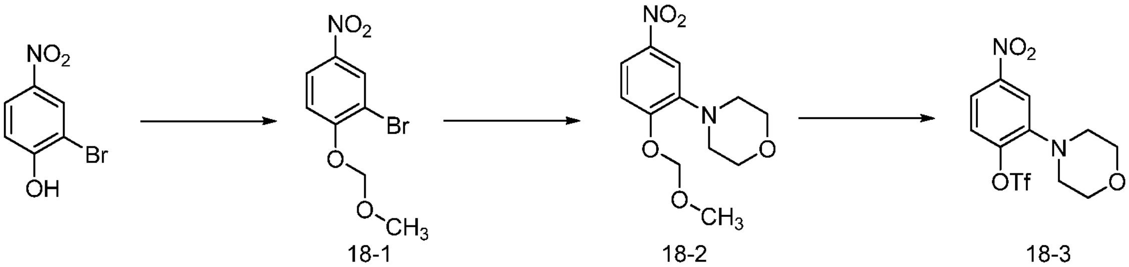

18-1 18-2 18-3

Sodium hydride (440 mg) was added to 2-bromo-4-nitro-phenol (2.0 g) in tetrahydrofuran (50 mL) and the mixture was stirred at room temperature for 1 hour. To the reaction mixture was added chloromethylmethylether (1.38 mL) and the mixture was further stirred at room temperature for 24 hours. A saturated aqueous solution of ammonium chloride was added to the reaction mixture and the mixture was extracted with diethylether. The organic layer was dried over anhydrous magnesium sulfate, filtered and concentrated under reduced pressure to give compound 18-1 (1.82 g). Compound 18-1 (1.0 g) was dissolved in a mixed solution of toluene (19.7 mL) and N,N-dimethylformamide (1.97 mL), morpholine (493 μL), palladium (II) acetate (85.7 mg), 2-dicyclohexylphosphino-2',4',6'-triisopropylbiphenyl (363 mg) and sodium tert-butoxide (550 mg) were added thereto, and the mixture was heated at 100°C for 1 hour using a microwave

reaction device [Initiator 60 EXP (400W), Biotage]. Water was added to the reaction mixture which was extracted with ethyl acetate. The organic layer was dried over anhydrous magnesium sulfate, filtered and concentrated under reduced pressure to give a residue, which was purified with silica gel column chromatography (hexane/ethyl acetate) to give compound 18-2 (502 mg). 4N Aqueous solution of hydrochloric acid/dioxane (4 mL) was added to compound 18-2 (206 mg) in methanol (5 mL) and the mixture was stirred at room temperature for 24 hours. The reaction mixture was concentrated under reduced pressure, dichloromethane (10.5 mL) was added thereto, and the mixture was cooled to 0°C. 2,6-Lutidine (183 μL) and trifluorometanesulfonic acid anhydride (207 μL) were added thereto, and the mixture was stirred for 1 hour. The reaction mixture was furter stirred at room temperature for 18 hours, an aqueous solution of potassium hydrogensulfate (2N) was added thereto, and the mixture was extracted with ethyl acetate. The organic layer was dried over anhydrous magnesium sulfate, filtered and concentrated under reduced pressure to give compound 18-3 (355 mg).

Compound 18-1: Analyzing method SC2, $t_R$ 1.021 min, obs MS[M+1] 262.3
Compound 18-2: Analyzing method SA3, $t_R$ 4.17 min, obs MS[M+1] 269.2
Compound 18-3: Analyzing method SC2, $t_R$ 1.117 min, obs MS[M+1] 357.2

Reference Example 19

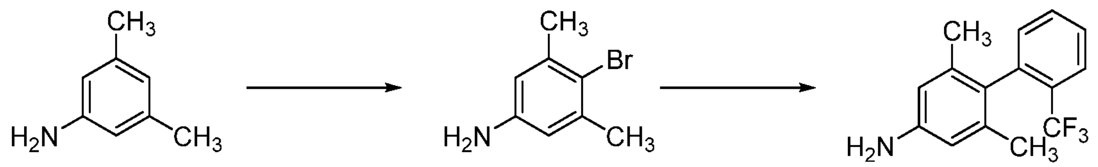

19-1 19-2

N-Bromosuccinimide (1.78 g) was added to 3,5-dimethylaniline (1.21 g) in acetonitrile (50 mL) and the mixture was stirred at room temperature for 8 hours. The reaction solution was concentrated under reduced pressure to give a residue, which was purified with silica gel column chromatography (hexane/ethyl acetate) to give compound 19-1 (1.78 g). A mixture of compound 19-1 (100 mg), tris(dibenzylideneacetone)dipalladium (0) (23 mg), 2-dicyclohexylphosphino-2',6-dimethoxybiphenyl (41 mg), potassium phosphate (424 mg) and 2-(trifluoromethyl)phenylboronic acid (190 mg) was stirred in toluene (1.0 mL) at 100°C for 32 hours. After cooling to room temperature, the reaction solution was diluted with ethyl acetate and filtered. The filtrate was concentrated under reduced pressure to give a residue, which was purified with silica gel column chromatography (hexane/ethyl acetate) to give compound 19-2 (51 mg).

Compound 19-1: $^1$H-NMR(CDCl$_3$) δ 2.31(d, J = 0.5 Hz, 6H), 3.53(bs, 2H), 6.44(d, J = 0.5 Hz, 2H).
Compound 19-2: $^1$H-NMR(CDCl$_3$) δ 1.85(s, 6H), 3.59(bs, 2H), 6.45(s, 2H), 7.17(d, J = 7.5 Hz, 1H), 7.44(t-like, J = 7.5 Hz, 1H), 7.56(t-like, J = 7.2 Hz, 1H), 7.75(d, J = 8.1 Hz, 1H).

Reference Example 20

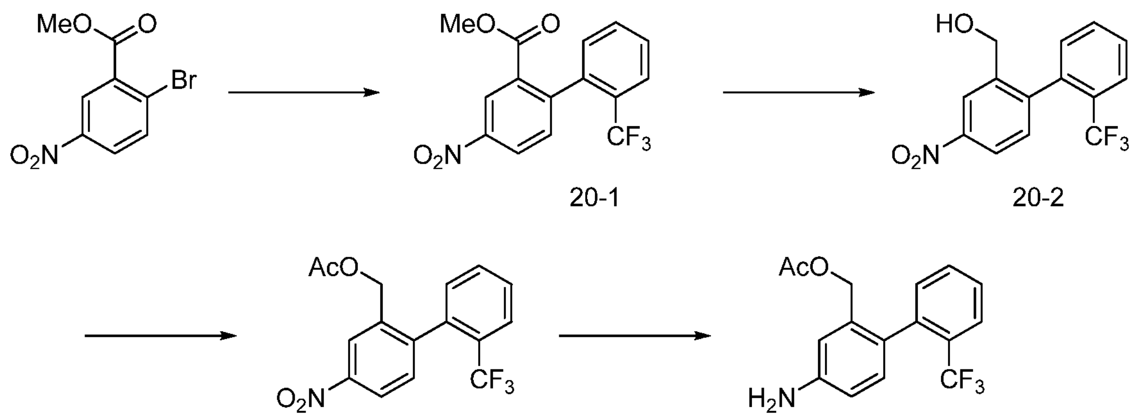

20-1 20-2

20-3 20-4

Compound 20-1 (26.7 g) was prepared from methyl 2-bromo-5-nitrobenzoate (26.0 g) in the same manner as the first step of Reference Example 11. Compound 20-1 (10.8 g) was dissolved in diethylether (220 mL), methanol (2.69 mL) was added thereto, and the mixture was cooled to 0°C. To the reaction mixture was added 2M solution of lithium

borohydride in tetrahydrofuran (33.2 mL), and the reaction mixture was warmed up to room temperature and stirred for 3 hours. After finishing the reaction, to the reaction mixture were added ice water and an aqueous solution of ammonium chrolide successively at 0°C, and the mixture was extracted with ethyl acetate. The organic layer was washed with saturated brine, dried over anhydrous sodium sulfate, filtered and concentrated under reduced pressure to give a residue, which was purified with silica gel column chromatography (hexane/ethyl acetate) to give compound 20-2 (9.67 g). Compound 20-3 (10.2 g) was prepared from compound 20-2 (9.67 g) in the same manner as the second step of Reference Example 9. Compound 20-3 (10.2g) was dissolved in a mixed solvent of tetrahydrofuran (190 mL) and ethanol (114 mL), an aqueous solution (38 mL) of ammonium chloride (7.34 g) and reduced iron (15.3 g) were added successively thereto, and the mixture was stirred at 75°C for 2.5 hours. After cooling, the reaction mixture was filtered through Celite, and Celite cake was washed with a mixture of tetrahydrofuran-ethanol (1:1). The filtrate was concentrated under reduced pressure and water and a mixture of ethyl acetate-tetrahydrofuran (1:1) were added thereto. The mixture was extracted, the aqueous layer was extracted with ethyl acetate, and the combined organic layers were washed with saturated brine, dried over anhydrous sodium sulfate, filtered and concentrated under reduced pressure to give a residue, which was purified with silica gel column chromatography (hexane/ethyl acetate) to give compound 20-4 (9.32 g).

Compound 20-1: $^1$H-NMR($CDCl_3$) δ 3.68(s, 3H), 7.19-7.20(m, 1H), 7.57(d, J = 8.5 Hz, 1H), 7.51-7.61(m, 2H), 7.74-7.76(m, 1H), 8.37(dd, J = 8.5, 2.4 Hz, 1H), 8.89(d, J = 2.2 Hz, 1H).
Compound 20-2: $^1$H-NMR($CDCl_3$) δ 1.75(bs, 1H), 4.42(s, 2H), 7.21(m, 1H), 7.33(d, J = 8.3 Hz, 1H), 7.53-7.63(m, 2H), 7.78-7.80(m, 1H), 8.15(dd, J = 8.4, 2.4 Hz, 1H), 8.50(d, J = 2.2 Hz, 1H).
Compound 20-3: $^1$H-NMR($CDCl_3$) δ 2.06(s, 3H), 4.83(t-like, J = 14.0 Hz, 2H), 7.26(m, 1H), 7.38(d, J = 8.3 Hz, 1H), 7.54-7.64(m, 2H), 7.78-7.81(m, 1H), 8.19(dd, J = 8.4, 2.4 Hz, 1H), 8.35(d, J = 2.4 Hz, 1H). Compound 20-4: $^1$H-NMR ($CDCl_3$) δ 1.97(s, 3H), 4.71(AB q, $J_{AB}$ = 29.8 Hz, 2H), 6.67(dd, J = 8.2, 2.5 Hz, 1H), 6.79(d, J = 2.6 Hz, 1H), 6.97(d, J = 8.1 Hz, 1H), 7.24-7.27(m, 1H), 7.41-7.53(m, 2H), 7.69-7.72(m, 1H).

Reference Example 21

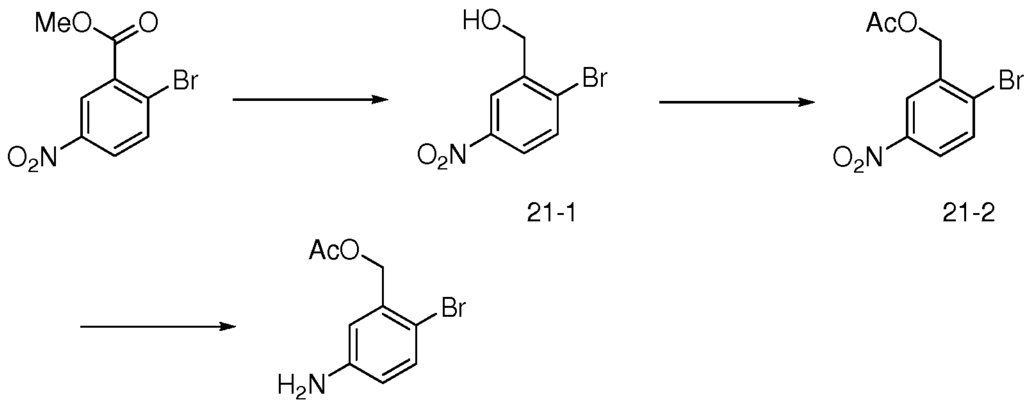

21-1 21-2

21-3

Compound 21-1 (3.14 g) was prepared from methyl 2-bromo-5-nitro-benzoate (4.94 g) in the same manner as the second step of Reference Example 20. Compound 21-2 (3.66 g) was prepared from compound 21-1 (3.14 g) in the same manner as the second step of Reference Example 9. Compound 21-3 (2.99 g) was prepared from compound 21-2 (3.46 g) in the same manner as the fourth step of Reference Example 20.

Compound 21-1: $^1$H-NMR($CDCl_3$) δ 2.12(br, 1H), 4.82(bs, 2H), 7.70(d, J = 8.8 Hz, 1H), 8.02(dd, J = 8.6, 2.8 Hz, 1H), 8.42(d, J = 2.8 Hz, 1H).
Compound 21-2: $^1$H-NMR($CDCl_3$) δ 2.20(s, 3H), 5.22(s, 2H), 7.75(d, J = 8.6 Hz, 1H), 8.03(dd, J = 8.7, 2.7 Hz, 1H), 8.25 (d, J = 2.8 Hz, 1H).
Compound 21-3: $^1$H-NMR($CDCl_3$) δ 2.12(s, 3H), 5.08(s, 2H), 6.52(dd, J = 8.4, 2.9 Hz, 1H), 6.73(d, J = 2.8 Hz, 1H), 7.29 (d, J = 8.4 Hz, 1H).

Reference Example 22

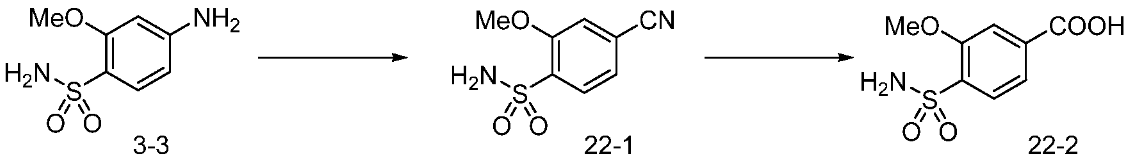

3-3 22-1 22-2

The compound obtained in Reference Example 3 (1.66 g) was dissolved in 2N aqueous solution of HCl (13.3 mL), an aqueous solution (6.6 mL) of sodium nitrite (0.6 g) was added dropwise thereto under ice-cooling over 30 minutes, and the mixture was stirred for 3 hours. Separately, an aqueous solution (10 mL) of potassium cyanide (2.45 g) was added to an aqueous solution (10 mL) of copper sulfate pentahydrate (2.0 g) at 70°C with stirring, and the mixture was cooled to room temperature. The resulting mixture was added to the above described aqueous solution of HCl at once under ice-cooling, and the mixture was heated to stir at 50°C for 30 minutes and then cooled to room temperature. Ethyl acetate (40 mL) was added thereto, and the mixture was filtered through Celite, an aqueous solution of sodium bicarbonate was added thereto, and the mixture was extracted with ethyl acetate. The organic layer was dried over anhydrous sodium sulfate, filtered and concentrated under reduced pressure to give a residue, which was purified with silica gel column chromatography (hexane/ethyl acetate) to give compound 22-1 (577 mg). Compound 22-1 (577 mg) was suspended in conc. aqueous solution of hydrochloric acid (19 mL) and the mixture was stirred at 120°C for 3 hours. The reaction solution was cooled to room temperature, concentrated under reduced pressure and the resulting residue was suspended in an aqueous solution of sodium hydrogencarbonate, filtered and dried under reduced pressure to give compound 22-2 (514 mg).

Compound 22-1: $^1$H-NMR(DMSO-$d_6$) $\delta$ 3.92(s, 3H), 7.38(s, 2H), 7.53(d, J = 8.0 Hz, 1H), 7.73(s, 1H), 7.86(d, J = 8.0 Hz, 1H).

Compound 22-2: $^1$H-NMR(DMSO-$d_6$) $\delta$ 3.95(s, 3H), 7.25(s, 2H), 7.60-7.62(m, 2H), 7.80-7.82(m, 1H).

Reference Example 23

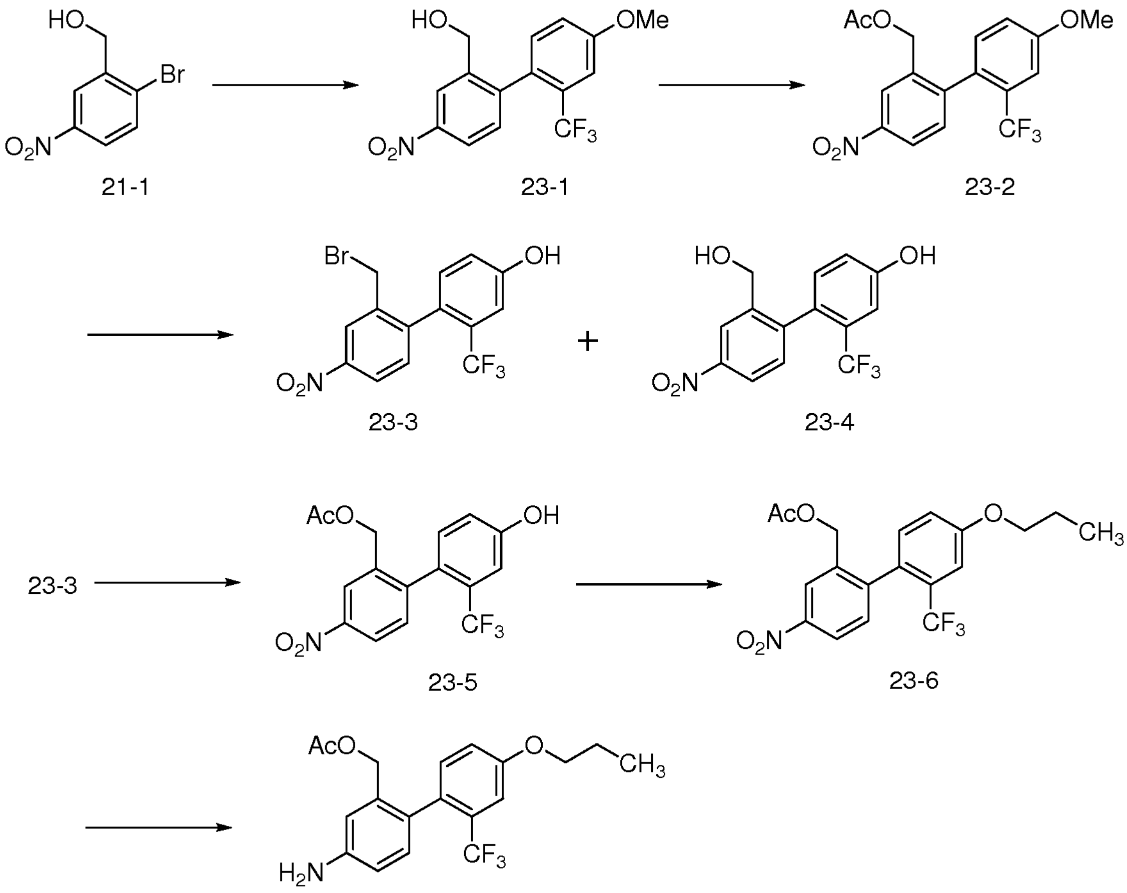

21-1 23-1 23-2

23-3 + 23-4

23-5 23-6

23-7

Compound 23-1 (299 mg) was prepared from compound 21-1 (232 mg), which was an intermediate of Reference Example 21, in the same manner as the first step of Reference Example 8. Compound 23-2 (331 mg) was prepared from compound 23-1 (297 mg) in the same manner as the second step of Reference Example 9. Compound 23-2 (330 mg) was suspended in dichloromethane (9 mL), and 1M solution of boron tribromide in dichloromethane (3.57 mL) was added thereto at -78°C. The mixture was warmed up to room temperature and stirred for 6 hours. After finishing the reaction, ice-water was added to the reaction mixture at 0°C and the mixture was extracted with ethyl acetate. The organic layer was washed with saturated brine, dried over anhydrous magnesium sulfate, filtered and concentrated under reduced pressure to give a residue, which was purified with silica gel column chromatography (hexane/ethyl acetate) to give compound 23-3 (186 mg) and compound 23-4 (69 mg). Sodium acetate (410 mg) was added to a solution of compound 23-3 (184 mg) in acetic acid (5 mL) and the mixture was heated to reflux for 6 hours. The reaction solution was cooled to room temperature, and then concentrated under reduced pressure, water was added to the residue, which was extracted with ethyl acetate. The organic layer was washed with water and saturated brine successively, dried over anhydrous magnesium sulfate, filtered and concentrated under reduced pressure to give compound 23-5 (158 mg). Potassium carbonate (41 mg) and n-propyl bromide (20 μL) were added to compound 23-5 (53 mg) in DMF (1.5 mL), and the mixture was stirred at room temperature for 4 hours. Potassium iodide (25 mg) was further added thereto, and the reaction solution was filtered, water was added to the filtrate and the mixture was extracted with ethyl acetate. The organic layer was washed with saturated brine, dried over anhydrous sodium sulfate, filtered and concentrated under reduced pressure to give a residue, which was purified with silica gel column chromatography (hexane/ethyl acetate) to give compound 23-6 (50 mg). Compound 23-7 (30 mg) was prepared from compound 23-6 (48 mg) in the same manner as the forth step of Reference Example 20.

Compound 23-1: $^1$H-NMR($CDCl_3$) δ 1.73(br, 1H), 3.89(s, 3H), 4.43(s, 2H), 7.09(dd, J = 8.6, 2.4 Hz, 1H), 7.13(d, J = 8.4 Hz, 1H), 7.28(d, J = 2.2 Hz, 1H), 7.31(d, J = 8.4 Hz, 1H), 8.13(dd, J = 8.3, 2.5 Hz, 1H), 8.48(d, J = 2.2 Hz, 1H).

Compound 23-2: $^1$H-NMR($CDCl_3$) δ 2.07(s, 3H), 3.89(s, 3H), 4.83(AB q, $J_{AB}$ = 21.9 Hz, 2H), 7.10(dd, J = 8.6, 2.4 Hz, 1H), 7.15(d, J = 8.6 Hz, 1H), 7.27(d, J = 2.4 Hz, 1H), 7.36(d, J = 8.4 Hz, 1H), 8.17(dd, J = 8.4, 2.4 Hz, 1H), 8.33 (d, J = 2.0 Hz, 1H).

Compound 23-3: $^1$H-NMR($CDCl_3$) δ 4.21(AB q, $J_{AB}$ = 97.1 Hz, 2H), 5.45(s, 1H), 7.08(dd, J = 8.3, 2.3 Hz, 1H), 7.24-7.27(m, 2H), 7.34(d, J = 8.4 Hz, 1H), 8.14(dd, J = 8.4, 2.4 Hz, 1H), 8.38(d, J = 2.4 Hz, 1H).

Compound 23-4: $^1$H-NMR(DMSO-$d_6$) δ 4.08-4.30(m, 2H), 5.55(t, J = 5.3 Hz, 1H), 7.07-7.19(m, 3H), 7.37(d, J = 8.4 Hz, 1H), 8.12(dd, J = 8.2, 2.6 Hz, 1H), 8.37(d, J = 2.4 Hz, 1H), 10.36(s, 1H).

Compound 23-5: $^1$H-NMR($CDCl_3$) δ 2.08(s, 3H), 4.85(AB q, $J_{AB}$ = 22.6 Hz, 2H), 5.63(s, 1H), 7.04(dd, J = 8.4, 2.5 Hz, 1H), 7.11(d, J = 8.4 Hz, 1H), 7.23(d, J = 2.4 Hz, 1H), 7.36(d, J = 8.4 Hz, 1H), 8.17(dd, J = 8.4, 2.4 Hz, 1H), 8.38 (d, J = 2.2 Hz, 1H).

Compound 23-6: $^1$H-NMR($CDCl_3$) δ 1.06(t, J = 7.3 Hz, 3H), 1.79-1.91(m, 2H), 2.07(s, 3H), 3.99(t, J = 6.4 Hz, 2H), 3.99(AB q, $J_{AB}$ = 22.1 Hz, 2H), 7.08(dd, J = 8.6, 2.4 Hz, 1H), 7.13(d, J = 8.4 Hz, 1H), 7.27(d, J = 2.4 Hz, 1H), 7.36 (d, J = 8.3 Hz, 1H), 8.17(dd, J = 6.4, 2.4 Hz, 1H), 8.33(d, J = 2.0 Hz, 1H).

Compound 23-7: Analyzing method SA4, $t_R$ 3.49 min, obs MS[M+1] 368.1

Reference Example 24

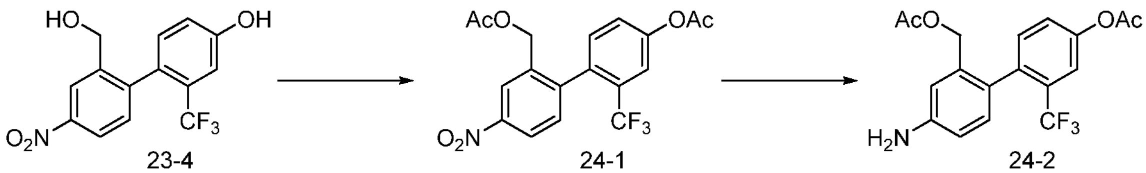

23-4 24-1 24-2

Compound 24-1 (62 mg) was prepared from compound 23-4 (67 mg), which was a by-product of Reference Example 23, in the same manner as the second step of Reference Example 9. Compound 24-2 (20 mg) was prepared from compound 24-1 (59 mg) in the same manner as the fourth step of Reference Example 20.
Compound 24-1: $^1$H-NMR($CDCl_3$) δ 2.06(s, 3H), 2.35(s, 3H), 4.85(s, 2H), 7.28(d, J = 8.4 Hz, 1H), 7.37-7.39(m, 2H), 7.54(d, J = 2.2 Hz, 1H), 8.19(dd, J = 8.4, 2.4 Hz, 1H), 8.34(d, J = 2.4 Hz, 1H).

Compound 24-2: Analyzing method SA4, $t_R$ 2.40 min, obs MS[M+1] 368.1

Reference Example 25

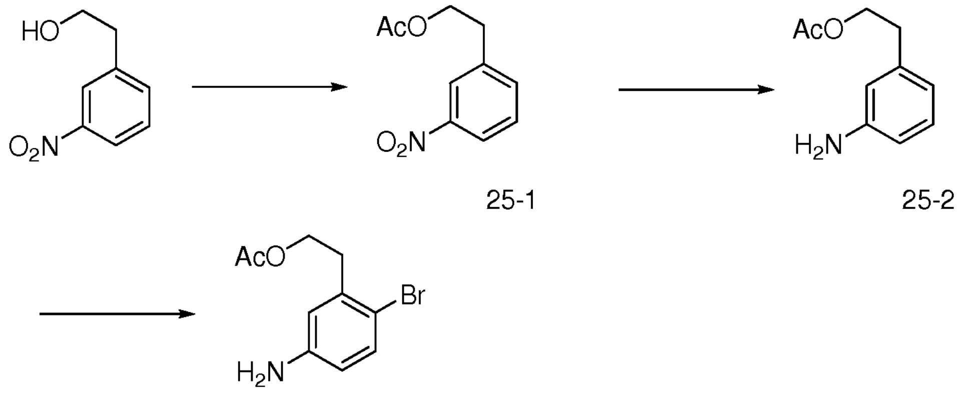

25-1 25-2

25-3

3-Nitrophenethyl alcohol (4.76 g) was dissolved in tetrahydrofuran (62 mL), acetic anhydride (4.03 mL) and 4-dimethylaminopyridine (5.21 g) were added thereto, and the mixture was stirred for 2 hours. The reaction mixture was acidified by addition of 2N aqueous solution of hydrochloric acid and extracted with ethyl acetate. The organic layer was washed with saturated brine, dried over anhydrous sodium sulfate, filtered and concentrated under reduced pressure to give compound 25-1 (5.95 g) as a crude product. Compound 25-1 (4.0 g) was dissolved in methanol (16 mL) and ethyl acetate (8 mL), 10% palladium-carbon (2.0 g) was added thereto, and the mixture was stirred under hydrogen atmosphere (3 atoms) overnight. The mixture was filtered through Celite and concentrated under reduced pressure to give compound 25-2 (0.77 g) as a crude product. Compound 25-2 (0.53 g) was dissolved in tetrahydrofuran (30 mL) and 2N aqueous solution of hydrochloric acid (1.5 mL), pyridinium bromide perbromide (946 mg) in tetrahydrofuran (45 mL) was added dropwise thereto under ice-cooling, and the mixture was stirred for 2 hours. The reaction mixture was poured into a saturated aqueous solution of sodium bicarbonate and extracted with ethyl acetate. The organic layer was washed with saturated brine, dried over anhydrous sodium sulfate, filtered and concentrated under reduced pressure to give a residue, which was purified with silica gel column chromatography (hexane/ethyl acetate) to give compound 25-3 (638 mg).

Compound 25-1: $^1$H-NMR($CDCl_3$) δ 2.08(s, 3H), 3.03(t, J = 8.0 Hz, 2H), 4.30(t, J = 8.0 Hz, 2H), 7.44-7.55(m, 2H), 8.09(bs, 1H).

Compound 25-2: $^1$H-NMR($CDCl_3$) δ 2.02(s, 3H), 2.83(t, J = 8.0 Hz, 2H), 4.24(t, J = 8.0 Hz, 2H), 6.58-6.65(m, 3H), 7.08 (bs, 1H).

Compound 25-3: $^1$H-NMR(DMSO-$d_6$) δ 2.50(s, 3H), 2.67-2.79(m, 2H), 4.10-4.16(m, 2H), 5.23(s, 2H), 6.34-6.40(m, 2H), 6.53(d, J = 4.0 Hz, 1H), 7.15(d, J = 9.0 Hz, 1H).

Reference Example 26

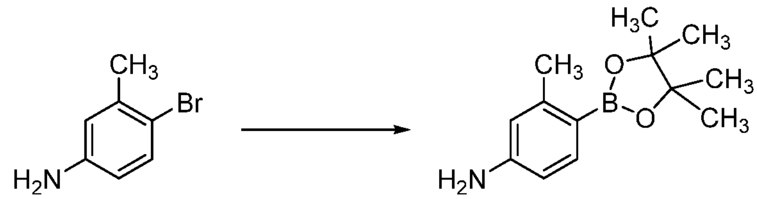

26

Palladium (II) acetate (300 mg), 2-dicyclophosphinebiphenyl (1.87 g), triethylamine (15.0 mL) and pinacolborane (11.6 mL) were added to 4-bromo-3-methylaniline (5.0 g) in dioxane (75 mL) and the mixture was stirred at 80°C for 24 hours. After adding water to the reaction mixture, the mixture was extracted with ethyl acetate. The organic layer was dried over anhydrous magnesium sulfate, filtered and concentrated under reduced pressure to give a residue, which was purified with silica gel column chromatography (hexane/ethyl acetate) to give compound 26 (4.85 g).

Compound 26: $^1$H-NMR($CDCl_3$) δ 1.31(s, 12H), 2.45(s, 3H), 3.74(br, 2H), 6.47-6.48(m, 2H), 7.59(d, J = 8.0 Hz, 1H).

Reference Example 27

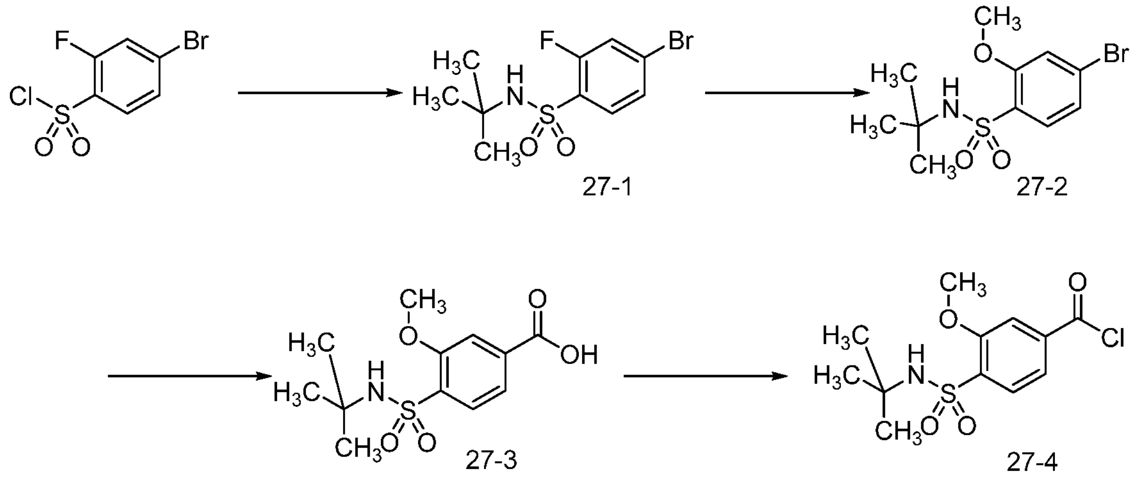

27-1 27-2 27-3 27-4

tert-Butylamine (58.0 mL) was added to 4-bromo-2-fluorobenzenesulfonyl chloride (50.0 g) in dichloromethane (180 mL) at 0°C and the mixture was stirred at room temperature for 2 hours. To the reaction mixture was added 2N aqueous solution of hydrochloric acid, and then chloroform and water were added thereto, and the mixture was separated. The organic layer was dried over anhydrous magnesium sulfate, filtered and concentrated under reduced pressure to give compound 27-1 (53.0 g). Sodium methoxide (27.7 g) was added to compound 27-1 (53.0 g) in N,N-dimethylacetamide (175 mL) and the mixture was stirred at room temperature for 4 hours. To the reaction mixture was added 2N aqueous solution of hydrochloric acid, and the mixture was stirred, filtered and dried under reduced pressure to give compound 27-2 (50.9 g). To compound 27-2 (10.0 g) in tetrahydrofuran (87.0 mL) was added 1M solution of methyl lithium in ether (34.1 mL) at -78°C and the mixture was stirred for 30 minutes. 2.7M n-Butyl lithium/hexane solution (12.7 mL) was added to the mixture above at -78°C and stirred for 1 hour, and dry ice was added thereto. The reaction solution was gradually warmed up to room temperature and stirred for 3 hours. Water, 1N aqueous solution of sodium hydroxide and ethyl acetate were added to the reaction mixture, and the mixture was extracted. The aqueous layer was acidified by addition of 6N aqueous solution of hydrochloric acid and extracted with ethyl acetate. The organic layer was dried over anhydrous magnesium sulfate, filtered and concentrated under reduced pressure to give compound 27-3 (7.80 g). To a solution of compound 27-3 (10.0 g) in dichloroethane (19.7 mL) were added oxalyl chloride (3.53 mL) and N,N-dimethylformamide (270 μL) at 0°C and the mixture was stirred at room temperature for 5 hours. The reaction mixture was concentrated under reduced pressure and the residue was azeotropically distilled with dichloroethane to give compound 27-4 (9.51 g).

Compound 27-2: Analyzing method SC2: $t_R$ 1.460 min, obs MS[M+1] 322.2

Compound 27-3: Analyzing method SC2: $t_R$ 0.741 min, obs MS[M+1] 288.3

Reference Example 28

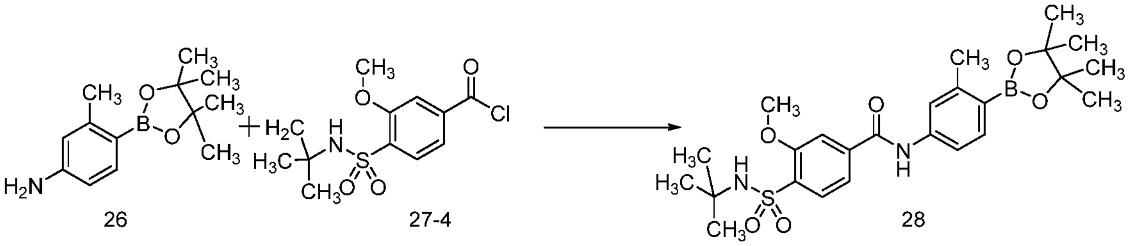

26 27-4 28

Compound 27-4 (5.33 g) prepared in Reference Example 27 and N-ethyl diisopropylamine (3.84 mL) were added to compound prepared in Reference Example 26 (4.84 g) in tetrahydrofuran (33 mL), and the mixture was stirred at room temperature for 24 hours. A saturated aqueous solution of ammonium chloride was added to the reaction mixture, and the mixture was extracted with ethyl acetate. The organic layer was washed with a saturated aqueous solution of sodium bicarbonate, then dried over anhydrous magnesium sulfate, filtered and concentrated under reduced pressure to give compound 28 (6.23 g).

Compound 28: Analyzing method SC2, $t_R$ 1.246 min, obs MS[M+1] 503.5

$^1$H-NMR(DMSO-$d_6$) δ 1.05(s, 9H), 1.35(s, 12H), 4.03(s, 3H), 7.19(s, 1H), 7.57-7.64(m, 5H), 7.86(d, J = 8.0 Hz, 1H), 10.4 (s, 1H).

Reference Example 29

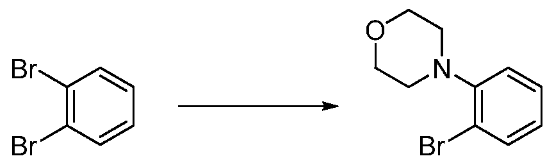

29

To toluene (70.6 mL) were added 1,2-dibromobenzene (3 g), palladium acetate (158 mg), 2,2'-diphenylphosphino-1,1'-binaphthyl (879 mg), sodium tert-butoxide (1.63 g) and morpholine (1.15 mL), and the mixture was stirred at 90°C overnight. The reaction mixture was cooled to room tempareture, filtered through Celite and concentrated under reduced pressure to give a residue, which was purified with silica gel column chromatography (hexane/ethyl acetate) to give compound 29 (500 mg).

$^1$H-NMR(DMSO-$d_6$) δ 4.95(s, 4H), 3.74(s, 4H), 6.99(t, J = 8.0 Hz, 1H), 7.17(d, J = 8.0 Hz, 1H), 7.35(t, J = 8.0 Hz, 1H), 7.59(d, J = 8.0 Hz, 1H).

Reference Example 30

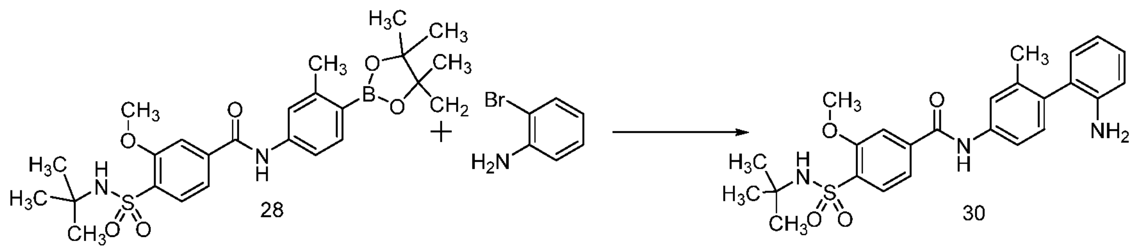

Compound prepared in Reference Example 28 (5.0 g), 2-bromoaniline (1.46 g), potassium carbonate (4.66 g) and [1,1'-bis(diphenylphosphino)ferrocene]palladium chloride (620 mg) were added to a mixed solvent of N,N-dimethylformamide (85 mL) and water (8.5 mL), and the mixture was stirred at 100°C for 20 hours. Water and ethyl acetate were added to the reaction mixture, the organic layer was extracted, dried over anhydrous magnesium sulfate, filtered and concentrated under reduced pressure to give a residue, which was purified with silica gel column chromatography (hexane/ethyl acetate) to give compound 30 (2.93 g).

Compound 30: Analyzing method SC2, $t_R$ 1.071 min, obs MS[M+1] 468.8

Reference Example 31

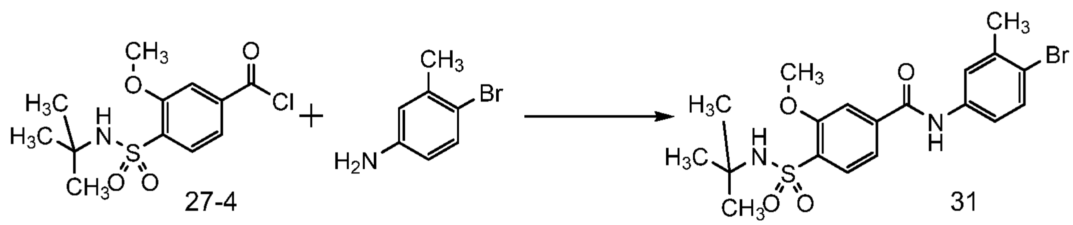

4-Bromo-3-methylaniline (2.76 g) and N-ethyl diisopropylamine (3.6 mL) were added to compound 27-4 (5.0 g) prepared in Reference Example 27 in tetrahydrofuran (31 mL) and the mixture was stirred at room temperature for 4 hours. A saturated aqueous solution of ammonium chloride was added to the reaction mixture which was extracted with ethyl acetate. The organic layer was washed with a saturated aqueous solution of sodium bicarbonate, dried over anhydrous magnesium sulfate, filtered and concentrated under reduced pressure to give compound 31 (7.8 g).

Compound 31: $^1$H-NMR(DMSO-$d_6$) δ 1.07(s, 9H), 2.34(s, 3H), 4.00(s, 3H), 7.19(s, 1H), 7.56-7.64(m, 4H), 7.75(m, 1H), 7.87(d, J = 8.0Hz, 1H), 10.42(s, 1H).

Reference Example 32

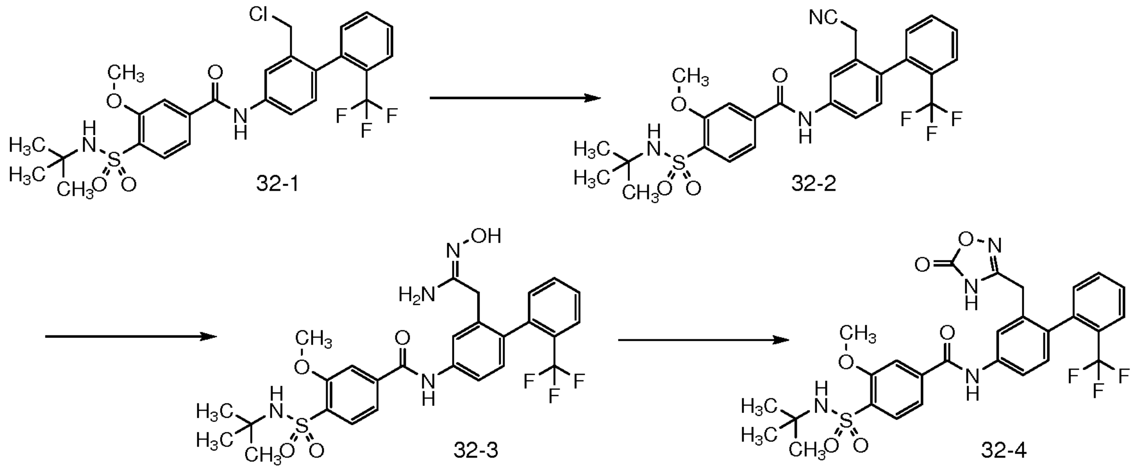

32-1 32-2 32-3 32-4

KCN (122 mg), potassium iodide (60 mg), 18-crown-6 (33 mg), MeCN (10 mL) and water (0.80 mL) was added to compound 32-1 (690 mg), which was prepared from compounds of Reference Example 27 and 20 in the same manner as examples 323 and 324, and the mixture was heated under reflux with stirring overnight. After finishing the reaction, water was added to the reaction mixture and extracted with ethyl acetate. The ethyl acetate layer was washed with water and brine, dried over anhydrous magnesium sulfate, filtered and concentrated under reduced pressure. The resulting residue was purified with silica gel column chromatography (hexane/ethyl acetate=1/1) to give compound 32-2 (510 mg). An aqueous solution of hydoxylamine (150 mg), sodium carbonate (245 mg), methanol (10 mL) and water (1 mL) were added to compound 32-2 (350 mg), and the mixture was heated under reflux with stirring for 5 hours. After finishing the reaction, water was added to the reaction mixture which was extracted with ethyl acetate. The ehtyl acetate-layer was washed with water and brine, dried over anhydrous magnesium sulfate, filtered and concentrated under reduced pressure to give crude compound 32-3 (330 mg). CDI (93 mg), DBU (0.090 mL) and methanol (7 mL) were added to the crude compound 32-3 (330 mg) and the mixture was stirred at room temperature for 4 hours. After finishing the reaction, water was added to the reaction mixture which was extracted with ethyl acetate. The ethyl acetate-layer was washed with water and brine, dried over anhydrous magnesium sulfate, filtered and concentrated to give a residue, which was purified with silica gel column chromatography (MeOH/$CHCl_3$= 5/95) to give compound 32-4 (130 mg).

Compound 32-2: Analyzing method SA2, $t_R$ 4.72 min, obs MS[M+1] 546.2
Compound 32-3: Analyzing method SA2, $t_R$ 4.62 min, obs MS[M+1] 579.1
Compound 32-4: Analyzing method SA2, $t_R$ 4.73 min, obs MS[M+1] 605.3

Reference Example 33

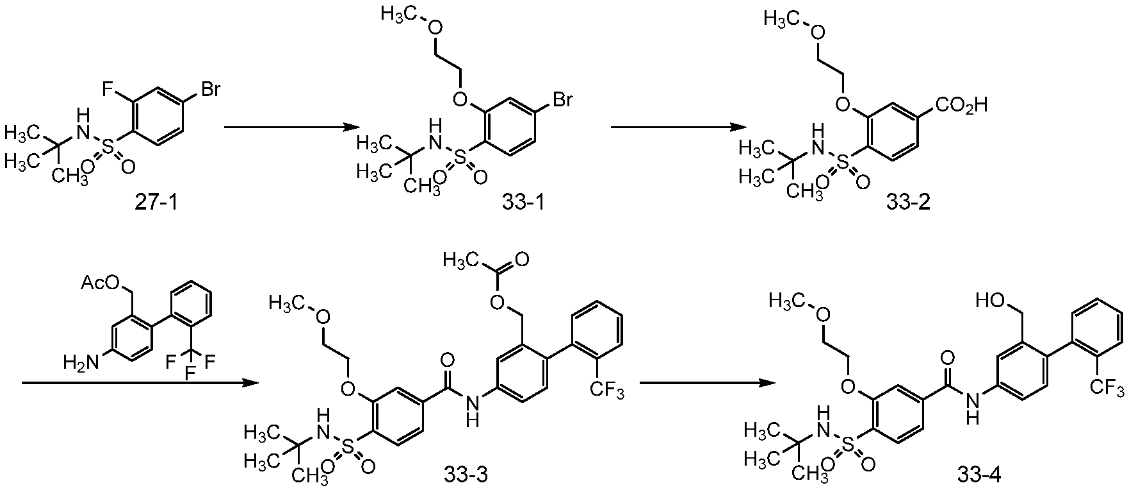

27-1 33-1 33-2 33-3 33-4

2-Methoxyethanol (2.3 ml), NaH (1.16 g) and DMA (50 ml) were added to compound 27-1 (30. g), which was an intermediate of Reference Example 27, and the mixture was stirred at room temperature overnight. After finishing the reaction, water was added to the reaction mixture which was extracted with ethyl acetate. The ethyl acetate layer was washed with water and brine, dried over anhydrous magnesium sulfate, filtered and concentrated to give a residue,

which was purified with silica gel column chromatography (hexane/ethyl acetate = 3/1) to give compound 33-1 (3.52 g). Compound 33-2 (2.32 g) was prepared from compound 33-1 (3.0 g) as a crude product in the same manner as the third step in Reference Example 27, and it was used in the next step without further purification. Oxalyl chloride (0.11 mL) and DMF (three drops) were added to the crude compound 33-2 (200 mg) and the mixture was stirred at room temperature for 1 hour. After finishing the reaction, solvent was evaporated, toluene (5 mL) was added to the residue, and the solvent was evaporated again. To the resulting residue, were added compound prepared in Reference Example 20 (185 mg), TEA (0.17 mL) and THF (5 mL) and the mixture was stirred at room temperature for 1 hour. After finishing the reaction, solvent was evaporated and the resulting residue was purified with silica gel column chromatography (hexane/ethyl acetate = 1/1) to give compound 33-3 (210 mg). 2N Aqueous solution of sodium hydroxide (3 mL), methanol (3 mL) and THF (3 mL) were added to compound 33-3 (210 mg) and the mixture was stirred at room temperature for 2 hour. After finishing the reaction, solvent was evaporated, 2N aqueous solution of hydrochloric acid (3 mL) was added to the residue which was extracted with ethyl acetate. The ethyl acetate layer was washed with water and brine, dried over anhydrous magnesium sulfate, filtered and concentrated to give compound 33-4 (150 mg).

Compound 33-1: Analyzing method SA2, $t_R$ 4.93 min, obs MS[M+1] 366.1
Compound 33-2: Analyzing method SA2, $t_R$ 4.92 min, obs MS[M+1] 332.1
Compound 33-3: Analyzing method SA2, $t_R$ 4.78 min, obs MS[M+1] 623.2
Compound 33-4: Analyzing method SA2, $t_R$ 4.68 min, obs MS[M+1] 581.2

Reference Example 34

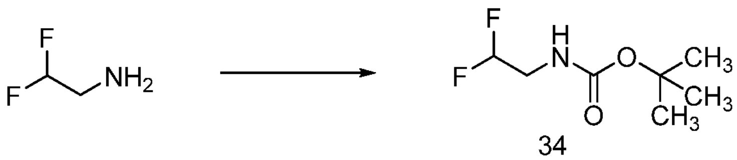

34

To 2,2-Difluoroethylamine (20.1 g) in 2-propanol (825 mL) were added di-tert-butyldicarbonate ($Boc_2O$) (86.6 g) and diiropropylethylamine (57.6 mL) successively at room temperature. The reaction mixture was warmed up to 50°C and stirred for 15 hours. The reaction mixture was cooled to room temperature and concentrated under reduced pressure. The resulting residue was dissolved in methanol (80 mL) and tetrahydrofuran (80 mL), 2N aqueous solution of sodium hydroxide (268 mL) was added dropwise and the mixture was stirred at room temperature for 2 hours. Chloroform was added to the reaction mixture which was extracted with chloroform. Chloroform layer was washed with water, dried over anhydrous sodium sulfate and concentrated under reduced pressure to give comopund 34 (42.7 g).
$^1$H-NMR(DMSO-$d_6$) $\delta$ 1.38(s, 9H), 3.22-3.36(m, 2H), 5.73-6.13(m, 1H), 7.24(brt, 1H).

Reference Example 35

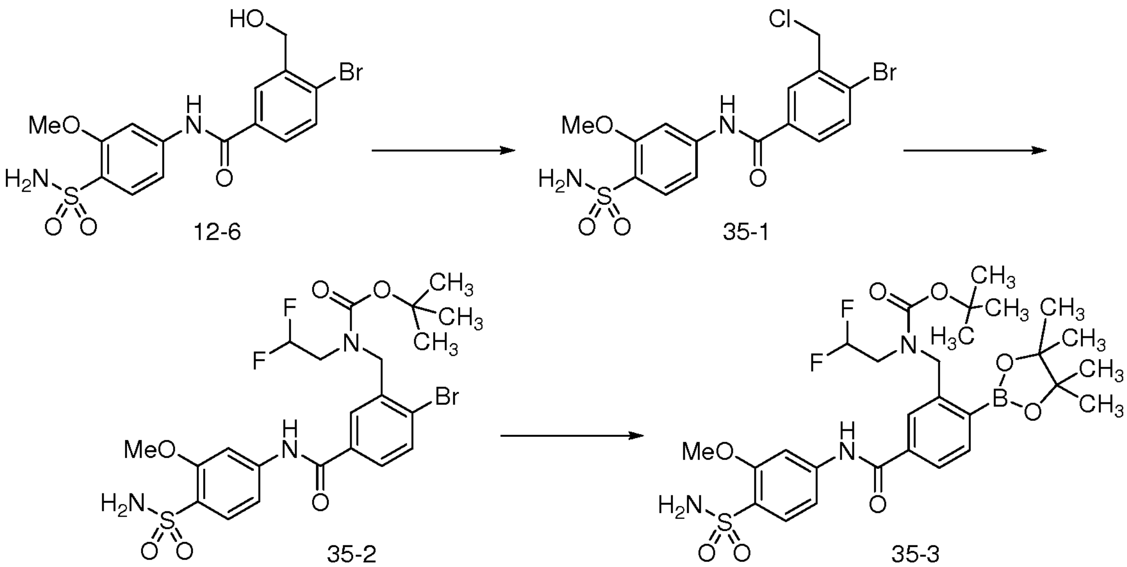

12-6 35-1 35-2 35-3

To a suspension of compound 12-6 (10 g) prepared in Reference Example 12 in ethyl acetate (200 mL) was

added thionyl chloride (100 mL) at room temperature. The mixture was heated at 70°C for 3 hours. The reaction mixture was cooled to room temperature, concentrated under reduced pressure and azeotropically distilled together with ethyl acetate three times. The resulting residue was suspended in ethyl acetate (33 mL) and hexane (26 mL), triturated by ultrasonic irradiation for 15 minutes, stirred at room temperature for 5 hours and the solid was filtrated. The collected solid was washed with a mixed solvent of ethyl acetate (80 mL) and hexane (100 mL), and hexane (60 mL) successively dried under reduced pressure to give compound 35-1 (9.97 g). Sodium hydride (60%; 7.04 g) was added to a suspension of a compound prepared in Reference Example 34 (7.97 g) in N-methyl pyrrolidone (80 mL), and the mixture was stirred at room temperature for 30 minutes. Compound 35-1 (9.97 g) was added to the resulting suspension and the mixture was stirred at room temperature for 1 hour. The reaction mixture was poured into a mixture of 1.2N aqueous solution of hydrochloric acid (360 mL) and ice (360 g) with vigorous stirring and the mixture was stirred at room temperature for 30 minutes. The resulting white solid was filtered, washed with water (400 mL) and hexane (60 mL) successively and dried under reduced pressure. The obtained solid was azeotropically distilled with toluene until it was dissolved in chloroform to give a homogenious solution, and then purified with silica gel column chromatography (hexane/ethyl acetate) to give compound 35-2 (9.78 g). Compound 35-2 (1.16 g) was treated in the same manner as Reference Example 14 to give a crude product, which was purified with silica gel column chromatography (hexane/ethyl acetate) to give compound 35-3 (846 mg).

Compound 35-1: $^1$H-NMR(DMSO-$d_6$) δ 3.89(s, 3H), 4.90(s, 2H), 7.00(s, 2H), 7.48(dd, J = 8.6, 2.0 Hz, 1H), 7.67-7.71(m, 2H), 7.88(s, 2H), 8.18(s, 1H), 10.62(s, 1H).
Compound 35-2: $^1$H-NMR(DMSO-$d_6$) δ 1.17(brd, J = 6.6 Hz, 9H), 3.68-3.74(m, 2H), 3.88(s, 3H), 4.55(s, 2H), 5.99-6.36 (m, 1H), 7.00(s, 2H), 7.46(dd, J = 8.6, 1.5 Hz, 1H), 7.68-7.74(m, 3H), 7.81-7.87(m, 2H), 10.58(s, 1H).
Compound 35-3: $^1$H-NMR(DMSO-$d_6$) δ 1.32-1.43(brm, 21H), 3.55-3.64(m, 2H), 3.88(s, 3H), 4.77(s, 2H), 5.99-6.27(m, 1H), 6.99(s, 2H), 7.49(d, J= 8.8 Hz, 1H), 7.67-7.93(m, 5H), 10.57(s, 1H).

Reference Example 36

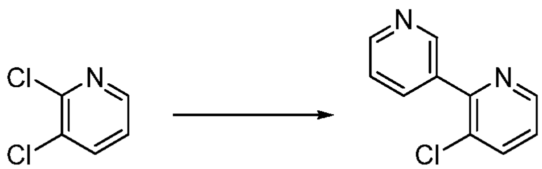

36

2,3-Dichloropyridine (300 mg), 3-pyridineboronic acid (298.7 mg), potassium carbonate (840 mg) and [1,1'-bis (diphenylphosphino)ferrocene]palladium chloride (148 mg) were added to a mixed solvent of toluene (2.6 mL) and ethanol (2.6 mL), and the mixture was heated in a sealed tube to 130°C for 1 hour by using a microwave synthesizer [Initiator 60 EXP (400W), Biotage]. Water and ethyl acetate were added to the reaction mixture, whichwas extracted, dried over anhydrous magnesium sulfate, filtered , and concentrated under reduced pressure to give a residue, which was purified with silica gel column chromatography (hexane/ethyl acetate) to give compound 36 (296 mg).
$^1$H-NMR($CDCl_3$) δ 7.29(dd, J=4.8, 8.0 Hz, 1H), 7.42(dd, J=4.8, 8.0 Hz, 1H), 7.84(dd, J=1.6, 8.0 Hz, 1H), 8.08(ddd, J=1.6, 9.6 Hz, 1H), 8.64(dd, J=1.2, 4.8 Hz, 1H), 8.68(dd, J=1.6, 4.8 Hz, 1H), 9.01(d, J=2.0 Hz, 1H).

Reference Example 37

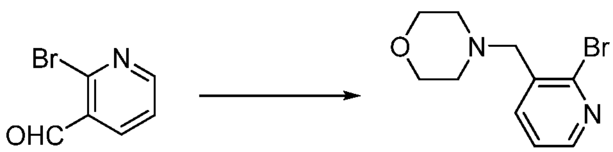

37

2-Bromo-3-formylpyridine (500 mg), morpholine (464 μL) and acetic acid (356 μL) were added to tetrahydrofuran (13 mL) and the mixture was stirred at room temperature for 1 hour. Sodium acetate (440 mg) and sodium triacetoxyborohydride (1.87 g) were added to the reaction mixture which was stirred at room temperature for 6 hours. The reaction mixture was neutralized by addition of a saturated aqueous solution of sodium bicarbonate, and water and ethyl acetate were added and extracted. The separated organic layer was dried over anhydrous magnesium sulfate, filtered, and concentrated under reduced pressure to give a residue, which was purified with silica gel column chromatography

(hexane/ethyl acetate) to give compound 37 (660 mg).
$^1$H-NMR($CDCl_3$) $\delta$ 2.60(t-like, J=6.4 Hz, 4H), 3.66(s, 2H), 3.76(t-like, J=6.4 Hz, 4H), 7.29(dd, J=6.0, 10.4 Hz, 1H), 7.85 (dd, J=2.8, 10.4 Hz, 1H), 8.31(dd, J=2.8, 6.4 Hz, 1H).

Reference Example 38

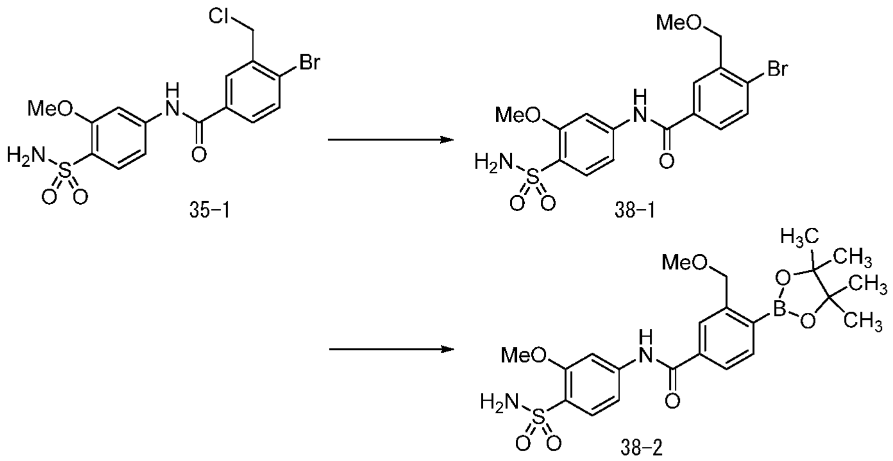

35-1

38-1

38-2

Compound 35-1 (500 mg), which was prepared in the first step of Reference Example 35, and sodium methoxide (498 mg) were dissolved in N-methyl-2-pyrrolidone (3.0 mL), and the mixture was stirred at room temperature for 4 hours. 2N Aqueous solution of hydrochloric acid and an excess amount of water were added to the reaction mixture, and the product was precipitated as a solid by using ultrasonic irradiation. The product was filtered, washed with water and dried to give compound 38-1 (471 mg). Compound 38-1 (420 mg), bis(pinacolato)diboran (696 mg), potassium acetate (346 mg) and 1,1'-bis(diphenylphosphino)ferrocene]dichloropalladium (215 mg) were added to dimethylsulfoxide (4.5 mL) and the mixture was stirred at 85°C for 6 hours. The reaction mixture was cooled to room temperature, water and ethyl acetate were added, and the extracted organic layer was dried over anhydrous magnesium sulfate and filtered. The filtrate was concentrated, chloroform (4 mL) and hexane (5 mL) were added and the product was precipitated as a solid by using ultrasonic irradiation. The product was filtered, washed with water and dried to give compound 38-2 (296 mg).

Compound 38-1: Analyzing method SC2, $t_R$ 1.229min, obs MS[M+1] 430.2

Compound 38-2: Analyzing method SA2, $t_R$ 5.35min, obs MS[M+1] 477.3

Reference Example 39

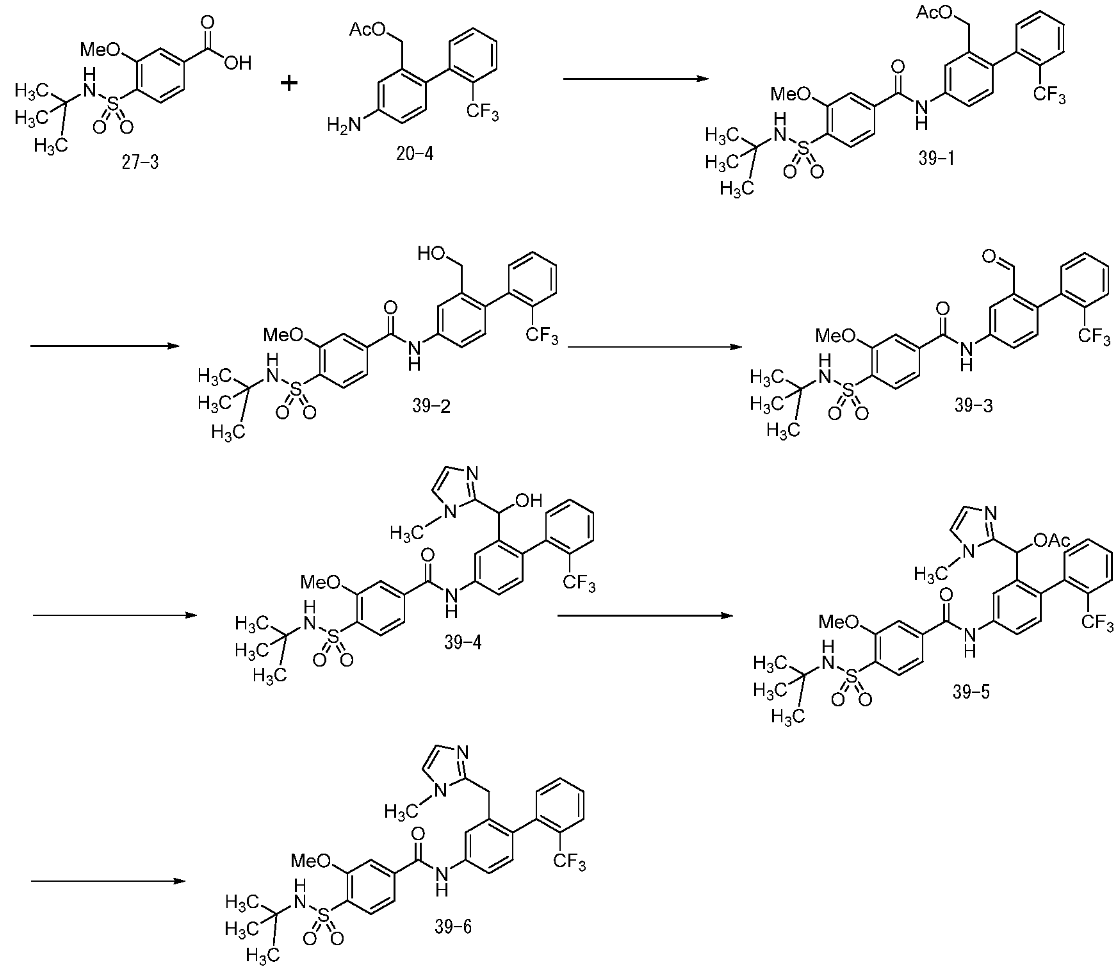

27-3 20-4 39-1 39-2 39-3 39-4 39-5 39-6

Compound 20-4 of Reference Example 20 (1.86 g), THF (10 mL). N,N-diisopropylethylamine (1.2 mL) and 2-chloro-1,3-dimethylimidazolidinium tetrafluoroborate (1.59 g) were added to compound 27-3 of Reference Example 27 (2.07 g) and the mixture was stirred at room temperature for 4 hours. Then, N,N-diisopropylethylamine (0.3 mL) and 2-chloro-1,3-dimethylimidazolidinium tetrafluoroborate (0.4 g) were added and the mixture was further stirred at room temperature for 15 hours. Water and a saturated aqueous solution of sodium bicarbonate were added to the reaction mixture and extracted with ethyl acetate. The organic layer was washed with brine, dried over anhydrous sodium sulfate, filtered and concentrated under reduced pressure. The resulting residue was purified with silica gel column chromatography (hexane/ethyl acetate) to give compound 39-1 (1.53 g). To compound 39-1 (1.53 g) in a mixed solvent of THF/MeOH (3 mL/3 mL) was added 2N aqueous solution of sodium hydroxide (2.6 mL) and the mixture was stirred at room temperature for 1 hour. 2N Aqueous solution of hydrochloric acid (3 mL) and water (100 mL) were added and the mixture was further stirred at room temperature for 30 minutes. The precipitated solid was filtered to give compound 39-2 (1.43 g). Manganese (IV) dioxide (400 mg) was added to compound 39-2 (100 mg) in dichloromethane (2 ml) and the mixture was stirred at room temperature for 17 hours. The reaction mixture was filtered through Celite and the filtrate was concentrated under reduced pressure to give compound 39-3 (120 mg). N-methyl imidazole (160 μL) in THF (4 mL) was cooled to -78°C, n-butyl lithium (0.74 mL) was added and the mixture was stirred at - 78°C for 15 minutes. Compound 39-3 (120 mg) in THF (3 mL) was added dropwise therein and the mixture was stirred under -50°C for 30 minutes. A saturated aqueous solution of ammonium chloride was added, the reaction mixture was warmed up to room temperature and extracted with ethyl acetate. The organic layer was washed with brine, dried over anhydrous sodium sulfate, filtered and concentrated under reduced pressure. The resulting residue was purified with silica gel column chromatography (chloroform/methanol) to give compound 39-4 (57 mg). Acetic anhydride (110 μL) and 4-dimethylaminopyridine (2 mg) were added to a THF-solution (1 mL) of compound 39-4 (36 mg) in THF (1 mL) and the mixture was stirred at room temperature for 5 hours. The reaction mixture was concentrated under reduced pressure to give compound 39-5 (38 mg). 10% Palladium-carbon (140 mg) was added to a methanol-solution (2 mL) of compound 39-5 (38 mg) in methanol (2 mL) and the mixture was stirred under hydrogen atmosphere (0.4 MPa) at room temperature for 16 hours. The reaction mixture was filtered through Celite, the filtrate was concentrated under reduced pressure and the residue was purified with silica gel column chromatography (chloroform/methanol) to give compound 39-6 (11 mg).

Compound 39-1: Analyzing method SC2, $t_R$ 1.20 min, obs MS[M+1] 579.1
Compound 39-2: Analyzing method SC2, $t_R$ 1.13 min, obs MS[M+1] 537.1
Compound 39-4: Analyzing method SC2, $t_R$ 0.86 min, obs MS[M+1] 617.0
Compound 39-5: Analyzing method SC2, $t_R$ 0.98 min, obs MS[M+1] 659.4
Compound 39-6: Analyzing method SC2, $t_R$ 0.87 min, obs MS[M+1] 601.7

Reference Example 40

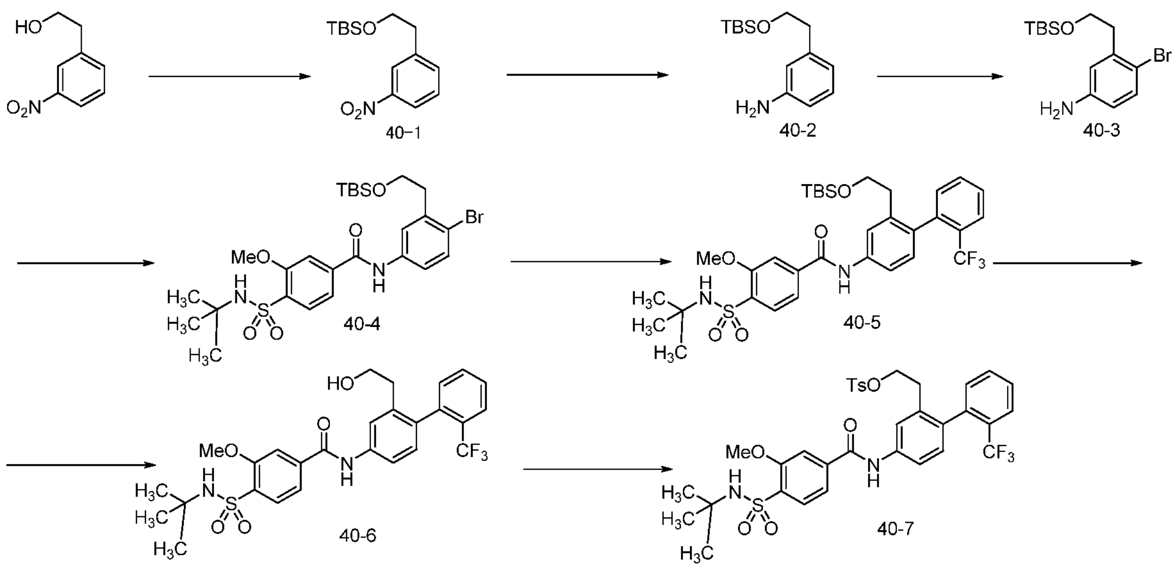

40-1 40-2 40-3 40-4 40-5 40-6 40-7

Triethylamine (3.3 mL) and tert-butyldimethylsilyl chloride (TBSCl) (2.34 g) were added to 3-nitrophenethyl alcohol (2 g) in DMF (25 mL) and the mixture was stirred at room temperature for 17 hours. Water and saturated brine were added to the reaction mixture, which was extracted with ethyl acetate. The organic layer was washed with brine, dried over anhydrous sodium sulfate, filtered and concentrated under reduced pressure to give compound 40-1 (3.49 g). 10% Palladium-carbon (2 g) and ammonium formate (3.7 g) were added to compound 40-1 (3.49 g) in ethanol (15 mL) and the mixture was stirred at 80°C for 2 hours. The reaction mixture was cooled to room tempearture, filtered through Celite and the filtrate was concentrated under reduced pressure. To the resulting residue, were added ethyl acetate, water and a saturated aqueous solution of sodium bicarbonate, the organic layer was separated and the aqueous layer was extracted with ethyl acetate. The combined organic layer was washed with brine, dried over anhydrous sodium sulfate, filtered and concentrated under reduced pressure to give compound 40-2 (2.77 g). Pyridinium bromide perbromide (2.88 g) was added to compound 40-2 (2.77 g) in THF (90 mL) at 0°C, and the reaction mixture was stirred at 0°C for 1 hour. A saturated aquesous solution of sodium biocarbonate and water were added successively to the reaction mixture, which was extracted with ethyl acetate. The organic layer was washed with saturated brine, dried over anhydrous sodium sulfate, filtered and concentrated under reduced pressure to give compound 40-3 (2.41 g). WSC monohydrochloride (700 mg), HOBt (490 mg) and triethylamine (0.5 mL) were added to compound 27-3 of Reference Example 27 (1.04 g) in DMF (6 mL) and compound 40-3 (1.00 g), and the mixture was stirred at room temperature for 15 hours. Water and a saturated aquesous solution of sodium biocarbonate were added successively to the reaction solution, which was extracted with ethyl acetate. The organic layer was washed with a saturated aquesous solution of sodium biocarbonate and saturated brine successively, dried over anhydrous sodium sulfate, filtered and concentrated under reduced pressure. The resulting residue was purified with silica gel column chromatography (hexane/ethyl acetate) to give compound 40-4 (1.24 g). Compound 40-4 (900 mg) was dissolved in a mixed solvent of 1,4-dioxane and water (8 mL/1 mL), 2-trifluoromethylphenylboronic acid (570 mg), cesium carbonarte (1.47 g) and $PdCl_2$(dppf) (110 mg) were added therein and the mixture was stirred at 100°C for 16 hours. The reaction mixture was cooled to room temperature, silicagel for removing a metal (SH silica, FUJI SILYSIA CHEMICAL Ltd) was added and the mixture was stirred at room temperature for 1 hour. The reaction mixture was filtered, the filtrate was concentrated under reduced pressure and the resulting residue was purified with silica gel column chromatography (hexane/ethyl acetate) to give compound 40-5 (934 mg). TBAF (1M solution in THF; 2.75 mL) was added to compound 40-5 (913 mg) in THF (6.5 mL) and the mixture was stirred at room temperature for 18 hours. 1M Aqueous solution of citric acid was added to the reaction mixture, which was extracted with ethyl acetate. The organic layer was washed with saturated brine, dried over anhydrous sodium sulfate, filtered and concentrated under reduced pressure. The resulting residue was purified with silica gel column chromatography (hexane/ethyl acetate) to give compound 40-6 (586 mg). Dichloromethane (4 mL), triethylamine (200 μL), p-toluenesulfonyl

chloride (200 mg) and 4-dimethylaminopyridine (9 mg) were added to compound 40-6 (386 mg) and the mixture was stirred at room temperature for 5 hours. Water and saturated brine were added to the reaction solution, which was extraceted with ethyl acetate. The organic layer was washed with saturated brine, dried over anhydrous sodium sulfate, filtered and concentrated under reduced pressure. The resulting residue was purified with silica gel column chromatography (hexane/ethyl acetate) to give compound 40-7 (386 mg).

Compound 40-3: Analyzing method SC1; $t_R$ 1.47 min, obs MS[M+1] 330.1
Compound 40-6: Analyzing method SC1; $t_R$ 1.16 min, obs MS[M+1] 551.3
Compound 40-7: Analyzing method SC2; $t_R$ 1.28 min, obs MS[M-1] 703.9

Reference Example 41

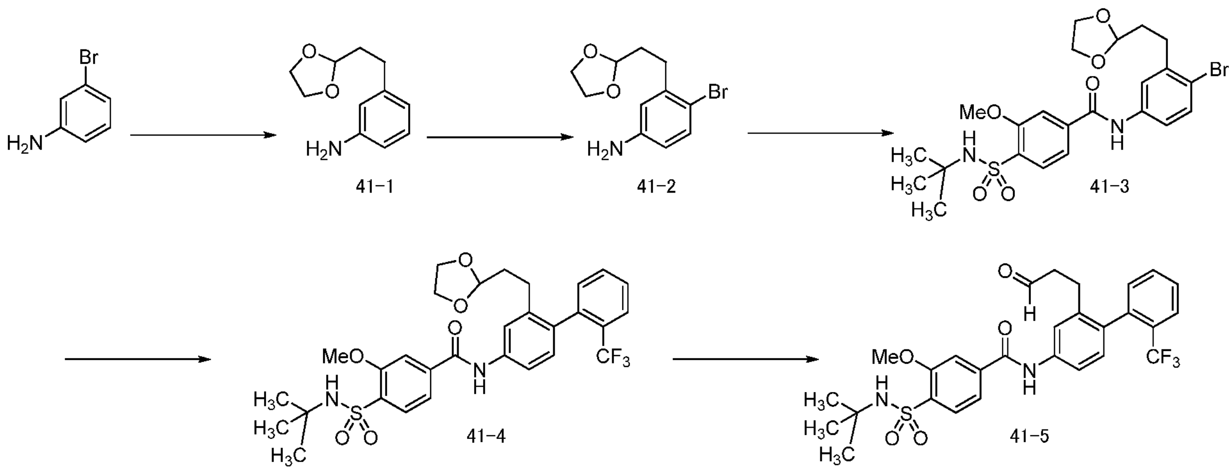

41-1 41-2 41-3 41-4 41-5

Bis(tri-tert-butylphosphine)palladium (85 mg) and (1,3-dioxolan-2-ylethyl)zinc bromide (0.5 M tetrahydrofuran-solution) (9.9 mL) were added to 3-bromoaniline (0.181 mL) in tetrahydrofuran (3.6 mL), and the mixture was stirred at room temperature for 20 minutes. The reaction mixture was poured into ice-water, the mixture was adjusted to basic condition by addition of a saturated aqueous solution of sodium carbonate and extracted with ethyl acetate. The organic layer was washed with saturated brine, dried over anhydrous magnesium sulfate, filtered and concentrated under reduced pressure. The resulting residue was purified with silica gel column chromatography (hexane/ethyl acetate) to give compound 41-1 (237 mg). Compound 41-1 (237 mg) was treated in the same manner as the third step of Reference Example 40 to give compound 41-2 (219 mg). Compound 41-2 (0.75 g), THF (6 mL), N,N-diisopropylethylamine (0.68 mL) and 2-chloro-1,3-dimethylimidazolinium tetrafluoroborate (0.9 g) were added to compound 27-3 of Reference Example 27 (1.2 g) and the mixture was stirred at room temperature for 2 hours. A saturated aqueous solution of sodium carbonate was added to the reaction mixture, which was extracted with ethyl acetate. The organic layer was washed with a saturated aqueous solution of sodium carbonate, water and saturated brine, dried over anhydrous sodium sulfate, filtered and concentrated under reduced pressure. Chloroform was added to the resulting residue, concentrated under reduced pressure and diethyl ether was added. The precipitated solid was filtered to give compound 41-3 (0.94 g). Compound 41-3 (600 mg) was dissolved in a mixed solvent of 1,4-dioxane and water (8 mL/1 mL), 2-trifluoromethylphenylboronic acid (316 mg), cesium carbonarte (1.08 g) and [1,1'-bis(diphenylphosphino)ferrocene]dichloropalladium (II) (81 mg) were added therein and the mixture was stirred at 100°C for 16 hours. The reaction mixture was cooled to room temperature, silica gel for removing a metal (SH silica, FUJI SILYSIA CHEMICAL Ltd) was added and the mixture was stirred at room temperature for 1 hour. The reaction mixture was filtered, the filtrate was concentrated under reduced pressure and the resulting residue was purified with silica gel column chromatography (hexane/ethyl acetate) to give compound 41-4 (326 mg). Acetone (3 mL), water (0.3 mL) and p-toluenesulfonic acid monohydrate (10 mg) were added to compound 41-4 (157 mg) and the mixture was heated to reflux for 5 hours. The reaction mixture was cooled to room temperature, a saturated aqueous solution of sodium bicarbonate was added thereto, and the mixture was extracted with ethyl acetate. The organic layer was washed with saturated brine, dried over anhydrous sodium sulfate, filtered and concentrated under reduced pressure to give compound 41-5 (146 mg).

Compound 41-4: Analyzing method SC2, $t_R$ 1.23 min, obs MS[M+1] 607.2
Compound 41-5: Analyzing method SC2, $t_R$ 1.16 min, obs MS[M+1] 563.2

Reference Example 42

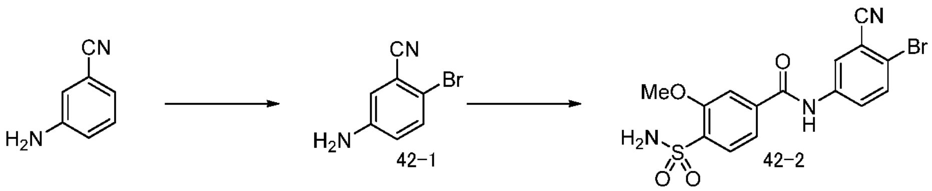

42-1 42-2

3-Aminobenzonitrile (2 g) was treated in the same manner as the third step of Reference Example 40 to give compound 42-1 (1.26 g). Compound 42-1 (630 mg) and compound 22-2 prepared in Reference Example 22 (961 mg) were dissolved in DMF (15 mL), WSC monohydrochloride (797 mg), HOBt (558 mg) and triethylamine (0.58 mL) were added thereto and the mixture was stirred at room temperature for 22 hours. Water and 1M aqueous solution of hydrochloric acid were added and extracted with ethyl acetate. The oragnic layer was washed with 1M aqueous solution of hydrochloric acid, a saturated aqueous solution of sodium bicarbonate, and saturated brine successively, dried over anhydrous sodium sulfate, filtered and concentrated under reduced pressure. The resulting residue was purified with silica gel column chromatography (chloroform/methanol) to give compound 42-2 (175 mg).

Compound 42-1: Analyzing method SC2, $t_R$ 0.67 min, obs MS[M+1] 198.1
Compound 42-2: Analyzing method SC2, $t_R$ 0.81 min, obs MS[M-1] 408.0

Reference Example 43

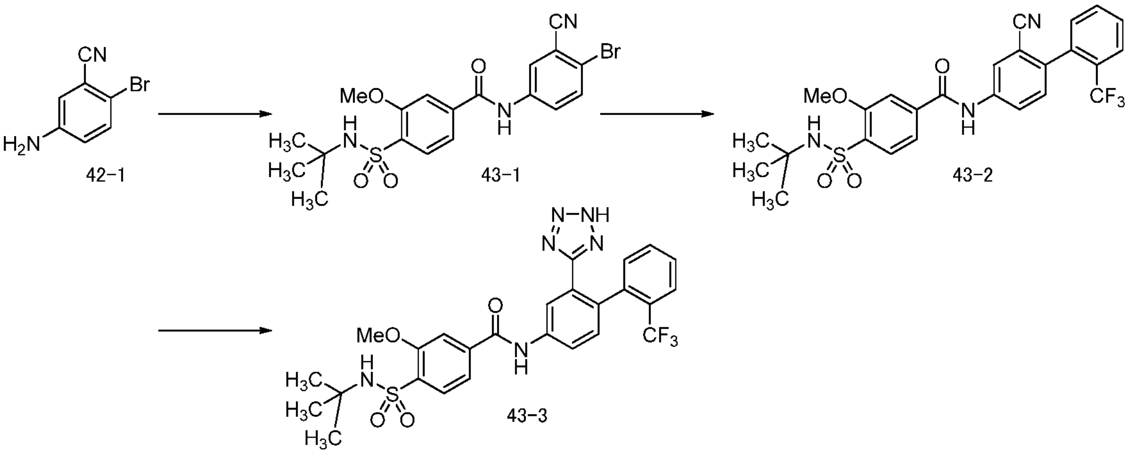

42-1 43-1 43-2 43-3

Compound 42-1 (311 mg) prepared in Reference Example 42 was treated in the same manner as the third step of Reference Example 41 to give compound 43-1 (0.94 g). Compound 43-1 (547 mg) was treated in the same manner as the forth step of Reference Example 41 to give compound 43-2 (233 mg). Sodium azide (61 mg), ammonium chloride (50 mg) and DMF (1 mL) were added to compound 43-2 (50 mg), and the mixture was stirred at 120°C for 22 hours. A saturated aqueous solution of ammonium chloride was added and extracted with ethyl acetate. The oragnic layer was washed with saturated brine, dried over anhydrous sodium sulfate, filtered and concentrated under reduced pressure. The resulting residue was purified with silica gel column chromatography (chloroform/methanol) to give compound 43-3 (35 mg).

Compound 43-1: Analyzing method SC2, $t_R$ 1.10 min, obs MS[M-1] 464.1
Compound 43-2: Analyzing method SC2, $t_R$ 1.16 min, obs MS[M+1] 532.3
Compound 43-3: Analyzing method SC2, $t_R$ 1.09 min, obs MS[M-1] 573.9

Reference Example 44

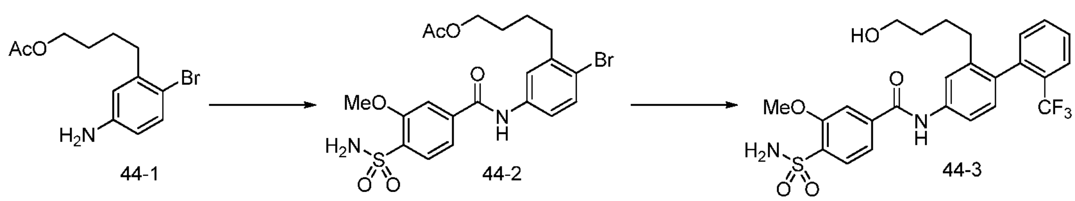

44-1 44-2 44-3

Compound 44-2 was prepared in the same manner as the second step of Reference Example 42 from compound 44-1 which was prepared in the same manner as the first and second steps of Reference Example 41, and compound 22-2 of Reference Example 22. Compound 44-2 (480 mg) was dissolved in a mixed solvent of 1,4-dioxane and water (8 mL/1 mL), 2-trifluoromethylphenylboronic acid (274 mg), 2M aqueous solution of sodium hydroxide (4.8 mL) and [1,1'-bis(diphenylphosphino)ferrocene]dichloropalladium (II) (70 mg) were added therein and the mixture was stirred at 100°C for 4 hours. The reaction mixture was cooled to room temperature, silica gel for removing a metal (SH silica, FUJI SILYSIA CHEMICAL Ltd) was added and the mixture was stirred at room temperature for 1 hour. The reaction mixture was filtered, the filtrate was concentrated under reduced pressure and the resulting residue was purified with silica gel column chromatography (chloroform/methanol) to give compound 44-3 (213 mg).
Compound 44-3: Analyzing method SC2, $t_R$ 1.04 min, obs MS[M-1] 521.3
Reference Example 45
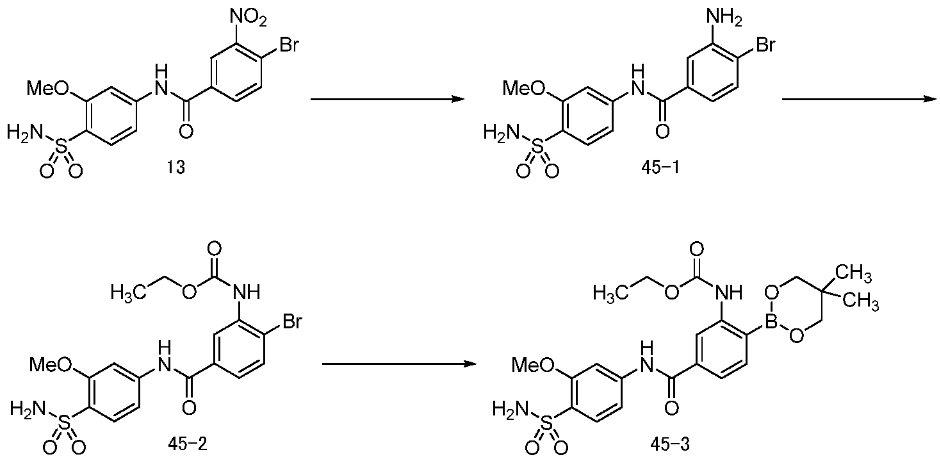

13

45-1

45-2

45-3

Reduced iron (3.2 g), ammonium chloride (3.1 g) and THF/EtOH/$H_2O$ (40 mL/40 mL/ 16 mL) were added to compound I (6.2 g) of Reference Example 13 and the mixture was stirred under nitrogen atmosphere at 65°C for 3 hours. The reaction mixture was cooled to room temperature, DMF (50 mL) was added thereto and the mixture was stirred at room temperature for 20 minutes. The reaction mixture was filtered through Celite, and the filtrate was extracted with ethyl acetate. The organic layer was washed with a saturated aqueous solution of sodium bicarbonate and saturated brine successively, dried over anhydrous sodium sulfate, filtered and concentrated under reduced pressure. Chloroform, diethyl ether and hexane were added to the precipitated solid, which was filtered and dried to give compound 45-1 (7.6 g). Compound 45-1 (5 g) was suspended in a mixed solvent of diiropropylethylamine/THF (8 mL/30 mL), ethyl chloroformate (4 mL) was added thereto and the mixture was stirred at room temperature for 20 hours. Water was added to the reaction mixture and extracted with ehtyl acetate. The organic layer was washed with saturated brine, dried over anhydrous sodium sulfate, filtered and concentrated under reduced pressure. Diethyl ether, ethyl acetate and hexane were added to the resulting solid, which was filtered and dried to give compound 45-2. Compound 45-2 (3 g) was dissolved in 1,4-dioxane/DMSO (50 mL/5 mL), potassium acetate (1.8 g), and [1,1'-bis(diphenylphosphino)ferrocene]-dichloropalladium (II) (1.4 g) were added therein and the mixture was stirred at 80°C for 2 hours. The reaction mixture was cooled to room temperature, water, a saturated aqueous solution of ammonium chloride and saturated brine were added thereto and the product was extracted with ethyl acetate. The organic layer was washed with saturated brine twice, dried over anhydrous sodium sulfate, filtered and concentrated under reduced pressure. Ethyl acetate, chloroform/methanol (8/1) and silica gel for removing a metal (SH silica, FUJI SILYSIA CHEMICAL Ltd) were added to the residue and the mixture was stirred at room temperature for 30 minutes. The reaction mixture was filtered, the filtrate was concentrated under reduced pressure and the residue was crystalized by adding chloroform, diethyl ether and hexane. The precipitated solid was filtered to give compound 45-3 (3.2 g).
Compound 45-1: Analyzing method SC1, $t_R$ 1.06 min, obs MS[M+1] 400.2
Compound 45-2: Analyzing method SC1, $t_R$ 1.22 min, obs MS[M-1] 471.4
Compound 45-3: $^1$H-NMR(DMSO-$d_6$) $\delta$ 0.73(s, 3H), 0.97(s, 3H), 1.25(t, J = 7.1 Hz, 3H), 3.13(s, 2H), 3.74(s, 2H), 3.88 (s, 3H), 4.17(q, J = 7.1 Hz, 1H), 6.99(s, 2H), 7.48-7.71(m, 6H), 8.18(s, 1H), 10.53(s, 1H).
Example 1:
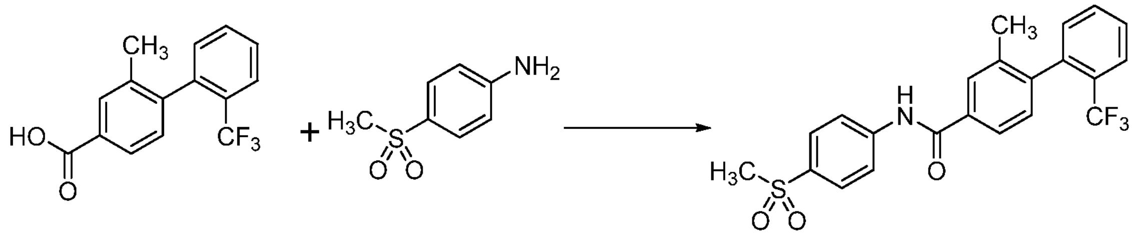

To a suspension of the compound (70 mg) prepared in Reference Example 1 in toluene (1 mL) were added dropwise thionyl chloride (55 μL) and DMF (2 μL), and the mixture was stirred for 1 hour at 95°C. After cooling to room temperature, the reaction mixture was concentrated under reduced pressure. The mixture was azeotropically distilled with toluene, and then tetrahydrofuran (1.5 mL) was added thereto. To the mixture were added 4-(methylsulfonyl)aniline (52 mg) and diisopropylethylamine (66 μL) and the mixture was stirred for 15.5 hours at 80°C to 90°C in a sealed tube. After cooling to room temperature, the reaction mixture was concentrated under reduced pressure to give a residue. The residue was purified with silica gel column chromatography (chloroform/methanol) to give the titled compound (85 mg) as a white solid.
$^1$H-NMR (DMSO-$d_6$) δ2.06 (s, 3H), 3.18 (s, 3H), 7.29 (d, J = 7.9 Hz, 1H), 7.35 (d, J = 7.5 Hz, 1H), 7.66 (t-like, J = 7.5 Hz, 1H), 7.73-7.93 (m, 6H), 8.05-8.08 (m, 2H), 10.72 (s, 1H).

Using the compound prepared in Reference Example 1 and treating it in the same manner as Example 1, the compounds of the following Examples 2-7 were prepared. The compound prepared in Reference Example 3 was also used in Example 7.

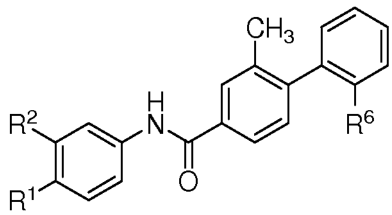

| Example | $R^1$ | $R^2$ | $R^6$ |
|---|---|---|---|
| 2 | $NHSO_2Me$ | H | $CF_3$ |
| 3 | $SO_2NH(C{=}NH)NH_2$ | H | $CF_3$ |
| 4 | $SO_2NH(CO)NH_2$ | H | $CF_3$ |
| 5 | $SO_2NH_2$ | H | $CF_3$ |
| 6 | $SO_2NHAc$ | H | $CF_3$ |
| 7 | $SO_2NH_2$ | OMe | $CF_3$ |

Example 2:
$^1$H-NMR (DMSO-$d_6$) δ2.05 (s, 3H), 2.95 (s, 3H), 7.18-7.21 (m, 2H), 7.26 (d, J = 7.9 Hz, 1H), 7.34 (d, J = 7.7 Hz, 1H), 7.65 (t-like, J = 7.7 Hz, 1H), 7.73-7.80 (m, 4H), 7.86-7.89 (m, 2H), 9.60 (s, 1H), 10.30 (s, 1H).

Example 3:
Analyzing method SA1, $t_R$ 3.65 min, obs MS [M+1] 477.3

Example 4:
$^1$H-NMR (DMSO-$d_6$) δ2.06 (s, 3H), 6.36 (br, 2H), 7.29 (d, J = 8.1 Hz, 1H), 7.35 (d, J = 7.5 Hz, 1H), 7.65 (t-like, J = 7.8 Hz, 1H), 7.76 (t-like, J = 7.3 Hz, 1H), 7.81 (dd, J = 7.9, 1.5 Hz, 4H), 7.86-7.89 (m, 4H), 7.99-8.02 (m, 2H), 10.50 (s, 1H), 10.69 (s, 1H).

Example 5:
$^1$H-NMR (DMSO-$d_6$) δ2.06 (s, 3H), 7.20-7.30 (m, 3H), 7.35 (d, J = 7.2 Hz, 1H), 7.65 (t-like, J = 7.8 Hz, 1H), 7.73-7.82 (m, 4H), 7.87-7.89 (m, 2H), 7.95-7.98 (m, 2H), 10.61 (bs, 1H).

Example 6:
$^1$H-NMR (DMSO-$d_6$) δ1.91 (s, 3H), 2.06 (s, 3H), 7.29 (d, J = 8.1 Hz, 1H), 7.34 (d, J = 7.5 Hz, 1H), 7.65 (t-like, J = 7.5 Hz, 1H), 7.76 (t-like, J = 8.1 Hz, 1H), 7.81 (d, J = 8.1 Hz, 1H), 7.87-7.91 (m, 4H), 8.00-8.03 (m, 2H), 10.71 (s, 1H), 12.01 (s, 1H).

Example 7:
$^1$H-NMR (DMSO-$d_6$) δ2.06 (s, 3H), 3.89 (s, 3H), 6.99 (s, 2H), 7.29 (d, J = 8.1 Hz, 1H), 7.35 (d, J = 7.3 Hz, 1H), 7.53 (dd, J= 8,4, 1.8 Hz, 1H), 7.63-7.89 (m, 7H), 10.56 (s, 1H).
Example 8:
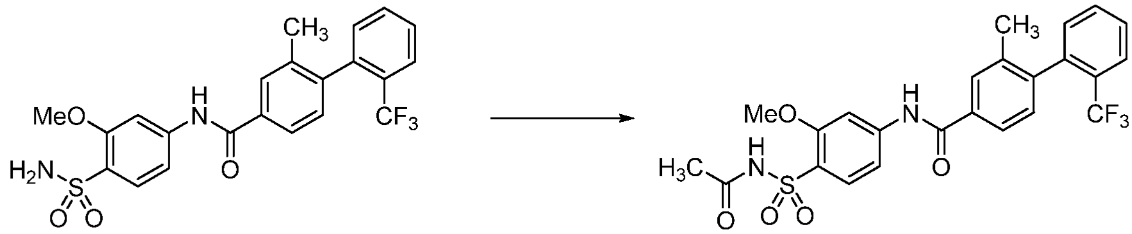

To a solution of the compound (23 mg) prepared in Example 7 in acetonitrile (1 mL) were added acetic anhydride (21 μL) and concentrated sulfuric acid (0.5 μL), and the mixture was stirred for 22 hours at 60°C. After cooling to room temperature, water was added thereto and the mixture was extracted with a mixed solvent of ethyl acetate-tetrahydrofuran (2: 1). The organic layer was dried over anhydrous magnesium sulfate, filtered and concentrated under reduced pressure. The resulting residue was purified with silica gel column chromatography (chloroform/ethyl acetate) to give the titled compound (19 mg) as a white solid.
Analyzing method SA1, $t_R$ 3.90 min, obs MS [M+1] 507.3
Example 9:
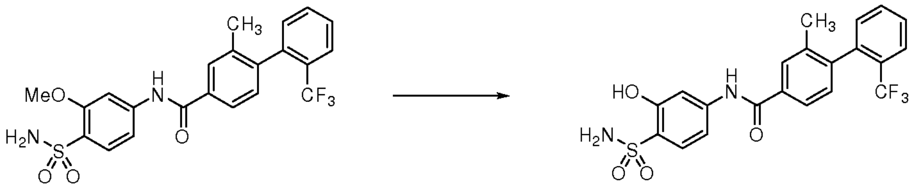

To a suspension of the compound (23 mg) prepared in Example 7 in dichloromethane (2 mL) was added 1M boron tribromide in dichloromethane (0.15 mL), and then the mixture was warmed to room temperature and stirred for 24 hours. After finishing the reaction, methanol was added to the reaction mixture at 0°C, and then the mixture was concentrated under reduced pressure. The resulting residue was purified with silica gel column chromatography (chloroform/methanol) to give the titled compound as a white solid (3 mg).
Analyzing method SA1, $t_R$ 3.76 min, obs MS [M+1] 451.3
Example 10:
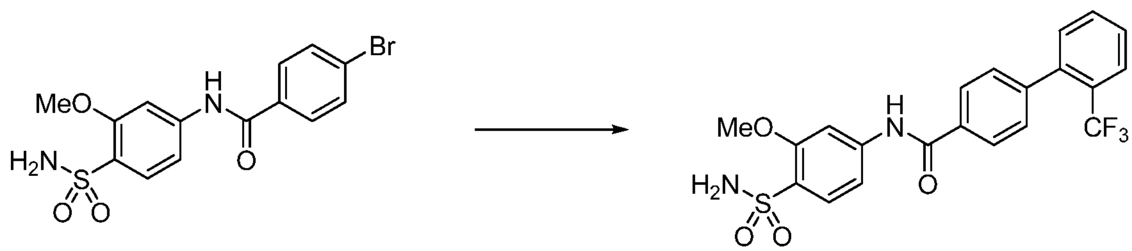

A mixture of the compound of Reference Example 4 (74 mg), tris(dibenzylideneacetone)-dipalladium (0) (18 mg), 2-dicyclohexylphosphino-2',6-dimethoxybiphenyl (31 mg), potassium phosphate (162 mg) and 2-(trifluoromethyl)phenylboronic acid (73 mg) in toluene (2.0 mL) was stirred for 22 hours at 100°C. After cooling to room temperature, the reaction mixture was diluted with a mixed solvent of ethyl acetate-methanol, and filtered. The filtrate was concentrated under reduced pressure. The resulting residue was purified with silica gel column chromatography (chloroform/ethyl acetate) to give the titled compound (51 mg) as a white solid.
$^1$H-NMR (DMSO-$d_6$) δ3.89 (s, 3H), 6.99 (s, 2H), 7.44-7.54 (m, 4H), 7.63-7.79 (m, 4H), 7.87 (d, J = 7.7 Hz, 1H), 8.01-8.04

(m, 2H), 10.60 (s, 1H).

Example 11:

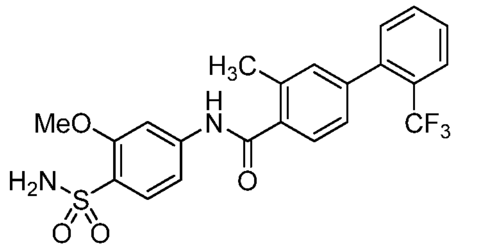

Using 4-bromo-2-methylbenzoic acid and treating it in the same manner as Example 10, the titled compound was prepared.

Analyzing method SA3, $t_R$ 4.40 min, obs MS [M+1] 465.0

Example 12:

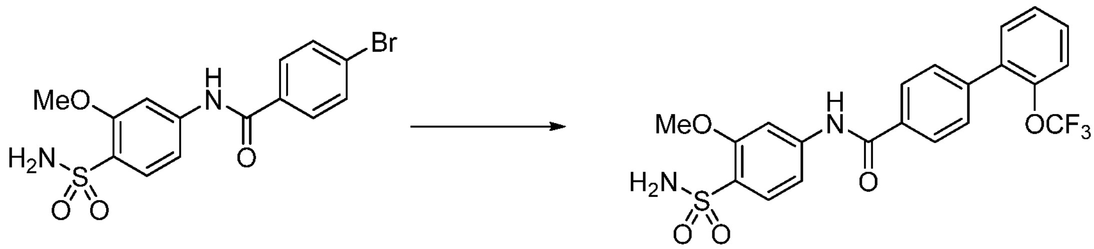

A mixture of the compound of Reference Example 4 (50 mg), [1,1'-bis(diphenylphosphino)-ferrocene]dichloropalladium (II) (9.5 mg), potassium carbonate (72 mg) and 2-(trifluoromethoxy)-phenylboronic acid (54 mg) was stirred for 24 hours at 80°C in DMF (1.3 mL). After cooling to room temperature, water was added thereto and the mixture was extracted with ethyl acetate. After washing the organic layer with saturated brine, it was dried over anhydrous sodium sulfate, filtered and concentrated under reduced pressure. The resulting residue was purified with preparative thin layer chromatography (chloroform/methanol = 30/1) to give the titled compound (19 mg) as a white solid.

Analyzing method SA2, $t_R$ 3.67 min, obs MS [M+1] 467.3

Using the compound prepared in Reference Example 4 and treating it in the same manner as Example 12, the compounds of the following Examples 13-16 were prepared.

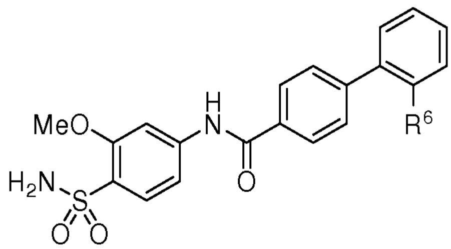

| Example | $R^6$ | obs MS [M+1] | $t_R$ (min) | Analytical Condition |
|---|---|---|---|---|
| 13 | OMe | 413.2 | 3.40 | SA2 |
| 14 | $CH_3$ | 397.2 | 3.55 | SA2 |
| 15 | F | 401.4 | 3.51 | SA2 |

Example 16:

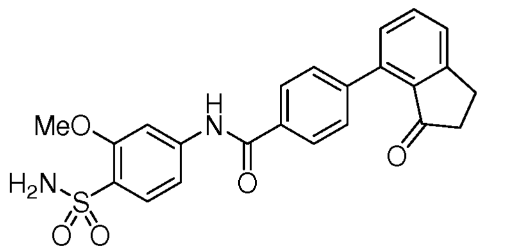

Analyzing method SA2, $t_R$ 3.19 min, obs MS [M+1] 4367.0

Example 17:

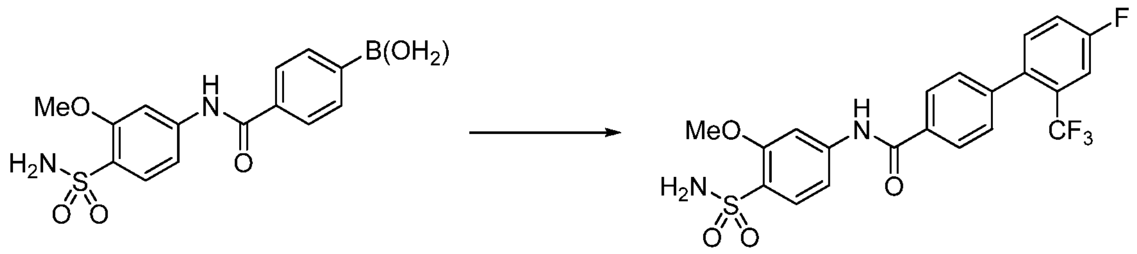

A mixture of the compound of Reference Example 5 (50 mg), [1,1'-bis(diphenylphosphino)-ferrocene]dichloropalladium (II) (11 mg), potassium carbonate (79 mg) and 2-(trifluoromethoxy)-phenylboronic acid 2-bromo-5-fluorobenzotrifluoride (70 mg) was stirred for 16 hours at 80°C in DMF (1.4 mL). After cooling to room temperature, the reaction mixture was filtered and the filter cake was washed with ethyl acetate. The combined filtrate was concentrated under reduced pressure. The resulting residue was purified with preparative reversed-phase HPLC to give the titled compound (15 mg) as a white solid.

Analyzing method SA2, $t_R$ 3.65 min, obs MS [M+1] 469.2

Example 18:

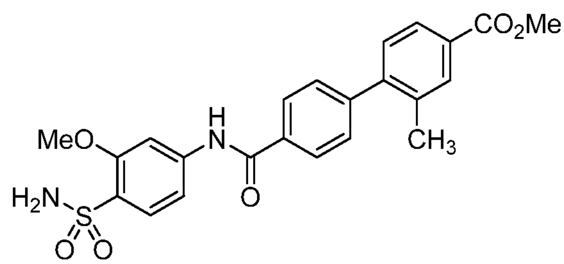

Using the compound of Reference Example 5 and treating it in the same manner as Example 17, the titled compound (18 mg) was prepared.

Analyzing method SA2, $t_R$ 3.51 min, obs MS [M+1] 455.2

Example 19:

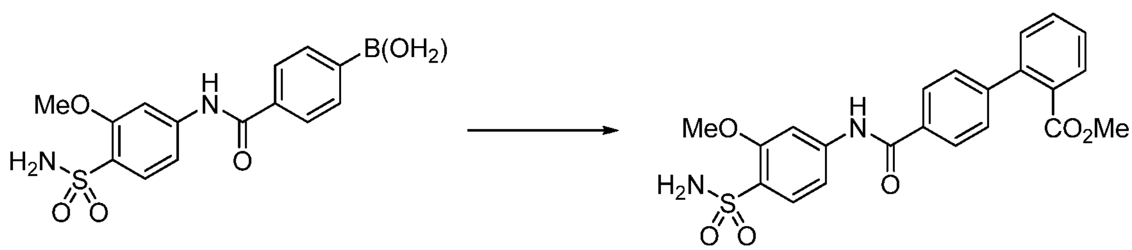

A mixture of the compound of Reference Example 5 (77 mg), dichlorobis(tri-o-tolylphosphine)palladium (II) (11 mg), potassium carbonate (79 mg) and methyl 2-bromo-benzoate (95 mg) was stirred for 8 hours at 80°C in a mixed solvent of DMF (2 mL)-water (0.2 mL). After cooling to room temperature, the reaction mixture was filtered and the filter cake was washed with ethyl acetate. The combined filtrate was concentrated under reduced pressure. The resulting residue was purified with preparative reversed-phase HPLC to give the titled compound (26 mg) as a white solid.
Analyzing method SB 1, $t_R$ 3.70 min, obs MS [M+1] 441.5

Using the compound of Reference Example 5 and treating it in the same manner as Example 19, the compounds of the following Examples 20-22 were prepared.

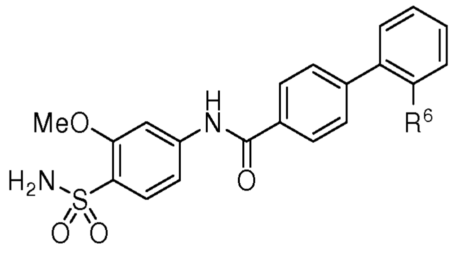

| Example | $R^6$ | obs MS [M+1] | $t_R$ (min) | Analytical Condition |
|---|---|---|---|---|
| 20 | $CO_2Et$ | 455.5 | 3.85 | SB1 |
| 21 | CN | 408.3 | 3.61 | SB1 |
| 22 | 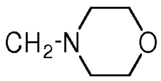 | 482.5 | 2.70 | SB1 |

Example 23:

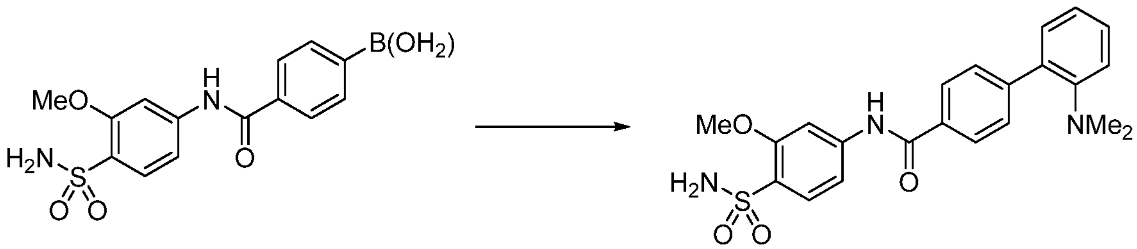

A mixture of the compound of Reference Example 5 (30 mg), [1,1'-bis(diphenylphosphino)ferrocene]dichloropalladium (II) (12 mg), potassium carbonate (47 mg) and 2-bromo-N,N-dimethylaniline (34 mg) was stirred for 5 hours at 80°C in a mixed solvent of DMF-water (10:1) (0.86 mL). After cooling to room temperature, PL-BnSH MP-Resin (Polymer supported benzylmercaptan, Polymer Laboratories) was added thereto, and the mixture was shaken at room temperature. The insoluble material and resin were separated by filtration, and the filtrate was concentrated under reduced pressure. The resulting residue was purified with preparative reversed-phase HPLC to give the titled compound (5.2 mg) as a white solid.
Analyzing method SB 1, $t_R$ 2.84 min, obs MS [M+1] 426.5

Using the compound of Reference Example 5 and treating it in the same manner as Example 23, the compounds of the following Examples 24-47 were prepared.

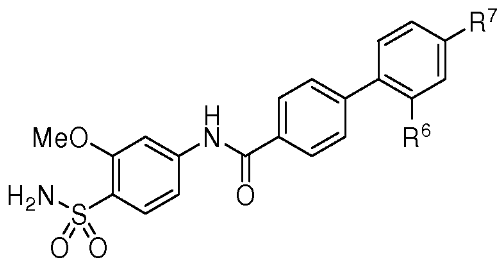

| Example | $R^6$ | $R^7$ | obs MS [M+1] | $t_R$ (min) | Analytical Condition |
|---|---|---|---|---|---|
| 24 | $CO_2Me$ | OMe | 471.4 | 3.76 | SB1 |
| 25 | $CF_3$ | Me | 465.4 | 4.34 | SB1 |
| 26 | $CF_3$ | OH | 467.3 | 3.65 | SB1 |

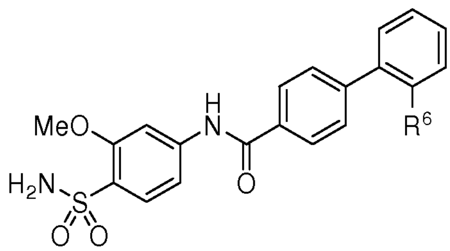

| Example | $R^6$ | obs MS [M+1] | $t_R$ (min) | Analytical Condition |
|---|---|---|---|---|
| 27 | $CH_2CN$ | 422.5 | 3.67 | SB1 |
| 28 | $CH_2OMe$ | 427.3 | 3.82 | SB1 |
| 29 | $CH_2CO_2Me$ | 455.4 | 3.77 | SB1 |
| 30 | $CHF_2$ | 433.2 | 3.98 | SB1 |
| 31 | $CO_2t$-Bu | 483.6 | 4.18 | SB1 |
| 32 | 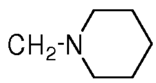 | 480.1 | 2.90 | SB1 |
| 33 | 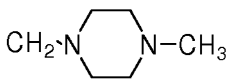 | 495.5 | 2.78 | SB1 |
| 34 | 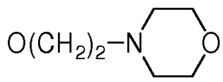 | 512.5 | 2.97 | SB1 |
| 35 | 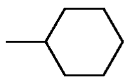 | 465.5 | 4.73 | SB1 |
| 36 | i-Pr | 425.3 | 4.34 | SB1 |
| 37 | $CH_2NMe_2$ | 440.5 | 2.80 | SB1 |
| 38 | $OCHF_2$ | 449.3 | 3.93 | SB1 |
| 39 | Oi-Pr | 441.5 | 4.17 | SB1 |
| 40 | $OCF_2CF_2H$ | 499.2 | 4.15 | SB1 |
| 41 | 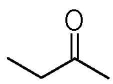 | 439.4 | 3.63 | SB1 |
| 42 | 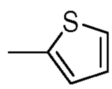 | 465.3 | 4.30 | SB1 |

(continued)

| Example | $R^6$ | obs MS [M+1] | $t_R$ (min) | Analytical Condition |
|---|---|---|---|---|
| 43 | 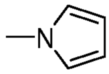 | 448.4 | 4.10 | SB1 |

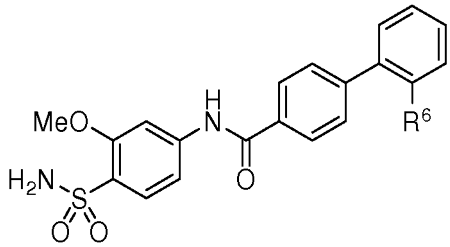

| Example | $R^6$ | obs MS [M+1] | $t_R$ (min) | Analytical Condition |
|---|---|---|---|---|
| 44 | 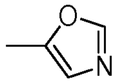 | 450.2 | 3.69 | SB1 |
| 45 | 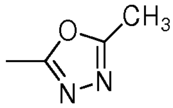 | 465.4 | 3.67 | SB1 |
| 46 | 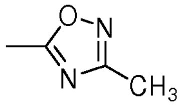 | 465.5 | 3.71 | SB1 |
| 47 | 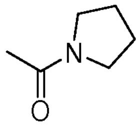 | 480.4 | 3.36 | SB1 |

Example 48:

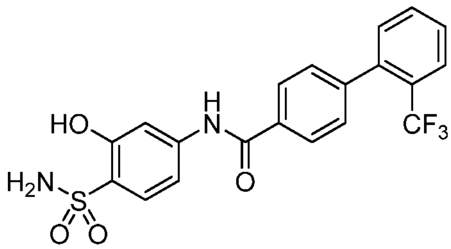

To a suspension of the compound (324 mg) prepared in Reference Example 2 in dichloromethane (4 mL) was added oxalyl chloride (109 μL) at 0°C-5°C, and DMF (1 μL) was added dropwise thereto. The reaction mixture was warmed to room temperature and stirred for 2 hours. The reaction mixture was concentrated under reduced pressure, and then after azeotropically distillation with toluene, pyridine (4 mL) was added. The compound (350 mg) prepared in Reference Example 6 was added thereto and the reaction mixture was stirred for 2.5 hours at room temperature. Water was added to the reaction mixture, which was extracted with ethyl acetate. After washing the organic layer with saturated brine, it was dried over anhydrous sodium sulfate, filtered and concentrated under reduced pressure. The resulting residue was purified with silica gel column chromatography (hexane/ethyl acetate). Desilylation was progressed at the post-processing stage and at column purification step to give the titled compound (194 mg) as a white solid.

Analyzing method SA2, $t_R$ 3.53 min, obs MS [M+1] 437.3

Example 49:

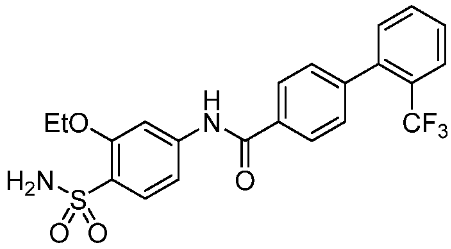

Using the compound (50 mg) prepared in Reference Example 2 and the compound (55 mg) prepared in Reference Example 7, acyl chloride was prepared in the same manner as Example 48, and the condensation reaction was performed in tetrahydrofuran in the same manner as Example 1, and then the resulting compound was purified with preparative reversed-phase HPLC to give the titled compound (32 mg) as a white solid.

Analyzing method SA2, $t_R$ 3.74 min, obs MS [M+1] 465.3

Example 50:

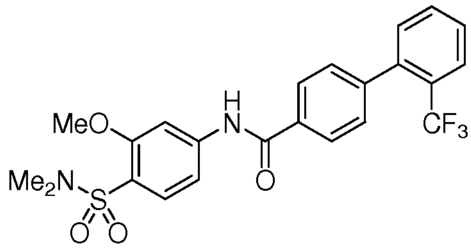

To a solution of the compound of Example 10 (50 mg) in DMF (0.5 mL) were added potassium carbonate (61 mg) and methyl iodide (15 μL), and then the reaction mixture was stirred for 4 hours at room temperature. Water was added to the reaction mixture, which was extracted with ethyl acetate. After washing the organic layer with saturated brine, it was dried over anhydrous sodium sulfate, filtered, and concentrated under reduced pressure. The resulting residue was purified with preparative reversed-phase HPLC to give the titled compound (15 mg) as a white solid.

Analyzing method SA2, $t_R$ 3.90 min, obs MS [M+1] 479.3

Using corresponding various primary amines instead of ammonia and treatingt them in the same manner as Reference Example 3 and Example 49, the compounds of the following Examples 51-55 were prepared.

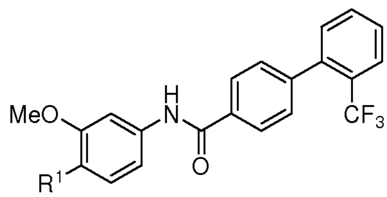

| Example | $R^1$ | obs MS [M+1] | $t_R$ (min) | Analytical Condition | Example No. | $R^1$ |
|---|---|---|---|---|---|---|
| 51 | $SO_2NHMe$ | 465.4 | 3.81 | SB2 | 54 | $SO_2NH(CH_2)_2OMe$ |
| 52 | $SO_2NHi\text{-}Pr$ | 493.4 | 4.20 | SB2 | 55 | $SO_2NH(CH_2)_3$– 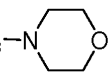 |
| 53 | $SO_2NH(CH_2)_3NMe_2$ | 536.2 | 3.01 | SA2 | | |

Example 54:

$^1$H-NMR (DMSO-$d_6$) δ2.91 (q, J = 6.0 Hz, 2H), 3.14 (s, 3H), 3.27 (t, J = 6.0 Hz, 2H), 3.89 (s, 3H), 7.08 (t, J = 6.0 Hz, 1H), 7.45 (d, J = 7.8 Hz, 1H), 7.50 (d, J = 8.0 Hz, 2H), 7.54 (dd, J = 8.8, 1.7 Hz, 1H), 7.65 (d, J = 7.6 Hz, 1H), 7.69 (d, J = 8.8 Hz, 1H), 7.75 (d, J = 8.5 Hz, 1H), 7.77 (d, J = 5.4 Hz, 1H), 7.87 (d, J = 7.6 Hz, 1H), 8.02 (d, J = 8.3 Hz, 2H), 10.64 (s, 1H).

Example 55:

$^1$H-NMR (DMSO-$d_6$) δ1.75-1.82 (m, 2H), 2.80 (q, J = 6.5 Hz, 2H), 3.01-3.10 (m, 4H), 3.37 (d, J = 12.0 Hz, 2H), 3.60 (t, J = 11.7 Hz, 2H), 3.91 (s, 3H), 3.96 (d, J = 10.7 Hz, 2H), 7.33 (t, J = 6.0 Hz, 1H), 7.45 (d, J = 7.6 Hz, 1H), 7.51 (d, J = 8.1 Hz, 2H), 7.55 (dd, J = 8.7, 1.6 Hz, 1H), 7.66 (t-like, J = 7.7 Hz, 1H), 7.71 (d, J = 8.8 Hz, 1H), 7.76 (d, J = 7.3 Hz, 1H), 7.79 (d, J = 1.7 Hz, 1H), 7.88 (d, J = 7.8 Hz, 1H), 8.02 (d, J = 8.3 Hz, 2H), 10.67 (s, 1H).

Example 56:

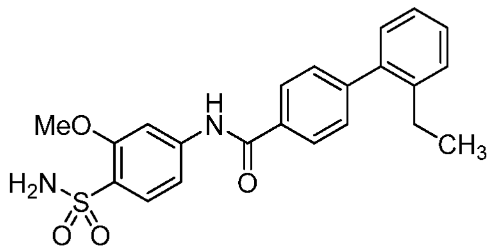

To a solution of the compound of Reference Example 4 (39 mg) in tetrahydrofuran (3 mL) were sequentially added bis(tri-tert-butylphosphine)palladium(O) (10 mg) and a 0.5 M solution of 2-ethylphenylzinc iodide in tetrahydrofuran (1.2 mL), and the mixture was stirred for 20 hours at room temperature. To the reaction mixture was added triufluoroacetic acid (75 μL), which was concentrated under reduced pressure. The resulting residue was purified with preparative reversed-phase HPLC to give the titled compound (8.6 mg) as a white solid.

Analyzing method SB 1, $t_R$ 4.30 min, obs MS [M+1] 411.5

Using the corresponding various organic zinc reagents instead of 2-ethylphenylzinc iodide and treating them in the same manner as Example 56, the compounds of the following Examples 57-58 were prepared.

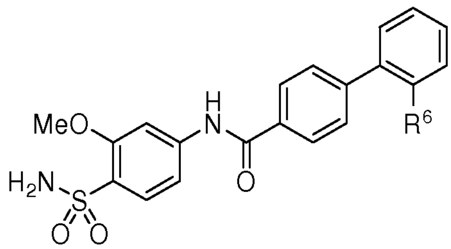

| Example | $R^6$ | obs MS [M+1] | $t_R$ (min) | Analytical Condition |
|---|---|---|---|---|
| 57 | SMe | 429.4 | 4.09 | SB1 |
| 58 | Cl | 417.4 | 4.11 | SB1 |

Example 59:

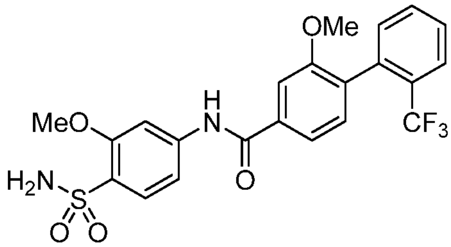

Using the compound (60 mg) prepared in Reference Example 3 and the compound (80 mg) prepared in Reference Example 8, and treating them in the same manner as Example 49, the titled compound (40 mg) was obtained as a white solid.
Analyzing method SA2, $t_R$ 3.55 min, obs MS [M+1] 481.3

Example 60:

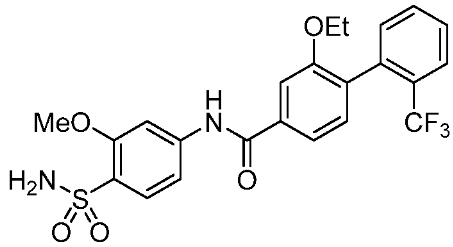

Using iodoethane instead of iodomethane and treating it in the same manner as Reference Example 8 and Example 59, the titled compound was prepared.
Analyzing method SA2, $t_R$ 3.67 min, obs MS [M+1] 495.3

Example 61:

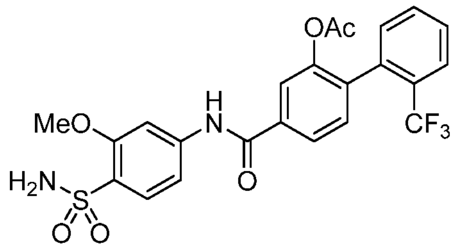

Using the compound (283 mg) prepared in Reference Example 3 and the compound (413 mg) prepared in Reference Example 9, and treating them in the same manner as Example 1, the titled compound (366 mg) was prepared as a white solid.
Analyzing method SA2, $t_R$ 3.44 min, obs MS [M+1] 509.3

Example 62:

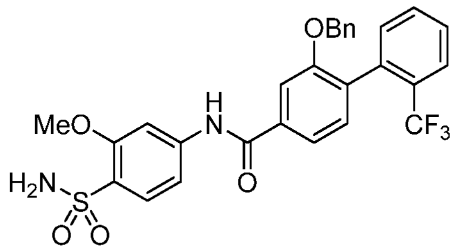

Using the compound (734 mg) prepared in Reference Example 3 and the compound (1.2 g) prepared in Reference Example 10, and treating them in the same manner as Example 1, the titled compound (1.4 g) was prepared as a white solid.
$^1$H-NMR (DMSO-$d_6$) δ3.90 (s, 3H), 5.17 (AB q, $J_{AB}$ = 26.7 Hz, 2H), 7.00 (s, 2H), 7.17-7.35 (m, 6H), 7.40 (d, J = 7.5 Hz, 1H), 7.49 (dd, J = 8.7, 1.7 Hz, 2H), 7.59-7.74 (m, 6H), 7.83 (d, J = 7.7 Hz, 1H), 10.57 (s, 1H).

Example 63:

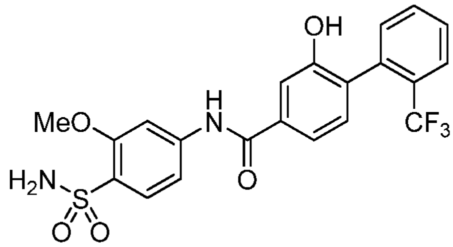

The compound (366 mg) of Example 61 was dissolved in a mixed solvent of tetrahydrofuran-methanol (1:1) (8 mL), a 1N aqueous solution of sodium hydroxide (1.1 mL) was added dropwise thereto, and then the mixture was stirred for 3 hours at room temperature. The reaction mixture was acidified by adding 2N aqueous solution of hydrochloric acid, the solvent was concentrated under reduced pressure, and then the residue was extracted with ethyl acetate and 1N aqueous solution of hydrochloric acid. After washing the organic layer with saturated brine, it was dried over anhydrous sodium sulfate, filtered, and concentrated under reduced pressure to give the titled compound (331 mg) as a white solid. Alternatively, using the compound of Example 62 (1.4 g), the titled compound (1.2 g) was prepared as a white solid according to the same hydrogenation reaction as that used in the second step in Reference Example 3.
Analyzing method SA2, $t_R$ 3.30 min, obs MS [M+1] 467.3

Example 64:

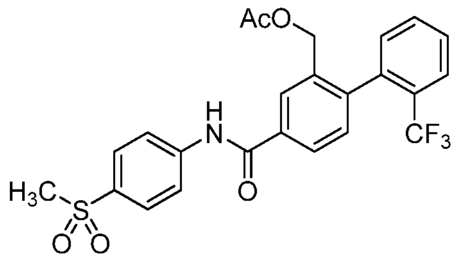

To a suspension of the compound of Reference Example 11 (342 mg) in toluene (5 mL) were added dropwise thionyl chloride (221 μL) and DMF (7 μL), and the mixture was stirred for 2 hours at 85°C. After cooling to room temperature, the reaction mixture was concentrated under reduced pressure, and azeotropically distilled with toluene, and then tetrahydrofuran (5 mL) was added thereto. To the solution were added 4-(methylsulfonyl)aniline (231 mg) and diisopropylethylamine (528 μL), the mixture was stirred for 16 hours at room temperature. 1N aqueous solution of hydrochloric acid was added thereto, and the mixture was extracted with ethyl acetate. The organic layer was washed with saturated brine, and then dried over anhydrous sodium sulfate, filtered, and concentrated under reduced pressure. The resulting residue was purified with silica gel column chromatography (hexane/ethyl acetate) to give the titled compound (465 mg) as a white solid.
$^1$H-NMR (DMSO-$d_6$) δ1.93 (s, 3H), 3.19 (s, 3H), 4.82 (AB q, $J_{AB}$ = 29.5 Hz, 2H), 7.40 (d, J = 8.0 Hz, 2H), 7.67 (t-like, J = 7.8 Hz, 1H), 7.76 (t-like, J = 7.0 Hz, 1H), 7.88-7.93 (m, 3H), 7.99-8.00 (m, 1H), 8.05-8.07 (m, 3H), 10.82 (s, 1H).

Example 65:

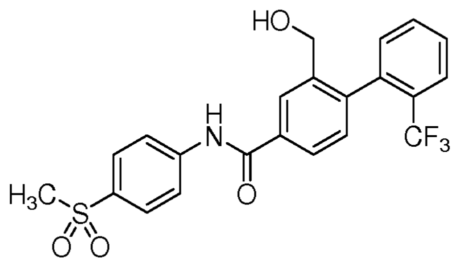

Using the compound (465 mg) prepared in Reference Example 64 and treating it in the same manner as Example 63, the titled compound (402 mg) was obtained as a white solid.
Analyzing method SA2, $t_R$ 3.34 min, obs MS [M+1] 450.2

$^1$H-NMR (DMSO-$d_6$) δ3.18 (s, 3H), 4.13-4.25 (m, 2H), 5.31 (t, J = 5.4 Hz, 1H), 7.28 (d, J = 7.8 Hz, 1H), 7.36 (d, J = 7.3 Hz, 1H), 7.66 (t-like, J = 7.7 Hz, 1H), 7.74 (t-like, J = 7.4 Hz, 1H), 7.86-7.92 (m, 4H), 8.06-8.08 (m, 2H), 8.17 (s, 1H), 10.79 (s, 1H).

Example 66-Example 69:

Using corresponding various anilines instead of 4-(methylsulfonyl)anilin and treating it in the same manner as Example 64-65, the compounds of the following Examples 66-69 were prepared.

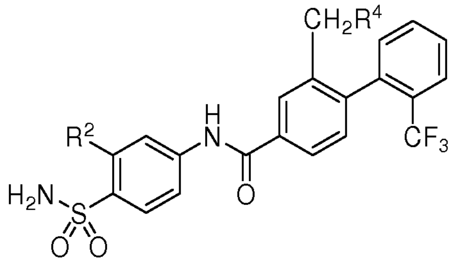

| Example | $R^2$ | $CH_2R^4$ |
|---|---|---|
| 66 | H | $CH_2OAc$ |
| 67 | H | $CH_2OH$ |
| 68 | OMe | $CH_2OAc$ |
| 69 | OMe | $CH_2OH$ |

Example 66:
$^1$H-NMR (DMSO-$d_6$) δ1.93 (s, 3H), 4.82 (AB q, $J_{AB}$ = 30.4 Hz, 2H), 7.29 (s, 2H), 7.39 (d, J = 3.9 Hz, 1H), 7.41 (d, J = 3.2 Hz, 1H), 7.67 (t-like, J = 7.7 Hz, 1H), 7.75 (t-like, J = 7.3 Hz, 1H), 7. 81 (d, J = 8.8 Hz, 2H), 7. 89 (d, J = 8.0 Hz, 1H), 7. 95-8.00 (m, 3H), 8.07 (d, J = 1.7 Hz, 1H), 10.71 (s, 1H).

Example 67:
$^1$H-NMR (DMSO-$d_6$) δ4.12-4.26 (m, 2H), 5.30 (t, J = 5.3 Hz, 1H), 7.26-7.28 (m, 2H), 7.36 (d, J = 7.3 Hz, 1H), 7.63-7.89 (m, 6H), 7.98 (d, J = 8.8 Hz, 2H), 8.16 (s, 1H), 10.69 (s, 1H).

Example 68:
$^1$H-NMR (DMSO-$d_6$) δ1.93 (s, 3H), 3.90 (s, 3H), 4.82 (AB q, $J_{AB}$ = 25.9 Hz, 2H), 7.01 (s, 2H), 7.38-7.41 (m, 2H), 7.50-7.53 (m, 1H), 7.65-7.78 (m, 4H), 7.89 (d, J = 7.5 Hz, 1H), 7.98-8.00 (m, 1H), 8.06 (s, 1H), 10.66 (s, 1H).

Example 69:
$^1$H-NMR (DMSO-$d_6$) δ3.89 (s, 3H), 4.12-4.26 (m, 2H), 5.30 (t, J = 5.2 Hz, 1H), 7.00 (s, 2H), 7.28 (d, J = 7.9 Hz, 1H), 7.37 (d, J = 7.5 Hz, 1H), 7.55 (dd, J = 8.6, 1.7 Hz, 1H), 7.63-7.77 (m, 4H), 7.87 (d, J = 7.9 Hz, 2H), 8.16 (s, 1H), 10.64 (s, 1H).

Example 70:

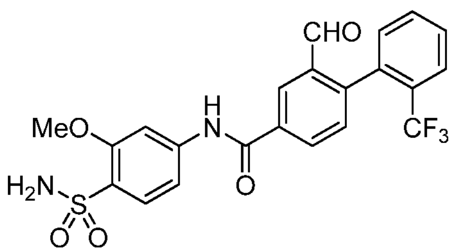

To a mixture of the compound (3.12 g) of Example 69 and diisopropylethylamine (3.39 mL) in a mixed solvent of dichloromethane (28 mL)-dimethylsulfoxide (14 mL) was added sulfur trioxide-pyridine complex (3.10g) under ice-cooling, and the mixture was stirred for 35 minutes at 0°C-5°C. Iced water was added to the reaction mixture, which was extracted with ethyl acetate. After washing the organic layer three times with saturated brine, it was dried over anhydrous sodium

sulfate, filtered, and then concentrated under reduced pressure. The resulting residue was purified with silica gel column chromatography (hexane/ethyl acetate) to give the titled compound (2.26 g).
$^1$H-NMR ($CDCl_3$) δ4.00 (s, 3H), 5.16 (s, 2H), 7.07 (dd, J = 8.5, 1.9 Hz, 1H), 7.32-7.34 (md, J = 6.4 Hz, 1H), 7.52 (d, J = 8.1 Hz, 1H), 7.61-7.65 (m, 2H), 7.79-7.83 (m, 3H), 8.24 (dd, J = 8.1, 1.8 Hz, 1H), 8.50 (m, 2H), 9.69 (s, 1H).

Example 71:

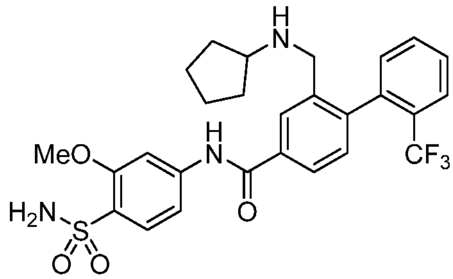

To a solution of the compound of Example 70 (30 mg) in tetrahydrofuran (1 mL) were added acetic acid (10 μL), cyclopentylamine (9.3 μL) and sodium triacetoxyborohydride (27 mg), and then the mixture was stirred for 23 hours at room temperature. An aqueous solution of sodium bicarbonate was added to the reaction mixture, which was extracted with chloroform. The organic layer was washed with water, and then concentrated under reduced pressure. The resulting residue was purified with preparative reversed-phase HPLC and further demineralization by using PL-$HCO_3$ MP SPE Device (a column filled with Polymer supported Hydrogen carbonate, Polymer Laboratories) to give the titled compound (14 mg) as a white solid.
Analyzing method SB 1, $t_R$ 3.29 min, obs MS [M+1] 548.7

Examples 72-80:

Using the corresponding various amines instead of cyclopentylamine, and treating them in the same manner as Example 71, the compounds of the following Examples 72-80 were prepared.

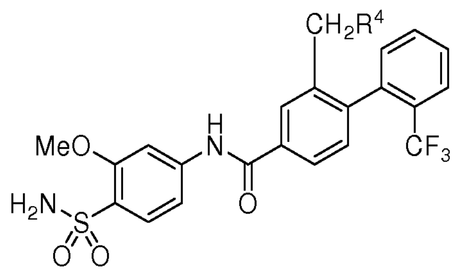

| Example | $CH_2R^4$ | obs MS [M+1] | $t_R$ (min) | Analytical Condition |
|---|---|---|---|---|
| 72 | $CH_2NMe_2$ | 508.1 | 2.90 | SA1 |
| 73 | $CH_2NEt_2$ | 536.5 | 3.17 | SB1 |
| 74 | $CH_2N(CH_2CH_2OH)_2$ | 568.4 | 2.97 | SB1 |
| 75 | 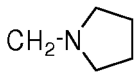 | 534.6 | 3.15 | SB1 |
| 76 | 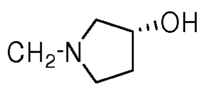 | 550.4 | 3.04 | SB1 |
| 77 | 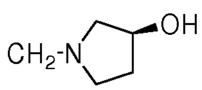 | 550.2 | 3.06 | SB1 |
| 78 | 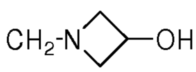 | 536.4 | 3.03 | SB1 |

(continued)

| Example | $CH_2R^4$ | obs MS [M+1] | $t_R$ (min) | Analytical Condition |
|---|---|---|---|---|
| 79 | 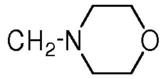 | 550.5 | 3.09 | SB1 |
| 80 | 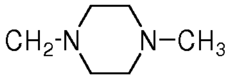 | 563.5 | 3.18 | SB1 |

Example 81:

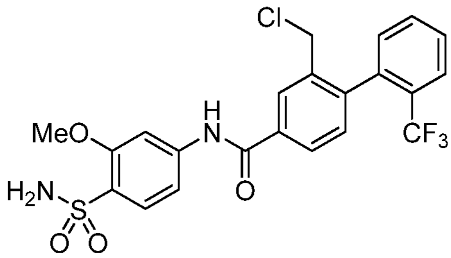

To a suspension of the compound (45 mg) obtained in Example 69 in dichloromethane (3 mL) was added thionyl chloride (95 μL) at room temperature, and the mixture was heated for 3.5 hours at 50°C. The reaction mixture was concentrated under reduced pressure at 50°C, and the resulting residue was purified with silica gel column chromatography (hexane/ ethyl acetate) to give the titled compound (16 mg).

$^1$H-NMR (DMSO-$d_6$) δ3.90 (s, 3H), 4.32 (d, J = 12 Hz, 1H),4.65 (d, J = 12 Hz, 1H), 7.00 (s, 2H), 7.38-8.18 (m, 10H), 10.6 (s, 1H).

Example 82:

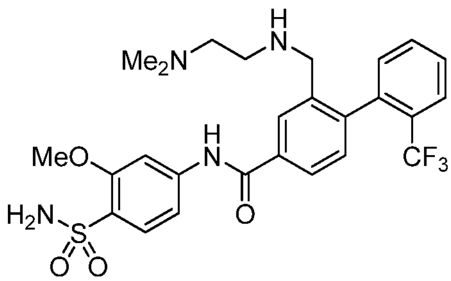

To a suspention of the compound of Example 81 (30 mg) in ethanol (1 mL) were added potassium carbonate (42 mg) and N,N-dimethylethylenediamine (66 μL), and then the mixture was heated for 5 minutes at 150°C in a sealed tube by using a microwave synthesizer (Initiator 60 EXP (400W), Biotage). After cooling, water was added thereto, and the mixture was extracted with chloroform. The organic layer was washed with water , and then cecncentrated under reduced pressure. The resulting residue was purified with preparative reversed-phase HPLC and further demineralization by using PL-$HCO_3$ MP SPE Device (a column filled with Polymer supported Hydrogen carbonate, Polymer Laboratories) to give the titled compound (10 mg) as a white solid.

Analyzing method SB 1, $t_R$ 2.84 min, obs MS [M+1] 551.7

Example 83-Example 94:

Using the corresponding various amines instead of N,N-dimethylethylenediamine and treating it in the same manner as Example 82, the compounds of the following Examples 83-94 were prepared.

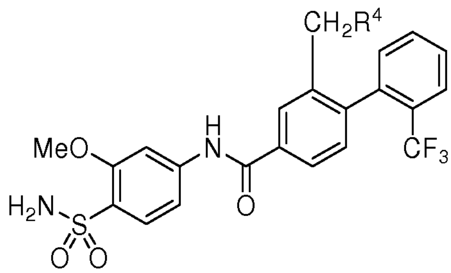

| Example | $CH_2R^4$ | obs MS [M+1] | $t_R$ (min) | Analytical Condition |
|---|---|---|---|---|
| 83 | $CH_2NHMe$ | 494.5 | 3.06 | SB1 |
| 84 | $CH_2NHEt$ | 508.4 | 3.12 | SB1 |
| 85 | $CH_2NHi\text{-}Pr$ | 522.3 | 3.18 | SB1 |
| 86 | $CH_2NHt\text{-}Bu$ | 536.5 | 3.26 | SB1 |
| 87 | $CH_2NHCHEt_2$ | 550.4 | 3.36 | SB1 |
| 88 | $CH_2NH(CH_2)_2OMe$ | 538.6 | 3.14 | SB1 |
| 89 | $CH_2NH(CH_2)_2OH$ | 524.4 | 3.01 | SB1 |
| 90 | 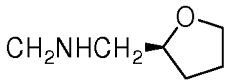 | 564.5 | 3.20 | SB1 |
| 91 | 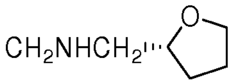 | 564.4 | 3.22 | SB1 |
| 92 | 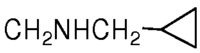 | 534.7 | 3.25 | SB1 |
| 93 | 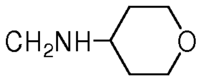 | 564.4 | 3.12 | SB1 |
| 94 | 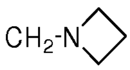 | 520.7 | 3.10 | SB1 |

Example 95:

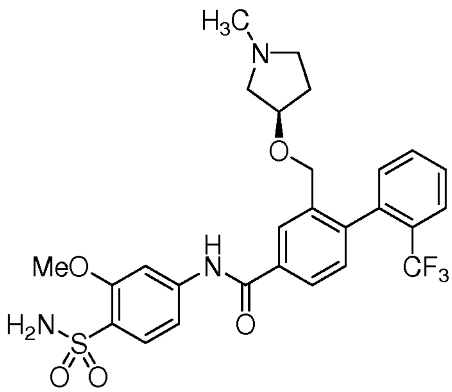

To a solution of (R)-(-)-1-methyl-3-hydroxypyrrolidine (0.14 mL) in N-methylpyrrolidone (NMP) (0.4 mL) was added sodium hydroxide (60 %, 12 mg), and the solution mixture was stirred for 20 minutes at room temperature. Then, the compound of Example 81 (30 mg) was added thereto, and the mixture was stirred for 66 hours at room temperature. Acetic acid was added to the reaction mixture, which was further dilulted with methanol, and filtered. The filtrate was concentrated under reduced pressure, and the resulting residue was purified with preparative reversed-phase HPLC and further demineralization by using PL-$HCO_3$ MP SPE Device (a column filled with Polymer supported Hydrogen carbonate, Polymer Laboratories) to give the titled compound (7.4 mg) as a white solid.

Analyzing method SB 1, $t_R$ 3.02 min, obs MS [M+1] 564.4

Example 96-Example 106:

Using corresponding various amines instead of (R)-(-)-1-methyl-3-hydroxypyrrolidine and treating it in the same manner as Example 95, the compounds of the following Examples 96-106 were prepared.

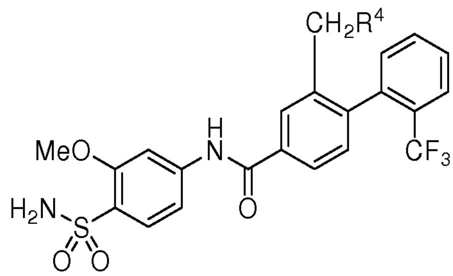

| Example | $CH_2R^4$ | obs MS [M+1] | $t_R$ (min) | Analytical Condition |
|---|---|---|---|---|
| 96 | 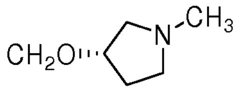 | 564.4 | 3.03 | SB1 |
| 97 | 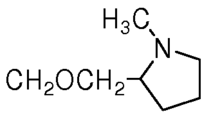 | 578.5 | 3.05 | SB1 |
| 98 | 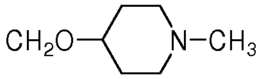 | 578.4 | 3.02 | SB1 |
| 99 | 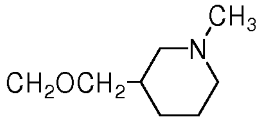 | 592.4 | 3.31 | SB1 |
| 100 | $CH_2O(CH_2)_2NMe_2$ | 552.4 | 3.00 | SB1 |
| 101 | 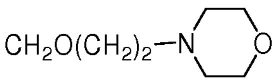 | 594.4 | 3.22 | SB1 |
| 102 | $CH_2O(CH_2)_2OH$ | 525.5 | 3.74 | SB1 |
| 103 | $CH_2O(CH_2)_3OH$ | 539.5 | 3.83 | SB1 |
| 104 | $CH_2OCH_2CH(OMe)_2$ | 569.1 | 4.10 | SB1 |
| 105 | $CH_2OCH_2CH(CH_2OH)_2$ | 569.2 | 3.58 | SB1 |
| 106 | $CH_2O(CH_2)_2NMe(CH_2)_2OH$ | 582.3 | 3.18 | SB1 |

Example 107:

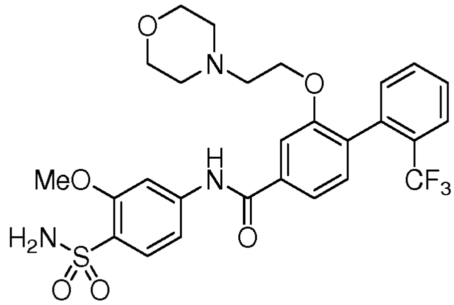

To a solution of triphenylphosphine (51 mg) in tetrahydrofuran (0.9 mL) were sequentially added 2.2 M diethyldiazodicarboxylate (DEAD) in toluene (92 μL) and N-(2-hydroxyethyl)morpholine (71 μL), which was stirred for 1.5 hours at room temperature. Then, the compound (30 mg) of Example 63 in tetrahydrofuran (0.2 mL) was added thereto, which was stirred for 18 hours at room temperature. The reaction mixture was concentrated under reduced pressure, and the resulting residue was purified with preparative reversed-phase HPLC and further demineralization by using PL-$HCO_3$ MP SPE Device (a column filled with Polymer supported Hydrogen carbonate, Polymer Laboratories) to give the titled compound (5.3 mg) as a white solid.
Analyzing method SB 1, $t_R$ 3.18 min, obs MS [M+1] 580.3

Example 108-Example 120:

Using the corresponding various alcohols instead of N-(2-hydroxyethyl)morpholine and treating it in the same manner as Example 107, the compounds of the following Examples 108-120 were prepared.

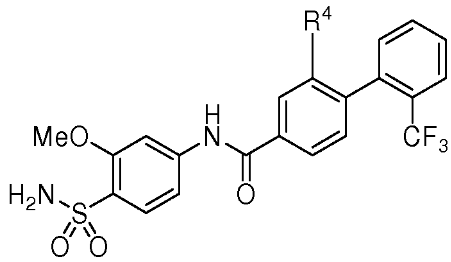

| Example | $R^4$ | obs MS [M+1] | $t_R$ (min) | Analytical Condition |
|---|---|---|---|---|
| 108 | $O(CH_2)_2NMe_2$ | 538.4 | 2.96 | SA1 |
| 109 | $O(CH_2)_2OBn$ | 601.1 | 4.18 | SA1 |
| 110 | $O(CH_2)_3OH$ | 525.6 | 3.72 | SB1 |
| 111 | 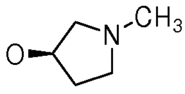 | 550.5 | 3.19 | SB1 |
| 112 | 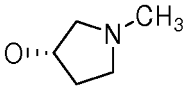 | 550.4 | 3.19 | SB1 |
| 113 | 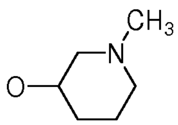 | 564.4 | 3.23 | SB1 |
| 114 | 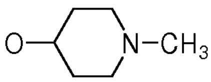 | 564.4 | 3.24 | SB1 |
| 115 | 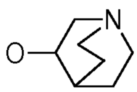 | 576.5 | 3.39 | SB1 |
| 116 | 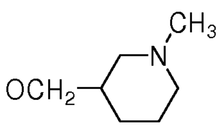 | 578.3 | 3.28 | SB1 |
| 117 | 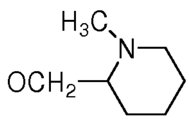 | 578.3 | 3.29 | SB1 |
| 118 | 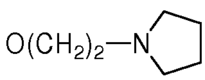 | 564.4 | 3.25 | SB1 |
| 119 | 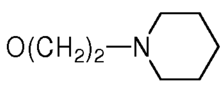 | 578.4 | 3.31 | SB1 |

(continued)

| Example | $R^4$ | obs MS [M+1] | $t_R$ (min) | Analytical Condition |
|---|---|---|---|---|
| 120 | 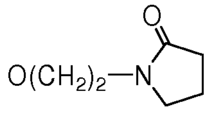 | 578.2 | 3.77 | SB1 |

Example 121:

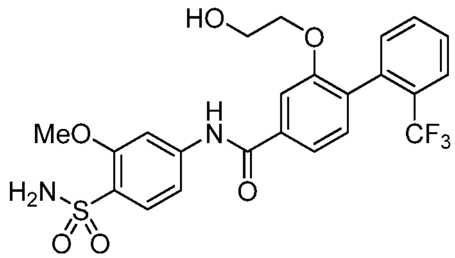

Using the compound of Example 109 (179 mg) and treating it in the same hydrogenation reaction as the second step in Reference Example 3, the titled compound (80 mg) was obtained as a white solid. $^1$H-NMR (DMSO-$d_6$) δ3.50-3.55 (m, 2H), 3.90 (s, 3H), 3.98-4.25 (m, 2H), 4.70 (t, J = 5.3 Hz, 1H), 7.00 (s, 2H), 7.28 (d, J = 7.7 Hz, 1H), 7.36 (d, J = 7.7 Hz, 1H), 7.49 (dd, J = 8.5, 1.7 Hz, 1H), 7.58-7.62 (m, 3H), 7.68-7.75 (m, 3H), 7.82 (d, J = 7.7 Hz, 1H), 10.57 (s, 1H).

Example 122:

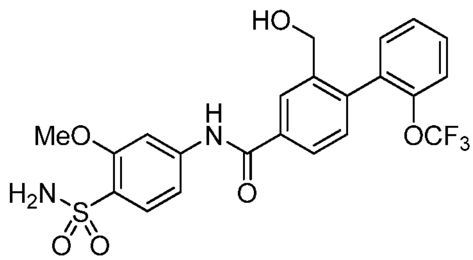

A mixture of the compound of Reference Example 12 (50 mg), [1,1'-bis(diphenylphosphino)ferrocene]dichloropalladium (II) (8.8 mg), potassium carbonate (66 mg) and 2-(trifluoromethoxy)phenylboronic acid (49 mg) was stirred for 21 hours at 80°C in a mixed solvent of DMF (1.2 mL) - water (0.12 mL). After cooling to room temperature, water was added thereto and the mixture was extracted with ethyl acetate. After washing the organic layer with water and saturated brine, it was dried over anhydrous sodium sulfate, filtered, and concentrated under reduced pressure. The resulting residue was purified with silica gel column chromatography (chloroform/methanol) and precipited by adding diethylether and hexane, and the precipitate was collected by filtration and dried under reduced pressure to give the titled compound (48 mg) as a white solid.

Analyzing method SA1, $t_R$ 3.57 min, obs MS [M+1] 497.2

$^1$H-NMR (DMSO-$d_6$) δ3.89 (s, 3H), 4.30-4.31 (m, 2H), 5.32 (t, J = 5.3 Hz, 1H), 6.99 (s, 2H), 7.33 (d, J = 7.9 Hz, 1H), 7.42-7.61 (m, 5H), 7.70 (d, J = 8.6 Hz, 1H), 7.73 (d, J = 1.7 Hz, 1H), 7.91 (dd, J = 7.9, 1.7 Hz, 1H), 8.19 (s, 1H), 10.64 (s, 1H).

Example 123-Example 128:

Using corresponding various substituted phenylboronic acids instead of 2-(trifluoromethoxy)phenylboronic acid and treating it in the same manner as Example 122, the compounds of the following Examples 123-128 were prepared.

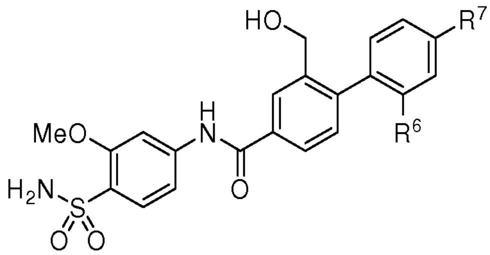

| Example | $R^6$ | $R^7$ | obs MS [M+1] | $t_R$ (min) | Analytical Condition |
|---|---|---|---|---|---|
| 123 | i-Pr | H | 455.5 | 3.67 | SA1 |
| 124 | 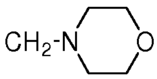 | H | 512.4 | 2.63 | SA1 |
| 125 | SMe | H | 459.1 | 3.42 | SA1 |
| 126 | Et | H | 441.5 | 3.53 | SA1 |
| 127 | $CF_3$ | F | 499.2 | 3.51 | SA1 |
| 128 | $CF_3$ | OMe | 511.2 | 3.51 | SA1 |

Example 129:

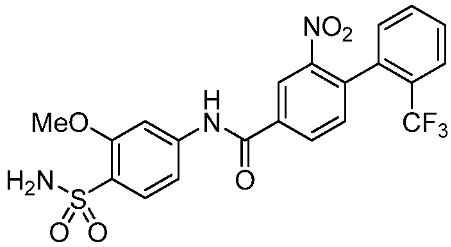

Using the compound of Reference Example 13 (215 mg) and 2-(trifluoromethyl)phenylboronic acid (190 mg), and treating them in the same manner as Example 122, the titled compound (175 mg) was prepared as a white solid.

$^1$H-NMR (DMSO-$d_6$) δ3.91 (s, 3H), 7.04 (s, 2H), 7.47-7.54 (m, 2H), 7.69-7.90 (m, 6H), 8.36 (dd, J = 8.1, 1.8 Hz, 1H), 8.75 (d, J = 1.7 Hz, 1H), 10.88 (s, 1H).

Example 130:

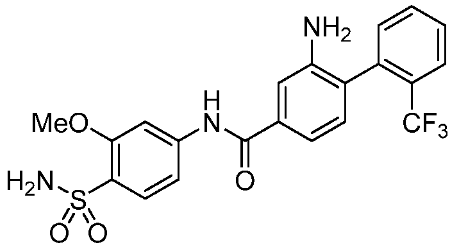

Using the compound of Example 129 (137 mg), the titled compound (122 mg) was prepared as a white solid according to the same hydrogenation reaction as those used in the second step in Reference Example 3.

$^1$H-NMR (DMSO-$d_6$) δ3.88 (s, 3H), 4.84 (s, 2H), 6.96-6.98 (m, 3H), 7.13 (dd, J = 7.9, 1.7 Hz, 1H), 7.25 (d, J = 1.5 Hz, 1H), 7.34 (d, J = 7.5 Hz, 1H), 7.52 (dd, J = 8.6, 1.8 Hz, 1H), 7.60-7.76 (m, 4H), 7.86 (d, J = 7.7 Hz, 1H), 10.44 (s, 1H).

Example 131:

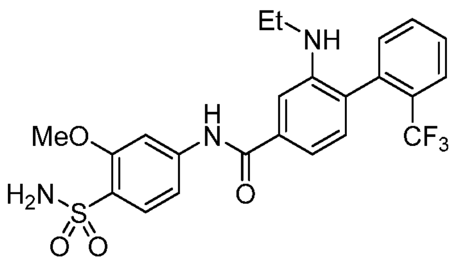

To a solution of the compound (43 mg) of Example 130 in tetrahydrofuran (1 mL) were added acetic acid (9.2 μL) and acetaldehyde (5.2 μL), the mixture was stirred for 1 hour at room temperature. Then, sodium acetate (13 mg) and sodium triacetoxyborohydride (57 mg) were added to the reaction mixture, which was stirred for 22 hours at room temperature. After adding an aqueous solution of ammonia to the reaction solution under ice-cooling, the solution was diluted with water and extracted with ethyl acetate. After washing the organic layer with saturated brine, it was dried over anhydrous magnesium sulfate, filtered, and then concentrated under reduced pressure. The resulting residue was purified with silica gel column chromatography (hexane/ethyl acetate) to give the titled compound (21 mg) as a white solid.
Analyzing method SA1, $t_R$ 3.90 min, obs MS [M+1] 494.1

Example 132:

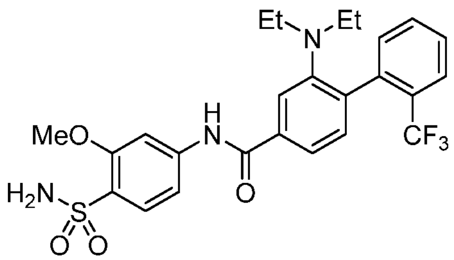

To a solution of the compound of Example 130 (43 mg) in tetrahydrofuran (1 mL) were added acetic acid (9.2 μL) and acetaldehyde (16 μL), the mixture was stirred for 1 hour at room temperature. Then, sodium acetate (13 mg) and sodium triacetoxyborohydride (57 mg) were added to the reaction mixture, which was stirred for 19 hours at room temperature. After adding further acetaldehyde (68 μL) and sodium triacetoxyborohydride (220 mg) to the solution, the mixture was stirred for 48 hours at room temperature. After adding an aqueous solution of ammonia to the reaction mixture under ice-cooling, the mixture was diluted with water and extracted with ethyl acetate. After washing the organic layer with saturated brine, it was dried over anhydrous magnesium sulfate, filtered, and then concentrated under reduced pressure. The resulting residue was purified with silica gel column chromatography (hexane/ethyl acetate) to give the titled compound (31 mg) as a white solid.
Analyzing method SA1, $t_R$ 3.38 min, obs MS [M+1] 522.2

Examples 133-134:

The compounds of the following Examples 133 and Example 134 were prepared in the same manner as Example 64-65.

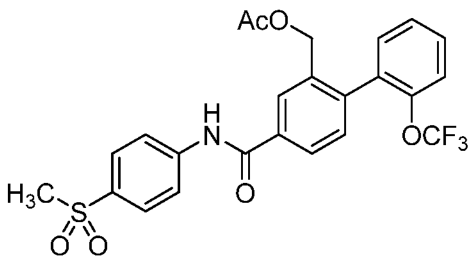

Example 133: $^1$H-NMR (DMSO-$d_6$) $\delta$1.93 (s, 3H), 3.19 (s, 3H), 4.92 (AB q, $J_{AB}$ = 32.2 Hz, 2H), 7.44-7.62 (m, 5H), 7.91-7.93 (m, 2H), 8.02-8.09 (m, 4H), 10.81 (s, 1H).

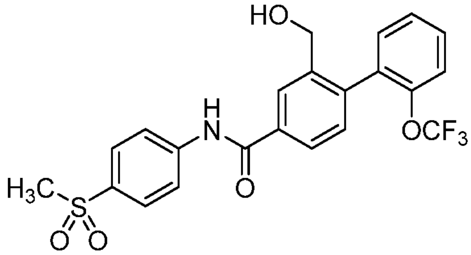

Example 134: $^1$H-NMR (DMSO-$d_6$) $\delta$3.19 (s, 3H), 4.30 (br.m, 2H), 5.33 (br.t, 1H), 7.34 (d, J = 7.8 Hz, 1H), 7.43 (d, J = 7.3 Hz, 1H), 7.49-7.52 (m, 2H), 7.57-7.61 (m, 1H), 7.90-7.93 (m, 3H), 8.06-8.08 (m, 2H), 8.20 (s, 1H), 10.79 (s, 1H).

Example 135-Example 241:

Using the comound prepared from the compound of Example 133 in the same manner as Example 81 and various amines, as well as using N,N-diisopropylethylamine or sodium hydroxide as a base under the condition of Example 82, and in DMF as a solvent or in the absence of a solvent, by appropriately heating at 70-100°C without using a microwave, the compounds of the following Examples 135-241 were prepared according to the same procesures as those used in Example 82.

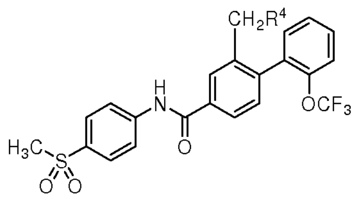

| Example | $CH_2R^4$ | obs MS $[M+1]^+$ | $t_R$ (min) | Analytical Condition | Example | $CH_2R^4$ | obs MS $[M+1]^+$ | $t_R$ (min) | Analytical Condition |
|---|---|---|---|---|---|---|---|---|---|
| 135 | 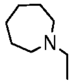 | 546.8 | 2.81 | SD1 | 149 | 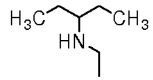 | 535.0 | 2.82 | SD1 |
| 136 | 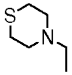 | 551.0 | 2.82 | SD1 | 150 | 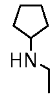 | 533.4 | 3.55 | SD2 |
| 137 | 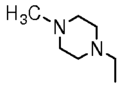 | 548.4 | 3.18 | SD2 | 151 | 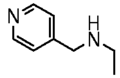 | 556.2 | 3.38 | SD2 |
| 138 | 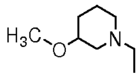 | 563.5 | 3.83 | SD2 | 152 | 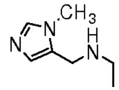 | 559.0 | 2.12 | SD3 |
| 139 | 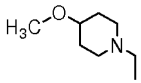 | 563.5 | 3.75 | SD2 | 153 | 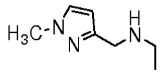 | 559.4 | 3.34 | SD2 |
| 140 | 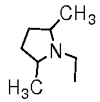 | 547.5 | 4.04 | SD2 | 154 | 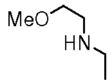 | 523.0 | 2.77 | SD1 |
| 141 | 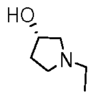 | 535.4 | 3.35 | SD2 | 155 | 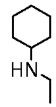 | 547.0 | 3.00 | SD1 |
| 142 | 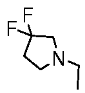 | 555.4 | 3.80 | SD2 | 156 | 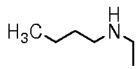 | 521.0 | 2.87 | SD1 |
| 143 | 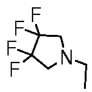 | 591.0 | 3.69 | SD1 | 157 | 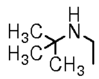 | 521.2 | 2.92 | SD1 |
| 144 | 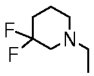 | 569.6 | 3.84 | SD2 | 158 | 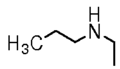 | 507.4 | 3.33 | SD2 |
| 145 | 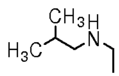 | 521.2 | 2.81 | SD1 | 159 | 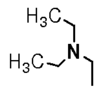 | 521.4 | 3.80 | SD2 |
| 146 | 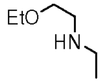 | 537.4 | 3.50 | SD2 | 160 | 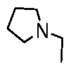 | 519.4 | 3.55 | SD2 |

(continued)

| Example | $CH_2R^4$ | obs MS $[M+1]^+$ | $t_R$ (min) | Analytical Condition | Example | $CH_2R^4$ | obs MS $[M+1]^+$ | $t_R$ (min) | Analytical Condition |
|---|---|---|---|---|---|---|---|---|---|
| 147 | 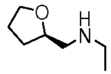 | 549.2 | 3.51 | SD2 | 161 | 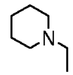 | 533.5 | 3.97 | SD2 |
| 148 | 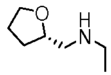 | 549.4 | 3.53 | SD2 | 162 | 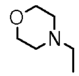 | 535.4 | 3.67 | SD2 |

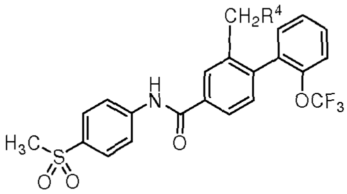

| Example | $CH_2R^4$ | obs MS [M+1] | $t_R$ (min) | Analytical Condition | Example | $CH_2R^4$ | obs MS $[M+1]^+$ | $t_R$ (min) | Analytical Condition |
|---|---|---|---|---|---|---|---|---|---|
| 163 | 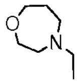 | 549.4 | 3.71 | SD2 | 177 | 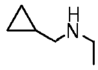 | 519.6 | 3.35 | SD2 |
| 164 | 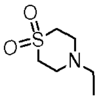 | 583.4 | 3.49 | SD2 | 178 | 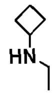 | 519.6 | 3.51 | SD2 |
| 165 | 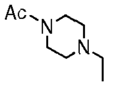 | 576.4 | 3.41 | SD2 | 179 | 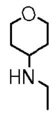 | 549.5 | 3.42 | SD2 |
| 166 | 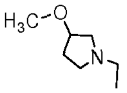 | 549.2 | 3.73 | SD2 | 180 | 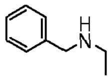 | 555.4 | 3.77 | SD2 |
| 167 | 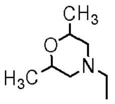 | 563.4 | 3.84 | SD2 | 181 | 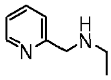 | 556.2 | 3.46 | SD2 |
| 168 | 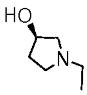 | 535.2 | 3.37 | SD2 | 182 | 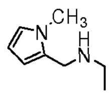 | 558.6 | 3.66 | SD2 |
| 169 | 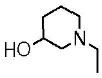 | 549.4 | 3.55 | SD2 | 183 | 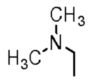 | 493.5 | 3.63 | SD2 |
| 170 | 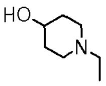 | 549.6 | 3.37 | SD2 | 184 | 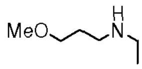 | 537.5 | 3.28 | SD2 |
| 171 | 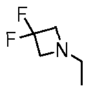 | 541.5 | 3.70 | SD2 | 185 | 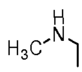 | 479.4 | 3.06 | SD2 |
| 172 | 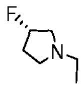 | 537.2 | 3.77 | SD2 | 186 | 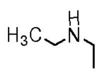 | 493.3 | 3.17 | SD2 |
| 173 | 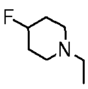 | 551.2 | 3.84 | SD2 | 187 | 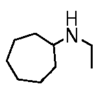 | 561.6 | 3.78 | SD2 |
| 174 | 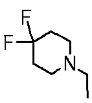 | 569.6 | 3.84 | SD2 | 188 | 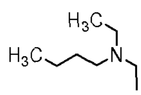 | 549.6 | 4.19 | SD2 |
| 175 | 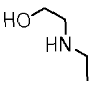 | 509.6 | 3.12 | SD2 | 189 | 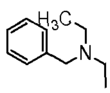 | 583.6 | 4.18 | SD2 |
| 176 | 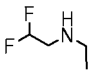 | 529.4 | 3.63 | SD2 | 190 | 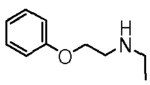 | 585.6 | 3.79 | SD2 |

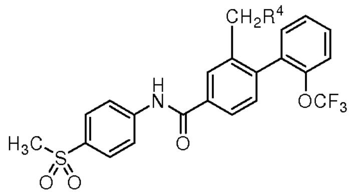

| Example | $CH_2R^4$ | obs MS $[M+1]^+$ | $t_R$ (min) | Analytical Condition | Example | $CH_2R^4$ | obs MS $[M+1]^+$ | $t_R$ (min) | Analytical Condition |
|---|---|---|---|---|---|---|---|---|---|
| 191 | 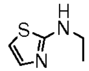 | 548.0 | 2.99 | SD2 | 205 | 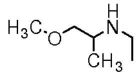 | 537.0 | 3.57 | SD2 |
| 192 | 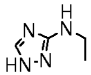 | 532.0 | 2.99 | SD2 | 206 | 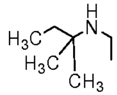 | 535.0 | 3.61 | SD2 |
| 193 | 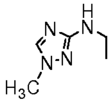 | 546.0 | 3.29 | SD2 | 207 | 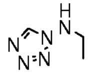 | 518.6 | 3.35 | SD2 |
| 194 | 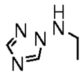 | 517.0 | 3.28 | SD2 | 208 | 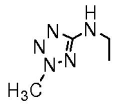 | 547.6 | 3.44 | SD2 |
| 195 | 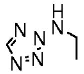 | 517.0 | 3.31 | SD2 | 209 | 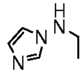 | 516.0 | 3.31 | SD2 |
| 196 | 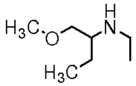 | 551.0 | 3.14 | SD1 | 210 | 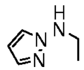 | 514.0 | 3.55 | SD2 |
| 197 | 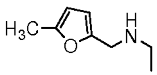 | 559.0 | 3.73 | SD2 | 211 | 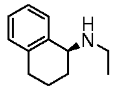 | 595.6 | 4.08 | SD2 |
| 198 | 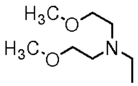 | 581.0 | 3.79 | SD2 | 212 | 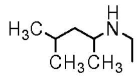 | 549.0 | 3.76 | SD2 |
| 199 | 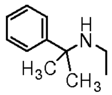 | 583.0 | 3.96 | SD2 | 213 | 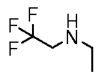 | 547.0 | 3.68 | SD2 |
| 200 | 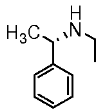 | 569.0 | 3.85 | SD2 | 214 | 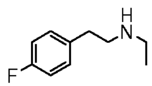 | 587.0 | 3.75 | SD2 |
| 201 | 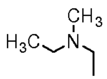 | 507.0 | 3.71 | SD2 | 215 | 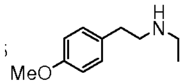 | 599.6 | 3.71 | SD2 |
| 202 | 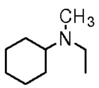 | 561.0 | 4.19 | SD2 | 216 | 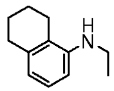 | 595.6 | 4.07 | SD2 |

(continued)

| Example | $CH_2R^4$ | obs MS $[M+1]^+$ | $t_R$ (min) | Analytical Condition | Example | $CH_2R^4$ | obs MS $[M+1]^+$ | $t_R$ (min) | Analytical Condition |
|---|---|---|---|---|---|---|---|---|---|
| 203 | 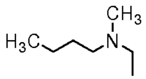 | 535.0 | 4.08 | SD2 | 217 | 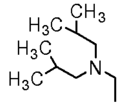 | 577.6 | 2.80 | SD2 |
| 204 | 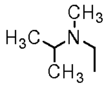 | 521.0 | 3.86 | SD2 | 218 | 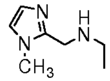 | 559.6 | 3.30 | SD2 |

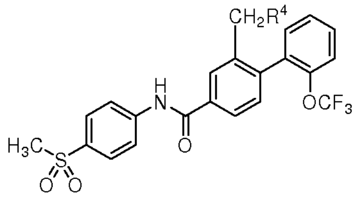

| Example | $CH_2R^4$ | obs MS $[M+1]^+$ | $t_R$ (min) | Analytical Condition | Example | $CH_2R^4$ | obs MS $[M+1]^+$ | $t_R$ (min) | Analytical Condition |
|---|---|---|---|---|---|---|---|---|---|
| 219 | 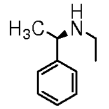 | 569.0 | 2.30 | SD2 | 231 | 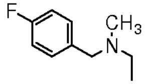 | 587.5 | 4.05 | SD2 |
| 220 | 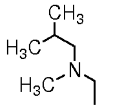 | 535.0 | 2.34 | SD2 | 232 | 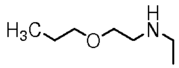 | 551.0 | 3.64 | SD2 |
| 221 | 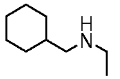 | 561.0 | 2.14 | SD2 | 233 | 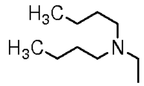 | 577.0 | 2.98 | SD1 |
| 222 | 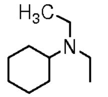 | 575.0 | 2.58 | SD2 | 234 | 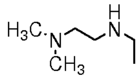 | 536.6 | 3.02 | SD2 |
| 223 | 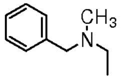 | 569.0 | 2.27 | SD2 | 235 | 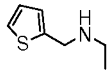 | 575.5 | 3.77 | SD2 |
| 224 | 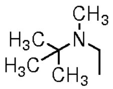 | 535.0 | 2.17 | SD2 | 236 | 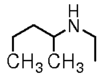 | 535.0 | 9.39 | SD2 |
| 225 | 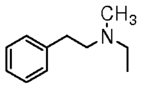 | 583.0 | 4.08 | SD2 | 237 | 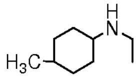 56· (High polar isomer) | 561.6 | 3.75 | SD2 |
| 226 | 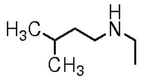 | 535.0 | 3.58 | SD2 | 238 | 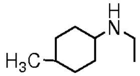 56· (Low polar isomer) | 561.6 | 3.92 | SD2 |
| 227 | 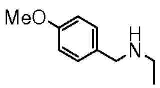 | 585.0 | 3.66 | SD2 | 239 | 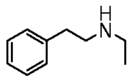 | 569.6 | 3.75 | SD2 |
| 22 | 28 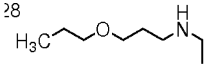 | 565.0 | 3.53 | SD2 | 240 | 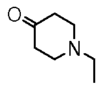 | 547.6 | 3.56 | SD2 |
| 229 | 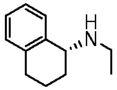 | 595.0 | 4.11 | SD2 | 241 | 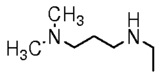 | 550.6 | 3.02 | SD2 |
| 230 | 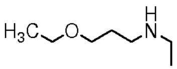 | 551.5 | 3.37 | SD2 | | | | | |

Examples 242-243:

Using methylamine instead of ammonia and treating it in the same manner as Reference Example 3 as well as Examples 68 and 69, the compounds of the following Examples 242-243 were prepared.

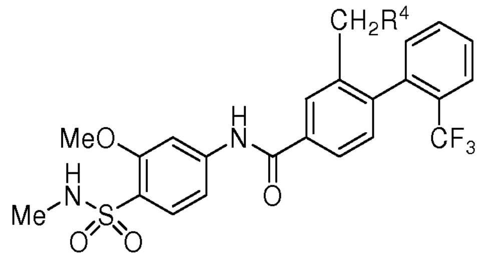

| Example | $CH_2R^4$ | obs MS [M+1] | $t_R$ (min) | Analytical Condition |
|---|---|---|---|---|
| 242 | $CH_2OH$ | 481.4 | 3.21 | SA2 |
| 243 | $CH_2OAc$ | 523.4 | 3.60 | SA2 |

Example 244:

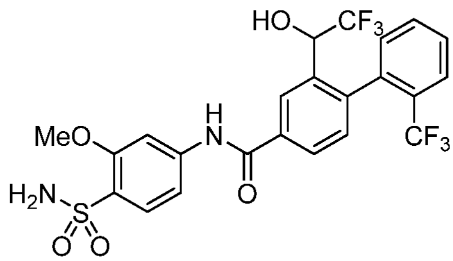

To a solution of the compound (60 mg) obtained in Example 70 in DMF (1 mL) were added lithium acetate (24.7 mg) and trimethylsilylated trifluoromethane (110 μL) under ice-cooling, the mixture was stirred for 2 hours. A saturated aqueous solution of ammonium chloride was added to the reaction mixture, which was extracted with ethyl acetate. After washing the organic layer with saturated brine, it was dried over anhydrous sodium sulfate, filtered and concentrated under reduced pressure. The resulting residue was purified with silica gel column chromatography to give the titled compound (15 mg) as a white solid.

$^1$H-NMR (DMSO-$d_6$) δ3.90 (s, 3H), 4.60 (m, 1H), 7.02-8.32 (m, 13H), 10.7 (s, 1H).

Example 245:

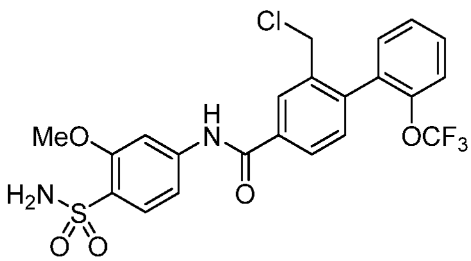

Using the compound (200 mg) obtained in Example 122 and treating it in the same manner as Example 81, the titled

compound (189 mg) was prepared.
$^1$H-NMR (DMSO-$d_6$) δ4.01 (s, 3H), 4.38 (d, J = 16 Hz, 1H),4.61 (d, J = 16 Hz, 1H), 7.24-7.26 (m, 3H), 7.44-7.70 (m, 6H), 7.82 (m, 2H), 8.03 (s, 1H), 10.5 (s, 1H).

Examples 246-250:

Using various amines and the compound prepared in Example 245, and treating them in the same manner as Example 82, the compounds of the following Example 246-Example 250 were prepared.

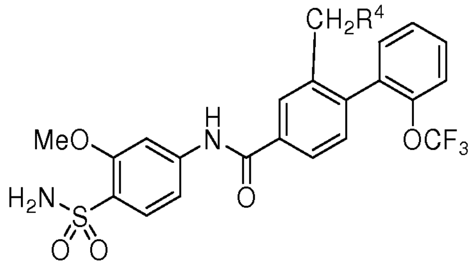

| Example | $CH_2R^4$ | $^1$H-NMR(DMSO-$d_6$) δ |
|---|---|---|
| 246 | 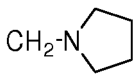 | 1.58-1.70(m, 4H), 2.20-2.40(m, 4H), 3.40(s, 2H), 4.01(s, 3H), 7.13-7.95(m, 15H), 10.5(s, 1 H). |

| Example | $CH_2R^4$ | obs MS [M+1] | $t_R$ (min) | Analytical Condition |
|---|---|---|---|---|
| 247 | 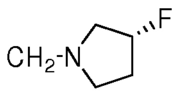 | 568.5 | 2.96 | SA2 |
| 248 | 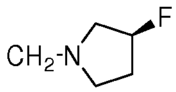 | 568.4 | 2.99 | SA2 |
| 249 | $CH_2NHCH_2CHF_2$ | 561.0 | 0.87 | SC1 |
| 250 | $CH_2NHCH_2CH_2F$ | 542.9 | 0.69 | SC1 |

Example 251:

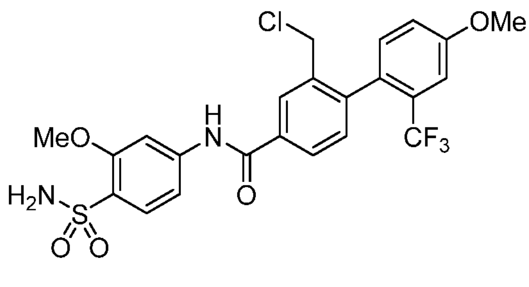

Using the compound (150 mg) prepared in Example 128 and treating it in the same manner as Example 81, the titled compound (146 mg) was prepared.
$^1$H-NMR (DMSO-$d_6$) δ3.90 (s, 3H), 4.35 (d, J = 16 Hz, 1H),4.65 (d, J = 16 Hz, 1H), 7.70 (s, 1H), 7.35-7.38 (m, 4H), 7.53 (d, J=12 Hz, 1H), 7.69-7.22 (m, 2H), 7.97 (dd, J = 12Hz,, 4 Hz), 8.15 (d, J = 4Hz, 1H), 10.6(s, 1H).

Example 252-Example 253:

The compound 12-4, which was an intermediate in Reference Example 12, and 2-(trifluoromethoxy)phenylboronic acid or 2-(trifluoromethyl)-4-methoxyphenylboronic acid were treated in the same manner as the first step in Reference Example 11, and the resulting compound and 4-sulfamidoaniline were treated in the same manner as the fifth step in Reference Example 12. The resulting compound was treated in the same manner as the sixth step in Reference Example 12 and Example 81 to prepare the compounds of the following Examples 252-253.

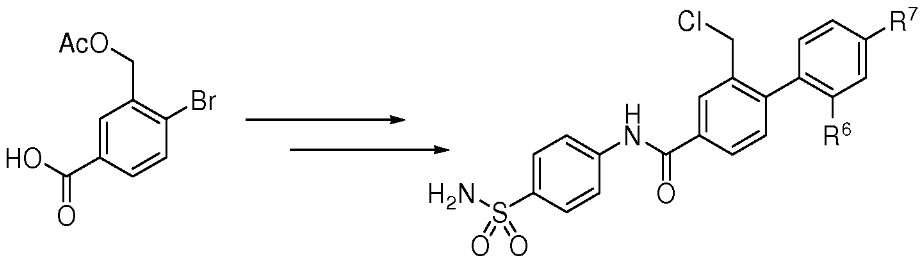

| Example | $R^6$ | $R^7$ | $^1$H-NMR(DMSO-$d_6$) $\delta$ |
|---|---|---|---|
| 252 | $OCF_3$ | H | 4.45(d, J = 12 Hz, 1H),4.71(d, J = 12 Hz, 1H), 7.29(s, 2H), 7.42-7.65(m, 5H), 7.82(d, J = 12Hz, 2H), 8.03(d, J = 8 Hz, H), 8.19(s, 1H), 10.7(s, 1H). |
| 253 | $CF_3$ | OMe | 3.90(s, 3H), 4.33(d, J = 12 Hz, 1 H),4.65(d, J = 12 Hz, 1 H), 7.28-7.37(m, 5H), 7.95-7.98 (m, 3H), 8.16(s, 1 H), 10.7(s, 1 H). |

Example 254:

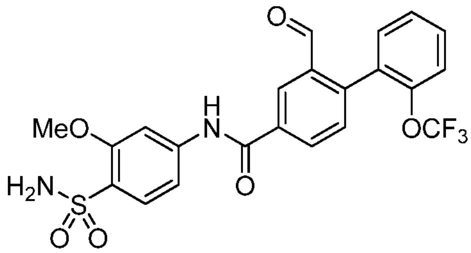

Using the compound prepared in Example 122 and treating it in the same manner as Example 70, the titled compound (1.27 g) was prepared.

$^1$H-NMR (DMSO-$d_6$) $\delta$3.91 (s, 3H), 7.01 (s, 2H), 7.53-7.74 (m, 9H), 8.32-8.54 (m, 2H), 9.83 (s, 1H), 10.8 (s, 1H).

Example 255:

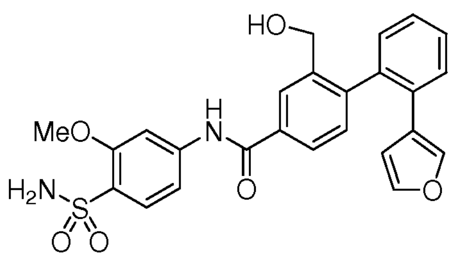

The titled compound was prepared from the compound 14 prepared in Reference Example 14 and the compound 15 prepared in Reference Example 15 in the same manner as the first step in Reference Example 11 and the sixth step in Reference Example 12.

Analyzing method SB3, $t_R$ 1.021 min, obs MS [M+1] 479.4

Examples 256-260:

The compounds of the following Examples 256-260 were prepared from the compound 14 prepared in Reference Example 14 in the same manner as Example 255.

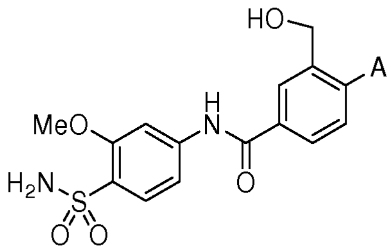

| Example | A | obs MS [M+1] | $t_R$ (min) | Analytical Condition |
|---|---|---|---|---|
| 256 | 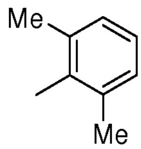 | 441.6 | 4.16 | SB3 |
| 257 | 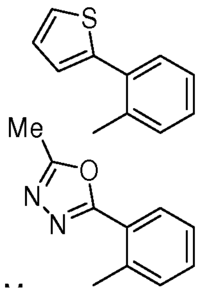 | 495.4 | 4.54 | SB3 |
| 258 | | 495.0 | 2.15 | SB3 |
| 259 | 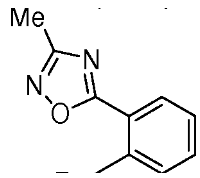 | 495.1 | 2.20 | SB3 |
| 260 | 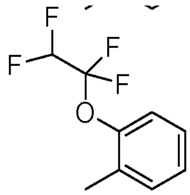 | 529.4 | 5.52 | SB1 |

Examples 261-263:

4-Sulfamidoaniline was prepared in the same manner as Reference Example 13, and the resulting compound and 2-(trifluoromethyl)-4-methoxyphenylboronic acid, 2-(trifluoromethoxy)phenylboronic acid or 2-trifluoromethylphenylboronic acid were treated in the same manner as Example 129-130 to prepare the compounds of the following Examples 261-263.

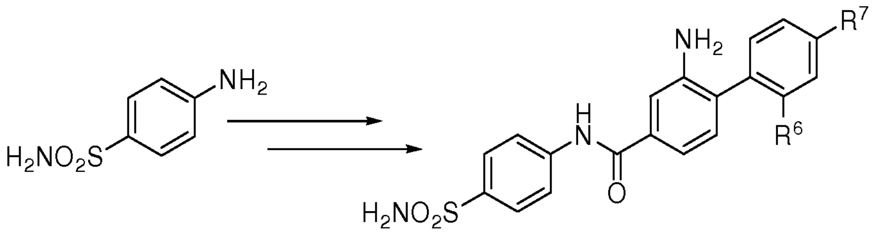

| Example | $R^6$ | $R^7$ | obs MS [M+1] | $t_R$ (min) | Analytical Condition |
|---|---|---|---|---|---|
| 261 | $CF_3$ | H | 436.5 | 3.63 | SB3 |
| 262 | $OCF_3$ | H | 452.0 | 4.22 | SB3 |
| 263 | $CF_3$ | OMe | 466.5 | 4.14 | SB3 |

Examples 264-265:

Using the compound prepared in Example 262 and treating it in the same manner as Example 131-132, the compounds of the following Examples 264-265 were prepared.

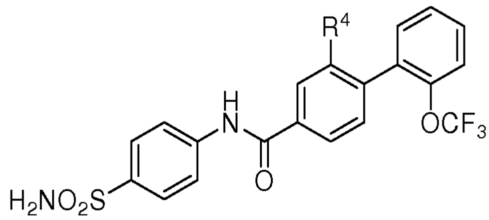

| Example | $R^4$ | obs MS [M+1] | $t_R$ (min) | Analytical Condition |
|---|---|---|---|---|
| 264 | NH(Et) | 480.5 | 4.68 | SB3 |
| 265 | $NEt_2$ | 508.6 | 3.92 | SB3 |

Example 266:

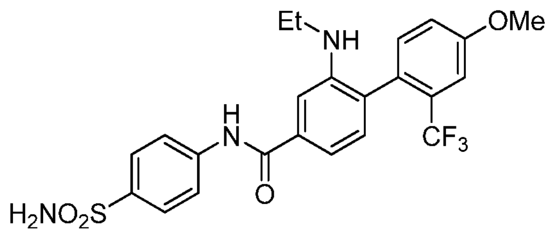

Using the compound prepared in Example 263 and treating it in the same manner as Example 131, the titled compound was prepared.

Analyzing method SB3, $t_R$ 4.82min, obs MS {M+1} 494.6

Examples 267-270:

Using the compound prepared in Examples 81, 245, 251-253 and 2,2-difluoroethylamine, and treating them in the same manner as Example 82, the compounds of the following Examples 267-270 were prepared.

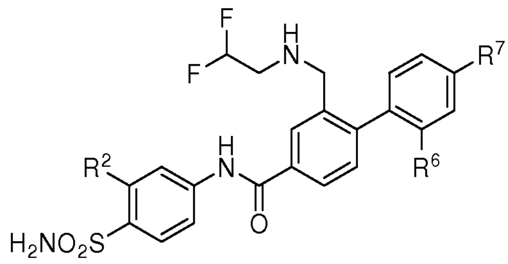

| Example | $R^6$ | $R^7$ | $R^2$ | obs MS [M+1] | $t_R$ (min) | Analytical Condition |
|---|---|---|---|---|---|---|
| 267 | $OCF_3$ | H | H | 530.4 | 5.05 | SB1 |
| 268 | $CF_3$ | H | OMe | 544.5 | 4.86 | SB1 |
| 269 | $CF_3$ | OMe | H | 544.4 | 5.02 | SB1 |
| 270 | $CF_3$ | OMe | OMe | 574.5 | 5.03 | SB1 |

Examples 271-273:

Using the compound 17 (80 mg) prepared in Reference Example 17, 2-trifluoromethyl-4-aminobromobenzene and 2-trifluoromethyl-4-hydroxybromobenzene or 3-(2-bromophenyl)-5-methyl-1,2,4-oxadiazole, were treated in the same manner as the first step in Reference Example 11, the resulting product was reacted with trifluoroacetic acid (4 mL) at room temperature, and the reaction solution was concentrated under reduced pressure, suspended in a saturated aqueous solution of sodium bicarbonate, filtered, and dried under reduced pressure to give the compounds of the following the compound of Examples 271-273.

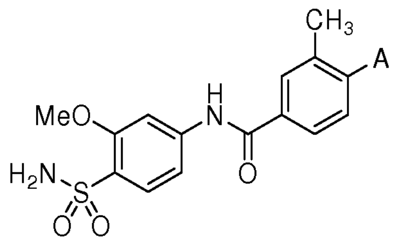

| Example | A | obs MS [M+1] | $t_R$ (min) | Analytical Condition |
|---|---|---|---|---|
| 271 | 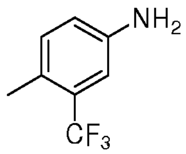 | 480.4 | 5.19 | SB1 |

| Example | A | $^1H$-NMR(DMSO-$d_6$) $\delta$ |
|---|---|---|
| 272 | 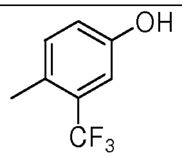 | 2.06(s, 3H), 3.89(s, 3H), 6.99(s, 2H), 7.11-7.25(m, 3H), 7.52(d, J = 8.0 Hz, 1H), 7.68-7.85(m, 4H), 10.2(s, 1H), 10.5(s, 1H). |

(continued)

| Example | A | $^1$H-NMR(DMSO-$d_6$) $\delta$ |
|---|---|---|
| 273 | 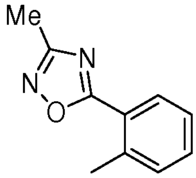 | 2.03(s, 3H), 2.43(s, 3H), 3.89(s, 3H), 7.00(s, 2H), 7.26(d, J = 8.0 Hz, 1 H), 7.41 (d, J = 8.0 Hz, 1H), 7.53(d, J = 8.0 Hz, 1H),7.65-7.87(m, 6H), 8.13(d, J = 8.0 Hz, 1H), 10.5(s, 1H). |

Examples 274-283:

Using the compound 17 prepared in Reference Example 17 (100 mg) and 5-(2-chlorophenyl)pyrimidine (75.8 mg) were treated in the same manner as the first step in Reference Example 11, the resulting product was reacted with trifluoroacetic acid (4 mL) at room temperature, and the reaction mixture was concentrated under reduced pressure, suspended in saturated aqueous solution of sodium bicarbonate, filtered, and dried under reduced pressure to give the compound of Example 274 (45.7 mg). According to the same proccedures above, the compounds of the following Examples 275-283 were prepared.

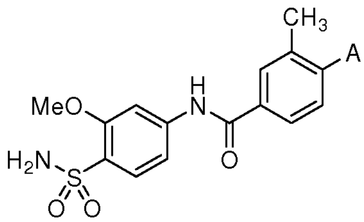

| Example | A | obs MS [M+1] | $t_R$ (min) | Analytical Condition | Example | A | obs MS [M+1] | $t_R$ (min) | Analytical Condition |
|---|---|---|---|---|---|---|---|---|---|
| 274 | 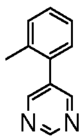 | 475.3 | 0.908 | SC2 | 279 | 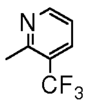 | 466.3 | 0.874 | SC2 |
| 275 | 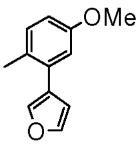 | 493.5 | 1.10 | SC2 | 280 | 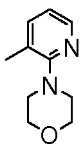 | 483.4 | 0.784 | SC2 |
| 276 | 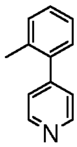 | 474.7 | 0.818 | SC2 | 281 | 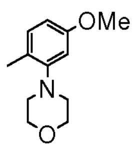 | 512.5 | 1.04 | SC2 |
| 277 | 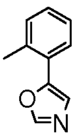 | 464.4 | 0.997 | SC2 | 282 | 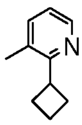 | 452.7 | 0.748 | SC2 |
| 278 | 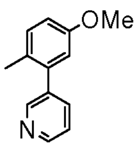 | 504.1 | 0.920 | SC2 | 283 | 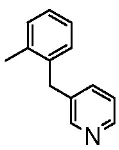 | 488.7 | 0.846 | SC2 |

Example 284:

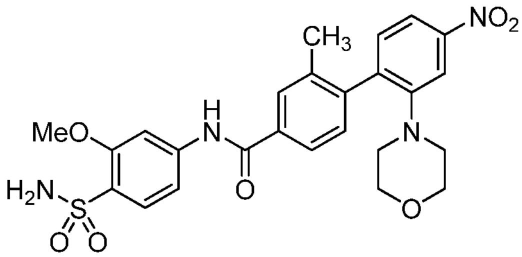

The compound 18-3 prepared in Reference Example 18 (140 mg) and the compound 17 prepared in Reference Example 17 (100 mg) were treated in the same manner as the first step in Reference Example 11. After the crude product was reacted with trifluoroacetic acid (4 mL) at room temperature, the reaction mixture was concentrated under reduced pressure, suspended in saturated aqueous solution of sodium bicarbonate, filtered, and dried under reduced pressure to give the titled compound (23.0 mg). Analyzing method SC2, $t_R$ 1.020 min, obs MS [M+1] 527.4

Example 285:

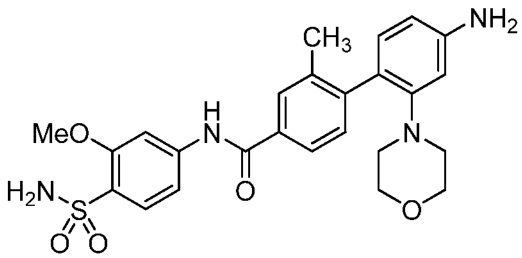

To a solution of the compound (23.0 mg) prepared in Example 284 in methanol (1 mL) was added 10 % palladium on carbon catalyst (23.0 mg), and the mixture was stirred for 4 hours at room temperature under hydrogen atmosphere. The reaction solution was filtered, and then dried under reduced pressure to give the titled compound (8.8 mg). Analyzing method SC2, $t_R$ 0.643 min, obs MS [M+1] 497.8

Examples 286-287:

Using the compound prepared in Example 245 and treating it in the same manner as Example 82, the compounds of the following Examples 286-287 were prepared.

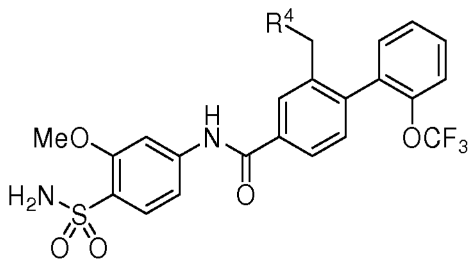

| Example | $R^4$ | obs MS $[M+1]^+$ | $t_R$ (min) | Analytical Condition |
|---|---|---|---|---|
| 286 | 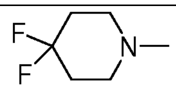 | 600.0 | 1.042 | SC2 |

(continued)

| Example | $R^4$ | obs MS $[M+1]^+$ | $t_R$ (min) | Analytical Condition |
|---|---|---|---|---|
| 287 | 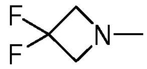 | 572.4 | 1.093 | SC2 |

Examples 288-310:

Using various amines and the compound obtained in Example 245, and treating them in the same manner as Example 82 provided that using N,N-diisopropylethylamine as a base and DMF as a solvent under heating condition at 100°C, the compounds of the following Examples 288-310 were prepared.

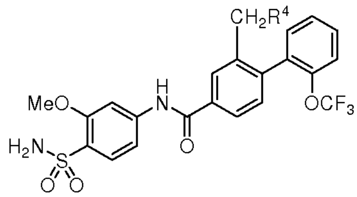

| Example | $CH_2R^4$ | obs MS $[M+1]^+$ | $t_R$ (min) | Analytical Condition | Example | $CH_2R^4$ | obs MS $[M+1]^+$ | $t_R$ (min) | Analytical Condition |
|---|---|---|---|---|---|---|---|---|---|
| 288 | 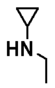 | 536.2 | 3.71 | SA2 | 299 | 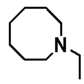 | 592.5 | 3.79 | SA2 |
| 289 | 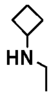 | 550.2 | 3.76 | SA2 | 300 | 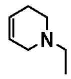 | 562.2 | 3.64 | SA2 |
| 290 | 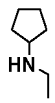 | 564.5 | 3.81 | SA2 | 301 | 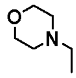 | 566.1 | 3.70 | SA2 |
| 291 | 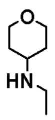 | 580.1 | 3.71 | SA2 | 302 | 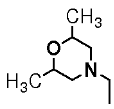 | 594.1 | 3.79 | SA2 |
| | | | | | 303 | 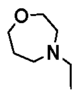 | 580.1 | 3.62 | SA2 |
| 292 | 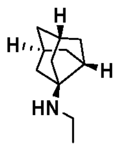 | 616.3 | 4.04 | SA2 | 304 | 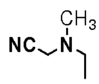 | 549.3 | 4.46 | SA2 |
| 293 | 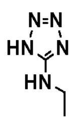 | 564.2 | 4.27 | SA2 | 305 | 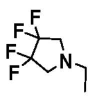 | 622.2 | 4.77 | SA2 |
| 294 | 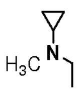 | 550.2 | 3.65 | SA2 | 306 | 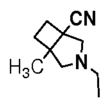 | 615.2 | 4.68 | SA2 |
| 295 | 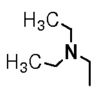 | 552.1 | 3.54 | SA2 | 307 | 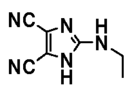 | 612.1 | 4.38 | SA2 |

(continued)

| Example | $CH_2R^4$ | obs MS $[M+1]^+$ | $t_R$ (min) | Analytical Condition | Example | $CH_2R^4$ | obs MS $[M+1]^+$ | $t_R$ (min) | Analytical Condition |
|---|---|---|---|---|---|---|---|---|---|
| 296 | 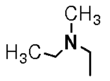 | 538.4 | 3.61 | SA2 | 308 | 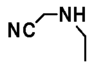 | 534.1 | 4.39 | SA2 |
| 297 | 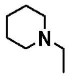 | 564.5 | 3.66 | SA2 | 309 | 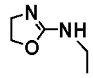 | 565.3 | 4.46 | SA2 |
| 298 | 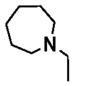 | 549.8 | 3.71 | SA2 | 310 | 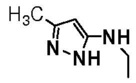 | 562.2 | 3.74 | SA2 |

Example 311:

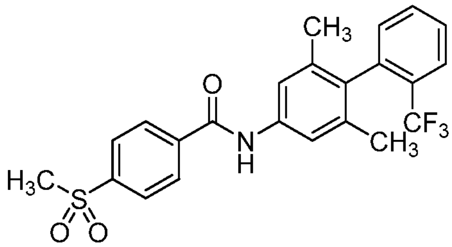

To a suspension of 4-(methylsulfonyl) benzoate (100 mg) in toluene (2 mL) were added dropwise thionyl chloride (110 μL) and DMF (3.7 μL), and the mixture was stirred for 2 hours at 90°C. After cooling to room temperature, the reaction solution was concentrated under reduced pressure. Then, after azeotropically distillation with toluene, tetrahydrofuran (1.5 mL) was added thereto, and further the compound (66 mg) prepared in Reference Example A1 and diisopropylethylamine (55 μL) were added thereto, and the mixture was stirred for 15 hours at room temperature. The reaction mixture was concentrated under reduced pressure, and the resulting residue was purified with silica gel column chromatography (hexane/ethyl acetate) to give the titled compound (112 mg) as a white solid.

$^1$H-NMR (DMSO-$d_6$) δ1.87 (s, 6H), 3.32 (s, 3H), 7.25 (d, J = 7.5 Hz, 1H), 7.55 (s, 2H), 7.62 (t-like, J = 7.8 Hz, 1H), 7.77 (t-like, J = 7.5 Hz, 1H), 7.87 (d, J = 7.7 Hz, 1H), 8.08 (d, J = 8.4 Hz, 2H), 8.18 (d, J = 8.4 Hz, 2H), 10.43 (s, 1H).

Example 312:

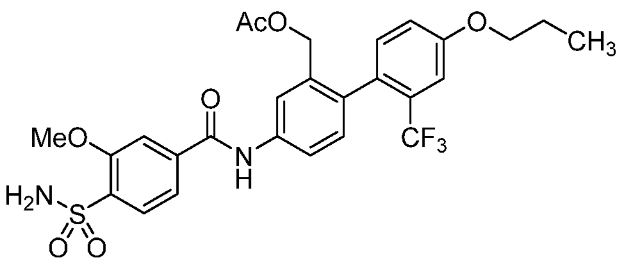

To a solution of the compound 22-2 (28 mg) prepared in Reference Example 22 and the compound 23-7 (30 mg) prepared in Reference Example 23 in DMF (0.81 mL) were added WSCI•HCl (19 mg), HOBt (13 mg) and DIEA (13.5 μL), and the mixture was stirred for 24 hours at room temperature. After adding water, the mixture was extracted with ethyl acetate, and the organic layer was washed with saturated brine. The organic layer was dried over sodium sulfate, concentrated under reduced pressure. The resulting residue was purified with silica gel column chromatography (hexane/ethyl acetate) to give the titled compound (30 mg) as a white solid.

Analyzing method SA3, $t_R$ 4.78 min, obs MS [M+1] 581.0

Example 313:

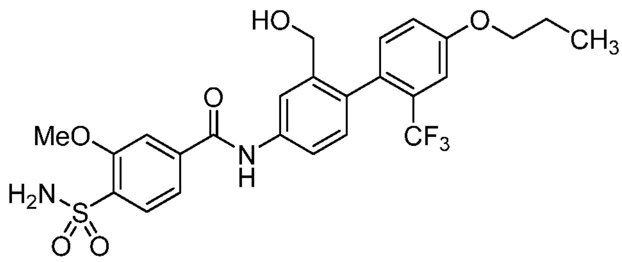

The compound of Example 312 (29 mg) was dissolved in a mixture of tetrahydrofuran-methanol (1:1) (1 mL), a 1N aqueous solution of sodium hydroxide (0.2 mL) was added dropwise into the mixture, and the mixture was stirred for 2 hours at room temperature. The reaction mixture was acidified by adding 2N aqueous solution of hydrochloric acid, and the solvent was concentrated under reduced pressure, and then the resulting residue was extracted with ethyl acetate and water. After washing the organic layer with saturated brine, it was dried over anhydrous sodium sulfate, filtered, and concentrated under reduced pressure. The resulting residue was purified with silica gel column chromatography (hexane/ ethyl acetate) to give the titled compound (18 mg) as a white solid.
Analyzing method SA3, $t_R$ 4.57 min, obs MS [M+1] 539.2

Example 314:

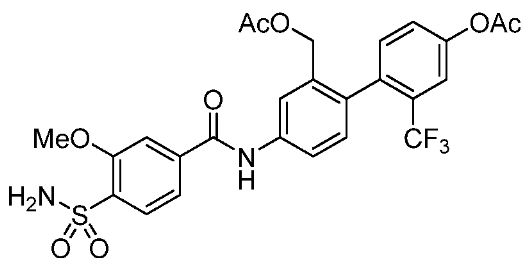

Using the compound 22-2 (19 mg) prepared in Reference Example 22 and the compound 24-2 (18 mg) prepared in Reference Example 24, and treating them in the same manner as Example 312, the titled compound (11 mg) was prepared.
Analyzing method SA3, $t_R$ 4.18 min, obs MS [M+1] 581.3

Example 315:

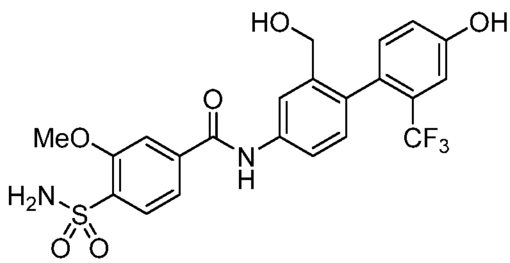

Using the compound (10 mg) prepared in Example 314 and treating it in the same manner as Example 313, the titled compound (6.1 mg) was prepared as a white solid.
Analyzing method SA3, $t_R$ 3.80 min, obs MS [M+1] 497.5

Examples 316-321:

The compounds of the following Examples 316-321 were prepared in the same manner as Examples 312-315.

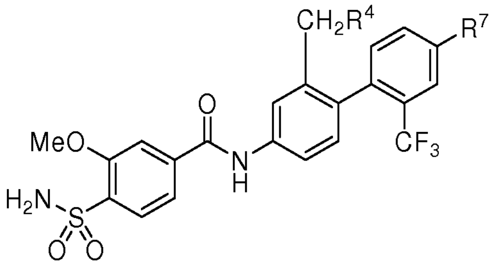

| Example | $CH_2R^4$ | $R^7$ | obs MS [M+1] | $t_R$ (min) | Analytical Condition |
|---|---|---|---|---|---|
| 316 | $CH_2OAc$ | $O(CH_2)_2NMe_2$ | 610.1 | 3.13 | SA3 |
| 317 | $CH_2OH$ | $O(CH_2)_2NMe_2$ | 568.4 | 2.92 | SA3 |

| Example | $CH_2R^4$ | $R^7$ | $^1$H-NMR(DMSO-$d_6$) $\delta$ |
|---|---|---|---|
| 318 | $CH_2OAc$ | $O(CH_2)_2OAc$ | 1.97(s, 3H), 2.05(s, 3H), 4.00(s, 3H), 4.34-4.36(m, 4H),4.73(m,2H), 7.17 (d, J = 8.4 Hz, 1H), 7.25-7.34(m, 5H), 7.61-7.64(m, 1H), 7.68(s, 1H), 7.78-7.81 (m, 1H), 7.85-7.88(m, 2H), 10.53(s, 1H). |
| 319 | $CH_2OAc$ | $OCH_2CO_2$t-Bu | 1.42(s, 9H), 1.96(s, 3H), 4.00(s, 3H), 4.73(AB q, JAB = 21.0 Hz, 2H), 4.84(s, 2H), 7.18(d, J = 8.4 Hz, 1 H), 7.23-7.31 (m, 5H), 7.61-7.64(m, 1H), 7.68(s, 1 H), 7.79(dd, J = 8.2, 2.0 Hz, 1 H), 7.85-7.87(m, 2H), 1 0.53 (s, 1H). |
| 320 | $CH_2OH$ | $O(CH_2)_2OH$ | 3.75(m, 2H), 4.01 (s, 3H), 4.05-4.17(m, 2H), 4.93(t-like, J = 5.5 Hz, 1H), 5.17(t-like, J = 5.2 Hz, 1H), 7.05(d, J = 8.1 Hz, 1H), 7.25-7.29(m, 5H), 7.62-7.65(m, 1 H), 7.70(s, 1H), 7.73-7.76(m, 1 H), 7.86(d, J = 8.1 Hz, 1H), 7.91 (m, 1H), 10.46(s, 1H). |
| 321 | $CH_2OH$ | $OCH_2CO_2H$ | 4.01 (s, 3H), 4.06-4.20(m, 2H), 4.34(bs, 1 H), 4.82(s, 2H), 5.18(br, 1H), 7.06(d, J = 8.3 Hz, 1H), 7.24-7.27(m, 5H), 7.62-7.64(m, 1H), 7.70(s, 1H), 7.72-7.76(m, 1H), 7.85(d, J = 8.1 Hz, 1H), 7.91 (m, 1H), 1 0.46(s, 1H). |

Example 322:

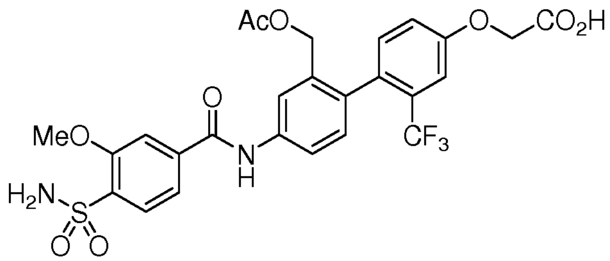

The compound of Example 319 (33 mg) was dissolved in dichloromethane (0.5 mL), trifluoroacetic acid (33 μL) was added to the obtained solution, and then the mixture was stirred for 3 days at room temperature. The reaction mixture was concentrated under reduced pressure and the resulting residue was purified with silica gel column chromatography (chloroform/methanol) to give the titled compound (26 mg) as a white solid.

$^1$H-NMR (DMSO-$d_6$) $\delta$1.96 (s, 3H), 4.00 (s, 3H), 4.73 (m, 2H), 4.85 (s, 2H), 7.18 (d, J = 8.4 Hz, 1H), 7.26-7.30 (m, 5H), 7.61-7.64 (m, 1H), 7.68 (s, 1H), 7.78-7.81 (m, 1H), 7.85-7.88 (m, 2H), 10.53 (s, 1H), 13.15 (bs, 1H).

Example 323:

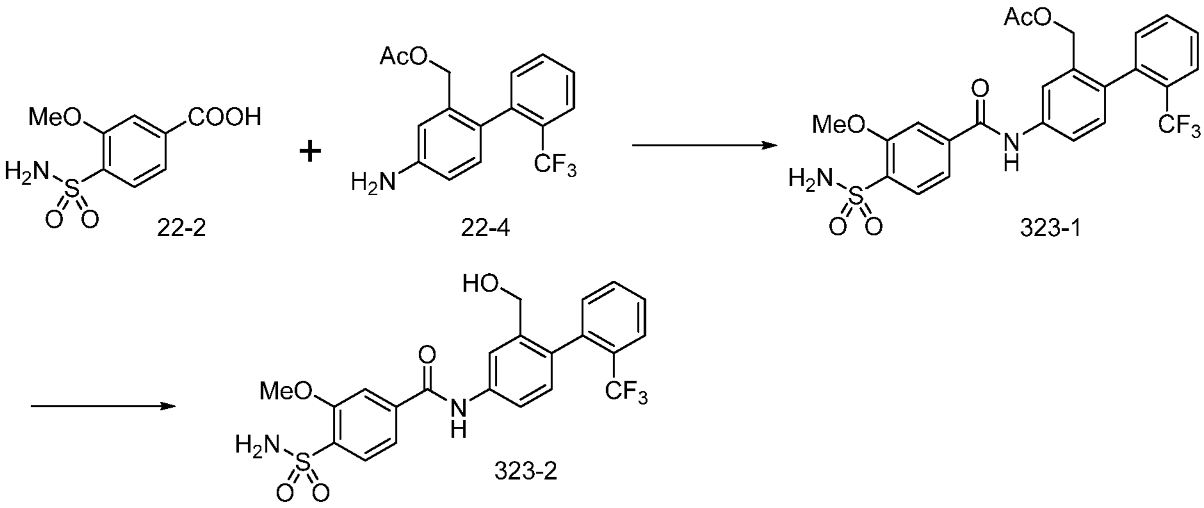

22-2 22-4 323-1

323-2

The compound 323-1 was prepared in the same manner as Example 311 from the compound 20-4 prepared in Reference Example 20 and the compound 22-2 prepared in Reference Example 22. To a suspension of the compound 323-1 (77 mg) in methanol (2 mL) and THF was added an aqueous solution of sodium hydroxide (1N, 1 mL) at room temperature, and the mixture was stirred for 1 hour. An aqueous solution of hydrochloric acid (3N, 20 mL) was added thereto, and the resulting suspension was filtered, dried under reduced pressure to give the compound 323-2 (65 mg) as a white powder.

The compound 323-1:$^1$H-NMR (DMSO-$d_6$) δ1.94 (s, 3H), 4.01 (s, 3H), 4.68-4.78 (m, 1H), 7.18 (m, 3H), 7.38 (d, J = 8.0Hz, 1H), 7.60-7.84 (m, 8H), 10.5 (s, 1H).

The compound 323-2:$^1$H-NMR (DMSO-$d_6$) δ4.01-4.20 (m, 5H), 5.18 (bs, 1H), 7.07 (d, J = 12Hz, 1H), 7.24 (s, 1H), 7.34 (d, J = 12Hz, 1H), 7.58-7.94 (m, 8H), 10.4 (s, 1H).

Example 324:

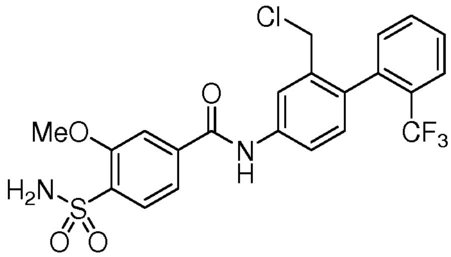

The titled compound (0.51 g) was prepared from the compound 323-2 prepared in Example 323 (1 g) in the same manner as Example 81.

$^1$H-NMR (DMSO-$d_6$) δ4.01 (s, 3H), 4.23 (d, J = 16 Hz, 1H), 4.55 (d, J = 16 Hz, 1H), 7.19-7.42 (m, 4H), 7.41 (d, J = 8.0 Hz, 1H), 7.62-7.88 (m, 7H), 8.01 (s, 1H), 10.5 (s, 1H).

Example 325:

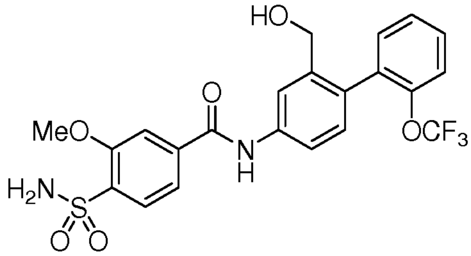

Using the compound 22-2 (200 mg) prepared in Reference Example 22 and the compound 21-3 (337 mg) prepared in Reference Example 21, the same reactions as those used in Example 311 were conducted, and then using 2-(trifluoromethoxy)phenylboronic acid, the same reactions as those used in Example 12 were conducted, and then the titled compound (137 mg) was prepared in the same manner as the second step in Example 323.
$^1$H-NMR (DMSO-$d_6$) δ4.03 (s, 3H), 4.25 (d, J = 4.0 Hz, d), 3.21 (t, J =4.0 Hz , 1H), 7.13-7.87 (m 11H), 7.96 (s, 1H), 10.4 (s, 1H).

Example 326:

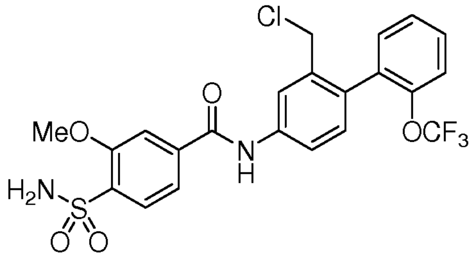

The titled compound (106 mg) was prepared from the compound (123 mg) prepared in Example 325 in the same manner as Example 81.
$^1$H-NMR (DMSO-$d_6$) δ4.01 (s, 3H), 4.39 (d, J = 12 Hz, 1H), 4.61 (d, J = 12 Hz, 1H), 7.24-7.88 (m, 11H), 8.03 (s, 1H), 10.5 (s, 1H).

Example 327:

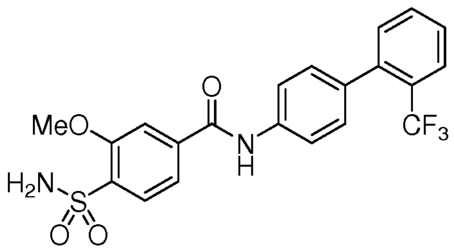

Using the compound 22-2 (152 mg) prepared in Reference Example 22 and 4-bromophenylaniline (169 mg) and conducting the reaction in the same manner as Example 311, and then using 2-(trifluoromethoxy)phenylboronic acid and treating it in the same manner as Example 12, the titled compound (42 mg) was prepared.
$^1$H-NMR (DMSO-$d_6$) δ3.89 (s, 3H), 7.00 (s, 2H), 7.40-8.26 (m, 11H), 10.6 (s, 1H).

Examples 328-330:

Using corresponding various amines and the compound prepared in Example 324, the compounds of the following Examples 328-330 were prepared in the same manner as Example 82.

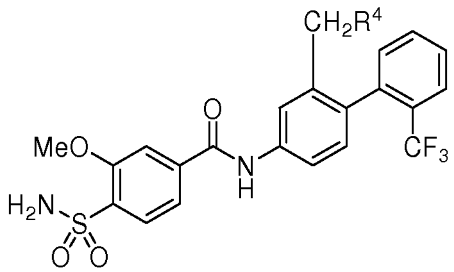

| Example | $CH_2R^4$ | $^1$H-NMR(DMSO-$d_6$) $\delta$ |
|---|---|---|
| 328 | 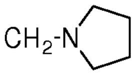 | 1.63(bs, 4H), 2.32(bs, 4H), 3.20-3.30(m, 5H), 4.01 (s, 3H), 7.09(d, J =8.0 Hz, 1H), 7.24-7.34(m, 3H), 7.60-7.90(m, 8H), 10.4(s, 1H). |
| 329 | 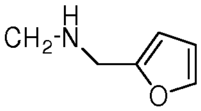 | 3.55(s, 2H), 4.01 (s, 3H), 6.10(d, J = 4.0 Hz, 1H9, 6.30(d, J = 4.0 Hz, 1H), 7.09(d, J = 8.0 Hz, 1H), 7.25-7.36(m, 3H), 7.47-7.94(m, 10H), 10.4(s, 1H). |
| 330 | 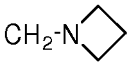 | 2.95-3.10(m, 6H), 3.99(s, 3H), 7.02-7.83(m, 10H). |

Example 331:

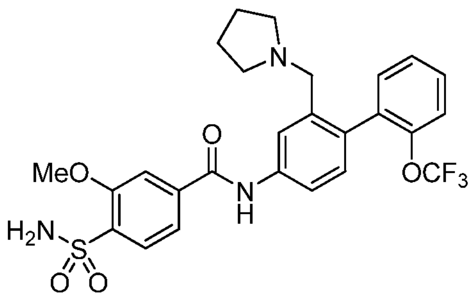

Using pyrrolidine and the compound prepared in Example 326, and treating them in the same manner as Example 82, the titled compound was prepared.

$^1$H-NMR (DMSO-$d_6$) $\delta$1.63 (bs, 4H), 2.31 (bs, 4H), 3.31 (s, 2H), 4.01 (s, 3H), 7.15 (d, J = 8.0 Hz, 1H), 7.24 (s, 2H), 7.41-7.92 (m, 9H), 10.4 (s, 1H).

Example 332:

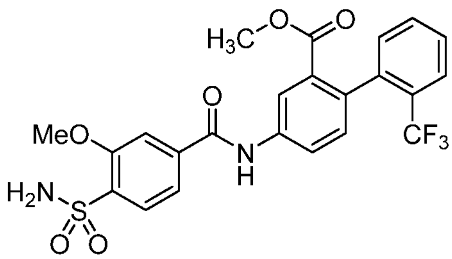

Using the compound 22-2 (100 mg) prepared in Reference Example 22 and 4-bromo-3-(methoxycarbonyl)phenylaniline (166 mg) and conducting the reactions in the same manner as Example 311, and then using 2-(trifluoromethoxy)phenylboronic acid and treating it in the same manner as Example 12, the titled compound (91 mg) was prepared.

$^1$H-NMR (DMSO-$d_6$) $\delta$3.55 (s, 3H), 4.01 (s, 3H), 7.26-7.31 (m, 4H), 7.56-7.88 (m, 5H), 8.08 (d, J = 12 Hz, 1H), 8.41 (s, 1H), 10.6 (s, 1H).

Example 333:

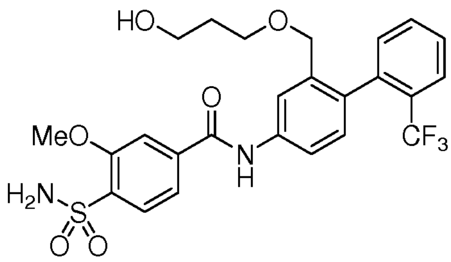

Using propylene glycol and the compound prepared in Example 327, and treating them in the same manner as Example 95, the titled compound was prepared.

Analyzing method SA2, $t_R$ 3.30 min, obs MS [M+1] 539.2

Example 334:

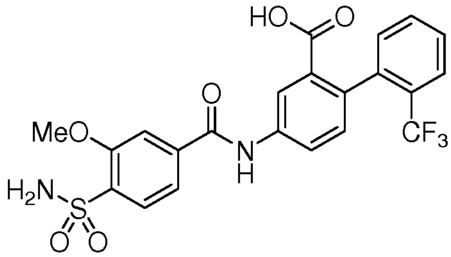

Using the compound prepared in Example 332 (20 mg) and treating it in the same manner as the second step in Example 323, the titled compound was prepared.

Analyzing method SA2, $t_R$ 3.21 min, obs MS [M+1] 495.3

Example 335:

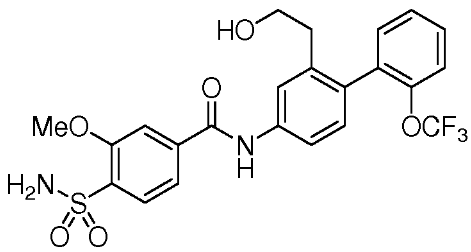

Using the compound 22-2 (200 mg) prepared in Reference Example 22 and the compound 25-3 (222 mg) prepared in Reference Example 25, the same reactions as those used in Example 311 were conducted, and then using 2-(trifluoromethoxy)phenylboronic acid, the same reactions as those used in Example 12 were conducted to give the titled compound (36 mg) in the same manner as the second step in Example 323.

$^1$H-NMR (DMSO-$d_6$) $\delta$2.49-2.56 (m, 2H), 3.41-3.46 (m, 2H), 4.00 (s, 3H), 4.62 (t, J = 4.0 Hz, 1H), 7.13 (d, J = 8.0 Hz, d), 7.25 (s, 2H), 7.39-7.87 (m, 9H), 10.4 (s, 1H).

Examples 336-338:

Using each of three compounds, 3-ethyl-4-bromoaniline, 3-methyl-4-bromoaniline or 3-methoxy-4-bromoaniline, obtained by brominating 3-ethylaniline, 3-methylaniline or 3-methoxyaniline, respectively, in the same manner as the third step in Reference Example 25, a condensation reaction with the compound 22-2 obtained in Reference Example 22 was conducted in the same manner as Example 311. Each of the resulting three compounds and 2-(N-morpholinomethyl)phenylboronic acid were treated in the same addition reaction as those in Example 12 to give the compounds of the following Examples 336-338.

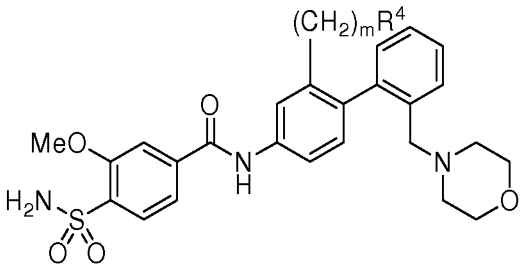

| Example | $(CH_2)_mR^4$ | $^1$H-NMR(DMSO-$d_6$) $\delta$ |
|---|---|---|
| 336 | Et | 1.00(t, J =8.0 Hz, 3H), 2.18-2.40(m, 6H), 3.18(bs, 2H), 3.49(bs, 4H), 4.01(s, 3H), 7.02-7.90 (m, 12H), 10.4(s, 1H). |
| 337 | Me | 1.99(s, 3H), 2.17(bs, 4H), 3.10-3.25(m, 2H), 3.48(bs, 4H), 4.00(s, 3H), 7.08(t, J = 8.0 Hz, 2H), 7.25(m, 10H), 10.3(s, 1H). |
| 338 | OMe | 2.17(bs, 4H), 3.10-3.30(m, 2H), 3.33(bs, 2H), 3.69(s, 3H), 4.01(s, 3H), 7.09(d, J = 8.0 Hz, 1H), 7.26-7.68(m, 9H), 7.87(d, J = 8.0 Hz, 1H), 10.4(s, 1H). |

Example 339:

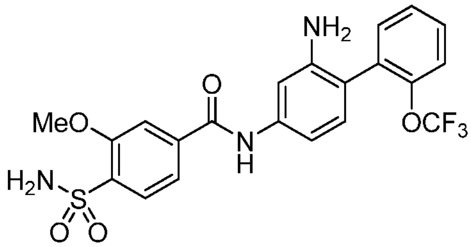

Using the compound 22-2 prepared in Reference Example 22 and 3-nitro-4-bromoaniline, the same reactions as those used in Example 311 were conducted. Then, after conducting in the same manner as the third step in Reference Example 21, the titled compound was prepared from 2-(trifluoromethoxy)phenylboronic acid by using the same reactions as those used in Example 12. $^1$H-NMR (DMSO-$d_6$) $\delta$4.00 (s, 3H), 4.73 (s, 2H), 6.87 (d, J = 12 Hz, 1H), 6.97 (d, J = 8.0 Hz, 1H), 7.24 (s, 3H), 7.39-7.87 (m, 7H), 10.2 (s, 1H).

Examples 340-347:

The compound 28 prepared in Reference Example 28 and the compound 29 prepared in Reference Example 29 were treated in the same manner as the first step in Reference Example 8. After the crude product was reacted with trifluoroacetic acid at room temperature, the reaction mixture was concentrated under reduced pressure, suspended in an aqueous solution of saturated sodium bicarbonate filtered, and dried under reduced pressure to give the following Examples 340. Using pyrrolidine or piperazine instead of morpholine in Reference Example 29, 5-fluoro-1,2-dibromobenzene or 4,5-difluoro-1,2-dibromobenzene instead of 1,2-dibromobenzene in Reference Example 29 and 2,6-dimethylmorpholine or cyclopentylamine instead of morpholine in Reference Example 29, each of the compounds of the following Examples 341-346 was prepared in the same manner as Example 340. In addition, using 2-trifluoromethyl-4-amino-bromobenzene instead of the compound of Reference Example 29, the compounds of the following Example 347 was prepared in the same manner as Example 340.

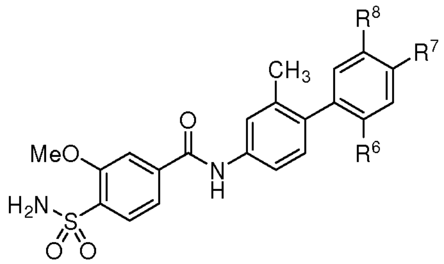

| Example | $R^6$ | $R^7$ | $R^8$ | $^1$H-NMR(DMSO-$d_6$) $\delta$ |
|---|---|---|---|---|
| 340 | 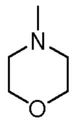 | H | H | 2.15(s, 3H), 2.50-2.77(m, 4H), 3.42(br, 4H), 4.01(s, 3H), 7.06-7.34(m, 7H), 7.61-7.69(m, 5H), 7.85(d, J = 8.0 Hz, 1H), 10.3(s, 1H). |
| 341 | 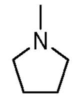 | H | H | |
| 342 | 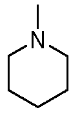 | H | H | 1.15-1.33(m, 6H), 2.13(s, 3H), 2.49-2.76(m, 4H), 4.01 (s, 3H), 7.03-7.30(m, 7H), 7.03-7.30(m, 4H), 7.85(d, J = 8.0 Hz, 1H), 10.3(s, 1H). |
| 343 | 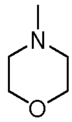 | H | F | 1.98(s, 3H), 2.62-2.73(m, 4H), 3.38(br, 4H), 4.00(s, 3H), 6.94 (dd, J = 12 Hz, 4.0 Hz, 5H), 7.05-7.24(m, 5H), 7.61-7.68(m, 4H), 7.85(d, J = 8.0 Hz, 1H), 10.3(s, 1H). |
| 344 | 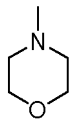 | F | F | 2.15(s, 3H), 2.63-2.73(m, 4H), 3.38(br, 4H), 4.00(s, 3H), 7.11-7.24(m, 5H), 7.60-7.68(m, 4H), 7.85(d, J = 8.0 Hz, 1H), 10.3(s, 1H). |
| 345 | 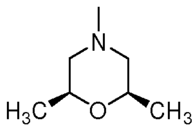 | H | H | 0.83(d, J = 8.0 Hz, 1H), 1.00(d, J = 8.0 Hz, 1H), 2.02-2.48(m, 5H), 2.70-2.90(m, 2H), 3.10-3.48(m, 2H), 4.00(s, 3H), 7.03-7.33(m, 7H), 7.57-7.86(m, 5H), 10.3(s, 1H). |
| 346 | 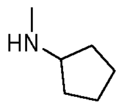 | H | H | 1.20-1.30(m, 2H), 1.50(br, 4H), 1.80-2.10(m, 5H), 3.38(br, 1H), 3.75(br, 1H), 4.00(s, 3H), 6.64-7.24(m, 7H), 7.60-7.87(m, 5H), 10.3(s, 1H). |
| 347 | $CF_3$ | $NH_2$ | H | 1.98(s, 3H), 4.00(s, 3H), 5.58(s, 2H), 6.76-7.05(m, 4H), 7.24(s, 2H), 7.55-7.65(m, 4H), 7.85(d, J =8.0 Hz, 1H), 10.3(s, 1H). |

Example 341:

Analyzing method SC2, $t_R$ 1.096min, obs MS [M+1] 466.4

Example 348:

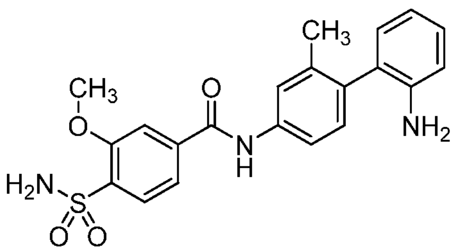

After the compound (150 mg) prepared in Reference Example 30 was reacted with trifluoroacetic acid (2 mL) at room

temperature, the mixture was concentrated under reduced pressure, and the resulting product was suspended in a saturated aqueous solution of sodium bicarbonate. The suspension was filtered, the resulting solution was dried under reduced pressure to give the titled compound (132 mg).
Analyzing method SC2, $t_R$ 0.835 min, obs MS [M+1] 412.7

Example 349:

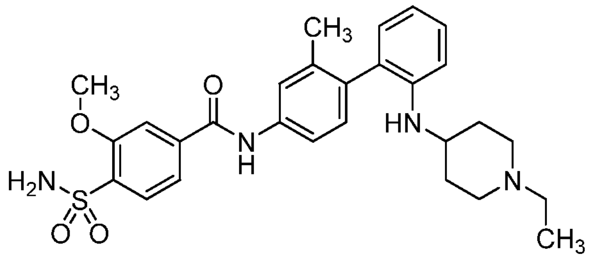

To the compound (50 mg) prepared in Example 348 in dichloroethane (500 μL) were added ethyl-4-piperidone (16.5 μL), acetic acid (7.0 μL) and sodium triacetoxyborohydride (36.5 mg), and the mixture was stirred for 4 hours at room temperature. A saturated aqueous solution of ammonium chloride was added to the reaction mixture which was extracted with ethyl acetate. The organic layer was dried over anhydrous magnesium sulfate, filtered, and then concentrated under reduced pressure. The resulting residue was purified with silica gel column chromatography (chloroform/methanol) to give the titled compound (9.7 mg).
Analyzing method SC2, $t_R$ 0.695 min, obs MS [M+1] 523.1

Examples 350-351:

Using the compound prepared in Example 348, tetrahydro-4H-pyran-4-one, and acetone or acetaldehyde, each of the compounds of the following Examples 350-352 was prepared in the same manner as Example 349.

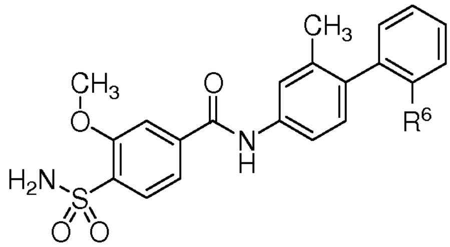

| Example | $R^6$ | obs MS [M+1] | $t_R$ (min) | Analytical Condition |
|---|---|---|---|---|
| 350 | 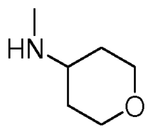 | 496.4 | 1.072 | SC2 |
| 351 | 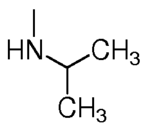 | 454.7 | 1.116 | SC2 |
| 352 | 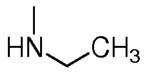 | 469.3 | 0.661 | SC2 |

Example 353:

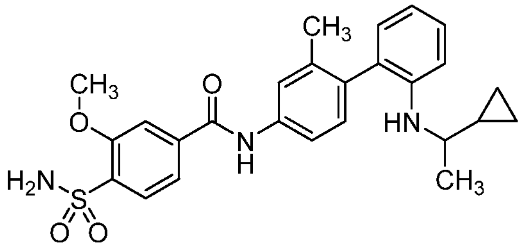

To the compound (200 mg) prepared in Example 348 in methanol (7.5 mL) were added cyclopropylmethylketone (225 μL), picoline borane (258 mg) and acetic acid (3.75 mL), the mixture was stirred for 29 hours at room temperature. 1N Aqueous solution of hydrochloric acid was added to the reaction mixture for neutralizing it, and then the mixture was extracted with ethyl acetate. The organic layer was dried over anhydrous magnesium sulfate, filtered, and then concentrated under reduced pressure. The resulting residue was purified with silica gel column chromatography (chloroform/methanol) to give the titled compound (17.2 mg).
Analyzing method SC2, $t_R$ 1.189 min, obs MS [M+1] 480.4

Examples 354-403:

Using the compounds prepared in Example 324 and Example 326 as well as corresponding various amines, and treating them in the same manner as Example 82, the compounds of the following Examples 354-403 were prepared, provided that under the same conditions as those used in Example 82 wherein N,N-diisopropylethylamine was used as a base, DMF or DMSO was used as a solvent, and the mixture was appropriately headed at 100°C without using a microwave, In addition, the compound of Example 373 was obtained as a byproduct in the preparation of the compound of Example 372.

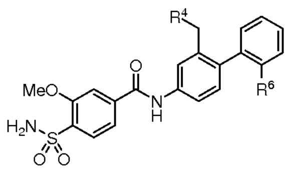

| Example | $R^4$ | $R^6$ | obs MS $[M+1]^+$ | $t_R$ (min) | Analytical Condition | Example | $R^4$ | $R^6$ | obs MS $[M+1]^+$ | $t_R$ (min) | Analytical Condition |
|---|---|---|---|---|---|---|---|---|---|---|---|
| 354 | 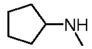 | $CF_3$ | 547.9 | 0.834 | SC1 | 379 | 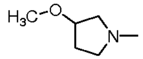 | $CF_3$ | 564.3 | 0.708 | SC2 |
| 355 | 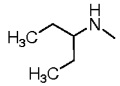 | $CF_3$ | 550.0 | 0.864 | SC1 | 380 | 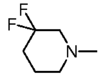 | $CF_3$ | 585.3 | 1.140 | SC2 |
| 356 | *i*-PrNH | $CF_3$ | 521.8 | 0.792 | SC1 | 381 | 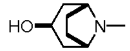 | $CF_3$ | 590.7 | 0.688 | SC2 |
| 357 | 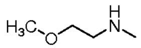 | $CF_3$ | 537.9 | 0.783 | SC1 | 382 | 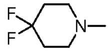 | $CF_3$ | 584.6 | 0.959 | SC2 |
| 358 | 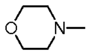 | $CF_3$ | 549.8 | 0.783 | SC1 | 383 | 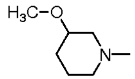 | $CF_3$ | 578.3 | 0.715 | SC2 |
| 359 | 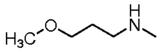 | $CF_3$ | 552.4 | 0.815 | SC1 | 384 | 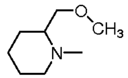 | $CF_3$ | 591.9 | 0.722 | SC2 |
| 360 | 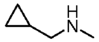 | $CF_3$ | 533.9 | 0.763 | SC1 | 385 | 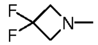 | $CF_3$ | 555.6 | 1.070 | SC2 |
| 361 | $Me_2N$ | $CF_3$ | 507.8 | 0.761 | SC1 | 386 | 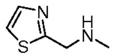 | $CF_3$ | 577.5 | 0.741 | SC2 |
| 362 | 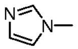 | $CF_3$ | 530.8 | 0.763 | SC1 | 387 | 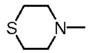 | $CF_3$ | 566.6 | 0.764 | SC2 |
| 363 | CN | $CF_3$ | 490.2 | 1.065 | SC1 | 388 | 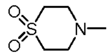 | $CF_3$ | 598.3 | 0.966 | SC2 |
| 364 | 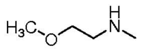 | $OCF_3$ | 553.9 | 0.795 | SC1 | 389 | 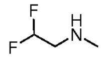 | $CF_3$ | 544.3 | 0.789 | SC2 |
| 365 | 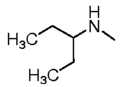 | $OCF_3$ | 566.4 | 1.169 | SC3 | 390 | 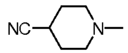 | $CF_3$ | 573.6 | 0.690 | SC2 |
| 366 | *n*-BuNH | $CF_3$ | 535.9 | 0.818 | SC1 | 391 | 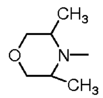 | $CF_3$ | 578.4 | 0.808, 0.914 | SC2 |
| 367 | 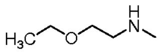 | $CF_3$ | 551.9 | 0.804 | SC1 | | | | | | |
| 368 | 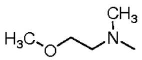 | $CF_3$ | 551.9 | 0.718 | SC2 | 392 | 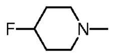 | $CF_3$ | 565.9 | 0.687 | SC2 |
| 369 | 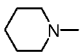 | $CF_3$ | 547.9 | 0.725 | SC2 | 393 | 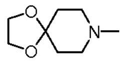 | $CF_3$ | 606.0 | 0.726 | SC2 |
| 370 | $NH_2$ | $CF_3$ | 479.9 | 0.700 | SC2 | 394 | 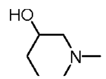 | $CF_3$ | 564.0 | 0.658 | SC2 |

(continued)

| Example | R4 | R6 | obs MS [M+1]+ | $t_R$ (min) | Analytical Condition | Example | R4 | R6 | obs MS [M+1]+ | $t_R$ (min) | Analytical Condition |
|---|---|---|---|---|---|---|---|---|---|---|---|
| 371 | 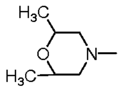 | $CF_3$ | 578.4 | 0.839 | SC2 | 395 | 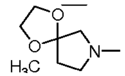 | $CF_3$ | 592.7 | 0.707 | SC2 |
| 372 | 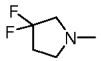 | $CF_3$ | 570.3 | 3.77 | SA2 | 396 | 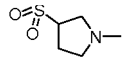 | $CF_3$ | 612.1 | 0.765 | SC2 |
| 373 | EtO | $CF_3$ | 509.3 | 4.38 | SA2 | 397 | 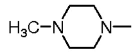 | $CF_3$ | 562.9 | 0.701 | SC2 |
| 374 | 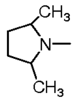 | $CF_3$ | 562.5 | 0.708 | SC2 | 398 | 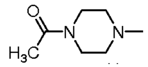 | $CF_3$ | 591.7 | 0.765 | SC2 |
| 375 | 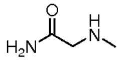 | $CF_3$ | 537.6 | 0.651 | SC2 | 399 | 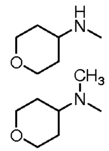 | $CF_3$ | 564.1 | 0.664 | SC2 |
| 376 | 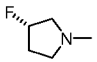 | $CF_3$ | 552.0 | 0.691 | SC2 | 400 | | $CF_3$ | 578.4 | 0.654 | SC2 |
| | | | | | | 401 | 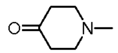 | $CF_3$ | 562.3 | 0.657 | SC2 |
| 377 | 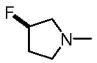 | $CF_3$ | 551.9 | 0.681 | SC2 | 402 | 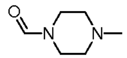 | $CF_3$ | 577.4 | 0.788 | SC2 |
| 378 | 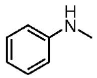 | $CF_3$ | 556.2 | 1.185 | SC2 | 403 | 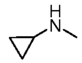 | $CF_3$ | 520.7 | 0.683 | SC2 |

Example 404:
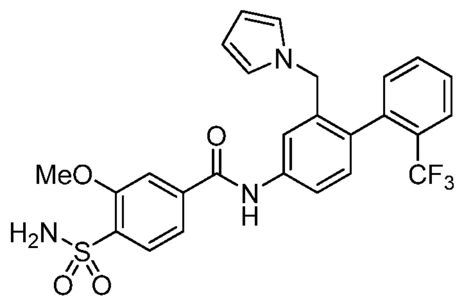

To a suspension of pyrrol (22 mg) in DMF (1.5 mL) was added sodium hydroxide at 0°C, the mixture was stirred for 20 minutes at 0°C, and after adding the compound prepared in Example 324 (30 mg) thereto, the mixture was further stirred for 2 hours at room temperature. A 1N aqueous solution of citric acid was added thereto, and the mixture was extracted with ethyl acetate. The organic layer was washed with saturated brine, dried over anhydrous sodium sulfate. The reaction mixture was filtered and concentrated under reduced pressure. The resulting product was purified with silica gel column chromatography (chloroform/methanol) to give the titled compound (24 mg).
Analyzing method SC2, $t_R$ 1.168 min, obs MS [M+1] 530.3
Examples 405-410
The compounds of the following Examples 405-410 were prepared in the same manner as Example 404.
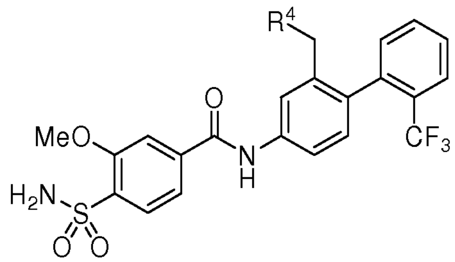

| Example | $R^4$ | obs MS $[M+1]^+$ | $t_R$ (min) | Analytical Condition |
|---|---|---|---|---|
| 405 | 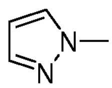 | 531.1 | 1.040 | SC2 |
| 406 | 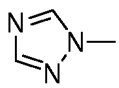 | 532.2 | 0.917 | SC2 |
| 407 | 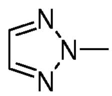 | 532.2 | 1.033 | SC2 |
| 408 | 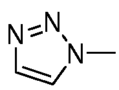 | 532.2 | 0.943 | SC2 |
| 409 | 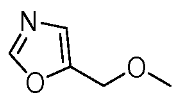 | 562.3 | 1.005 | SC2 |
| 410 | 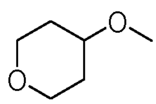 | 563.0 $[M-H]^-$ | 1.113 | SC2 |

Examples 411-412:

Using the compound 32-4 prepared in Reference Example 32 and treating it with trifluoroacetic acid at room temperature in the same manner as Example 348, the compound of the following Example 411 was prepared. In addition, the compound 32-4 prepared in Reference Example 32 was firstly methylated in the same manner as the second step in Reference Example 8, and then it was also treated with trifluoroacetic acid to give the compound of the following Example 412.

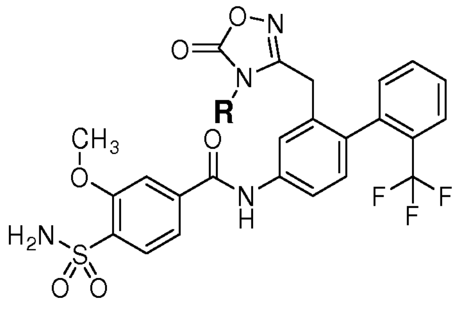

| Example | R | obs MS [M+1] | $t_R$ (min) | Analytical Condition |
|---|---|---|---|---|
| 411 | H | 549.3 | 4.3 | SA2 |
| 412 | $CH_3$ | 563.3 | 4.32 | SA2 |

Example 413:

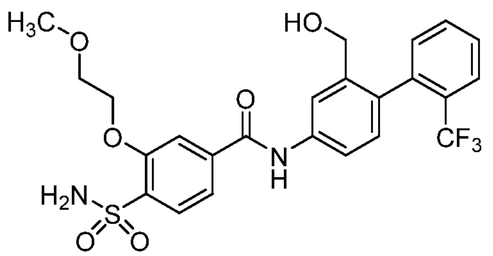

Using the compound 33-4 prepared in Reference Example 33 and treating it with trifluoroacetic acid at room temperature, the compound of Example 413 was prepared.

Analyzing method SC2, $t_R$ 0.951 min, obs MS [M+1] 525.4

Example 414:

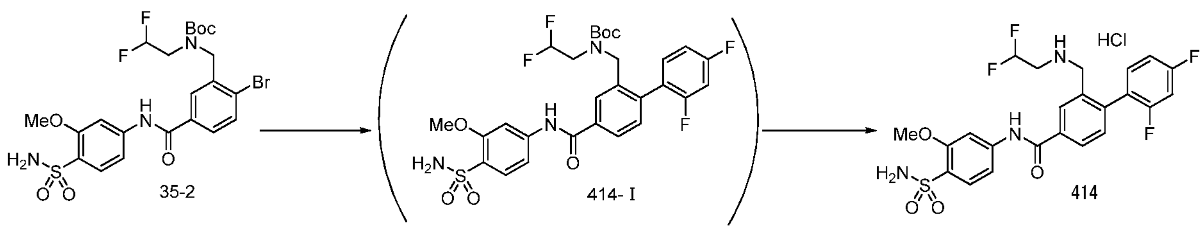

35-2 414- I 414

To the compound 35-2 (1 g) prepared in Reference Example 35 were added 2,4-difluorophenylboronic acid (546 mg), potassium phosphate (1.1 g), palladium acetate (70 mg), 2-dicyclohexylphosphino-2',6'-dimethoxybiphenyl (SPhos) (142 mg) and THF (13 mL), the mixture was stirred for 10 minutes at room temperature. Water (0.8 mL) was added thereto and the mixture was stirred for 1 hour at 90°C under microwave irradiation using a microwave reaction

apparatus (Initiator 60 EXP (400W), Biotage). After cooling to room temperature, the mixture was filtered through Celite and concentrated under reduced pressure. The resulting residue was purified with silica gel column chromatography (chloroform/methanol) to give Boc-protected compound 414-1 (1.06 g). 4N-HCl/dioxane was added to the compound 414-I (1.06 g), and the mixture was stirred for 2 hours at room temperature. Hexane (20 mL) was added thereto, and the mixture was further stirred for 1 hours, and then the precipitated solid was collected by filtration, dried under reduced pressure to give the titled compound (840 mg).
Analyzing method SB 1, $t_R$ 2.96 min, obs MS [M+1] 512.2

Examples 415-437:

The compounds of Examples 415-437 were prepared in the same manner as Example 414.

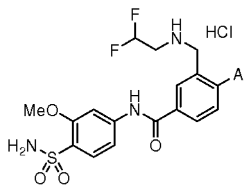

| Example | A | obs MS $[M+1]^+$ | $t_R$ (min) | Analytical Condition | Example | A | obs MS $[M+1]^+$ | $t_R$ (min) | Analytical Condition |
|---|---|---|---|---|---|---|---|---|---|
| 415 | 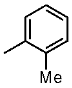 | 490.4 | 2.98 | SB1 | 426 | 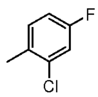 | 528.3 | 3.65 | SB1 |
| 416 | 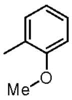 | 506.3 | 2.90 | SB1 | 427 | 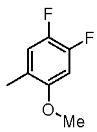 | 542.2 | 3.07 | SB1 |
| 417 | 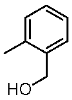 | 506.2 | 2.74 | SB1 | 428 | 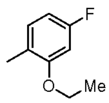 | 538.3 | 3.17 | SB1 |
| 418 | 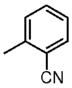 | 501.3 | 2.77 | SB1 | 429 | 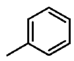 | 476.2 | 3.03 | SB1 |
| 419 | 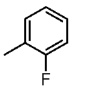 | 494.5 | 2.87 | SB1 | 430 | 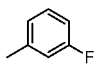 | 494.5 | 3.12 | SB1 |
| 420 | 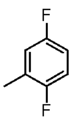 | 506.2 | 2.74 | SB1 | 431 | 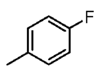 | 494.5 | 3.11 | SB1 |
| 421 | 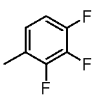 | 530.2 | 3.07 | SB1 | 432 | 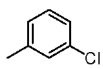 | 510.4 | 3.24 | SB1 |
| | | | | | 433 | 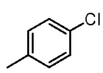 | 510.4 | 3.26 | SB1 |
| 422 | 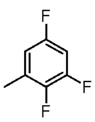 | 530.2 | 3.03 | SB1 | 434 | 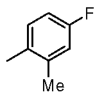 | 562.9 | 0.701 | SB1 |
| 423 | 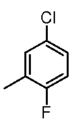 | 528.3 | 3.08 | SB1 | 435 | 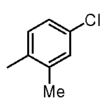 | 524.3 | 3.25 | SB1 |

(continued)

| Example | A | obs MS $[M+1]^+$ | $t_R$ (min) | Analytical Condition | Example | A | obs MS $[M+1]^+$ | $t_R$ (min) | Analytical Condition |
|---|---|---|---|---|---|---|---|---|---|
| 424 | 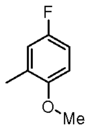 | 524.3 | 2.97 | SB1 | 436 | 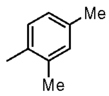 | 504.3 | 3.18 | SB1 |
| 425 | 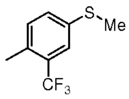 | 590.1 | 3.31 | SB1 | 437 | 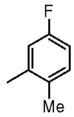 | 508.4 | 3.08 | SB1 |

Example 438:

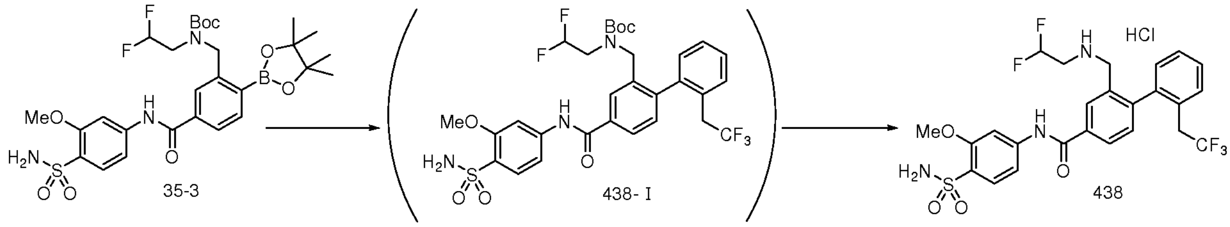

To the compound 35-3 (100 mg) prepared in Reference Example 35 were added 1-bromo-2-(2,2,2-trifluoroethyl)-benzene (57 mg), potassium phosphate (68 mg), palladium acetate (3.6 mg), 2-dicyclohexylphosphino-2',6'-dimethoxybiphenyl (SPhos) (13 mg) and THF (1.6 mL), the mixture was stirred for 10 minutes at room temperature. Water (0.16 mL) was added thereto and the mixture was stirred for 1 hour at 90°C under microwave irradiation using a microwave reaction apparatus (Initiator 60 EXP (400W), Biotage). After cooling to room temperature, the solution was filtered through Celite, and concentrated under reduced pressure. The resulting residue was purified with silica gel column chromatography (chloroform/methanol) to give Boc-protected compound 438-I. 4N-HCl/dioxane (1 mL) was added to the compound 438-I, and the mixture was stirred for 2 hours at room temperature. After concentration under reduced pressure, diethylether and hexane were added thereto, and the precipitated solid was collected by filtration to give the titled compound (25 mg). Analyzing method SB 1, $t_R$ 3.18 min, obs MS [M+1] 558.2

Examples 439-444:

The compounds of Examples 439-444 were prepared in the same manner as Example 438.

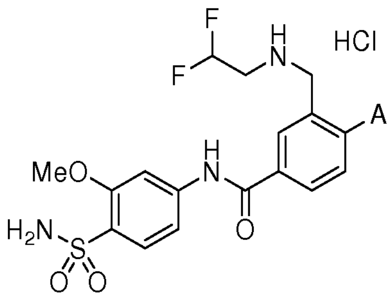

| Example | A | obs MS $[M+1]^+$ | $t_R$ (min) | Analytical Condition |
|---|---|---|---|---|
| 439 | 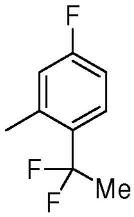 | 558.2 | 3.10 | SB1 |
| 440 | 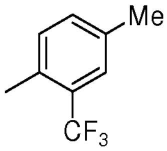 | 558.2 | 3.35 | SB1 |
| 441 | 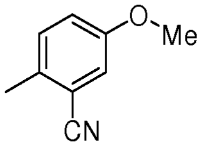 | 531.2 | 3.09 | SB1 |
| 442 | 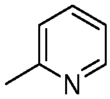 | 477.3 | 2.85 | SB1 |
| 443 | 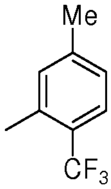 | 558.3 | 3.41 | SB1 |
| 444 | 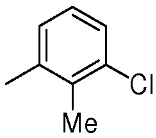 | 504.3 | 3.24 | SB1 |

Examples 445-466:

The compounds of the following Examples 445-466 were prepared in the same manner as Example 414.

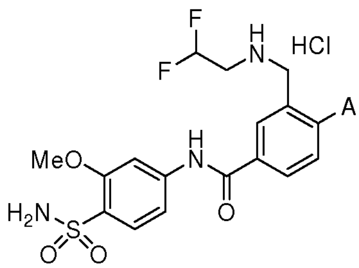

| Example | A | obs MS [M+1] | $t_R$ (min) | Analytical Condition | Example | A | obs MS [M+1] | $t_R$ (min) | Analytical Condition |
|---|---|---|---|---|---|---|---|---|---|
| 445 | 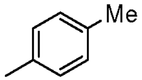 | 490.4 | 3.19 | SB1 | 456 | 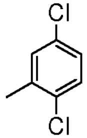 | 544.1 | 3.32 | SB1 |
| 446 | 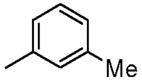 | 490.4 | 3.18 | SB1 | 457 | 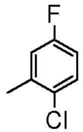 | 528.3 | 3.08 | SB1 |
| 447 | 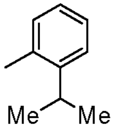 | 518.4 | 1.21 | SC2 | 458 | 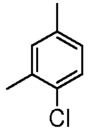 | 524.3 | 3.18 | SB1 |
| 448 | 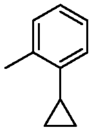 | 516.4 | 3.30 | SB1 | 459 | 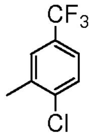 | 578.0 | 3.47 | SB1 |
| 449 | 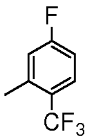 | 562.1 | 3.15 | SB1 | 460 | 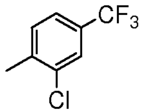 | 578.1 | 3.38 | SB1 |
| 450 | 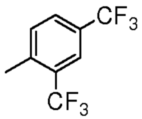 | 612.2 | 3.42 | SB1 | 461 | 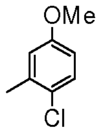 | 540.4 | 3.24 | SB1 |
| 451 | 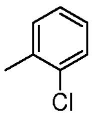 | 510.4 | 1.063 | SC2 | 462 | 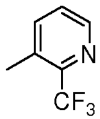 | 545.4 | 2.89 | SB1 |
| 452 | 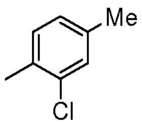 | 524.3 | 3.19 | SB1 | 463 | 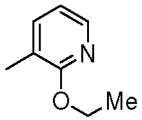 | 521.6 | 2.82 | SB1 |

(continued)

| Example | A | obs MS [M+1] | $t_R$ (min) | Analytical Condition | Example | A | obs MS [M+1] | $t_R$ (min) | Analytical Condition |
|---|---|---|---|---|---|---|---|---|---|
| 453 | 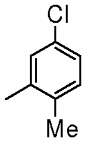 | 524.3 | 3.21 | SB1 | 464 | 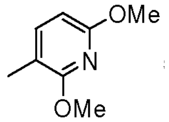 | 537.2 | 2.99 | SB1 |
| 454 | 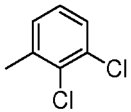 | 544.1 | 3.34 | SB1 | 465 | 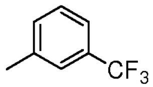 | 544.3 | 3.23 | SB1 |
| 455 | 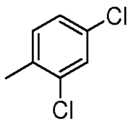 | 544.2 | 3.26 | SB1 | 466 | 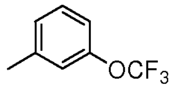 | 560.3 | 3.28 | SB1 |

Examples 467-486:

The compounds of the following Examples 467-486 were prepared in the same manner as Example 438.

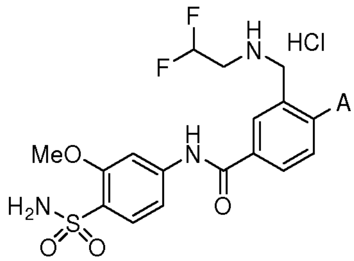

| Example | A | obs MS [M+1] | $t_R$ (min) | Analytical Condition | Example | A | obs MS [M+1] | $t_R$ (min) | Analytical Condition |
|---|---|---|---|---|---|---|---|---|---|
| 467 | 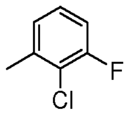 | 528.3 | 3.10 | SB1 | 477 | 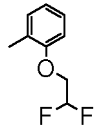 | 556.2 | 3.04 | SB1 |
| 468 | 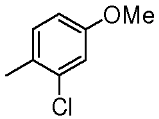 | 540.3 | 3.24 | SB1 | 478 | 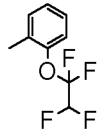 | 592.3 | 1.179 | SC2 |
| 469 | 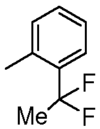 | 540.3 | 1.096 | SC2 | 479 | 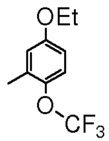 | 604.1 | 3.40 | SB1 |
| 470 | 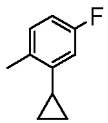 | 534.2 | 3.39 | SB1 | 480 | 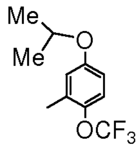 | 618.2 | 3.49 | SB1 |
| 471 | 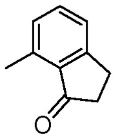 | 530.3 | 3.16 | SB1 | 481 | 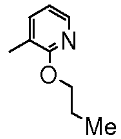 | 535.3 | 2.98 | SB1 |
| 472 | 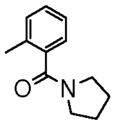 | 573.4 | 2.95 | SB1 | 482 | 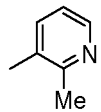 | 491.2 | 2.54 | SB1 |
| 473 | 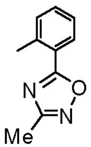 | 558.2 | 2.94 | SB1 | 483 | 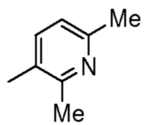 | 505.3 | 2.54 | SB1 |
| 474 | 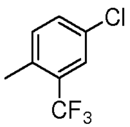 | 578.1 | 3.34 | SB1 | 484 | 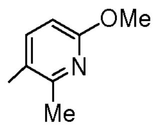 | 521.4 | 2.65 | SB1 |

(continued)

| Example | A | obs MS [M+1] | $t_R$ (min) | Analytical Condition | Example | A | obs MS [M+1] | $t_R$ (min) | Analytical Condition |
|---|---|---|---|---|---|---|---|---|---|
| 475 | 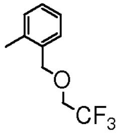 | 588.2 | 3.22 | SB1 | 485 | 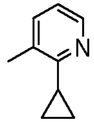 | 517.5 | 2.78 | SB1 |
| 476 | 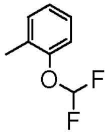 | 542.0 | 3.00 | SB1 | 486 | 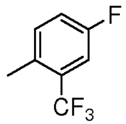 | 562.2 | 3.88 | SA3 |

Examples 487-496:

Using the compound 21-3 of Reference Example 21 and the compound 22-2 of Reference Example 22, and treating them in the same manner as the fifth and sixth steps in Reference Example 12 or as Example 323, and then treating them in the same manner as the first and second steps in Reference Example 35, and further treating the resulting compound in the same mannner as Example 414, the compounds of the following Examples were prepared.

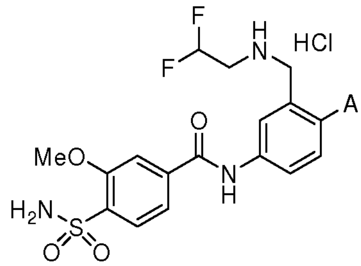

| Example | A | obs MS [M+1] | $t_R$ (min) | Analytical Condition | Example | A | obs MS [M+1] | $t_R$ (min) | Analytical Condition |
|---|---|---|---|---|---|---|---|---|---|
| 487 | 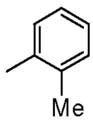 | 490.4 | 3.07 | SB1 | 492 | 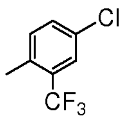 | 578.3 | 3.37 | SB1 |
| 488 | 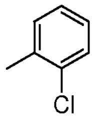 | 510.3 | 3.08 | SB1 | 493 | 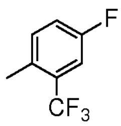 | 562.2 | 3.26 | SB1 |
| 489 | 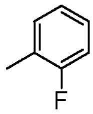 | 494.4 | 2.98 | SB1 | 494 | 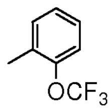 | 561.3 | 1.108 | SC2 |
| 490 | 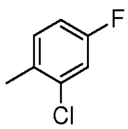 | 528.3 | 3.16 | SB1 | 495 | 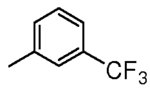 | 544.3 | 3.26 | SB1 |
| 491 | 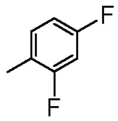 | 512.3 | 3.06 | SB1 | 496 | 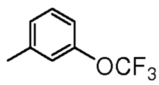 | 560.3 | 3.31 | SB1 |

Example 497:

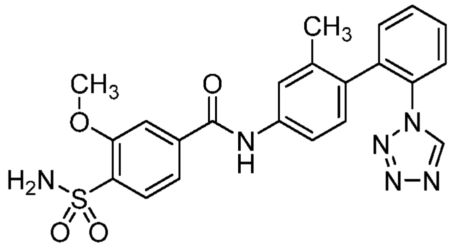

To the compound 30 (120 mg) prepared in Reference Example 30 were added triethyl orthoformate (1.27 mL), sodium azide (166 mg) and acetic acid (300 μL), and the mixture was stirred for 2 days at 80°C. Water was added to the reaction mixture, which was extracted with ethyl acetate. The organic layer was dried over anhydrous magnesium sulfate, filtered,

and then concentrated under reduced pressure. The resulting residue was purified with silica gel column chromatography (chloroform/methanol). The crude product was reacted with trifluoroacetic acid (2 mL) at room temperature, and then the mixture was concentrated under reduced pressure. The resulting residue was purified with silica gel column chromatography (chloroform/methanol) to give the titled compound (34.8 mg).
Analyzing method SC2, $t_R$ 0.811 min, obs MS [M+1] 465.3

Examples 498-499:

Using the compound 31 (60 mg) prepared in Reference Example 31 and 2-trifluoromethylphenylboronic acid (75 mg) or 2-trifluoromethoxyphenylboronic acid (82 mg), treating them in the same manner as the first step of Reference Example 8, the compounds of the following Example 498 (47.6 mg) and Example 499 (60.8 mg) were prepared, respectively.

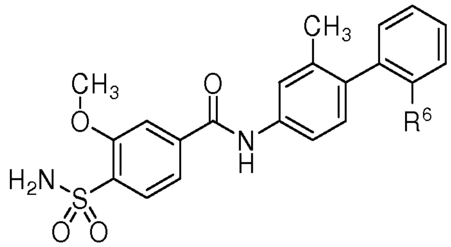

| Example | $R^6$ | obs MS [M+1] | $t_R$ (min) | Analytical Condition |
|---|---|---|---|---|
| 498 | $CF_3$ | 465.4 | 1.086 | SC2 |
| 499 | $OCF_3$ | 481.3 | 1.117 | SC2 |

Example 500-Example 527:

Using the compound of Example 133 prepared in the same manner as Example 81 and various amines, treating them in the same manner as Example 82 wherein N,N-diisopropylethylamine or sodium hydroxide was used as a base and DMF was used as a solvent or in the absence of a solvent, by appropriately heating at 70-100°C without microwave irradiation, the compounds of the following Examples 500-527 were prepared.

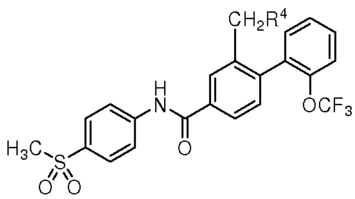

| Example | $CH_2R^4$ | obs MS [M+1]$^+$ | $t_R$ (min) | Analytical Condition | Example | $CH_2R^4$ | obs MS [M+1]$^+$ | $t_R$ (min) | Analytical Condition |
|---|---|---|---|---|---|---|---|---|---|
| 500 | 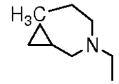 | 561.6 | 4.24 | SD2 | 514 | 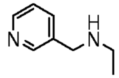 | 556.0 | 3.40 | SD2 |
| 501 | 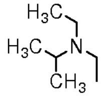 | 535.4 | 4.00 | SD2 | 515 | 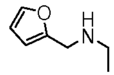 | 545.0 | 3.64 | SD2 |
| 502 | 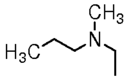 | 521.4 | 3.90 | SD2 | 516 | 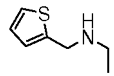 | 561.0 | 3.77 | SD2 |
| 503 | 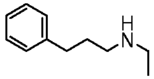 | 583.6 | 3.72 | SD2 | 517 | 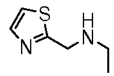 | 562.0 | 3.50 | SD2 |
| 504 | 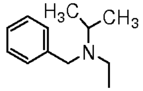 | 597.4 | 2.43 | SD2 | 518 | 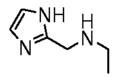 | 545.0 | 3.20 | SD2 |
| 505 | 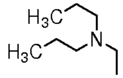 | 549.4 | 4.34 | SD2 | 519 | 9 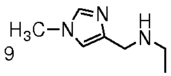 | 559.6 | 3.10 | SD2 |
| 506 | 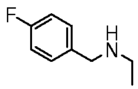 | 573.3 | 1.96 | SD2 | 520 | 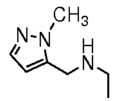 | 559.0 | 3.38 | SD2 |
| 507 | 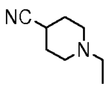 | 558.0 | 2.78 | SD1 | 521 | 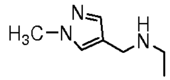 | 559.6 | 3.14 | SD2 |
| 508 | 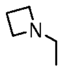 | 505.0 | 3.36 | SD2 | 522 | 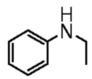 | 541.0 | 3.82 | SD2 |
| 509 | 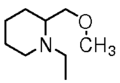 | 577.0 | 3.97 | SD2 | 523 | 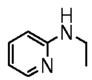 | 542.0 | 2.98 | SD2 |
| 510 | 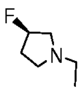 | 537.0 | 3.75 | SD2 | 524 | 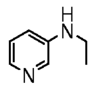 | 542.0 | 2.97 | SD2 |
| 511 | 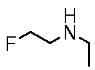 | 511.0 | 3.50 | SD2 | 525 | 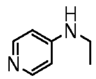 | 542.0 | 2.96 | SD2 |
| 512 | 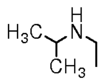 | 507.0 | 3.30 | SD2 | 526 | 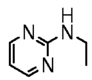 | 543.0 | 3.50 | SD2 |

(continued)

| Example | $CH_2R^4$ | obs MS $[M+1]^+$ | $t_R$ (min) | Analytical Condition | Example | $CH_2R^4$ | obs MS $[M+1]^+$ | $t_R$ (min) | Analytical Condition |
|---|---|---|---|---|---|---|---|---|---|
| 513 | 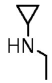 | 505.0 | 3.65 | SD2 | 527 | 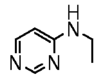 | 543.0 | 2.95 | SD2 |

Example 528-Example 529:

Using 2,2,2-trifluoroethylamine and treating it in the same manner as Example 354-403, the compounds of the following Examples 528-529 were prepared.

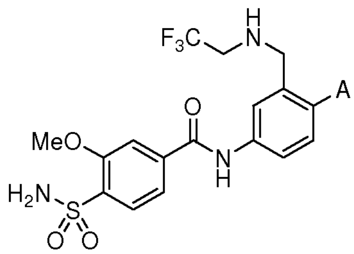

| Example | A | obs MS [M+1] | $t_R$ (min) | Analytical Condition | Example | A | obs MS [M+1] | $t_R$ (min) | Analytical Condition |
|---|---|---|---|---|---|---|---|---|---|
| 528 | 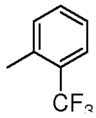 | 562.3 | 1.477 | SC2 | 529 | 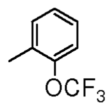 | 578.3 | 1.575 | SC2 |

Example 530:

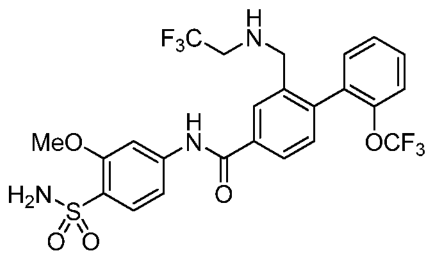

Using the compound of Example 254 and 2,2,2-trifluoroethylamine, and treating them in the same manner as Example 71, the compound of Example 530 was prepared.

Analyzing method SC2, $t_R$ 1.427 min, obs MS [M+1] 578.4

Examples 531-537:

Using the compound 17 (100 mg) prepared in Reference Example 17 and the compound 36 (75.9 mg) prepared in Reference Example 36, and treating them in the same manner as Examples 271-273, the compound of Example 531 (59.4 mg) was prepared. Using the compound 17 (100 mg) prepared in Reference Example 17 and the compound 37 (102.3 mg) prepared in Reference Example 37, the compound of Example 532 (25.5 mg) was prepared. In the same manner, the compounds of Examples 533-537 were prepared.

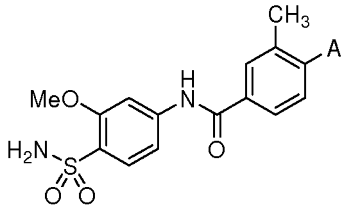

| Example | A | obs MS [M+1] | $t_R$ (min) | Analytical Condition | Example | A | obs MS [M+1] | $t_R$ (min) | Analytical Condition |
|---|---|---|---|---|---|---|---|---|---|
| 531 | 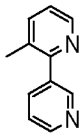 | 475.1 | 3.16 | SA2 | 535 | 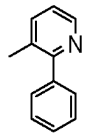 | 474.3 | 1.178 | SC2 |
| 532 | 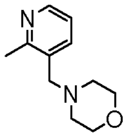 | 497.4 | 0.588 | SC2 | 536 | 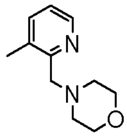 | 497.7 | 0.562 | SC2 |
| 533 | 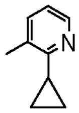 | 438.5 | 4.08 | SB1 | 537 | 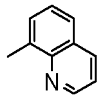 | 448.7 | 1.179 | SC2 |
| 534 | 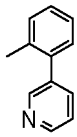 | 474.7 | 0.915 | SC2 | | | | | |

Example 538:

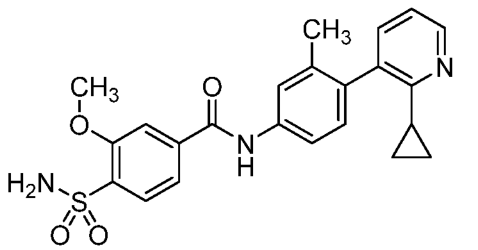

Using the compound 28 (100 mg) prepared in Reference Example 28 and treating it in the same manner as Examples 271-273, the titled compound (45.7 mg) was prepared.

Analyzing method SC2, $t_R$ 0.938 min, obs MS [M+1] 438.0

Examples 539-544:

Using the compound 38-1 prepared in Reference Example 38 and treating it in the same manner as Example 414, the compounds of Examples 539-541 were prepared.

Using the compound 38-2 prepared in Reference Example 38 and treating it in the same manner as Example 438, the compounds of Examples 542-544 were prepared.

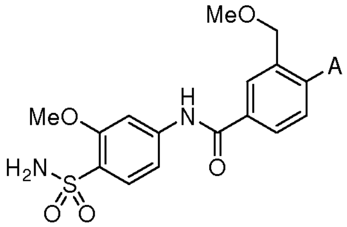

| Example | A | obs MS [M+1] | $t_R$ (min) | Analytical Condition | Example | A | obs MS [M+1] | $t_R$ (min) | Analytical Condition |
|---|---|---|---|---|---|---|---|---|---|
| 539 | 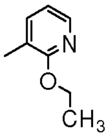 | 472.4 | 3.63 | SB1 | 542 | 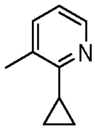 | 468.3 | 4.03 | SB1 |
| 540 | 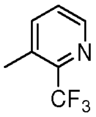 | 496.1 | 3.85 | SB1 | 543 | 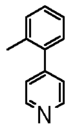 | 504.3 | 4.44 | SB1 |
| 541 | 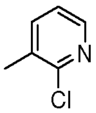 | 462.1 | 1.136 | SC2 | 544 | 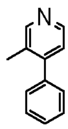 | 504.3 | 4.56 | SB1 |

Examples 545-546:

Using the compound 21-3 of Reference Example 21 and the compound 22-2 of Reference Example 22, and treating them in the same manner as the fifth and sixth steps in Reference Example 12 or Example 323, and then treating in the same manner in Reference Example 38 and Examples 542-544, the compounds of Examples 545-546 were prepared.

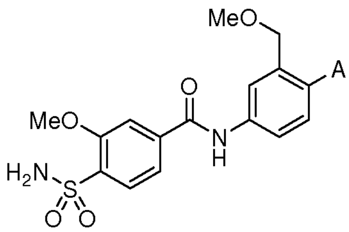

| Example | A | obs MS [M+1] | $t_R$ (min) | Analytical Condition | Example | A | obs MS [M+1] | $t_R$ (min) | Analytical Condition |
|---|---|---|---|---|---|---|---|---|---|
| 545 | 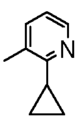 | 468.3 | 3.90 | SB1 | 546 | 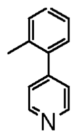 | 504.3 | 4.53 | SB1 |

Example 547:

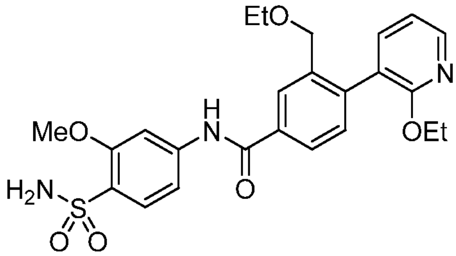

Using sodium ethoxide instead of sodium methoxide and treating it in the same manner as Reference Example 38, and then using 2-ethoxypyridine-3-phenylboronic acid and treating it in the same manner as Example 414, the titled compound was prepared.

Examples 548-549:

Using 4-bromo-3-methoxybenzoic acid and the compound 3-3 of Reference Example 3, and treating them in the same manner as the fifth step in Reference Example 12, and then treating in the same manner as the second step in Reference Example 38 as well as the same manner as Examples 542-544, the compounds of Examples 548-549 were prepared.

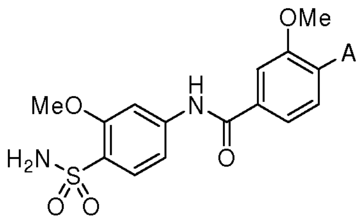

| Example | A | obs MS [M+1] | $t_R$ (min) | Analytical Condition | Example | A | obs MS [M+1] | $t_R$ (min) | Analytical Condition |
|---|---|---|---|---|---|---|---|---|---|
| 548 | 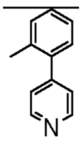 | 490.4 | 4.78 | SB1 | 549 | 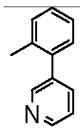 | 490.3 | 1.025 | SC2 |

Example 550

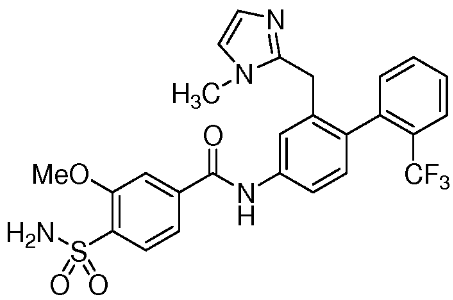

The compound 39-6 (11 mg) prepared in Reference Example 39 was stirred in TFA (1 mL) for 3 hours at room temperature. After finishing the reaction, the reaction mixture was concentrated under reduced pressure, and then ethyl acetate (1 mL) and triethylamine (1 mL) were added thereto, and the mixture was concentrated under reduced pressure. The resulting residue was purified with silica gel column chromatography (chloroform/methanol) to give the titled compound (2.2 mg).

Analyzing method SC2, $t_R$ 0.665 min, obs MS [M+1] 545.3

Example 551

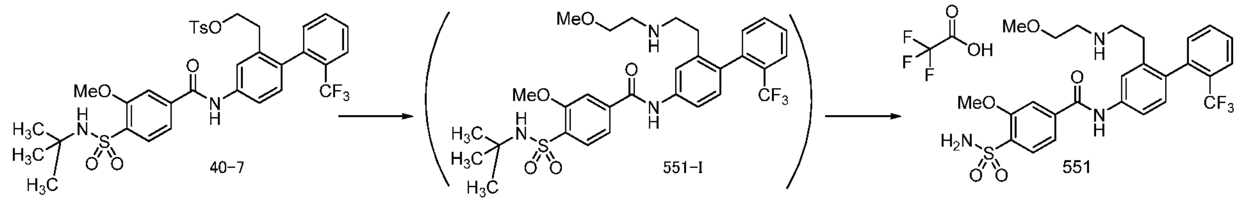

To the compound 40-7 (210 mg) prepared in Reference Example 40 in ethanol (2.5 mL) were added potassium carbonate (412 mg) and 2-methoxyethylamine (260 μL), and the mixture was stirred for 30 minutes at 150°C under microwave irradiation. The reaction mixture was filtered and concentrated under reduced pressure. The resulting residue was purified with silica gel column chromatography (chloroform/methanol) to give the compound 551-I (149 mg). Trifluoroacetic acid (TFA) (3 mL) was added to the compound 551-I (100 mg) and the mixture was stirred for 4 hours at room temperature. The reaction mixture was concentrated under reduced pressure, and then chloroform was added thereto, and the mixture was concentrated under reduced pressure. Chloroform was added to the residue, and the mixture was again concentrated under reduced pressure, a solid crystallized by adding diethylether was collected by filtration to give the titled compound 551 (87 mg).

The compound 551-I: Analyzing method SC2, $t_R$ 0.93 min, obs MS [M+1] 608.7

The compound 551: Analyzing method SC2, $t_R$ 0.73 min, obs MS [M+1] 552.6

Example 552

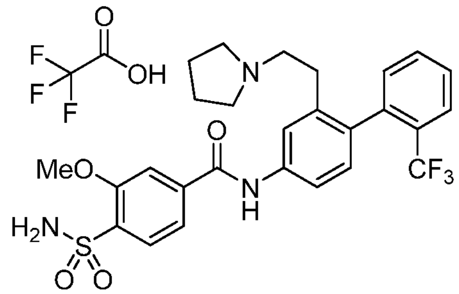

The titled compound was prepared in the same manner as Example 61.

Analyzing method SC2, $t_R$ 0.71 min, obs MS [M+1] 548.7

Example 553

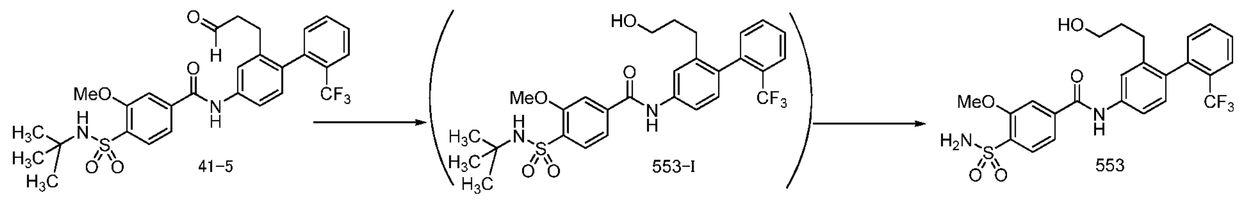

To the compound 41-5 (46 mg) prepared in Reference Example 41 in methanol (2 mL) was added sodium borohydride (16 mg) at 0°C and the mixture was stirred for 30 minutes at 0°C. Water and a saturated aqueous solution of ammonium chloride were added thereto, and the mixture was extracted with ethyl acetate. The organic layer was washed with saturated brine, dried over sodium sulfate, filtered, and concentrated under reduced pressure. The residue was purified with silica gel column chromatography (hexane/ethyl acetate) to give the compound 553-I (38 mg). Trifluoroacetic acid (TFA) (1.5 mL) was added to the compound 553-I (38 mg), the mixture was stirred for 4 hours at room temperature. The reaction mixture was concentrated under reduced pressure, and methanol (1 mL) and 4M aqueous solution of sodium hydroxide (1 mL) were added thereto, and then, the mixture was stirred for 40 minutes at room

temperature. 1M aqueous solution of hydrochloric acid was added to the mixture which was extracted with ethyl acetate. The organic layer was washed with saturated brine, dried over sodium sulfate, filtered, and concentrated under reduced pressure. The residue was purified with silica gel column chromatography (chloroform/methanol) to give the titled compound 553 (16 mg).

Analyzing method SC2, $t_R$ 1.00 min, obs MS [M-1] 507.1

Example 554

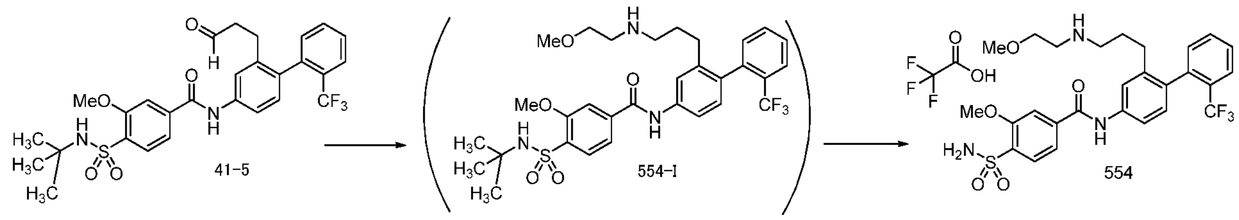


To the compound 41-5 (73 mg) prepared in Reference Example 41 were added methanol (2 mL), acetic acid (0.2 mL) and 2-methoxyethylamine (112 μL), and the mixture was stirred for 5 minutes at room temperature. Picoline borane (69 mg) was added thereto, and the mixture was stirred for 1.5 hours, and then 1M aqueous solution of hydrochloric acid was added thereto, and further stirred for 30 minutes at room temperature. A saturated aqueous solution of sodium bicarbonate was added thereto, and the mixture was extracted with ethyl acetate. The organic layer was washed with saturated brine, dried over sodium sulfate, filtered, and concentrated under reduced pressure. The residue was purified with silica gel column chromatography (chloroform/methanol) to give the compound 554-I (43 mg). Trifluoroacetic acid (TFA) (1.5 mL) was added to the compound 554-I (43 mg), and the mixture was stirred for 3 hours at room temperature. The reaction mixture was concentrated under reduced pressure, and chloroform was added thereto, and then the mixture was again concentrated under reduced pressure. Diethylether/hexane was added to the resulting residue, and the precipitated solid was collected by filtration to give the titled compound 554 (39 mg).

The compound 554-I: Analyzing method SC2, $t_R$ 0.93 min, obs MS [M+1] 622.1

The compound 554: Analyzing method SC2, $t_R$ 0.76 min, obs MS [M+1] 566.3

Example 555

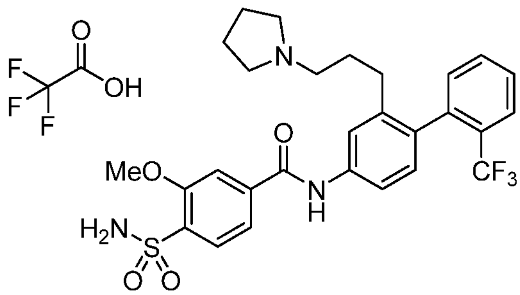

The titled compound was obtained in the same manner as Example 554.

Analyzing method SC2, $t_R$ 0.73 min, obs MS [M+1] 561.9

Example 556

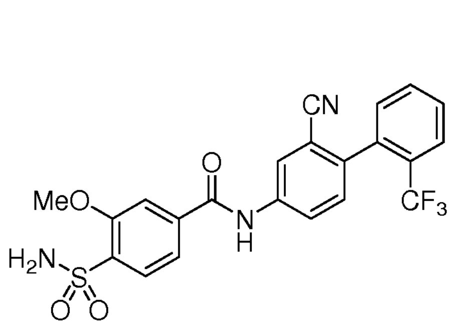

To the compound 42-2 (170 mg) preparedin Reference Example 42 in a mixture of 1,4-dioxane/water (5 mL/0.5 mL), 2-trifluoromethylphenylboronic acid (157 mg), cesium carbonate (405 mg) and [1,1'-bis(diphenylphosphino)ferrocene]dichloropalladium (II) (30 mg) were added, and the mixture was stirred for 22 hours at 100°C. The reaction mixture was cooled to room temperature, 1M aqueous solution of citric acid was added thereto, and the mixture was extracted with ethyl acetate. After washing the organic layer with saturated brine, it was dried over sodium sulfate, filtered, and concentrated under reduced pressure. The resulting residue was purified with silica gel column chromatography (hexane/ethyl acetate) to give the titled compound (60 mg).
Analyzing method SC2, $t_R$ 0.97 min, obs MS [M-1] 474.4

Example 557

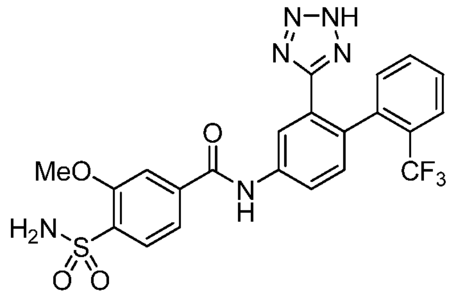

To the compound 43-3 (30 mg) prepared in Reference Example 43 was added trifluoroacetic acid (TFA) (1 mL), and the mixture was stirred for 5 hours at room temperature. The reaction mixture was concentrated under reduced pressure, and purified with silica gel column chromatography (chloroform/methanol) to give the titled compound (15 mg).
Analyzing method SC2, $t_R$ 0.88 min, obs MS [M-1] 517.0

Example 558

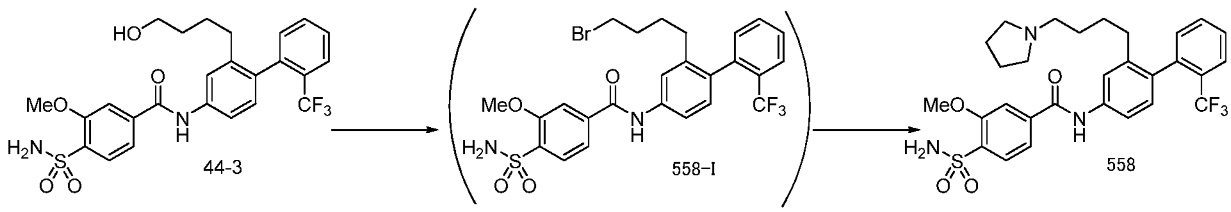

44-3 558-I 558

To the compound 44-3 (30 mg) prepared in Reference Example 44 were added carbon tetrachloride (1.5 mL) and phosphorus tribromide (54 μL), and the mixture was heated to reflux for 1.5 hours. After cooling to room temperature, water was added thereto, and the mixture was extracted with chloroform. The organic layer was washed with saturated brine, dried over sodium sulfate, filtered, and concentrated under reduced pressure to give the compound 558-I (34 mg). To the compound 558-I (34 mg) were added pyrrolidine (48 μL), potassium carbonate (79 mg) and ethanol (1.5 mL), the mixture was stirred for 30 minutes at 150°C under microwave irradiation. After cooling to room temperature, 1M aqueous solution of citric acid was added thereto, and the mixture was extracted with ethyl acetate. The organic layer was washed with saturated brine, dried over sodium sulfate, filtered, and concentrated under reduced pressure. The residue was purified with amino silica gel column chromatography (chloroform/methanol) to give the titled compound (10 mg).
Analyzing method SC2, $t_R$ 0.79 min, obs MS [M-1] 576.3

Example 559

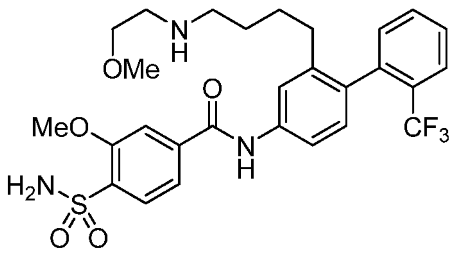

The titled compound was prepared in the same manner as Example 558.
Analyzing method SC2, $t_R$ 0.82 min, obs MS [M-1] 580.2

Example 560

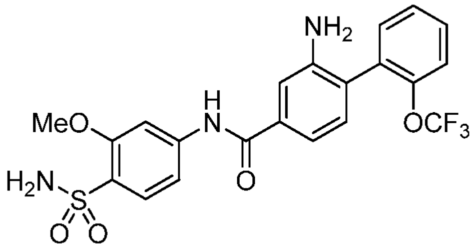

The titled compound was prepared in the same manner as Example 130
The compound 101: Analyzing method SC1, $t_R$ 1.25 min, obs MS [M+1] 482.0

Examples 561-577
The following derivatives were prepared in the same manner as Example 131 and Example 132.

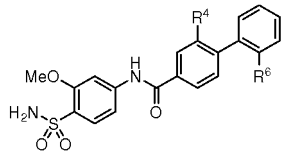

| Example | $R^4$ | $R^6$ | obs MS $[M+1]^+$ | $t_R$ (min) | Analytical Condition |
|---|---|---|---|---|---|
| 561 | 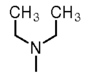 | $OCF_3$ | 538.1 | 1.20 | SC2 |
| 562 | 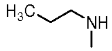 | $OCF_3$ | 566.4 | 1.32 | SC2 |
| 563 | 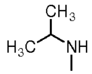 | $OCF_3$ | 524.4 | 1.14 | SC2 |
| 564 | 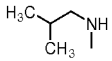 | $OCF_3$ | 538.4 | 1.20 | SC2 |
| 565 | 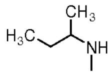 | $OCF_3$ | 538.4 | 1.20 | SC2 |
| 566 | 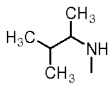 | $OCF_3$ | 552.5 | 1.24 | SC2 |
| 567 | 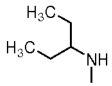 | $OCF_3$ | 552.5 | 1.25 | SC2 |
| 568 | 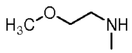 | $OCF_3$ | 540.4 | 1.04 | SC2 |
| 569 | 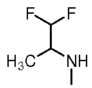 | $OCF_3$ | 560.4 | 1.11 | SC2 |
| 570 | 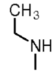 | $OCF_3$ | 510.3 | 1.09 | SC2 |
| 571 | 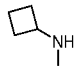 | $OCF_3$ | 536.2 | 1.18 | SC2 |
| 572 | 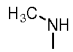 | $OCF_3$ | 496.3 | 1.02 | SC2 |
| 573 | 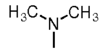 | $OCF_3$ | 510.3 | 1.13 | SC2 |
| 574 | 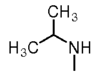 | Et | 496.3 | 1.20 | SC2 |
| 575 | 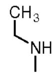 | Et | 510.3 | 1.15 | SC2 |
| 576 | 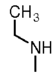 | /Pr | 510.3 | 1.20 | SC2 |
| 577 | 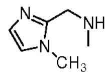 | $OCF_3$ | 576.1 | 0.71 | SC2 |

Example 578

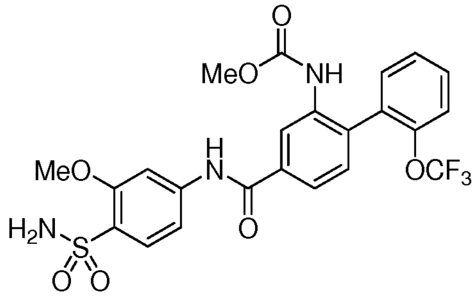

To the compound (40 mg) of Example 560 in THF (1 mL) were added diisopropylethylamine (21 μL) and methyl chloroformate (8 μL), and the mixture was stirred for 50 hours at room temperature. The reaction mixture was concentrated under reduced pressure and the resulting residue was purified with silica gel column chromatography (chloroform/methanol) to give the titled compound (48 mg).

Analyzing method SC2, $t_R$ 0.97 min, obs MS [M-1] 538.3

Examples 579-581

The compounds of the following Examples were prepared in the same manner as Example 578.

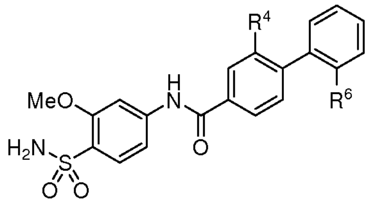

| Example | $R^4$ | $R^6$ | obs MS $[M-1]^-$ | $t_R$ (min) | Analytical Condition |
|---|---|---|---|---|---|
| 579 | 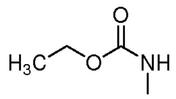 | $OcF_3$ | 566.1 | 1.40 | SC1 |
| 580 | 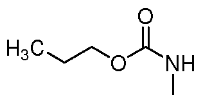 | $OCF_3$ | 524.4 | 1.44 | SC1 |
| 581 | 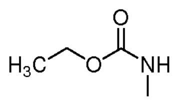 | Et | 496.2 | 1.46 | SC1 |

Example 582

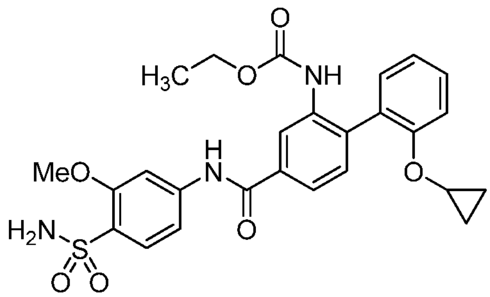

To the compound 45-3 (80 mg) prepared in Reference Example 45 in a mixture of 1,4-dioxane/wate (1 mL/0.1 mL) were added 2-cyclopropoxyphenyl iodide (82 mg), potassium carbonate (66 mg) and [1,1'-bis(diphenylphosphino) ferrocene]dichloropalladium (II) (12 mg), and the mixture was stirred for 3 hours at 80°C. The reaction mixture was cooled to room temperature, ethyl acetate and metal scavenger silica gel (SH silica, FUJI SILYSIA CHEMICAL Ltd.) were added thereto, and the mixture was stirred for 1 hour at room temperature. The reaction mixture was filtered and concentrated under reduced pressure. The resulting residue was purified with silica gel column chromatography (chloroform/methanol) to give the titled compound (25 mg).
Analyzing method SC1, $t_R$ 1.41 min, obs MS [M+1] 526.3

Example 583

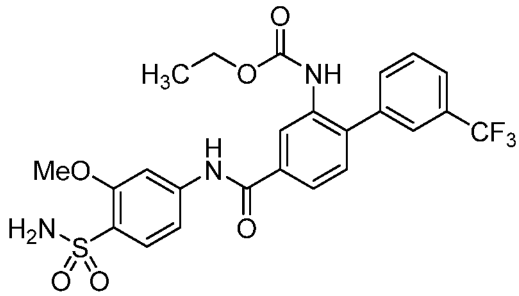

Using the compound 45-3 prepared in Reference Example 45 and treating it in the same manner as Example 582, the titled compound was prepared.
Analyzing method SC1, $t_R$ 1.46 min, obs MS [M+1] 536.0

Example 584

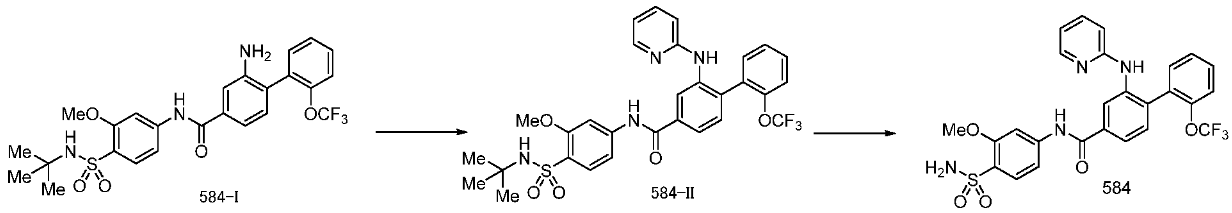

584-I 584-II 584

To a suspension of the compound 584-I (80 mg) prepared in the same manner as Reference Example 17 and Example 130 in toluene (1.5 mL) were added 2-chloropyridine (34 mg), palladium acetate (7 mg), 2-dicyclohexylphosphino-2',4',6'-triisopropylbiphenyl (XPhos) (28 mg) and sodium tert-butoxide (57 mg), and the mixture was stirred for 18 hours at 110°C. After cooling to room temperature, a saturated aqueous solution of ammonium chloride was added thereto, and the mixture was extracted with ethyl acetate. The organic layer was washed with saturated brine, dried over sodium sulfate, filtered, and then concentrated under reduced pressure. The resulting residue was purified with silica gel column chromatography (chloroform/methanol) to give the compound 584-II (39 mg). To the compound 584-II (39 mg) was added trifluoroacetic acid (TFA) (1.5 mL), and the mixture was stirred for 2 hours at room temperature. The reaction mixture was concentrated under reduced pressure, and purified with reverse phase column chromatography (methanol/water/TFA) to give the titled compound 584.

The compound 584-II: Analyzing method SC2, $t_R$ 1.05 min, obs MS [M+1] 615.1
The compound 584: Analyzing method SC2, $t_R$ 0.77 min, obs MS [M+1] 559.3

Example 585-Example 587:

Using corresponding phenylboronic acids with various substituents instead of 2-(trifluoromethoxy)phenylboronic acid, the compounds of the following Examples 585-587 were prepared in the same manner as Example 122.

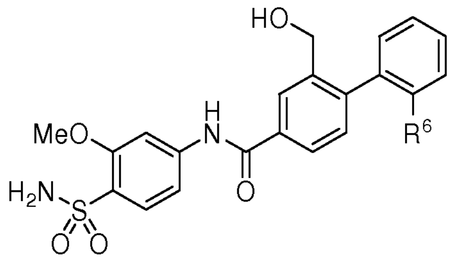

| Example | $R^6$ | obs MS [M+1] | $t_R$ (min) | Analytical Condition |
|---|---|---|---|---|
| 585 | F | 431.4 | 4.28 | SA3 |
| 586 | Cl | 447.1 | 4.38 | SA3 |
| 587 | $CH_3$ | 427.2 | 4.45 | SA3 |

Examples 588-590:

The compounds of the following Examples 588-590 were prepared in the same manner as Example 122 or Example 255.

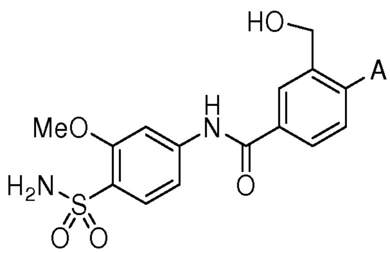

| Example | A | obs MS [M+1] | $t_R$ (min) | Analytical Condition |
|---|---|---|---|---|
| 588 | 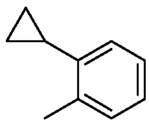 | 453.5 | 5.66 | SB1 |
| 589 | 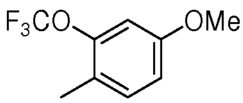 | 527.5 | 3.84 | SB1 |
| 590 | 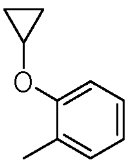 | 469.5 | 3.65 | SB1 |

Example 591:

The compound of the following Example 591 was prepared in the same manner as Example 487-496.

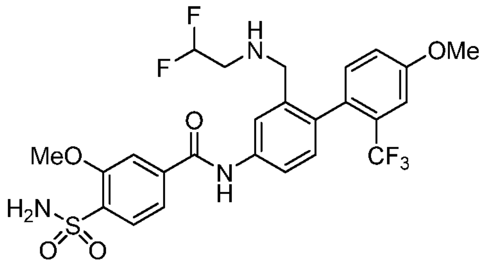

Analyzing method SC2, $t_R$ 0.802 min, obs MS [M+1] 574.1

Examples 592-594:

The compounds of the following Examples 592-594 were prepared in the same manner as Example 414.

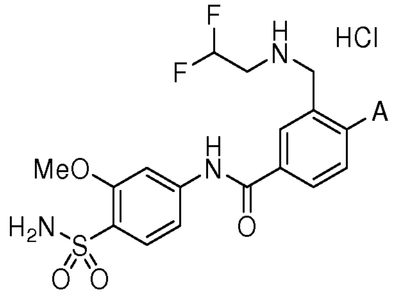

| Example | A | obs MS $[M+1]^+$ | $t_R$ (min) | Analytical Condition |
|---|---|---|---|---|
| 592 | 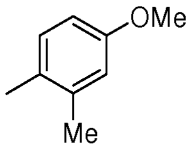 | 520.5 | 3.05 | SB1 |
| 593 | 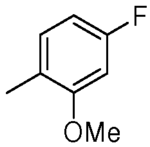 | 524.3 | 3.02 | SB1 |
| 594 | 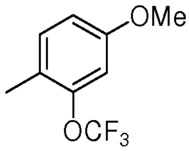 | 590.5 | 3.32 | SB1 |

<u>Test example 1: Binding inhibition test for aldosterone receptor</u>

A rat was layed open in flank regions (on both sides) to remove both adrenal glands. After stitching up the incision, the rat was returnd to a breeding cage again. After removing the kidney from the rat reared for about 3 to 5 days, the kidney was homogenized in 0.1 M Tris-HCl, 0.25M sucrose, 0.1M $Na_2MoO_4$ and 2 mM DTT buffer solution (pH7.4). The resultant was centrifuged at 105,000 g for 30 minutes, and then a supernatant was collected as a renal soluble fraction.

The rat renal soluble fraction (0.5 to 1.5 mg), a labeled ligand [$^3$H] aldosterone (2 nM) and a test article were reacted in the presence of 2 $\mu$M RU-486 (glucocorticoid receptor antagonist) in 100 $\mu$L of total amounts at 4°C for about 18 hours. Afterwards, to the reaction solution was added 50 $\mu$L of 5% dextran-coated activated charcoal, and the mixture was immingled, and then centrifuged at 3,000 g for 5 minutes. Then, a supernatant (100 $\mu$L) was collected and mixed with

a liquid scintillator ACS-II (Amersham) (3.5 mL), and then analyzed its radioactivity by a liquid scintillation counter (manufactured by Packard, Tri Carb 2700TR). A non-specific binding was obtained by addition of 2 μM aldosterone to a reaction solution. A binding inhibition rate (%) to the labeled ligand was calculated as a test result in the test article concentration in the range from 0.1 nM to 10000 nM according to the following equation.

$$[0709]\ \text{Binding inhibition rate (\%)} = 100-\{(B-N)/(B_0-N)\} \times 100$$

B: labeled ligand binding amounts in the presence of a test article and a labeled ligand
$B_0$: labeled ligand binding amounts in the absence of a test article
N: labeled ligand binding amounts in the presence of excess aldosterone (2 μM)

$IC_{50}$ value (MR $IC_{50}$ value; μM) corresponding to a concentration of a test article which inhibits 50% was calculated from a concentration-response curve.
Test example 1 was carried out for compounds obtained in the Examples. Results of binding inhibition test for aldosterone receptor are shown in Table 1 to Table 3.

[Table 1]

| Example | MR $IC_{50}$ (μM) | Example | MR $IC_{50}$ (μM) | Example | MR $IC_{50}$ (μM) |
|---|---|---|---|---|---|
| 7 | 0.35 | 75 | 0.88 | 163 | <10 |
| 10 | 0.50 | 79 | 0.32 | 171 | <10 |
| 12 | 0.61 | 81 | 0.12 | 174 | 0.94 |
| 14 | 4.7 | 85 | 0.54 | 176 | 0.39 |
| 17 | 1.7 | 88 | 0.41 | 180 | 0.28 |
| 19 | 3.3 | 91 | 0.26 | 182 | 0.35 |
| 20 | 0.55 | 92 | 0.41 | 189 | <10 |
| 22 | 1.6 | 94 | 0.82 | 190 | <10 |
| 24 | 2.4 | 99 | 2.0 | 195 | <10 |
| 26 | 0.49 | 101 | 0.29 | 198 | 0.17 |
| 28 | 3.0 | 103 | 0.13 | 202 | 0.76 |
| 31 | 0.25 | 104 | 0.082 | 206 | <10 |
| 35 | 0.35 | 109 | 0.19 | 207 | <10 |
| 36 | 1.4 | 120 | 3.6 | 210 | 0.16 |
| 39 | 1.2 | 121 | 0.52 | 211 | 0.91 |
| 40 | 0.39 | 122 | 0.06 | 218 | 0.37 |
| 42 | <1 | 126 | 0.31 | 221 | <10 |
| 46 | 3.0 | 127 | 0.22 | 226 | <10 |
| 50 | 0.70 | 128 | 0.14 | 229 | <0.1 |
| 51 | 0.20 | 131 | 0.17 | 231 | <10 |
| 56 | 0.52 | 132 | 0.29 | 235 | <10 |
| 57 | 0.65 | 136 | 0.40 | 243 | 0.031 |
| 59 | 0.26 | 142 | 0.42 | 244 | 0.68 |
| 60 | 0.20 | 144 | 0.50 | 247 | 0.21 |
| 62 | 1.9 | 147 | <10 | 249 | 0.26 |

(continued)

| Example | MR $IC_{50}$ (μM) | Example | MR $IC_{50}$ (μM) | Example | MR $IC_{50}$ (μM) |
|---|---|---|---|---|---|
| 64 | 0.40 | 153 | 0.47 | 250 | 0.33 |
| 65 | 0.25 | 154 | 1.3 | 257 | 0.084 |
| 68 | 0.21 | 159 | 0.29 | 259 | 0.45 |
| 69 | 0.22 | 160 | 2.0 | 260 | 0.44 |
| 72 | 0.53 | 162 | 0.66 | 265 | 0.36 |

[Table 2]

| Example | MR $IC_{50}$ (μM) | Example | MR $IC_{50}$ (μM) | Example | MR $IC_{50}$ (μM) |
|---|---|---|---|---|---|
| 268 | 0.99 | 346 | 1.1 | 440 | <10 |
| 270 | 0.65 | 351 | 1.2 | 443 | <10 |
| 271 | 0.37 | 355 | 1.0 | 448 | <10 |
| 272 | 0.40 | 357 | 0.13 | 451 | 0.55 |
| 279 | 4.1 | 363 | 0.75 | 454 | <10 |
| 282 | 0.73 | 364 | 0.54 | 457 | <10 |
| 286 | 0.18 | 365 | 0.46 | 460 | <10 |
| 287 | 0.25 | 371 | 0.45 | 463 | <10 |
| 288 | 0.12 | 373 | 0.10 | 469 | <10 |
| 289 | 0.36 | 376 | 0.38 | 470 | <10 |
| 293 | 0.27 | 380 | 1.0 | 474 | <10 |
| 294 | 0.022 | 385 | 0.63 | 478 | <10 |
| 295 | 0.10 | 387 | 0.36 | 481 | <10 |
| 297 | 0.19 | 389 | 0.56 | 486 | <10 |
| 299 | 0.14 | 392 | 0.45 | 488 | <10 |
| 302 | 0.061 | 393 | 0.52 | 493 | 0.52 |
| 303 | 0.15 | 395 | 0.19 | 494 | 0.23 |
| 305 | 0.15 | 403 | 0.63 | 499 | 1.2 |
| 309 | 0.23 | 405 | 0.21 | 500 | 0.19 |
| 313 | 2.2 | 407 | 0.26 | 505 | 0.29 |
| 315 | 0.42 | 410 | 0.52 | 508 | <10 |
| 320 | 2.5 | 414 | 1.7 | 510 | 0.28 |
| 323-2 | 0.25 | 415 | 0.63 | 513 | 0.70 |
| 324 | 0.19 | 419 | 1.2 | 515 | 0.87 |
| 329 | 0.22 | 424 | <10 | 517 | 0.39 |
| 333 | 0.28 | 426 | 0.58 | 520 | 2.9 |
| 335 | 0.35 | 429 | <10 | 526 | 2.2 |
| 337 | 1.1 | 431 | <10 | 528 | <10 |
| 342 | 0.47 | 432 | <10 | 531 | <10 |

(continued)

| Example | MR $IC_{50}$ ($\mu$M) | Example | MR $IC_{50}$ ($\mu$M) | Example | MR $IC_{50}$ ($\mu$M) |
|---|---|---|---|---|---|
| 345 | 0.14 | 438 | <10 | 533 | 1.6 |

[Table 3]

| Example | MR $IC_{50}$ ($\mu$M) | Example | MR $IC_{50}$ ($\mu$M) | Example | MR $IC_{50}$ ($\mu$M) |
|---|---|---|---|---|---|
| 537 | 5.4 | 560 | 0.24 | 581 | <10 |
| 543 | <10 | 561 | 0.10 | 583 | 1.3 |
| 545 | <10 | 562 | 0.23 | 590 | <10 |
| 547 | <10 | 577 | <10 | 592 | <10 |
| 550 | 0.62 | 578 | <10 | 593 | <10 |

Results of the above Test example 1 for Example compounds 1 and 66 which were described in the prior art (the above mentioned Patent document 6) are shown in Table 4.

[Table 4]

| Comparative example | Compound | MR $IC_{50}$ ($\mu$M) | Inhibition rates (%) in 100 $\mu$M |
|---|---|---|---|
| 1 | 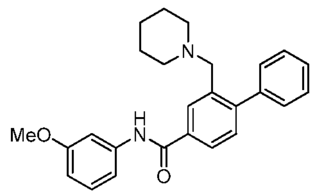 | >10 | 7 |
| 2 | 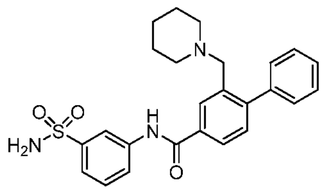 | >10 | 11 |

Both comparative examples 1 and 2 showed very few binding affinities for aldosterone receptor. On the other hand, the compound of the present invention showed strong binding affinities for aldosterone receptor.

Test example 2: Antagonism *(in vivo* antagonist activity) against reduction of Na/K ratios in deoxycorticosterone acetate (DOCA) induced urine of normal rat

A rat was encaged in a metabolism cage and was collected urine for 8 hours. When a test article was orally administered, it was administered in 5 ml/kg of dosage solution amounts using 0.5% methylcellulose as a solvent. DOCA was dissolved in corn oil in 0.3 mg/ml, and was subcutaneously administered in 1 ml/kg (0.3 mg/kg). Administration timing for a test article was 30 minutes before DOCA administration. 20 ml/kg of Solution amounts of saline was orally administered when administering DOCA in order to stabilize urine amounts and to secure enough amounts. Collected urine was analyzed its amounts, and then electrolyte (sodium: $Na^+$ and potassium: $K^+$) concentrations in urine were analyzed by an automatic biochemical analyzer (manufactured by JEOL Ltd., JCA-BM1650) to calculate ratios of $N^{a+}$ and $K^+$.

Test example 2 was carried out for Example compounds and aldosterone receptor antagonist eplerenone, and Example compounds and eplerenone suppressed reduction of Na/K ratios in DOCA-induced urine in a dose-dependent manner.

INDUSTRIAL APPLICABILITY

The compounds of the present invention have high binding affinities for aldosterone receptors. The compounds of the present invention also show pharmacological effects caused by antagonistic activities or partial agonistic activities as an aldosterone receptor regulator. Thus, the compounds of the present invention are usuful for preventing and/or treating diseases such as hypertension, stroke, arrhythmia, heart failure, cardiomegaly, arteriosclerosis, vascular restenosis, renal fibrosis, cardiac infarction, diabetes complication, renal disease, edema, primary aldosteronism, inflammation, insulin resistance, sleep apnea syndrome, non-alcoholic steatohepatitis, Cushing's syndrome.

## Claims

**1.** A compound of formula (1), or a pharmaceutically acceptable salt thereof.

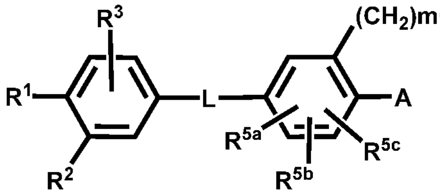

(1)

[wherein A is any one of groups of the following formulae (a) to (e):

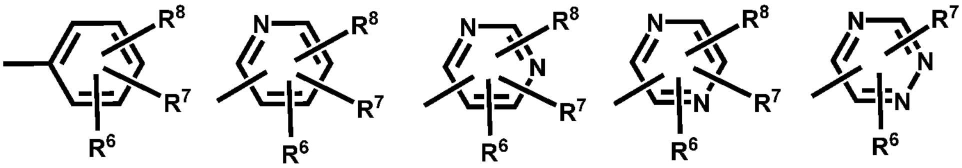

(a) (b) (c) (d) (e) ;

L is -CONH-, or -NHCO-;
$R^1$ is optionally substituted aminosulfonyl group, optionally substituted $C_{1-6}$ alkylsulfonyl group, or optionally substituted $C_{1-6}$ alkylsulfonylamino group;
$R^2$ is hydrogen atom, halogen atom, hydroxy group, optionally substituted $C_{1-6}$ alkyl group, optionally substituted $C_{1-6}$ alkoxy group, or optionally substituted 5- or 6-membered monocyclic heteroaryl group;
$R^3$ is hydrogen atom, or halogen atom;
$R^4$ is hydrogen atom, halogen atom, hydroxy group, cyano group, nitro group, formyl group, carboxyl group, optionally substituted $C_{1-6}$ alkyl group, optionally substituted amino group, optionally substituted $C_{1-6}$ alkoxy group, optionally substituted $C_{1-6}$ alkoxycarbonyl group, optionally substituted 4-to 7-membered heterocyclic group, optionally substituted 5- or 6-membered monocyclic heteroaryl group, optionally substituted $C_{7-14}$ aralkyloxy group, optionally substituted $C_{1-6}$ alkylcarbonyloxy group, optionally substituted $C_{1-6}$ alkylsulfonyloxy group, optionally substituted 4- to 8-membered cyclic amino group, optionally substituted 4- to 7-membered saturated heterocyclic oxy group, optionally substituted $C_{1-6}$ alkylcarbonylamino group, optionally substituted $C_{3-10}$ cycloalkylcarbonylamino group, optionally substituted $C_{1-6}$ alkoxycarbonylamino group, optionally substituted 5- or 6-membered monocyclic heteroarylcarbonylamino group, optionally substituted 4- to 7-membered saturated heterocyclic carbonylamino group, or optionally substituted aminocarbonylamino group;
$R^{5a}$, $R^{5b}$ and $R^{5c}$ are each independently hydrogen atom, halogen atom, optionally substituted amino group, or optionally substituted $C_{1-6}$ alkyl group;
$R^6$ is halogen atom, cyano group, optionally substituted $C_{1-6}$ alkyl group, amino group (in which the amino may be optionally substituted with the same or different 1 to 2 groups selected from the group consisting of $C_{1-6}$ alkyl, $C_{3-10}$ cycloalkyl, $C_{3-10}$ cycloalkyl-$C_{1-4}$ alkyl, saturated heterocycle which may be optionally substituted with $C_{1-6}$ alkyl, 5- or 6-membered saturated heterocyclic-$C_{1-4}$ alkyl, 5- or 6-membered monocyclic heteroaryl, and 5- or 6-membered monocyclic heteroaryl-$C_{1-4}$ alkyl), hydroxy group, optionally substituted $C_{1-6}$ alkoxy group, optionally substituted $C_{3-10}$ cycloalkyl group, optionally substituted $C_{3-10}$ cycloalkoxy group, optionally substituted $C_{1-6}$ alkylthio group, optionally substituted $C_{1-6}$ alkoxycarbonyl group, optionally substituted 4- to 7-membered cyclic amino group, optionally substituted 4- to 7-membered cyclic aminocarbonyl group, optionally substituted $C_{6-10}$ aryl group, optionally substituted 5- to 10-membered monocyclic or polycyclic heteroaryl group, optionally substituted 5- or 6-membered monocyclic heteroaryloxy group, optionally substituted 4- to 7-membered saturated heterocyclic group, or optionally substituted 4- to 7-membered saturated heterocyclic oxy group;
$R^7$ and $R^8$ are each independently hydrogen atom, halogen atom, hydroxy group, cyano group, nitro group, amino group (in which the amino may be optionally substituted with the same or different 1 to 2 groups selected from the group consisting of $C_{1-6}$ alkyl, $C_{3-10}$ cycloalkyl, $C_{3-10}$ cycloalkyl-$C_{1-4}$ alkyl, 5-or 6-membered saturated heterocycle, 5- or 6-membered saturated heterocyclic-$C_{1-4}$ alkyl, 5- or 6-membered monocyclic heteroaryl, and 5- or 6-membered monocyclic heteroaryl-$C_{1-4}$ alkyl), optionally substituted $C_{1-6}$ alkyl group, optionally substituted $C_{1-6}$ alkoxy group, optionally substituted $C_{1-6}$ alkylthio group, optionally substituted $C_{1-6}$ alkoxycarbonyl group, or optionally substituted $C_{1-6}$ alkylcarbonyloxy group, or
any one of $R^6$, $R^7$ and $R^8$ is hydrogen atom, and the remaining two groups are adjacent each other and may combine each other together with the ring atoms to which they bind to form $C_{3-7}$ cycloalkyl ring, 5- or 6-membered saturated heterocycle, or 5- or 6-membered heteroaryl ring;
m is an integer of 0 to 6;
provided that 4-(methylsulfonyl)-N-[3'-(trifluoromethyl)biphenyl-4-yl]benzamide, and 2'-methyl-5'-(5-methyl-1,3,4-oxadiazol-2-yl)-N-[4-[(methylsulfonyl)amino]phenyl]biphenyl-4-carboxamide are excluded. ]

**2.** The compound of claim 1, or a pharmaceutically acceptable salt thereof, wherein A is a group of formula (a).

**3.** The compound of either claim 1 or 2, or a pharmaceutically acceptable salt thereof, wherein L is - NHCO-.

**4.** The compound of either claim 1 or 2, or a pharmaceutically acceptable salt thereof, wherein L is - CONH-.

**5.** The compound of any one of claims 1 to 4, or a pharmaceutically acceptable salt thereof, wherein $R^1$ is

1: aminosulfonyl group (in which the amino may be optionally substituted with the same or different 1 to 2 groups selected from the group consisting of

(a) $C_{1-6}$ alkyl (in which the group may be optionally substituted with

(i) amino (in which the amino may be optionally substituted with the same or different 1 to 2 $C_{1-6}$ alkyl),
(ii) $C_{1-6}$ alkoxy, or
(iii) 4- to 7-membered cyclic amino),

(b) $C_{1-6}$ alkylcarbonyl,
(c) aminocarbonyl, and
(d) $-C(=NH)-NH_2$),

2: $C_{1-6}$ alkylsulfonyl group, or
3: $C_{1-6}$ alkylsulfonylamino group, or a pharmaceutically acceptable salt thereof.

**6.** The compound of claim 5, wherein $R^1$ is aminosulfonyl group, or a pharmaceutically acceptable salt thereof.

**7.** The compound of claim 5, wherein $R^1$ is $C_{1-6}$ alkylsulfonyl group, or a pharmaceutically acceptable salt thereof.

**8.** The compound of any one of claims 1 to 7, wherein $R^2$ is hydrogen atom, halogen atom, hydroxy group, $C_{1-6}$ alkyl group, or optionally substituted $C_{1-6}$ alkoxy group.

**9.** The compound of claim 8, or a pharmaceutically acceptable salt thereof, wherein $R^2$ is $C_{1-6}$ alkoxy group.

**10.** The compound of any one of claims 1 to 9, or a pharmaceutically acceptable salt thereof, wherein $R^3$ is hydrogen atom.

**11.** The compound of any one of claims 1 to 10, or a pharmaceutically acceptable salt thereof, wherein $R^4$ is

1: hydrogen atom,
2: halogen atom,
3: hydroxy group,

4: cyano group,
5: nitro group,
6: formyl group,
7: carboxyl group,
8: amino group (in which the amino may be optionally substituted with the same or different 1 to 2 groups selected from the group consisting of

(a) $C_{1-6}$ alkyl (in which the group may be optionally substituted with

(i) 1 to 3 fluorine atoms,
(ii) cyano,
(iii) hydroxy,
(iv) amino (in which the amino may be optionally substituted with the same or different 1 to 2 $C_{1-6}$ alkyl),
(v) $C_{1-6}$ alkoxy,
(vi) $C_{6-10}$ aryloxy,
(vii) $C_{7-14}$ aralkyl (in which the group may be optionally substituted with $C_{1-4}$ alkoxy), or
(viii) aminocarbonyl),

(b) $C_{3-10}$ cycloalkyl (in which the group may be optionally substituted with cyano, or $C_{1-4}$ alkyl),
(c) $C_{3-10}$ cycloalkyl-$C_{1-4}$ alkyl,
(d) $C_{6-10}$ aryl (in which the group may be optionally substituted with $C_{1-6}$ alkyl),
(e) $C_{7-14}$ aralkyl (in which the group may be optionally substituted with halogen atom, or $C_{1-4}$ alkoxy),
(f) 4- to 7-membered saturated heterocycle (in which the heterocycle may be optionally substituted with $C_{1-6}$ alkyl),
(g) 4- to 7-membered saturated heterocyclic-$C_{1-4}$ alkyl (in which the heterocycle may be optionally substituted with $C_{1-4}$ alkyl),
(h) 5- or 6-membered monocyclic heteroaryl (in which the group may be optionally substituted with the same or different 1 to 2 groups selected from the group consisting of cyano and $C_{1-4}$ alkyl), and
(i) 5- or 6-membered monocyclic heteroaryl-$C_{1-4}$ alkyl (in which the heteroaryl may be optionally substituted with $C_{1-4}$ alkyl)),

9: $C_{1-6}$ alkoxy group (in which the group may be optionally substituted with

(a) 1 to 2 hydroxy,
(b) 1 to 3 fluorine atoms,
(c) amino (in which the amino may be optionally substituted with the same or different 1 to 2 groups selected from the group consisting of

(i) $C_{1-6}$ alkyl (in which the group may be optionally substituted with hydroxy, $C_{1-4}$ alkoxy, or 5- or 6-membered monocyclic heteroaryl),
(ii) $C_{3-6}$ cycloalkyl, and
(iii) 4- to 7-membered saturated heterocyclic-$C_{1-4}$ alkyl),

(d) 1 to 2 $C_{1-6}$ alkoxy,
(e) 4- to 7-membered cyclic amino (in which the ring may be optionally substituted with the same or different 1 to 2 groups selected from the group consisting of

(i) hydroxy,
(ii) cyano,
(iii) 1 to 4 fluorine atoms,
(iv) $C_{1-6}$ alkyl (in which the group may be optionally substituted with 1 to 3 fluorine atoms, or $C_{1-6}$ alkoxy),
(v) $C_{1-6}$ alkoxy,
(vi) formyl,
(vii) $C_{1-6}$ alkylcarbonyl,
(viii) $C_{1-6}$ alkylsulfonyl, and
(ix) oxo),

(f) 4- to 7-membered saturated heterocycle (in which the ring may be optionally substituted with the same

or different group(s) selected from the group consisting of (i) to (ix) in the above (e)),
(g) 5- or 6-membered monocyclic heteroaryl (in which the ring may be optionally substituted with $C_{1-6}$ alkyl), or
(h) $C_{7-14}$ aralkyloxy),

10: $C_{1-6}$ alkoxycarbonyl group,
11: 4- to 7-membered heterocyclic group (in which the ring may be optionally substituted with the same or different group(s) selected from the group consisting of (i) to (ix) in the above (e)),
12: 5- or 6-membered monocyclic heteroaryl group (in which the ring may be optionally substituted with cyano, or $C_{1-6}$ alkyl),
13: $C_{7-14}$ aralkyloxy group,
14: $C_{1-6}$ alkylcarbonyloxy group,
15: $C_{1-6}$ alkylsulfonyloxy group,
16: 4- to 8-membered cyclic amino group (in which the ring may be optionally substituted with the same or different group(s) selected from the group consisting of (i) to (ix) in the above (e)),
17: saturated heterocyclic oxy group (in which the ring may be optionally substituted with the same or different 1 to 2 groups selected from the group consisting of $C_{1-6}$ alkyl which may be optionally substituted with 1 to 3 fluorine atoms, and oxo), or
18: $C_{1-6}$ alkylcarbonylamino group (in which the alkyl may be optionally substituted with

(a) hydroxy,
(b) 1 to 3 fluorine atoms,
(c) $C_{1-6}$ alkoxy, or
(d) $C_{1-6}$ alkylcarbonyloxy),

19: $C_{3-10}$cycloalkylcarbonylamino group,
20: $C_{1-6}$ alkoxycarbonylamino group,
21: 5- or 6-membered monocyclic heteroarylcarbonylamino group (in which the ring may be optionally substituted with $C_{1-6}$ alkyl),
22: 4- to 7-membered saturated heterocyclic carbonylamino group (in which the ring may be optionally substituted with $C_{1-6}$ alkyl which may be optionally substituted with 1 to 3 fluorine atoms),
23: mono- or di-$C_{1-6}$ alkylaminocarbonylamino group, or
24: $C_{1-6}$ alkyl group (in which the group may be optionally substituted with group(s) selected from 1 to 3 fluorine atoms, and hydroxy).

**12.** The compound of claim 11, or a pharmaceutically acceptable salt thereof, wherein $R^4$ is amino group (in which the amino is substituted with $C_{1-6}$ alkyl which is substituted with 1 to 3 fluorine atoms).

**13.** The compound of claim 12, or a pharmaceutically acceptable salt thereof, wherein $R^4$ is 2,2-difluoroethylamino.

**14.** The compound of any one of claims 1 to 13, or a pharmaceutically acceptable salt thereof, wherein $R^{5a}$, $R^{5b}$, and $R^{5c}$ are each independently

1: hydrogen atom,
2: halogen atom,
3: amino group (in which the amino may be optionally substituted with the same or different 1 to 2 groups selected from the group consisting of

(a) $C_{1-6}$ alkyl,
(b) $C_{3-10}$ cycloalkyl, and
(c) $C_{3-10}$cycloalkyl-$C_{1-4}$alkyl), or

4: $C_{1-6}$ alkyl group.

**15.** The compound of claim 14, or a pharmaceutically acceptable salt thereof, wherein all of $R^{5a}$, $R^{5b}$, and $R^{5c}$ are hydrogen atom.

**16.** The compound of any one of claims 1 to 15, or a pharmaceutically acceptable salt thereof, wherein $R^6$ is

1: halogen atom,
2: cyano group,
3: $C_{1-6}$ alkyl group (in which the group may be optionally substituted with

(a) hydroxy,
(b) cyano,
(c) oxo,
(d) 1 to 4 fluorine atoms,
(e) amino (in which the amino may be optionally substituted with the same or different 1 to 2 groups selected from the group consisting of

(i) $C_{1-6}$ alkyl (in which the group may be optionally substituted with 1 to 3 fluorine atoms, or $C_{1-6}$ alkoxy),
(ii) $C_{3-10}$ cycloalkyl, and
(iii) $C_{3-10}$ cycloalkyl-$C_{1-4}$ alkyl),

(f) 4- to 7-membered cyclic amino (in which the ring may be optionally substituted with

(i) 1 to 3 fluorine atoms,
(ii) $C_{1-6}$ alkyl, or
(iii) $C_{1-6}$ alkoxy),

(g) $C_{1-6}$ alkoxy (in which the group may be optionally substituted with 1 to 3 fluorine atoms),
(h) $C_{3-10}$ cycloalkoxy,
(i) 5- or 6-membered monocyclic heteroaryl,
(j) 5- or 6-membered monocyclic heteroaryloxy,
(k) 4- to 7-membered saturated heterocyclic oxy,
(l) $C_{1-6}$ alkylcarbonyl, or
(m) $C_{1-6}$ alkoxycarbonyl),

4: amino group (in which the amino may be optionally substituted with the same or different 1 to 2 groups selected from the group consisting of

(a) $C_{1-6}$ alkyl,
(b) $C_{3-10}$ cycloalkyl,
(c) $C_{3-10}$cycloalkyl-$C_{1-4}$alkyl, and
(d) 4- to 7-membered saturated heterocycle (in which the ring may be optionally substituted with $C_{1-6}$ alkyl)),

5: hydroxy group,
6: $C_{1-6}$ alkoxy group (in which the group may be optionally substituted with

(a) hydroxy,
(b) cyano,
(c) 1 to 6 fluorine atoms,
(d) amino (in which the amino may be optionally substituted with the same or different 1 to 2 groups selected from the group consisting of

(i) $C_{1-6}$ alkyl,
(ii) $C_{3-10}$cycloalkyl, and
(iii) $C_{3-10}$ cycloalkyl-$C_{1-4}$ alkyl),

(e) 4- to 7-membered cyclic amino (in which the ring may be optionally substituted with

(i) 1 to 3 fluorine atoms,
(ii) $C_{1-6}$ alkyl, or
(iii) $C_{1-6}$ alkoxy),

(f) $C_{1-6}$ alkoxy (in which the group may be optionally substituted with 1 to 3 fluorine atoms),
(g) $C_{3-10}$cycloalkyl, or

(h) 5- or 6-membered monocyclic heteroaryl),

7: $C_{3-10}$cycloalkyl group (in which the group may be optionally substituted with $C_{1-6}$ alkyl, or cyano),
8: $C_{3-10}$cycloalkoxy group,
9: $C_{1-6}$ alkylthio group,
10: $C_{1-6}$ alkoxycarbonyl group,
11: 4- to 7-membered cyclic amino group (in which the ring may be optionally substituted with

(a) 1 to 3 fluorine atoms,
(b) $C_{1-6}$ alkyl, or
(c) $C_{1-6}$ alkoxy),

12: 4- to 7-membered cyclic aminocarbonyl group,
13: $C_{6-10}$aryl group,
14: 5- or 6-membered monocyclic heteroaryl group (in which the ring may be optionally substituted with $C_{1-6}$ alkyl),
15: 5- or 6-membered monocyclic heteroaryloxy group,
16: 4- to 7-membered saturated heterocyclic group, or
17: 4- to 7-membered saturated heterocyclic oxy group.

**17.** The compound of claim 16, or a pharmaceutically acceptable salt thereof, wherein $R^6$ is halogen atom.

**18.** The compound of any one of claims 1 to 17, or a pharmaceutically acceptable salt thereof, wherein $R^7$ is

1: hydrogen atom,
2: halogen atom,
3: hydroxy group,
4: cyano group,
5: nitro group,
6: amino group (in which the amino may be optionally substituted with the same or different 1 to 2 groups selected from the group consisting of

(a) $C_{1-6}$ alkyl,
(b) $C_{3-10}$ cycloalkyl, and
(c) $C_{3-10}$ cycloalkyl-$C_{1-4}$ alkyl),

7: $C_{1-6}$ alkyl group (in which the group may be optionally substituted with 1 to 3 fluorine atoms),
8: $C_{1-6}$ alkoxy group (in which the group may be optionally substituted with

(a) hydroxy,
(b) carboxyl,
(c) 1 to 3 fluorine atoms,
(d) amino (in which the amino may be optionally substituted with the same or different 1 to 2 $C_{1-6}$ alkyl),
(e) $C_{1-6}$ alkoxycarbonyl, or
(f) $C_{1-6}$ alkylcarbonyloxy),

9: $C_{1-6}$ alkylthio group,
10: $C_{1-6}$ alkoxycarbonyl group, or
11: $C_{1-6}$ alkylcarbonyloxy group.

**19.** The compound of claim 18, or a pharmaceutically acceptable salt thereof, wherein $R^7$ is hydrogen atom.

**20.** The compound of any one of claims 1 to 19, or a pharmaceutically acceptable salt thereof, wherein $R^8$ is

1: hydrogen atom,
2: halogen atom, or
3: $C_{1-6}$ alkoxy group.

**21.** The compound of claim 20, or a pharmaceutically acceptable salt thereof, wherein $R^8$ is hydrogen atom.

**22.** The compound of any one of claims 1 to 21, or a pharmaceutically acceptable salt thereof, wherein m is 0 or 1.

**23.** The compound of claim 22, or a pharmaceutically acceptable salt thereof, wherein m is 0.

**24.** The compound of claim 22, or a pharmaceutically acceptable salt thereof, wherein m is 1.

**25.** The compound of claim 1, or a pharmaceutically acceptable salt thereof, which is any one selected from the following group:

2-(hydroxymethyl)-*N*-[4-(methylsulfonyl)phenyl]-2'-(trifluorophenyl)biphenyl-4-carboxamide,
2-(hydroxymethyl)-*N*-(3-methoxy-4-sulfamoylphenyl)-2'-(trifluorophenyl)biphenyl-4-carboxamide,
2-(hydroxymethyl)-*N*-(3-methoxy-4-sulfamoylphenyl)-2'-(trifluoromethoxy)biphenyl-4-carboxamide,
2'-ethyl-2-(hydroxymethyl)-*N*-(3-methoxy-4-sulfamoylphenyl)biphenyl-4-carboxamide,
4'-fluoro-2-(hydroxymethyl)-*N*-(3-methoxy-4-sulfamoylphenyl)-2'-(trifluorophenyl)biphenyl-4-carboxamide,
2-(hydroxymethyl)-4'-methoxy-*N*-(3-methoxy-4-sulfamoylphenyl)-2'-(trifluorophenyl)biphenyl-4-carboxamide,
2-(hydroxymethyl)-*N*-[4-(methylsulfonyl)phenyl]-2'-(trifluoromethoxy)biphenyl-4-carboxamide,
2-[(3,3-difluoropyrrolidin-1-yl)methyl]-*N*-[4-(methylsulfonyl)phenyl]-2'-(trifluoromethoxy)biphenyl-4-carboxamide,
2-[(3,3-difluoropiperidin-1-yl)methyl]-*N*-[4-(methylsulfonyl)phenyl]-2'-(trifluoromethoxy)biphenyl-4-carboxamide,
2-{[(2-methoxyethyl)amino]methyl}-*N*-[4-(methylsulfonyl)phenyl]-2'-(trifluoromethoxy)biphenyl-4-carboxamide,
2-[(diethylamino)methyl]-*N*-[4-(methylsulfonyl)phenyl]-2'-(trifluoromethoxy)biphenyl-4-carboxamide,
2-{[(3*S*)-3-fluoropyrrolidin-1-yl]methyl}-*N*-[4-(methylsulfonyl)phenyl]-2'-(trifluoromethoxy)biphenyl-4-carboxamide,
2-{[(2,2-difluoroethyl)amino]methyl}-*N*-[4-(methylsulfonyl)phenyl]-2'-(trifluoromethoxy)biphenyl-4-carboxamide,
2-{[bis(2-methoxyethyl)amino]methyl}-*N*-[4-(methylsulfonyl)phenyl]-2'-(trifluoromethoxy)biphenyl-4-carboxamide,
*N*-[4-(methylsulfonyl)phenyl]-2-{[(2,2,2-trifluoroethyl)amino]methyl}-2'-(trifluoromethoxy)biphenyl-4-carboxamide,
2-{[(2,2-difluoroethyl)amino]methyl}-N-(3-methoxy-4-sulfamoylphenyl)-2'-(trifluoromethoxy)biphenyl-4-carboxamide,
2-(hydroxymethyl)-*N*-(3-methoxy-4-sulfamoylphenyl)-2'-(1,1,2,2-tetrafluoroethoxy)biphenyl-4-carboxamide,
2- {[(2,2-difluoroethyl)amino]methyl}-*N*-(3-methoxy-4-sulfamoylphenyl)-2'-(trifluorophenyl)biphenyl-4-carboxamide,
2-{[(2,2-difluoroethyl)amino]methyl}-4'-methoxy-*N*-(3-methoxy-4-sulfamoylphenyl)-2'-(trifluorophenyl)biphenyl-4-carboxamide,
*N*-[2-(hydroxymethyl)-2'-(trifluorophenyl)biphenyl-4-yl]-3-methoxy-4-sulfamoylbenzamide,
*N*-[2-(hydroxymethyl)-2'-(trifluoromethoxy)biphenyl-4-yl]-3-methoxy-4-sulfamoylbenzamide,
*N*-[2-{[(2,2-difluoroethyl)amino]methyl}-2'-(trifluorophenyl)biphenyl-4-yl]-3-methoxy-4-sulfamoylbenzamide,
2- {[(2,2-difluoroethyl)amino]methyl}-2',4'-difluoro-*N*-(3-methoxy-4-sulfamoylphenyl)biphenyl-4-carboxamide,
2- {[(2,2-difluoroethyl)amino]methyl}-*N*-(3-methoxy-4-sulfamoylphenyl)-2'-methylbiphenyl-4-carboxamide,
2- {[(2,2-difluoroethyl)amino]methyl}-2'-fluoro-*N*-(3-methoxy-4-sulfamoylphenyl)biphenyl-4-carboxamide,
2- {[(2,2-difluoroethyl)amino]methyl}-2',5'-difluoro-*N*-(3-methoxy-4-sulfamoylphenyl)biphenyl-4-carboxamide,
5'-chloro-2- {[(2,2-difluoroethyl)amino]methyl}-2'-fluoro-*N*-(3-methoxy-4-sulfamoylphenyl)biphenyl-4-carboxamide,
2'-chloro-2-{[(2,2-difluoroethyl)amino]methyl}-4'-fluoro-*N*-(3-methoxy-4-sulfamoylphenyl)biphenyl-4-carboxamide,
2- {[(2,2-difluoroethyl)amino]methyl}-*N*-(3-methoxy-4-sulfamoylphenyl)-4'-methyl-2'-(trifluorophenyl)biphenyl-4-carboxamide,
2'-chloro-2- {[(2,2-difluoroethyl)amino]methyl}-*N*-(3-methoxy-4-sulfamoylphenyl)biphenyl-4-carboxamide,
2',4'-dichloro-2- {[(2,2-difluoroethyl)amino]methyl}-*N*-(3-methoxy-4-sulfamoylphenyl)biphenyl-4-carboxamide,
2', 5'-dichloro-2- {[(2,2-difluoroethyl)amino]methyl}-*N*-(3-methoxy-4-sulfamoylphenyl)biphenyl-4-carboxamide,
2'-cyclopropyl-2- {[(2,2-difluoroethyl)amino]methyl}-4'-fluoro-*N*-(3-methoxy-4-sulfamoylphenyl)-biphenyl-4-carboxamide,
4'-chloro-2- {[(2,2-difluoroethyl)amino]methyl}-*N*-(3-methoxy-4-sulfamoylphenyl)-2'-(trifluorophenyl)biphenyl-4-carboxamide,
2- {[(2,2-difluoroethyl)amino]methyl}-4'-fluoro-*N*-(3-methoxy-4-sulfamoylphenyl)-2'-(trifluorophenyl)biphenyl-4-carboxamide,
N-(2'-chloro-2- {[(2,2-difluoroethyl)amino]methyl}-4'-fluorobiphenyl-4-yl)-3-methoxy-4-sulfamoylbenzamide,
*N*-[2- {[(2,2-difluoroethyl)amino]methyl}-4'-fluoro-2'-(trifluorophenyl)biphenyl-4-yl]-3-methoxy-4-sulfamoylbenzamide,
2-{[(3*R*)-3-fluoropyrrolidin-1-yl]methyl}-*N*-[4-(methylsulfonyl)phenyl]-2'-(trifluoromethoxy)biphenyl-4-carboxamide,
2-{[(2-fluoroethyl)amino]methyl}-*N*-[4-(methylsulfonyl)phenyl]-2'-(trifluoromethoxy)biphenyl-4-carboxamide,
2-[(cyclopropylamino)methyl]-*N*-[4-(methylsulfonyl)phenyl] -2'-(trifluoromethoxy)biphenyl-4-carboxamide,
3-methoxy-4-sulfamoyl-*N*-[2-{[(2,2,2-trifluoroethyl)amino]methyl}-2'-(trifluorophenyl)biphenyl-4-yl]benzamide,
3-methoxy-4-sulfamoyl-*N*-[2-{[(2,2,2-trifluoroethyl)amino]methyl}-2'-(trifluoromethoxy)biphenyl-4-yl]benzamide,
2'-chloro-2-(hydroxymethyl)-*N*-(3-methoxy-4-sulfamoylphenyl)biphenyl-4-carboxamide,
2-(hydroxymethyl)-*N*-(3-methoxy-4-sulfamoylphenyl)-2'-methylbiphenyl-4-carboxamide,
2'-cyclopropyl-2-(hydroxymethyl)-*N*-(3-methoxy-4-sulfamoylphenyl)biphenyl-4-carboxamide,
2-(hydroxymethyl)-4'-methoxy-*N*-(3-methoxy-4-sulfamoylphenyl)-2'-(trifluoromethoxy)biphenyl-4-carboxamide,
2'-(cyclopropyloxy)-2-(hydroxymethyl)-*N*-(3-methoxy-4-sulfamoylphenyl)biphenyl-4-carboxamide,
*N*-[2- {[(2,2-difluoroethyl)amino]methyl}-4'-methoxy-2'-(trifluorophenyl)biphenyl-4-yl]-3-methoxy-4-sulfamoylbenzamide,
2-{[(2,2-difluoroethyl)amino]methyl}-4'-methoxy-*N*-(3-methoxy-4-sulfamoylphenyl)-2'-methylbiphenyl-4-carboxamide,
2- {[(2,2-difluoroethyl)amino]methyl}-4'-fluoro-2'-methoxy-*N*-(3-methoxy-4-sulfamoylphenyl)biphenyl-4-carboxamide, and
2-{[(2,2-difluoroethyl)amino]methyl}-4'-methoxy-*N*-(3-methoxy-4-sulfamoylphenyl)-2'-(trifluoromethoxy)biphenyl-4-carboxamide.

**26.** The compound of claim 25, which is any one selected from the following group, or a pharmaceutically acceptable salt thereof:

2-(hydroxymethyl)-*N*-(3-methoxy-4-sulfamoylphenyl)-2'-(trifluorophenyl)biphenyl-4-carboxamide,
2-{[(2,2-difluoroethyl)amino]methyl}-*N*-(3-methoxy-4-sulfamoylphenyl)-2'-(trifluoromethoxy)biphenyl-4-carboxamide,
*N*-[2-(hydroxymethyl)-2'-(trifluoromethoxy)biphenyl-4-yl]-3-methoxy-4-sulfamoylbenzamide,
2-{[(2,2-difluoroethyl)amino]methyl}-*N*-(3-methoxy-4-sulfamoylphenyl)-2'-methylbiphenyl-4-carboxamide,
2-{[(2,2-difluoroethyl)amino]methyl}-2'-fluoro-*N*-(3-methoxy-4-sulfamoylphenyl)biphenyl-4-carboxamide,
2'-chloro-2-{[(2,2-difluoroethyl)amino]methyl}-4'-fluoro-*N*-(3-methoxy-4-sulfamoylphenyl)biphenyl-4-carboxamide,
2'-chloro-2-{[(2,2-difluoroethyl)amino]methyl}-*N*-(3-methoxy-4-sulfamoylphenyl)biphenyl-4-carboxamide,
4'-chloro-2-{[(2,2-difluoroethyl)amino]methyl}-*N*-(3-methoxy-4-sulfamoylphenyl)-2'-(trifluorophenyl)-biphenyl-4-carboxamide, and
2-{[(2,2-difluoroethyl)amino]methyl}-4'-fluoro-*N*-(3-methoxy-4-sulfamoylphenyl)-2'-(trifluorophenyl)-biphenyl-4-carboxamide.

**27.** The compound of claim 25, or a pharmaceutically acceptable salt thereof, which is any one selected from the following group:

2-(hydroxymethyl)-*N*-[4-(methylsulfonyl)phenyl]-2'-(trifluorophenyl)biphenyl-4-carboxamide,
2-(hydroxymethyl)-*N*-[4-(methylsulfonyl)phenyl]-2'-(trifluoromethoxy)biphenyl-4-carboxamide,
2-{[(2,2-difluoroethyl)amino]methyl}-*N*-[4-(methylsulfonyl)phenyl]-2'-(trifluoromethoxy)biphenyl-4-carboxamide, and
2-{[(2-fluoroethyl)amino]methyl}-*N*-[4-(methylsulfonyl)phenyl]-2'-(trifluoromethoxy)biphenyl-4-carboxamide.

**28.** A pharmaceutical composition, comprising the compound of any one of claims 1 to 27 or a pharmaceutically acceptable salt thereof.

**29.** A medicament, comprising the compound of any one of claims 1 to 27 or a pharmaceutically acceptable salt thereof as the active ingredient.

**30.** A preventive or therapeutic agent for hypertension, stroke, arrhythmia, heart failure, cardiomegaly, arteriosclerosis, vascular restenosis, renal fibrosis, cardiac infarction, diabetes complication, renal disease, edema, primary aldosteronism, inflammation, insulin resistance, sleep apnea syndrome, non-alcoholic steatohepatitis, or Cushing's syndrome, which comprises the compound of any one of claims 1 to 27 or a pharmaceutically acceptable salt thereof.

**31.** A method for preventing or treating hypertension, stroke, arrhythmia, heart failure, cardiomegaly, arteriosclerosis, vascular restenosis, renal fibrosis, cardiac infarction, diabetes complication, renal disease, edema, primary aldosteronism, inflammation, insulin resistance, sleep apnea syndrome, non-alcoholic steatohepatitis, or Cushing's syndrome, which comprises administering an effective amount of the compound of any one of claims 1 to 27 or a pharmaceutically acceptable salt thereof to a patient in need.

**32.** Use of the compound of any one of claims 1 to 27 or a pharmaceutically acceptable salt thereof in the manufacture of a medicament for preventing or treating hypertension, stroke, arrhythmia, heart failure, cardiomegaly, arteriosclerosis, vascular restenosis, renal fibrosis, cardiac infarction, diabetes complication, renal disease, edema, primary aldosteronism, inflammation, insulin resistance, sleep apnea syndrome, non-alcoholic steatohepatitis, or Cushing's syndrome.

**INTERNATIONAL SEARCH REPORT**

International application No.
PCT/JP2011/065857

A. CLASSIFICATION OF SUBJECT MATTER
See extra sheet.

According to International Patent Classification (IPC) or to both national classification and IPC

B. FIELDS SEARCHED

Minimum documentation searched (classification system followed by classification symbols)
See extra sheet.

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)
CAplus(STN), REGISTRY(STN), JSTPlus/JMEDPlus/JST7580(JDreamII)

C. DOCUMENTS CONSIDERED TO BE RELEVANT

| Category* | Citation of document, with indication, where appropriate, of the relevant passages | Relevant to claim No. |
|---|---|---|
| A | WO 2004/110986 A1 (Yamanouchi Pharmaceutical Co., Ltd.), 23 December 2004 (23.12.2004), claims; page 64 & JP 4400566 B & US 2007/0167444 A1 & US 2009/0270365 A1 & EP 1632477 A1 | 1-30,32 |
| A | JP 2005-507910 A (Glaxo Group Ltd.), 24 March 2005 (24.03.2005), claims; paragraph [0149] & US 2004/0266839 A1 & EP 1435949 A1 & WO 2003/032986 A1 | 1-30,32 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>25 August, 2011 (25.08.11) | Date of mailing of the international search report<br>06 September, 2011 (06.09.11) |
| Name and mailing address of the ISA/<br>Japanese Patent Office<br>Facsimile No. | Authorized officer<br><br>Telephone No. |

Form PCT/ISA/210 (second sheet) (July 2009)

**INTERNATIONAL SEARCH REPORT**

International application No.
PCT/JP2011/065857

C (Continuation). DOCUMENTS CONSIDERED TO BE RELEVANT

| Category* | Citation of document, with indication, where appropriate, of the relevant passages | Relevant to claim No. |
|---|---|---|
| A | JP 2009-508835 A (Arrow Therapeutics Ltd.), 05 March 2009 (05.03.2009), claims; page 37; paragraph [0205] & US 2008/0255105 A1 & EP 1940786 A1 & WO 2007/031791 A1 | 1-30,32 |
| A | Bioorganic & Medicinal Chemistry Letters, 2008, 18, (1), p.324-328 | 1-30,32 |
| A | REGISTRY (STN) [online], 24 June 2008 (24.06.2008), (retrieval date 25 August 2011 (25.08.2011)), CAS Registry Number:1030238-85-7 | 1-30,32 |

Form PCT/ISA/210 (continuation of second sheet) (July 2009)

INTERNATIONAL SEARCH REPORT

International application No.
PCT/JP2011/065857

Continuation of A. CLASSIFICATION OF SUBJECT MATTER
(International Patent Classification (IPC))

*C07C311/08*(2006.01)i, *A61K31/167*(2006.01)i, *A61K31/18*(2006.01)i, *A61K31/216*(2006.01)i, *A61K31/235*(2006.01)i, *A61K31/277*(2006.01)i, *A61K31/341*(2006.01)i, *A61K31/351*(2006.01)i, *A61K31/381*(2006.01)i, *A61K31/397*(2006.01)i, *A61K31/40*(2006.01)i, *A61K31/4015*(2006.01)i, *A61K31/403*(2006.01)i, *A61K31/407*(2006.01)i, *A61K31/41*(2006.01)i, *A61K31/415*(2006.01)i, *A61K31/4164*(2006.01)i, *A61K31/4196*(2006.01)i, *A61K31/421*(2006.01)i, *A61K31/4245*(2006.01)i, *A61K31/426*(2006.01)i, *A61K31/438*(2006.01)i, *A61K31/439*(2006.01)i, *A61K31/44*(2006.01)i, *A61K31/4402*(2006.01)i, *A61K31/4406*(2006.01)i, *A61K31/4409*(2006.01)i, *A61K31/444*(2006.01)i, *A61K31/445*(2006.01)i, *A61K31/4453*(2006.01)i, *A61K31/45*(2006.01)i, *A61K31/46*(2006.01)i, *A61K31/495*(2006.01)i, *A61K31/505*(2006.01)i, *A61K31/5375*(2006.01)i, *A61K31/5377*(2006.01)i, *A61K31/54*(2006.01)i, *A61K31/55*(2006.01)i, *A61K31/553*(2006.01)i, *A61P1/16*(2006.01)i, *A61P3/10*(2006.01)i, *A61P5/38*(2006.01)i, *A61P5/42*(2006.01)i, *A61P7/10*(2006.01)i, *A61P9/00*(2006.01)i, *A61P9/04*(2006.01)i, *A61P9/06*(2006.01)i, *A61P9/10*(2006.01)i, *A61P9/12*(2006.01)i, *A61P11/00*(2006.01)i, *A61P13/12*(2006.01)i, *A61P29/00*(2006.01)i, *A61P43/00*(2006.01)i, *C07C311/46*(2006.01)i, *C07C311/54*(2006.01)i, *C07C311/64*(2006.01)i, *C07C317/40*(2006.01)i, *C07D205/04*(2006.01)i, *C07D207/06*(2006.01)i, *C07D207/08*(2006.01)i, *C07D207/10*(2006.01)i, *C07D207/12*(2006.01)i, *C07D207/27*(2006.01)i, *C07D207/327*(2006.01)i, *C07D207/335*(2006.01)i, *C07D209/52*(2006.01)i, *C07D211/22*(2006.01)i, *C07D211/38*(2006.01)i, *C07D211/42*(2006.01)i, *C07D211/46*(2006.01)i, *C07D211/62*(2006.01)i, *C07D211/70*(2006.01)i, *C07D211/74*(2006.01)i, *C07D213/38*(2006.01)i, *C07D213/40*(2006.01)i, *C07D213/56*(2006.01)i, *C07D213/61*(2006.01)i, *C07D213/64*(2006.01)i, *C07D213/74*(2006.01)i, *C07D215/14*(2006.01)i, *C07D231/12*(2006.01)i, *C07D231/38*(2006.01)i, *C07D233/56*(2006.01)i, *C07D233/64*(2006.01)i, *C07D233/90*(2006.01)i, *C07D239/26*(2006.01)i, *C07D239/42*(2006.01)i, *C07D249/04*(2006.01)i, *C07D249/08*(2006.01)i, *C07D249/14*(2006.01)i, *C07D257/04*(2006.01)i, *C07D257/06*(2006.01)i, *C07D263/14*(2006.01)i, *C07D263/28*(2006.01)i, *C07D263/32*(2006.01)i, *C07D265/30*(2006.01)i, *C07D267/10*(2006.01)i, *C07D271/06*(2006.01)i, *C07D271/10*(2006.01)i, *C07D277/20*(2006.01)i, *C07D277/28*(2006.01)i, *C07D277/42*(2006.01)i, *C07D295/08*(2006.01)i, *C07D295/12*(2006.01)i, *C07D295/14*(2006.01)i, *C07D295/18*(2006.01)i, *C07D307/14*(2006.01)i, *C07D307/52*(2006.01)i, *C07D307/54*(2006.01)i, *C07D309/12*(2006.01)i, *C07D309/14*(2006.01)i, *C07D333/20*(2006.01)i, *C07D333/24*(2006.01)i, *C07D451/06*(2006.01)i, *C07D453/02*(2006.01)i, *C07D491/113*(2006.01)i

(According to International Patent Classification (IPC) or to both national classification and IPC)

INTERNATIONAL SEARCH REPORT

International application No.
PCT/JP2011/065857

Continuation of B. FIELDS SEARCHED
Minimum documentation searched (International Patent Classification (IPC))

C07C311/08, A61K31/167, A61K31/18, A61K31/216, A61K31/235, A61K31/277, A61K31/341, A61K31/351, A61K31/381, A61K31/397, A61K31/40, A61K31/4015, A61K31/403, A61K31/407, A61K31/41, A61K31/415, A61K31/4164, A61K31/4196, A61K31/421, A61K31/4245, A61K31/426, A61K31/438, A61K31/439, A61K31/44, A61K31/4402, A61K31/4406, A61K31/4409, A61K31/444, A61K31/445, A61K31/4453, A61K31/45, A61K31/46, A61K31/495, A61K31/505, A61K31/5375, A61K31/5377, A61K31/54, A61K31/55, A61K31/553, A61P1/16, A61P3/10, A61P5/38, A61P5/42, A61P7/10, A61P9/00, A61P9/04, A61P9/06, A61P9/10, A61P9/12, A61P11/00, A61P13/12, A61P29/00, A61P43/00, C07C311/46, C07C311/54, C07C311/64, C07C317/40, C07D205/04, C07D207/06, C07D207/08, C07D207/10, C07D207/12, C07D207/27, C07D207/327, C07D207/335, C07D209/52, C07D211/22, C07D211/38, C07D211/42, C07D211/46, C07D211/62, C07D211/70, C07D211/74, C07D213/38, C07D213/40, C07D213/56, C07D213/61, C07D213/64, C07D213/74, C07D215/14, C07D231/12, C07D231/38, C07D233/56, C07D233/64, C07D233/90, C07D239/26, C07D239/42, C07D249/04, C07D249/08, C07D249/14, C07D257/04, C07D257/06, C07D263/14, C07D263/28, C07D263/32, C07D265/30, C07D267/10, C07D271/06, C07D271/10, C07D277/20, C07D277/28, C07D277/42, C07D295/08, C07D295/12, C07D295/14, C07D295/18, C07D307/14, C07D307/52, C07D307/54, C07D309/12, C07D309/14, C07D333/20, C07D333/24, C07D451/06, C07D453/02, C07D491/113

Minimum documentation searched (classification system followed by classification symbols)

Form PCT/ISA/210 (extra sheet) (July 2009)

**INTERNATIONAL SEARCH REPORT**

International application No.
PCT/JP2011/065857

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 31
because they relate to subject matter not required to be searched by this Authority, namely:
Claim 31 pertains to a method for treatment of the human body or animal body by surgery or therapy and thus relates to a subject matter on which the International Searching Authority is not required to carry out a search under the provisions of PCT Article 17(2)(a)(i) and PCT Rule 39.1(iv).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 06012642 A
- WO 07077961 A
- WO 08053300 A
- WO 08118319 A
- WO 08126831 A
- WO 04110986 A


### Non-patent literature cited in the description


- The Experimental Chemistry. Maruzen, vol. 22
- Protective Groups in Organic Synthesis. John Wiley & Sons Inc, 1999
- **R. C. LAROCK.** Comprehensive Organic Transformations. VCH publisher Inc, 1989